(19) Europäisches Patentamt
European Patent Office
Office européen des brevets

(11) EP 0 730 663 B1

(12) EUROPEAN PATENT SPECIFICATION

(45) Date of publication and mention of the grant of the patent:
**24.09.2003 Bulletin 2003/39**

(21) Application number: **95900441.7**

(22) Date of filing: **26.10.1994**

(51) Int Cl.[7]: **C12Q 1/68**, C07H 21/02, C07H 21/04

(86) International application number:
**PCT/US94/12305**

(87) International publication number:
**WO 95/011995 (04.05.1995 Gazette 1995/19)**

# (54) ARRAYS OF NUCLEIC ACID PROBES ON BIOLOGICAL CHIPS

FELDER VON NUKLEINSAEURESONDEN AUF BIOLOGISCHEN CHIPS

RESEAUX DE SONDES D'ACIDE NUCLEIQUE SUR DES MICROPLAQUETTES BIOLOGIQUES

(84) Designated Contracting States:
**CH DE FR GB IT LI NL**

(30) Priority: **26.10.1993 US 143312**
**02.08.1994 US 284064**

(43) Date of publication of application:
**11.09.1996 Bulletin 1996/37**

(73) Proprietor: **Affymetrix, Inc.**
**Santa Clara, CA 95051 (US)**

(72) Inventors:
- **CHEE, Mark**
  **Palo Alto, CA 94306 (US)**
- **CRONIN, Maureen T.**
  **Los Altos, CA 94024 (US)**
- **FODOR, Stephen P.A.**
  **Palo Alto, CA 94303 (US)**
- **GINGERAS, Thomas R.**
  **Santa Clara, CA 95054 (US)**
- **HUANG, Xiaohua C.**
  **Mountain View, CA 94043 (US)**
- **HUBBELL, Earl A.**
  **Mountain View, CA 94040 (US)**
- **LIPSHUTZ, Robert J.**
  **Palo Alto, CA 94301 (US)**
- **LOBBAN, Peter E.**
  **Palo Alto, CA 94301 (US)**
- **MIYADA, Charles Garrett**
  **San Jose, CA 95129 (US)**
- **MORRIS, MacDonald S.**
  **San Jose, CA 95172 (US)**
- **SHAH, Nila**
  **Saratoga, CA 95070 (US)**
- **SHELDON, Edward L.**
  **Menlo Park, CA 94025 (US)**

(74) Representative:
**Voelker, Ingeborg Carla Emmy et al**
**Uexküll & Stolberg**
**Patentanwälte**
**Beselerstrasse 4**
**22607 Hamburg (DE)**

(56) References cited:

| | |
|---|---|
| **WO-A-89/10977** | **WO-A-92/10588** |
| **WO-A-93/17126** | **US-A- 4 656 127** |
| **US-A- 5 002 867** | **US-A- 5 202 231** |

- **NATURE, Volume 313, issued 24 January 1985, RATNER et al., "Complete Nucleotide Sequence of the AIDS Virus, HTLV-III", pages 277-284.**
- **VIROLOGY, Volume 175, issued 1990, QUERAT et al., "Nucleotide Sequence Analysis of SA-OMVV, A Visna-Related Ovine Lentivirus: Phylogenetic History of Lentiviruses", pages 434-447.**
- **JOURNAL OF VIROLOGY, Volume 68, Number 6, issued June 1994, LUO et al., "Cellular Protein Modulates Effects of Human Immunodeficiency Virus Type 1 Rev", pages 3850-3856.**
- **CELL, Volume 40, issued January 1985, WAIN-HOBSON et al., "Nucleotide Sequence of the AIDS Virus, LAV", pages 9-17.**
- **JOURNAL OF BIOMOLECULAR STRUCTURE & DYNAMICS, Volume 11, Number 3, issued 1993, LIPSHUTZ, "Likelihood DNA Sequencing by Hybridization", pages 637-653.**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 0 730 663 B1

- **MAXIMUM ENTROPY AND BAYESIAN METHODS, (Paris), issued 1992, ELDER, "Analysis of DNA Oligonucleotide Hybridization Data by Maximum Entropy", pages 1-10.**
- **GENOMICS, Volume 13, issued 1992, SOUTHERN et al., "Analyzing and Comparing Nucleic Acid Sequences by Hybridization to Arrays of Oligonucleotides: Evaluation Using Experimental Models", pages 1008-1017.**

## Description

### Background of the Invention

### Field of the Invention

The present invention provides arrays of oligonucleotide probes immobilized in microfabricated patterns on silica chips for analyzing molecular interactions of biological interest. The invention therefore relates to diverse fields impacted by the nature of molecular interaction, including chemistry, biology, medicine, and medical diagnostics.

### Description of Related Art

Oligonucleotide probes have long been used to detect complementary nucleic acid sequences in a nucleic acid of interest (the "target" nucleic acid). In some assay formats, the oligonucleotide probe is tethered, *i.e.*, by covalent attachment, to a solid support, and arrays of oligonucleotide probes immobilized on solid supports have been used to detect specific nucleic acid sequences in a target nucleic acid. *See, e.g.*, PCT patent publication Nos. WO 89/10977 and 89/11548. Others have proposed the use of large numbers of oligonucleotide probes to provide the complete nucleic acid sequence of a target nucleic acid but failed to provide an enabling method for using arrays of immobilized probes for this purpose. *See* U.S. Patent Nos. 5,202,231 and 5,002,867 and PCT patent publication No. WO 93/17126.

The development of VLSIPS™ technology has provided methods for making very large arrays of oligonucleotide probes in very small arrays. *See* U.S. Patent No. 5,143,854 and PCT patent publication Nos. WO 90/15070 and 92/10092. U.S. Patent No. 6,284,460, filed June 25, 1993, describes methods for making arrays of oligonucleotide probes that can be used to provide the complete sequence of a target nucleic acid and to detect the presence of a nucleic acid containing a specific nucleotide sequence.

WO 92/10588 discloses various methods of using arrays of immobilized nucleic acids for investigating the sequence or expression of target polynucleotides.

Microfabricated arrays of large numbers of oligonucleotide probes, called "DNA chips" offer great promise for a wide variety of applications. New methods and reagents are required to realize this promise, and the present invention helps meet that need.

### SUMMARY OF THE INVENTION

The invention provides several strategies employing immobilized arrays of probes for comparing a reference sequence of known sequence with a target sequence showing substantial similarity with the reference sequence, but differing in the presence of, *e.g.*, mutations. In a first embodiment, the invention provides a tiling strategy employing an array of immobilized oligonucleotide probes comprising at least two sets of probes. A first probe set comprises a plurality of probes, each probe comprising a segment of at least three nucleotides exactly complementary to a subsequence of the reference sequence, the segment including at least one interrogation position complementary to a corresponding nucleotide in the reference sequence. A second probe set comprises a corresponding probe for each probe in the first probe set, the corresponding probe in the second probe set being identical to a sequence comprising the corresponding probe from the first probe set or a subsequence of at least three nucleotides thereof that includes the at least one interrogation position, except that the at least one interrogation position is occupied by a different nucleotide in each of the two corresponding probes from the first and second probe sets. The probes in the first probe set have at least two interrogation positions corresponding to two contiguous nucleotides in the reference sequence. One interrogation position corresponds to one of the contiguous nucleotides, and the other interrogation position to the other.

In a second embodiment, the invention provides a tiling strategy employing an array comprising four probe sets. A first probe set comprises a plurality of probes, each probe comprising a segment of at least three nucleotides exactly complementary to a subsequence of the reference sequence, the segment including at least one interrogation position complementary to a corresponding nucleotide in the reference sequence. Second, third and fourth probe sets each comprise a corresponding probe for each probe in the first probe set. The probes in the second, third and fourth probe sets are identical to a sequence comprising the corresponding probe from the first probe set or a subsequence of at least three nucleotides thereof that includes the at least one interrogation position, except that the at least one interrogation position is occupied by a different nucleotide in each of the four corresponding probes from the four probe sets. The first probe set often has at least 100 interrogation positions corresponding to 100 contiguous nucleotides in the reference sequence. Sometimes the first probe set has an interrogation position corresponding to every nucleotide in the reference sequence. The segment of complementarity within the probe set is usually about 9-21 nucleotides. Although probes may contain leading or trailing sequences in addition to the 9-21 sequences, many probes consist exclusively of a 9-21 segment of complementarity.

In a third embodiment, the invention provides immobilized arrays of probes tiled for multiple reference sequences. One such array comprises at least one pair of first and second probe groups, each group comprising first and second sets of probes as defined in the first embodiment. Each probe in the first probe set from the first group is exactly complementary to a subsequence of a first reference sequence, and each probe in the first probe set from the second group is exactly complementary to a subsequence of a second reference sequence. Thus, the first group of probes are tiled with respect to a first reference sequence and the second group of probes with respect to a second reference sequence. Each group of probes can also include third and fourth sets of probes as defined in the second embodiment. In some arrays of this type, the second reference sequence is a mutated form of the first reference sequence.

In a fourth embodiment, the invention provides arrays for block tiling. Block tiling is a species of the general tiling strategies described above. The usual unit of a block tiling array is a group of probes comprising a wildtype probe, a first set of three mutant probes and a second set of three mutant probes. The wildtype probe comprises a segment of at least three nucleotides exactly complementary to a subsequence of a reference sequence. The segment has at least first and second interrogation positions corresponding to first and second nucleotides in the reference sequence. The probes in the first set of three mutant probes are each identical to a sequence comprising the wildtype probe or a subsequence of at least three nucleotides thereof including the first and second interrogation positions, except in the first interrogation position, which is occupied by a different nucleotide in each of the three mutant probes and the wildtype probe. The probes in the second set of three mutant probes are each identical to a sequence comprising the wildtype probes or a subsequence of at least three nucleotides thereof including the first and second interrogation positions, except in the second interrogation position, which is occupied by a different nucleotide in each of the three mutant probes and the wildtype probe.

In a fifth embodiment, the invention provides methods of comparing a target sequence with a reference sequence using arrays of immobilized pooled probes. The arrays employed in these methods represent a further species of the general tiling arrays noted above. In these methods, variants of a reference sequence differing from the reference sequence in at least one nucleotide are identified and each is assigned a designation. An array of pooled probes is provided, with each pool occupying a separate cell of the array. Each pool comprises a probe comprising a segment exactly complementary to each variant sequence assigned a particular designation. The array is then contacted with a target sequence comprising a variant of the reference sequence. The relative hybridization intensities of the pools in the array to the target sequence are determined. The identity of the target sequence is deduced from the pattern of hybridization intensities. Often, each variant is assigned a designation having at least one digit and at least one value for the digit. In this case, each pool comprises a probe comprising a segment exactly complementary to each variant sequence assigned a particular value in a particular digit. When variants are assigned successive numbers in a numbering system of base m having n digits, n x (m-1) pooled probes are used are used to assign each variant a designation.

In a sixth embodiment, the invention provides a pooled probe for trellis tiling, a further species of the general tiling strategy. In trellis tiling, the identity of a nucleotide in a target sequence is determined from a comparison of hybridization intensities of three pooled trellis probes. A pooled trellis probe comprises a segment exactly complementary to a subsequence of a reference sequence except at a first interrogation position occupied by a pooled nucleotide N, a second interrogation position occupied by a pooled nucleotide selected from the group of three consisting of (1) M or K, (2) R or Y and (3) S or W, and a third interrogation position occupied by a second pooled nucleotide selected from the group. The pooled nucleotide occupying the second interrogation position comprises a nucleotide complementary to a corresponding nucleotide from the reference sequence when the second pooled probe and reference sequence are maximally aligned, and the pooled nucleotide occupying the third interrogation position comprises a nucleotide complementary to a corresponding nucleotide from the reference sequence when the third pooled probe and the reference sequence are maximally aligned. Standard IUPAC nomenclature is used for describing pooled nucleotides.

In trellis tiling, an array comprises at least first, second and third cells, respectively occupied by first, second and third pooled probes, each according to the generic description above. However, the segment of complementarity, location of interrogation positions, and selection of pooled nucleotide at each interrogation position may or may not differ between the three pooled probes subject to the following constraint. One of the three interrogation positions in each of the three pooled probes must align with the same corresponding nucleotide in the reference sequence. This interrogation position must be occupied by a N in one of the pooled probes, and a different pooled nucleotide in each of the other two pooled probes.

In a seventh embodiment, the invention provides arrays for bridge tiling. Bridge tiling is a species of the general tiling strategies noted above, in which probes from the first probe set contain more than one segment of complementarity. In bridge tiling, a nucleotide in a reference sequence is usually determined from a comparison of four probes. A first probe comprises at least first and second segments, each of at least three nucleotides and each exactly complementary to first and second subsequences of a reference sequences. The segments including at least one interrogation position corresponding to a nucleotide in the reference sequence. Either (1) the first and second subsequences are

noncontiguous in the reference sequence, or (2) the first and second subsequences are contiguous and the first and second segments are inverted relative to the first and second subsequences. The arrays further comprises second, third and fourth probes, which are identical to a sequence comprising the first probe or a subsequence thereof comprising at least three nucleotides from each of the first and second segments, except in the at least one interrogation position, which differs in each of the probes. In a species of bridge tiling, referred to as deletion tiling, the first and second subsequences are separated by one or two nucleotides in the reference sequence.

In an eighth embodiment, the invention provides arrays of probes for multiplex tiling. Multiplex tiling is a strategy, in which the identity of two nucleotides in a target sequence is determined from a comparison of the hybridization intensities of four probes, each having two interrogation positions. Each of the probes comprising a segment of at least 7 nucleotides that is exactly complementary to a subsequence from a reference sequence, except that the segment may or may not be exactly complementary at two interrogation positions. The nucleotides occupying the interrogation positions are selected by the following rules: (1) the first interrogation position is occupied by a different nucleotide in each of the four probes, (2) the second interrogation position is occupied by a different nucleotide in each of the four probes, (3) in first and second probes, the segment is exactly complementary to the subsequence, except at no more than one of the interrogation positions, (4) in third and fourth probes, the segment is exactly complementary to the subsequence, except at both of the interrogation positions.

In a ninth embodiment, the invention provides arrays of immobilized probes including helper mutations. Helper mutations are useful for, *e.g.*, preventing self-annealing of probes having inverted repeats. In this strategy, the identity of a nucleotide in a target sequence is usually determined from a comparison of four probes. A first probe comprises a segment of at least 7 nucleotides exactly complementary to a subsequence of a reference sequence except at one or two positions, the segment including an interrogation position not at the one or two positions. The one or two positions are occupied by helper mutations. Second, third and fourth mutant probes are each identical to a sequence comprising the wildtype probe or a subsequence thereof including the interrogation position and the one or two positions, except in the interrogation position, which is occupied by a different nucleotide in each of the four probes.

In a tenth embodiment, the invention provides arrays of probes comprising at least two probe sets, but lacking a probe set comprising probes that are perfectly matched to a reference sequence. Such arrays are usually employed in methods in which both reference and target sequence are hybridized to the array. The first probe set comprising a plurality of probes, each probe comprising a segment exactly complementary to a subsequence of at least 3 nucleotides of a reference sequence except at an interrogation position. The second probe set comprises a corresponding probe for each probe in the first probe set, the corresponding probe in the second probe set being identical to a sequence comprising the corresponding probe from the first probe set or a subsequence of at least three nucleotides thereof that includes the interrogation position, except that the interrogation position is occupied by a different nucleotide in each of the two corresponding probes and the complement to the reference sequence.

In an eleventh embodiment, the invention provides methods of comparing a target sequence with a reference sequence comprising a predetermined sequence of nucleotides using any of the arrays described above. The methods comprise hybridizing the target nucleic acid to an array and determining which probes, relative to one another, in the array bind specifically to the target nucleic acid. The relative specific binding of the probes indicates whether the target sequence is the same or different from the reference sequence. In some such methods, the target sequence has a substituted nucleotide relative to the reference sequence in at least one undetermined position, and the relative specific binding of the probes indicates the location of the position and the nucleotide occupying the position in the target sequence. In some methods, a second target nucleic acid is also hybridized to the array. The relative specific binding of the probes then indicates both whether the target sequence is the same or different from the reference sequence, and whether the second target sequence is the same or different from the reference sequence. In some methods, when the array comprises two groups of probes tiled for first and second reference sequences, respectively, the relative specific binding of probes in the first group indicates whether the target sequence is the same or different from the first reference sequence. The relative specific binding of probes in the second group indicates whether the target sequence is the same or different from the second reference sequence. Such methods are particularly useful for analyzing heterologous alleles of a gene. Some methods entail hybridizing both a reference sequence and a target sequence to any of the arrays of probes described above. Comparison of the relative specific binding of the probes to the reference and target sequences indicates whether the target sequence is the same or different from the reference sequence.

In a twelfth embodiment, the invention provides arrays of immobilized probes in which the probes are designed to tile a reference sequence from a human immunodeficiency virus. Reference sequences from either the reverse transcriptase gene or protease gene of HIV are of particular interest. Some chips further comprise arrays of probes tiling a reference sequence from a 16S RNA or DNA encoding the 16S RNA from a pathogenic microorganism. The invention further provides methods of using such arrays in analyzing a HIV target sequence. The methods are particularly useful where the target sequence has a substituted nucleotide relative to the reference sequence in at least one position, the substitution conferring resistance to a drug use in treating a patient infected with a HIV virus. The methods reveal the existence of the substituted nucleotide. The methods are also particularly useful for analyzing a mixture of

undetermined proportions of first and second target sequences from different HIV variants. The relative specific binding of probes indicates the proportions of the first and second target sequences.

In a thirteenth embodiment, the invention provides arrays of probes tiled based on reference sequence from a CFTR gene. A preferred array comprises at least a group of probes comprising a wildtype probe, and five sets of three mutant probes. The wildtype probe is exactly complementary to a subsequence of a reference sequence from a cystic fibrosis gene, the segment having at least five interrogation positions corresponding to five contiguous nucleotides in the reference sequence. The probes in the first set of three mutant probes are each identical to the wildtype probe, except in a first of the five interrogation positions, which is occupied by a different nucleotide in each of the three mutant probes and the wildtype probe. The probes in the second set of three mutant probes are each identical to the wildtype probe, except in a second of the five interrogation positions, which is occupied by a different nucleotide in each of the three mutant probes and the wildtype probe. The probes in the third set of three mutant probes are each identical to the wildtype probe, except in a third of the five interrogation positions, which is occupied by a different nucleotide in each of the three mutant probes and the wildtype probe. The probes in the fourth set of three mutant probes are each identical to the wildtype probe, except in a fourth of the five interrogation positions, which is occupied by a different nucleotide in each of the three mutant probes and the wildtype probe. The probes in the fifth set of three mutant probes are each identical to the wildtype probe, except in a fifth of the five interrogation positions, which is occupied by a different nucleotide in each of the three mutant probes and the wildtype probe. Preferably, a chip comprises two such groups of probes. The first group comprises a wildtype probe exactly complementary to a first reference sequence, and the second group comprises a wildtype probe exactly complementary to a second reference sequence that is a mutated form of the first reference sequence.

The invention further provides methods of using the arrays of the invention for analyzing target sequences from a CFTR gene. The methods are capable of simultaneously analyzing first and second target sequences representing heterozygous alleles of a CFTR gene.

In a fourteenth embodiment, the invention provides arrays of probes tiling a reference sequence from a p53 gene, an hMLH1 gene and/or an MSH2 gene. The invention further provides methods of using the arrays described above to analyze these genes. The method are useful, *e.g.*, for diagnosing patients susceptible to developing cancer.

In a fifteenth embodiment, the invention provides arrays of probes tiling a reference sequence from a mitochondrial genome. The reference sequence may comprise part or all of the D-loop region, or all, or substantially all, of the mitochondrial genome. The invention further provides method of using the arrays described above to analyze target sequences from a mitochondrial genome. The methods are useful for identifying mutations associated with disease, and for forensic, epidemiological and evolutionary studies.

BRIEF DESCRIPTION OF THE FIGURES

Fig. 1: Basic tiling strategy. The figure illustrates the relationship between an interrogation position (I) and a corresponding nucleotide (n) in the reference sequence, and between a probe from the first probe set and corresponding probes from second, third and fourth probe sets.

Fig. 2: Segment of complementarity in a probe from the first probe set.

Fig. 3: Incremental succession of probes in a basic tiling strategy. The figure shows four probe sets, each having three probes. Note that each probe differs from its predecessor in the same set by the acquisition of a 5' nucleotide and the loss of a 3' nucleotide, as well as in the nucleotide occupying the interrogation position.

Fig. 4: Exemplary arrangement of lanes on a chip. The chip shows four probe sets, each having five probes and each having a total of five interrogation positions (I1-I5), one per probe.

Fig. 5: Hybridization pattern of chip having probes laid down in lanes. Dark patches indicate hybridization. The probes in the lower part of the figure occur at the column of the array indicated by the arrow when the probes length is 15 and the interrogation position 7.

Fig. 6: Strategies for detecting deletion and insertion mutations. Bases in brackets may or may not be present.

Fig. 7: Block tiling strategy. The probe from the first probe set has three interrogation positions. The probes from the other probe sets have only one of these interrogation positions.

Fig. 8: Multiplex tiling strategy. Each probe has two interrogation positions.

Fig. 9. Helper mutation strategy. The segment of complementarity differs from the complement of the reference sequence at a helper mutation as well as the interrogation position.

Fig. 10 Layout of probes on the HV 407 chip. The figure shows successive rows of sequence each of which is subdivided into four lanes. The four lanes correspond to the A-, C-, G-and T-lanes on the chip. Each probe is represented by the nucleotide occupying its interrogation position. The letter "N" indicates a control probe or empty column. The different sized-probes are laid out in parallel. That is, from top-to-bottom, a row of 13 mers is followed

by a row of 15 mers, which is followed by a row of 17 mers, which is followed by a row of 19 mers.

Fig. 11 Fluorescence pattern of HV 407 hybridized to a target sequence (pPol19) identical to the chips reference sequence.

Fig. 12 Sequence read from HV 407 chip hybridized to pPol19 and 4MUT18 (separate experiments). The reference sequence is designated "wildtype." Beneath the reference sequence are four rows of sequence read from the chip hybridized to the pPol19 target, the first row being read from 13 mers, the second row from 15 mers, the third row from 17 mers and the fourth row from 19 mers. Beneath these sequences, there are four further rows of sequence read from the chip hybridized to the HXB2 target. Successive rows are read from 13 mers, 15 mers, 17 mers and 19 mers. Each nucleotide in a row is called from the relative fluorescence intensities of probes in A-, C-, G- and T-lanes. Regions of ambiguous sequence read from the chip are highlighted. The strain differences between the HBX2 sequence and the reference sequence that were correctly detected are indicated (*), and those that could not be called are indicated (o). (The nucleotide at position 417 was read correctly in some experiments). The location of some mutations known to be associated with drug resistance that occur in readable regions of the chip are shown above (codon number) and below (mutant nucleotide) the sequence designated "wildtype." The locations of primer used to amplify the target sequence are indicated by arrows.

Fig. 13: Detection of mixed target sequences. The mutant target differs from the wildtype by a single mutation in codon 67 of the reverse transcriptase gene. Each different sized group of probes has a column of four probes for reading the nucleotide in which the mutation occurs. The four probes occupying a column are represented by a single probe in the figure with the symbol (o) indicating the interrogation position, which is occupied by a different nucleotide in each probe.

Fig. 14: Fluorescence intensities of target bound to 13 mers and 15 mers for different proportions of mutant and wildtype target. The fluorescence intensities are from probes having interrogation positions for reading the nucleotide at which the mutant and wildtype targets diverge.

Fig. 15: Sequence read from protease chip from four clinical samples before and after treatment with ddI>.

Fig. 16: Block tiling array of probes for analyzing a CFTR point mutation. Each probe show actually represents four probes, with one probe having each of A, C, G or T at the interrogation position N. In the order shown, the first probe shown on the left is tiled from the wildtype reference sequence, the second probe from the mutant sequence, and so on in alternating fashion. Note that all of the probes are identical except at the interrogation position, which shifts one position between successive probes tiled from the same reference sequence (*e.g.*, the first, third and fifth probes in the left hand column.) The grid shows the hybridization intensities when the array is hybridized to the reference sequence.

Fig. 17: Hybridization pattern for heterozygous target. The figure shows the hybridization pattern when the array of the previous figure is hybridized to a mixture of mutant and wildtype reference sequences.

Fig. 18, in panels A, B, and C, shows an image made from the region of a DNA chip containing CFTR exon 10 probes; in panel A, the chip was hybridized to a wild-type target; in panel C, the chip was hybridized to a mutant ΔF508 target; and in panel B, the chip was hybridized to a mixture of the wild-type and mutant targets.

Fig. 19, in sheets 1 - 3, corresponding to panels A, B, and C of Fig. 18, shows graphs of fluorescence intensity versus tiling position. The labels on the horizontal axis show the bases in the wild-type sequence corresponding to the position of substitution in the respective probes. Plotted are the intensities observed from the features (or synthesis sites) containing wild-type probes, the features containing the substitution probes that bound the most target ("called"), and the feature containing the substitution probes that bound the target with the second highest intensity of all the substitution probes ("2nd Highest").

Fig. 20, in panels A, B, and C, shows an image made from a region of a DNA chip containing CFTR exon 10 probes; in panel A, the chip was hybridized to the wt480 target; in panel C, the chip was hybridized to the mu480 target; and in panel B, the chip was hybridized to a mixture of the wild-type and mutant targets.

Fig. 21, in sheets 1 - 3, corresponding to panels A, B, and C of Fig. 20, shows graphs of fluorescence intensity versus tiling position. The labels on the horizontal axis show the bases in the wild-type sequence corresponding to the position of substitution in the respective probes. Plotted are the intensities observed from the features (or synthesis sites) containing wild-type probes, the features containing the substitution probes that bound the most target ("called"), and the feature containing the substitution probes that bound the target with the second highest intensity of all the substitution probes ("2nd Highest").

Fig. 22, in panels A and B, shows an image made from a region of a DNA chip containing CFTR exon 10 probes; in panel A, the chip was hybridized to nucleic acid derived from the genomic DNA of an individual with wild-type ΔF508 sequences; in panel B, the target nucleic acid originated from a heterozygous (with respect to the ΔF508 mutation) individual.

Fig. 23, in sheets 1 and 2, corresponding to panels A and B of Fig. 22, shows graphs of fluorescence intensity versus tiling position. The labels on the horizontal axis show the bases in the wild-type sequence corresponding to the position of substitution in the respective probes. Plotted are the intensities observed from the features (or

synthesis sites) containing wild-type probes, the features containing the substitution probes that bound the most target ("called"), and the feature containing the substitution probes that bound the target with the second highest intensity of all the substitution probes ("2nd Highest").

Fig. 24: Hybridization of homozygous wildtype (A) and heterozygous (B) target sequences from exon 11 of the CFTR gene to a block tiling array designed to detect G551D and Q552X mutations in CFTR gene.

Fig. 25: Hybridization of homozygous wildtype (A) and ΔF508 mutant (B) target sequences from exon 10 of the CFTR gene to a block tiling array designed to detect mutations, ΔF508, ΔI507 and F508C.

Fig. 26: Hybridization of heterozygous mutant target sequences, ΔF508/F508C, to the array of Fig. 25.

Fig. 27 shows the alignment of some of the probes on a p53 DNA chip with a 12-mer model target nucleic acid.

Fig. 28 shows a set of 10-mer probes for a p53 exon 6 DNA chip.

Fig. 29 shows that very distinct patterns are observed after hybridization of p53 DNA chips with targets having different 1 base substitutions. In the first image in Fig. 29, the 12-mer probes that form perfect matches with the wild-type target are in the first row (top). The 12-mer probes with single base mismatches are located in the second, third, and fourth rows and have much lower signals.

Fig. 30, in graphs 2, 3, and 4, graphically depicts the data in Fig. 29. On each graph, the X ordinate is the position of the probe in its row on the chip, and the Y ordinate is the signal at that probe site after hybridization.

Fig. 31 shows the results of hybridizing mixed target populations of WT and mutant p53 genes to the p53 DNA chip.

Fig. 32, in graphs 1-4, shows (see Fig. 30 as well) the hybridization efficiency of a 10-mer probe array as compared to a 12-mer probe array.

Fig. 33 shows an image of a p53 DNA chip hybridized to a target DNA.

Fig. 34 illustrates how the actual sequence was read from the chip shown in Fig. 33. Gaps in the sequence of letters in the WT rows correspond to control probes or sites. Positions at which bases are miscalled are represented by letters in italic type in cells corresponding to probes in which the WT bases have been substituted by other bases.

Fig. 35 shows the human mitochondrial genome; "$O_H$" is the H strand origin of replication, and arrows indicate the cloned unshaded sequence.

Fig. 36 shows the image observed from application of a sample of mitochondrial DHA derived nucleic acid (from the mt4 sample) on a DNA chip.

Fig. 37 is similar to Fig. 36 but shows the image observed from the mt5 sample.

Fig. 38 shows the predicted difference image between the mt4 and mt5 samples on the DNA chip based on mismatches between the two samples and the reference sequence.

Fig. 39 shows the actual difference image observed for the mt4 and mt5 samples.

Fig. 40, in sheets 1 and 2, shows a plot of normalized intensities across rows 10 and 11 of the array and a tabulation of the mutations detected.

Fig. 41 shows the discrimination between wild-type and mutant hybrids obtained with the chip. A median of the six normalized hybridization scores for each probe was taken; the graph plots the ratio of the median score to the normalized hybridization score versus mean counts. A ratio of 1.6 and mean counts above 50 yield no false positives.

Fig. 42 illustrates how the identity of the base mismatch may influence the ability to discriminate mutant and wild-type sequences more than the position of the mismatch within an oligonucleotide probe. The mismatch position is expressed as % of probe length from the 3'-end. The base change is indicated on the graph.

Fig. 43 provides a 5' to 3' sequence listing of one target corresponding to the probes on the chip. X is a control probe. Positions that differ in the target (*i.e.*, are mismatched with the probe at the designated site) are in bold.

Fig. 44 shows the fluorescence image produced by scanning the chip described in Fig. 17 when hybridized to a sample.

Fig. 45 illustrates the detection of 4 transitions in the target sequence relative to the wild-type probes on the chip in Fig. 44.

Fig. 46: VLSIPS™ technology applied to the light directed synthesis of oligonucleotides. Light (hv) is shone through a mask ($M_1$) to activate functional groups (-OH) on a surface by removal of a protecting group (X). Nucleoside building blocks protected with photoremovable protecting groups (T-X, C-X) are coupled to the activated areas. By repeating the irradiation and coupling steps, very complex arrays of oligonucleotides can be prepared.

Fig. 47: Use of the VLSIPS™ process to prepare "nucleoside combinatorials" or oligonucleotides synthesized by coupling all four nucleosides to form dimers, trimers, and so forth.

Fig. 48: Deprotection, coupling, and oxidation steps of a solid phase DNA synthesis method.

Fig. 49: An illustrative synthesis route for the nucleoside building blocks used in the VLSIPS™ method.

Fig. 50: A preferred photoremovable protecting group, MeNPOC, and preparation of the group in active form.

Fig. 51: Detection system for scanning a DNA chip.

DETAILED DESCRIPTION OF THE INVENTION

The invention provides a number of strategies for comparing a polynucleotide of known sequence (a reference sequence) with variants of that sequence (target sequences). The comparison can be performed at the level of entire genomes, chromosomes, genes, exons or introns, or can focus on individual mutant sites and immediately adjacent bases. The strategies allow detection of variations, such as mutations or polymorphisms, in the target sequence irrespective whether a particular variant has previously been characterized. The strategies both define the nature of a variant and identify its location in a target sequence.

The strategies employ arrays of oligonucleotide probes immobilized to a solid support. Target sequences are analyzed by determining the extent of hybridization at particular probes in the array. The strategy in selection of probes facilitates distinction between perfectly matched probes and probes showing single-base or other degrees of mismatches. The strategy usually entails sampling each nucleotide of interest in a target sequence several times, thereby achieving a high degree of confidence in its identity. This level of confidence is further increased by sampling of adjacent nucleotides in the target sequence to nucleotides of interest. The number of probes on the chip can be quite large (*e. g.*, $10^5$-$10^6$). However, usually only a small proportion of the total number of probes of a given length are represented. Some advantage of the use of only a small proportion of all possible probes of a given length include: (i) each position in the array is highly informative, whether or not hybridization occurs; (ii) nonspecific hybridization is minimized; (iii) it is straightforward to correlate hybridization differences with sequence differences, particularly with reference to the hybridization pattern of a known standard; and (iv) the ability to address each probe independently during synthesis, using high resolution photolithography, allows the array to be designed and optimized for any sequence. For example the length of any probe can be varied independently of the others.

The present tiling strategies result in sequencing and comparison methods suitable for routine large-scale practice with a high degree of confidence in the sequence output.

I. GENERAL TILING STRATEGIES

A. Selection of Reference Sequence

The chips are designed to contain probes exhibiting complementarity to one or more selected reference sequence whose sequence is known. The chips are used to read a target sequence comprising either the reference sequence itself or variants of that sequence. Target sequences may differ from the reference sequence at one or more positions but show a high overall degree of sequence identity with the reference sequence (*e.g.*, at least 75, 90, 95, 99, 99.9 or 99.99%). Any polynucleotide of known sequence can be selected as a reference sequence. Reference sequences of interest include sequences known to include mutations or polymorphisms associated with phenotypic changes having clinical significance in human patients. For example, the CFTR gene and P53 gene in humans have been identified as the location of several mutations resulting in cystic fibrosis or cancer respectively. Other reference sequences of interest include those that serve to identify pathogenic microorganisms and/or are the site of mutations by which such microorganisms acquire drug resistance (*e.g.*, the HIV reverse transcriptase gene). Other reference sequences of interest include regions where polymorphic variations are known to occur (*e.g.*, the D-loop region of mitochondrial DNA). These reference sequences have utility for, *e.g.*, forensic or epidemiological studies. Other reference sequences of interest include p34 (related to p53), p65 (implicated in breast, prostate and liver cancer), and DNA segments encoding cytochromes P450 (*see* Meyer et al., *Pharmac. Ther*. 46, 349-355 (1990)). Other reference sequences of interest include those from the genome of pathogenic viruses (*e.g.,* hepatitis (A, B, or C), herpes virus (*e. g.*, VZV, HSV-1, HAV-6, HSV-II, and CMV, Epstein Barr virus), adenovirus, influenza virus, flaviviruses, echovirus, rhinovirus, coxsackie virus, cornovirus, respiratory syncytial virus, mumps virus, rotavirus, measles virus, rubella virus, parvovirus, vaccinia virus, HTLV virus, dengue virus, papillomavirus, molluscum virus, poliovirus, rabies virus, JC virus and arboviral encephalitis virus. Other reference sequences of interest are from genomes or episomes of pathogenic bacteria, particularly regions that confer drug resistance or allow phylogenic characterization of the host (*e.g.*, 16S rRNA or corresponding DNA). For example, such bacteria include chlamydia, rickettsial bacteria, mycobacteria, staphylococci, treptocci, pneumonococci, meningococci and conococci, klebsiella, proteus, serratia, pseudomonas, legionella, diphtheria, salmonella, bacilli, cholera, tetanus, botulism, anthrax, plague, leptospirosis, and Lymes disease bacteria. Other reference sequences of interest include those in which mutations result in the following autosomal recessive disorders: sickle cell anemia, β-thalassemia, phenylketonuria, galactosemia, Wilson's disease, hemochromatosis, severe combined immunodeficiency, alpha-1-antitrypsin deficiency, albinism, alkaptonuria, lysosomal storage diseases and Ehlers-Danlos syndrome. Other reference sequences of interest include those in which mutations result in X-linked recessive disorders: hemophilia, glucose-6-phosphate dehydrogenase, agammaglobulimenia, diabetes insipidus, Lesch-Nyhan syndrome, muscular dystrophy, Wiskott-Aldrich syndrome, Fabry's disease and fragile X-syndrome. Other reference sequences of interest includes those in which mutations result in the following autosomal

dominant disorders: familial hypercholesterolemia, polycystic kidney disease, Huntingdon's disease, hereditary spherocytosis, Marfan's syndrome, von Willebrand's disease, neurofibromatosis, tuberous sclerosis, hereditary hemorrhagic telangiectasia, familial colonic polyposis, Ehlers-Danlos syndrome, myotonic dystrophy, muscular dystrophy, osteogenesis imperfecta, acute intermittent porphyria, and von Hippel-Lindau disease.

The length of a reference sequence can vary widely from a full-length genome, to an individual chromosome, episome, gene, component of a gene, such as an exon, intron or regulatory sequences, to a few nucleotides. A reference sequence of between about 2, 5, 10, 20, 50, 100, 5000, 1000, 5,000 or 10,000, 20,000 or 100,000 nucleotides is common. Sometimes only particular regions of a sequence (*e.g.*, exons of a gene) are of interest. In such situations, the particular regions can be considered as separate reference sequences or can be considered as components of a single reference sequence, as matter of arbitrary choice.

A reference sequence can be any naturally occurring, mutant, consensus or purely hypothetical sequence of nucleotides, RNA or DNA. For example, sequences can be obtained from computer data bases, publications or can be determined or conceived *de novo.* Usually, a reference sequence is selected to show a high degree of sequence identity to envisaged target sequences. Often, particularly, where a significant degree of divergence is anticipated between target sequences, more than one reference sequence is selected. Combinations of wildtype and mutant reference sequences are employed in several applications of the tiling strategy.

B. Chip Design

1. Basic Tiling Strategy

The basic tiling strategy provides an array of immobilized probes for analysis of target sequences showing a high degree of sequence identity to one or more selected reference sequences. The strategy is first illustrated for an array that is subdivided into four probe sets, although it will be apparent that in some situations, satisfactory results are obtained from only two probe sets. A first probe set comprises a plurality of probes exhibiting perfect complementarity with a selected reference sequence. The perfect complementarity usually exists throughout the length of the probe. However, probes having a segment or segments of perfect complementarity that is/are flanked by leading or trailing sequences lacking complementarity to the reference sequence can also be used. Within a segment of complementarity, each probe in the first probe set has at least one interrogation position that corresponds to a nucleotide in the reference sequence. That is, the interrogation position is aligned with the corresponding nucleotide in the reference sequence, when the probe and reference sequence are aligned to maximize complementarity between the two. If a probe has more than one interrogation position, each corresponds with a respective nucleotide in the reference sequence. The identity of an interrogation position and corresponding nucleotide in a particular probe in the first probe set cannot be determined simply by inspection of the probe in the first set. As will become apparent, an interrogation position and corresponding nucleotide is defined by the comparative structures of probes in the first probe set and corresponding probes from additional probe sets.

In principle, a probe could have an interrogation position at each position in the segment complementary to the reference sequence. Sometimes, interrogation positions provide more accurate data when located away from the ends of a segment of complementarity. Thus, typically a probe having a segment of complementarity of length x does not contain more than x-2 interrogation positions. Since probes are typically 9-21 nucleotides, and usually all of a probe is complementary, a probe typically has 1-19 interrogation positions. Often the probes contain a single interrogation position, at or near the center of probe.

For each probe in the first set, there are, for purposes of the present illustration, three corresponding probes from three additional probe sets. *See* Fig. 1. Thus, there are four probes corresponding to each nucleotide of interest in the reference sequence. Each of the four corresponding probes has an interrogation position aligned with that nucleotide of interest. Usually, the probes from the three additional probe sets are identical to the corresponding probe from the first probe set with one exception. The exception is that at least one (and often only one) interrogation position, which occurs in the same position in each of the four corresponding probes from the four probe sets, is occupied by a different nucleotide in the four probe sets. For example, for an A nucleotide in the reference sequence, the corresponding probe from the first probe set has its interrogation position occupied by a T, and the corresponding probes from the additional three probe sets have their respective interrogation positions occupied by A, C, or G, a different nucleotide in each probe. Of course, if a probe from the first probe set comprises trailing or flanking sequences lacking complementarity to the reference sequences (*see* Fig. 2), these sequences need not be present in corresponding probes from the three additional sets. Likewise corresponding probes from the three additional sets can contain leading or trailing sequences outside the segment of complementarity that are not present in the corresponding probe from the first probe set. Occasionally, the probes from the additional three probe set are identical (with the exception of interrogation position (s)) to a contiguous subsequence of the full complementary segment of the corresponding probe from the first probe set. In this case, the subsequence includes the interrogation position and usually differs from the full-length probe only

in the omission of one or both terminal nucleotides from the termini of a segment of complementarity. That is, if a probe from the first probe set has a segment of complementarity of length n, corresponding probes from the other sets will usually include a subsequence of the segment of at least length n-2. Thus, the subsequence is usually at least 3, 4, 7, 9, 15, 21, or 25 nucleotides long, most typically, in the range of 9-21 nucleotides. The subsequence should be sufficiently long to allow a probe to hybridize detectably more strongly to a variant of the reference sequence mutated at the interrogation position than to the reference sequence.

The probes can be oligodeoxyribonucleotides or oligoribonucleotides, or any modified forms of these polymers that are capable of hybridizing with a target nucleic sequence by complementary base-pairing. Complementary base pairing means sequence-specific base pairing which includes *e.g.*, Watson-Crick base pairing as well as other forms of base pairing such as Hoogsteen base pairing. Modified forms include 2'-O-methyl oligoribonucleotides and so-called PNAs, in which oligodeoxyribonucleotides are linked via peptide bonds rather than phophodiester bonds. The probes can be attached by any linkage to a support (e.g., 3', 5' or via the base). 3' attachment is more usual as this orientation is compatible with the preferred chemistry for solid phase synthesis of oligonucleotides.

The number of probes in the first probe set (and as a consequence the number of probes in additional probe sets) depends on the length of the reference sequence, the number of nucleotides of interest in the reference sequence and the number of interrogation positions per probe. In general, each nucleotide of interest in the reference sequence requires the same interrogation position in the four sets of probes. Consider, as an example, a reference sequence of 100 nucleotides, 50 of which are of interest, and probes each having a single interrogation position. In this situation, the first probe set requires fifty probes, each having one interrogation position corresponding to a nucleotide of interest in the reference sequence. The second, third and fourth probe sets each have a corresponding probe for each probe in the first probe set, and so each also contains a total of fifty probes. The identity of each nucleotide of interest in the reference sequence is determined by comparing the relative hybridization signals at four probes having interrogation positions corresponding to that nucleotide from the four probe sets.

In some reference sequences, every nucleotide is of interest. In other reference sequences, only certain portions in which variants (*e.g.*, mutations or polymorphisms) are concentrated are of interest. In other reference sequences, only particular mutations or polymorphisms and immediately adjacent nucleotides are of interest. Usually, the first probe set has interrogation positions selected to correspond to at least a nucleotide (*e.g.,* representing a point mutation) and one immediately adjacent nucleotide. Usually, the probes in the first set have interrogation positions corresponding to at least 3, 10, 50, 100, 1000, or 20,000 contiguous nucleotides. The probes usually have interrogation positions corresponding to at least 5, 10, 30, 50, 75, 90, 99 or sometimes 100% of the nucleotides in a reference sequence. Frequently, the probes in the first probe set completely span the reference sequence and overlap with one another relative to the reference sequence. For example, in one common arrangement each probe in the first probe set differs from another probe in that set by the omission of a 3' base complementary to the reference sequence and the acquisition of a 5' base complementary to the reference sequence. *See* Fig. 3.

For conceptual simplicity, the probes in a set are usually arranged in order of the sequence in a lane across the chip. A lane contains a series of overlapping probes, which represent or tile across, the selected reference sequence (*see* Fig. 3). The components of the four sets of probes are usually laid down in four parallel lanes, collectively constituting a row in the horizontal direction and a series of 4-member columns in the vertical direction. Corresponding probes from the four probe sets (*i.e.*, complementary to the same subsequence of the reference sequence) occupy a column. Each probe in a lane usually differs from its predecessor in the lane by the omission of a base at one end and the inclusion of additional base at the other end as shown in Fig. 3. However, this orderly progression of probes can be interrupted by the inclusion of control probes or omission of probes in certain columns of the array. Such columns serve as controls to orient the chip, or gauge the background, which can include target sequence nonspecifically bound to the chip.

The probes sets are usually laid down in lanes such that all probes having an interrogation position occupied by an A form an A-lane, all probes having an interrogation position occupied by a C form a C-lane, all probes having an interrogation position occupied by a G form a G-lane, and all probes having an interrogation position occupied by a T (or U) form a T lane (or a U lane). Note that in this arrangement there is not a unique correspondence between probe sets and lanes. Thus, the probe from the first probe set is laid down in the A-lane, C-lane, A-lane, A-lane and T-lane for the five columns in Fig. 4. The interrogation position on a column of probes corresponds to the position in the target sequence whose identity is determined from analysis of hybridization to the probes in that column. Thus, $I_1$-$I_5$ respectively correspond to $N_1$-$N_5$ in Fig. 4. The interrogation position can be anywhere in a probe but is usually at or near the central position of the probe to maximize differential hybridization signals between a perfect match and a single-base mismatch. For example, for an 11 mer probe, the central position is the sixth nucleotide.

Although the array of probes is usually laid down in rows and columns as described above, such a physical arrangement of probes on the chip is not essential. Provided that the spatial location of each probe in an array is known, the data from the probes can be collected and processed to yield the sequence of a target irrespective of the physical arrangement of the probes on a chip. In processing the data, the hybridization signals from the respective probes can

be reassorted into any conceptual array desired for subsequent data reduction whatever the physical arrangement of probes on the chip.

A range of lengths of probes can be employed in the chips. As noted above, a probe may consist exclusively of a complementary segments, or may have one or more complementary segments juxtaposed by flanking, trailing and/or intervening segments. In the latter situation, the total length of complementary segment(s) is more important that the length of the probe. In functional terms, the complementarity segment(s) of the first probe sets should be sufficiently long to allow the probe to hybridize detectably more strongly to a reference sequence compared with a variant of the reference including a single base mutation at the nucleotide corresponding to the interrogation position of the probe. Similarly, the complementarity segment(s) in corresponding probes from additional probe sets should be sufficiently long to allow a probe to hybridize detectably more strongly to a variant of the reference sequence having a single nucleotide substitution at the interrogation position relative to the reference sequence. A probe usually has a single complementary segment having a length of at least 3 nucleotides, and more usually at least 5, 6, 7, 8, 9, 10, 11, 12, 13, 14, 15, 16, 17, 18, 19, 20, 21, 22, 23, 24, 25 or 30 bases exhibiting perfect complementarity (other than possibly at the interrogation position(s) depending on the probe set) to the reference sequence. In bridging strategies, where more than one segment of complementarity is present, each segment provides at least three complementary nucleotides to the reference sequence and the combined segments provide at least two segments of three or a total of six complementary nucleotides. As in the other strategies, the combined length of complementary segments is typically from 6-30 nucleotides, and preferably from about 9-21 nucleotides. The two segments are often approximately the same length. Often, the probes (or segment of complementarity within probes) have an odd number of bases, so that an interrogation position can occur in the exact center of the probe.

In some chips, all probes are the same length. Other chips employ different groups of probe sets, in which case the probes are of the same size within a group, but differ between different groups. For example, some chips have one group comprising four sets of probes as described above in which all the probes are 11 mers, together with a second group comprising four sets of probes in which all of the probes are 13 mers. Of course, additional groups of probes can be added. Thus, some chips contain, *e.g.,* four groups of probes having sizes of 11 mers, 13 mers, 15 mers and 17 mers. Other chips have different size probes within the same group of four probe sets. In these chips, the probes in the first set can vary in length independently of each other. Probes in the other sets are usually the same length as the probe occupying the same column from the first set. However, occasionally different lengths of probes can be included at the same column position in the four lanes. The different length probes are included to equalize hybridization signals from probes irrespective of whether A-T or C-G bonds are formed at the interrogation position.

The length of probe can be important in distinguishing between a perfectly matched probe and probes showing a single-base mismatch with the target sequence. The discrimination is usually greater for short probes. Shorter probes are usually also less susceptible to formation of secondary structures. However, the absolute amount of target sequence bound, and hence the signal, is greater for larger probes. The probe length representing the optimum compromise between these competing considerations may vary depending on *inter alia* the GC content of a particular region of the target DNA sequence, secondary structure, synthesis efficiency and cross-hybridization. In some regions of the target, depending on hybridization conditions, short probes (*e.g.*, 11 mers) may provide information that is inaccessible from longer probes (*e.g.*, 19 mers) and vice versa. Maximum sequence information can be read by including several groups of different sized probes on the chip as noted above. However, for many regions of the target sequence, such a strategy provides redundant information in that the same sequence is read multiple times from the different groups of probes. Equivalent information can be obtained from a single group of different sized probes in which the sizes are selected to maximize readable sequence at particular regions of the target sequence. The appropriate size of probes at different regions of the target sequence can be determined from, *e.g.*, Fig. 12, which compares the readability of different sized probes in different regions of a target. The strategy of customizing probe length within a single group of probe sets minimizes the total number of probes required to read a particular target sequence. This leaves ample capacity for the chip to include probes to other reference sequences.

The invention provides an optimization block which allows systematic variation of probe length and interrogation position to optimize the selection of probes for analyzing a particular nucleotide in a reference sequence. The block comprises alternating columns of probes complementary to the wildtype target and probes complementary to a specific mutation. The interrogation position is varied between columns and probe length is varied down a column. Hybridization of the chip to the reference sequence or the mutant form of the reference sequence identifies the probe length and interrogation position providing the greatest differential hybridization signal.

The probes are designed to be complementary to either strand of the reference sequence (*e.g.*, coding or non-coding). Some chips contain separate groups of probes, one complementary to the coding strand, the other complementary to the noncoding strand. Independent analysis of coding and noncoding strands provides largely redundant information. However, the regions of ambiguity in reading the coding strand are not always the same as those in reading the noncoding strand. Thus, combination of the information from coding and noncoding strands increases the overall accuracy of sequencing.

Some chips contain additional probes or groups of probes designed to be complementary to a second reference sequence. The second reference sequence is often a subsequence of the first reference sequence bearing one or more commonly occurring mutations or interstrain variations. The second group of probes is designed by the same principles as described above except that the probes exhibit complementarity to the second reference sequence. The inclusion of a second group is particular useful for analyzing short subsequences of the primary reference sequence in which multiple mutations are expected to occur within a short distance commensurate with the length of the probes (*i.e.*, two or more mutations within 9 to 21 bases). Of course, the same principle can be extended to provide chips containing groups of probes for any number of reference sequences. Alternatively, the chips may contain additional probe(s) that do not form part of a tiled array as noted above, but rather serves as probe(s) for a conventional reverse dot blot. For example, the presence of mutation can be detected from binding of a target sequence to a single oligomeric probe harboring the mutation. Preferably, an additional probe containing the equivalent region of the wildtype sequence is included as a control.

The chips are read by comparing the intensities of labelled target bound to the probes in an array. Specifically, a comparison is performed between each lane of probes (*e.g.*, A, C, G and T lanes) at each columnar position (physical or conceptual). For a particular columnar position, the lane showing the greatest hybridization signal is called as the nucleotide present at the position in the target sequence corresponding to the interrogation position in the probes. See Fig. 5. The corresponding position in the target sequence is that aligned with the interrogation position in corresponding probes when the probes and target are aligned to maximize complementarity. Of the four probes in a column, only one can exhibit a perfect match to the target sequence whereas the others usually exhibit at least a one base pair mismatch. The probe exhibiting a perfect match usually produces a substantially greater hybridization signal than the other three probes in the column and is thereby easily identified. However, in some regions of the target sequence, the distinction between a perfect match and a one-base mismatch is less clear. Thus, a call ratio is established to define the ratio of signal from the best hybridizing probes to the second best hybridizing probe that must be exceeded for a particular target position to be read from the probes. A high call ratio ensures that few if any errors are made in calling target nucleotides, but can result in some nucleotides being scored as ambiguous, which could in fact be accurately read. A lower call ratio results in fewer ambiguous calls, but can result in more erroneous calls. It has been found that at a call ratio of 1.2 virtually all calls are accurate. However, a small but significant number of bases (*e.g.*, up to about 10%) may have to be scored as ambiguous.

Although small regions of the target sequence can sometimes be ambiguous, these regions usually occur at the same or similar segments in different target sequences. Thus, for precharacterized mutations, it is known in advance whether that mutation is likely to occur within a region of unambiguously determinable sequence.

An array of probes is most useful for analyzing the reference sequence from which the probes were designed and variants of that sequence exhibiting substantial sequence similarity with the reference sequence (*e.g.*, several single-base mutants spaced over the reference sequence). When an array is used to analyze the exact reference sequence from which it was designed, one probe exhibits a perfect match to the reference sequence, and the other three probes in the same column exhibits single-base mismatches. Thus, discrimination between hybridization signals is usually high and accurate sequence is obtained. High accuracy is also obtained when an array is used for analyzing a target sequence comprising a variant of the reference sequence that has a single mutation relative to the reference sequence, or several widely spaced mutations relative to the reference sequence. At different mutant loci, one probe exhibits a perfect match to the target, and the other three probes occupying the same column exhibit single-base mismatches, the difference (with respect to analysis of the reference sequence) being the lane in which the perfect match occurs.

For target sequences showing a high degree of divergence from the reference strain or incorporating several closely spaced mutations from the reference strain, a single group of probes (*i.e.*, designed with respect to a single reference sequence) will not always provide accurate sequence for the highly variant region of this sequence. At some particular columnar positions, it may be that no single probe exhibits perfect complementarity to the target and that any comparison must be based on different degrees of mismatch between the four probes. Such a comparison does not always allow the target nucleotide corresponding to that columnar position to be called. Deletions in target sequences can be detected by loss of signal from probes having interrogation positions encompassed by the deletion. However, signal may also be lost from probes having interrogation positions closely proximal to the deletion resulting in some regions of the target sequence that cannot be read. Target sequence bearing insertions will also exhibit short regions including and proximal to the insertion that usually cannot be read.

The presence of short regions of difficult-to-read target because of closely spaced mutations, insertions or deletion, does not prevent determination of the remaining sequence of the target as different regions of a target sequence are determined independently. Moreover, such ambiguities as might result from analysis of diverse variants with a single group of probes can be avoided by including multiple groups of probe sets on a chip. For example, one group of probes can be designed based on a full-length reference sequence, and the other groups on subsequences of the reference sequence incorporating frequently occurring mutations or strain variations.

A particular advantage of the present sequencing strategy over conventional sequencing methods is the capacity simultaneously to detect and quantify proportions of multiple target sequences. Such capacity is valuable, *e.g.,* for diagnosis of patients who are heterozygous with respect to a gene or who are infected with a virus, such as HIV, which is usually present in several polymorphic forms. Such capacity is also useful in analyzing targets from biopsies of tumor cells and surrounding tissues. The presence of multiple target sequences is detected from the relative signals of the four probes at the array columns corresponding to the target nucleotides at which diversity occurs. The relative signals at the four probes for the mixture under test are compared with the corresponding signals from a homogeneous reference sequence. An increase in a signal from a probe that is mismatched with respect to the reference sequence, and a corresponding decrease in the signal from the probe which is matched with the reference sequence signal the presence of a mutant strain in the mixture. The extent in shift in hybridization signals of the probes is related to the proportion of a target sequence in the mixture. Shifts in relative hybridization signals can be quantitatively related to proportions of reference and mutant sequence by prior calibration of the chip with seeded mixtures of the mutant and reference sequences. By this means, a chip can be used to detect variant or mutant strains constituting as little as 1, 5, 20, or 25 % of a mixture of stains.

Similar principles allow the simultaneous analysis of multiple target sequences even when none is identical to the reference sequence. For example, with a mixture of two target sequences bearing first and second mutations, there would be a variation in the hybridization patterns of probes having interrogation positions corresponding to the first and second mutations relative to the hybridization pattern with the reference sequence. At each position, one of the probes having a mismatched interrogation position relative to the reference sequence would show an increase in hybridization signal, and the probe having a matched interrogation position relative to the reference sequence would show a decrease in hybridization signal. Analysis of the hybridization pattern of the mixture of mutant target sequences, preferably in comparison with the hybridization pattern of the reference sequence, indicates the presence of two mutant target sequences, the position and nature of the mutation in each strain, and the relative proportions of each strain.

In a variation of the above method, the different components in a mixture of target sequences are differentially labelled before being applied to the array. For example, a variety of fluorescent labels emitting at different wavelength are available. The use of differential labels allows independent analysis of different targets bound simultaneously to the array. For example, the methods permit comparison of target sequences obtained from a patient at different stages of a disease.

2. Omission of Probes

The general strategy outlined above employs four probes to read each nucleotide of interest in a target sequence. One probe (from the first probe set) shows a perfect match to the reference sequence and the other three probes (from the second, third and fourth probe sets) exhibit a mismatch with the reference sequence and a perfect match with a target sequence bearing a mutation at the nucleotide of interest. The provision of three probes from the second, third and fourth probe sets allows detection of each of the three possible nucleotide substitutions of any nucleotide of interest. However, in some reference sequences or regions of reference sequences, it is known in advance that only certain mutations are likely to occur. Thus, for example, at one site it might be known that an A nucleotide in the reference sequence may exist as a T mutant in some target sequences but is unlikely to exist as a C or G mutant. Accordingly, for analysis of this region of the reference sequence, one might include only the first and second probe sets, the first probe set exhibiting perfect complementarity to the reference sequence, and the second probe set having an interrogation position occupied by an invariant A residue (for detecting the T mutant). In other situations, one might include the first, second and third probes sets (but not the fourth) for detection of a wildtype nucleotide in the reference sequence and two mutant variants thereof in target sequences. In some chips, probes that would detect silent mutations (*i.e.*, not affecting amino acid sequence) are omitted.

In some chips, the probes from the first probe set are omitted corresponding to some or all positions of the reference sequences. Such chips comprise at least two probe sets. The first probe set has a plurality of probes. Each probe comprises a segment exactly complementary to a subsequence of a reference sequence except in at least one interrogation position. A second probe set has a corresponding probe for each probe in the first probe set. The corresponding probe in the second probe set is identical to a sequence comprising the corresponding probe form the first probe set or a subsequence thereof that includes the at least one (and usually only one) interrogation position except that the at least one interrogation position is occupied by a different nucleotide in each of the two corresponding probes from the first and second probe sets. A third probe set, if present, also comprises a corresponding probe for each probe in the first probe set except at the at least one interrogation position, which differs in the corresponding probes from the three sets. Omission of probes having a segment exhibiting perfect complementarity to the reference sequence results in loss of control information, *i.e.*, the detection of nucleotides in a target sequence that are the same as those in a reference sequence. However, similar information can be obtained by hybridizing a chip lacking probes from the first probe set to both target and reference sequences. The hybridization can be performed sequentially, or concurrently,

if the target and reference are differentially labelled. In this situation, the presence of a mutation is detected by a shift in the background hybridization intensity of the reference sequence to a perfectly matched hybridization signal of the target sequence, rather than by a comparison of the hybridization intensities of probes from the first set with corresponding probes from the second, third and fourth sets.

3. Wildtype Probe Lane

When the chips comprise four probe sets, as discussed *supra,* and the probe sets are laid down in four lanes, an A lane, a C-lane, a G lane and a T or U lane, the probe having a segment exhibiting perfect complementarity to a reference sequence varies between the four lanes from one column to another. This does not present any significant difficulty in computer analysis of the data from the chip. However, visual inspection of the hybridization pattern of the chip is sometimes facilitated by provision of an extra lane of probes, in which each probe has a segment exhibiting perfect complementarity to the reference sequence. *See* Fig. 4. This segment is identical to a segment from one of the probes in the other four lanes (which lane depending on the column position). The extra lane of probes (designated the wildtype lane) hybridizes to a target sequence at all nucleotide positions except those in which deviations from the reference sequence occurs. The hybridization pattern of the wildtype lane thereby provides a simple visual indication of mutations.

4. Deletion, Insertion and Multiple-Mutation Probes

Some chips provide an additional probe set specifically designed for analyzing deletion mutations. The additional probe set comprises a probe corresponding to each probe in the first probe set as described above. However, a probe from the additional probe set differs from the corresponding probe in the first probe set in that the nucleotide occupying the interrogation position is deleted in the probe from the additional probe set. *See* Fig. 6. Optionally, the probe from the additional probe set bears an additional nucleotide at one of its termini relative to the corresponding probe from the first probe set. The probe from the additional probe set will hybridize more strongly than the corresponding probe from the first probe set to a target sequence having a single base deletion at the nucleotide corresponding to the interrogation position. Additional probe sets are provided in which not only the interrogation position, but also an adjacent nucleotide is detected.

Similarly, other chips provide additional probe sets for analyzing insertions. For example, one additional probe set has a probe corresponding to each probe in the first probe set as described above. However, the probe in the additional probe set has an extra T nucleotide inserted adjacent to the interrogation position. *See* Fig. 6. Optionally, the probe has one fewer nucleotide at one of its termini relative to the corresponding probe from the first probe set. The probe from the additional probe set hybridizes more strongly than the corresponding probe from the first probe set to a target sequence having an A nucleotide inserted in a position adjacent to that corresponding to the interrogation position. Similar additional probe sets are constructed having C, G or T/U nucleotides inserted adjacent to the interrogation position. Usually, four such probe sets, one for each nucleotide, are used in combination.

Other chips provide additional probes (multiple-mutation probes) for analyzing target sequences having multiple closely spaced mutations. A multiple-mutation probe is usually identical to a corresponding probe from the first set as described above, except in the base occupying the interrogation position, and except at one or more additional positions, corresponding to nucleotides in which substitution may occur in the reference sequence. The one or more additional positions in the multiple mutation probe are occupied by nucleotides complementary to the nucleotides occupying corresponding positions in the reference sequence when the possible substitutions have occurred.

5. Block Tiling

As noted in the discussion of the general tiling strategy, a probe in the first probe set sometimes has more than one interrogation position. In this situation, a probe in the first probe set is sometimes matched with multiple groups of at least one, and usually, three additional probe sets. *See* Fig. 7. Three additional probe sets are used to allow detection of the three possible nucleotide substitutions at any one position. If only certain types of substitution are likely to occur (*e.g.*, transitions), only one or two additional probe sets are required (analogous to the use of probes in the basic tiling strategy). To illustrate for the situation where a group comprises three additional probe sets, a first such group comprises second, third and fourth probe sets, each of which has a probe corresponding to each probe in the first probe set. The corresponding probes from the second, third and fourth probes sets differ from the corresponding probe in the first set at a first of the interrogation positions. Thus, the relative hybridization signals from corresponding probes from the first, second, third and fourth probe sets indicate the identity of the nucleotide in a target sequence corresponding to the first interrogation position. A second group of three probe sets (designated fifth, sixth and seventh probe sets), each also have a probe corresponding to each probe in the first probe set. These corresponding probes

differ from that in the first probe set at a second interrogation position. The relative hybridization signals from corresponding probes from the first, fifth, sixth, and seventh probe sets indicate the identity of the nucleotide in the target sequence corresponding to the second interrogation position. As noted above, the probes in the first probe set often have seven or more interrogation positions. If there are seven interrogation positions, there are seven groups of three additional probe sets, each group of three probe sets serving to identify the nucleotide corresponding to one of the seven interrogation positions.

Each block of probes allows short regions of a target sequence to be read. For example, for a block of probes having seven interrogation positions, seven nucleotides in the target sequence can be read. Of course, a chip can contain any number of blocks depending on how many nucleotides of the target are of interest. The hybridization signals for each block can be analyzed independently of any other block. The block tiling strategy can also be combined with other tiling strategies, with different parts of the same reference sequence being tiled by different strategies.

The block tiling strategy offers two advantages over the basic strategy in which each probe in the first set has a single interrogation position. One advantage is that the same sequence information can be obtained from fewer probes. A second advantage is that each of the probes constituting a block (i.e., a probe from the first probe set and a corresponding probe from each of the other probe sets) can have identical 3' and 5' sequences, with the variation confined to a central segment containing the interrogation positions. The identity of 3' sequence between different probes simplifies the strategy for solid phase synthesis of the probes on the chip and results in more uniform deposition of the different probes on the chip, thereby in turn increasing the uniformity of signal to noise ratio for different regions of the chip. A third advantage is that greater signal uniformity is achieved within a block.

6. Multiplex Tiling

In the block tiling strategy discussed above, the identity of a nucleotide in a target or reference sequence is determined by comparison of hybridization patterns of one probe having a segment showing a perfect match with that of other probes (usually three other probes) showing a single base mismatch. In multiplex tiling, the identity of at least two nucleotides in a reference or target sequence is determined by comparison of hybridization signal intensities of four probes, two of which have a segment showing perfect complementarity or a single base mismatch to the reference sequence, and two of which have a segment showing perfect complementarity or a double-base mismatch to a segment. The four probes whose hybridization patterns are to be compared each have a segment that is exactly complementary to a reference sequence except at two interrogation positions, in which the segment may or may not be complementary to the reference sequence. The interrogation positions correspond to the nucleotides in a reference or target sequence which are determined by the comparison of intensities. The nucleotides occupying the interrogation positions in the four probes are selected according to the following rule. The first interrogation position is occupied by a different nucleotide in each of the four probes. The second interrogation position is also occupied by a different nucleotide in each of the four probes. In two of the four probes, designated the first and second probes, the segment is exactly complementary to the reference sequence except at not more than one of the two interrogation positions. In other words, one of the interrogation positions is occupied by a nucleotide that is complementary to the corresponding nucleotide from the reference sequence and the other interrogation position may or may not be so occupied. In the other two of the four probes, designated the third and fourth probes, the segment is exactly complementary to the reference sequence except that both interrogation positions are occupied by nucleotides which are noncomplementary to the respective corresponding nucleotides in the reference sequence.

There are number of ways of satisfying these conditions depending on whether the two nucleotides in the reference sequence corresponding to the two interrogation positions are the same or different. If these two nucleotides are different in the reference sequence (probability 3/4), the conditions are satisfied by each of the two interrogation positions being occupied by the same nucleotide in any given probe. For example, in the first probe, the two interrogation positions would both be A, in the second probe, both would be C, in the third probe, each would be G, and in the fourth probe each would be T or U. If the two nucleotides in the reference sequence corresponding to the two interrogation positions are different, the conditions noted above are satisfied by each of the interrogation positions in any one of the four probes being occupied by complementary nucleotides. For example, in the first probe, the interrogation positions could be occupied by A and T, in the second probe by C and G, in the third probe by G and C, and in the four probe, by T and A. *See* (Fig. 8).

When the four probes are hybridized to a target that is the same as the reference sequence or differs from the reference sequence at one (but not both) of the interrogation positions, two of the four probes show a double-mismatch with the target and two probes show a single mismatch. The identity of probes showing these different degrees of mismatch can be determined from the different hybridization signals. From the identity of the probes showing the different degrees of mismatch, the nucleotides occupying both of the interrogation positions in the target sequence can be deduced.

For ease of illustration, the multiplex strategy has been initially described for the situation where there are

two nucleotides of interest in a reference sequence and only four probes in an array. Of course, the strategy can be extended to analyze any number of nucleotides in a target sequence by using additional probes. In one variation, each pair of interrogation positions is read from a unique group of four probes. In a block variation, different groups of four probes exhibit the same segment of complementarity with the reference sequence, but the interrogation positions move within a block. The block and standard multiplex tiling variants can of course be used in combination for different regions of a reference sequence. Either or both variants can also be used in combination with any of the other tiling strategies described.

7. Helper Mutations

Occasionally small regions of a reference sequence give a low hybridization signal as a result of annealing of probes. The self-annealing reduces the amount of probe effectively available for hybridizing to the target. Although such regions of the target are generally small and the reduction of hybridization signal is usually not so substantial as to obscure the sequence of this region, this concern can be avoided by the use of probes incorporating helper mutations. The helper mutation(s) serve to break-up regions of internal complementarity within a probe and thereby prevent annealing. Usually, one or two helper mutations are quite sufficient for this purpose. The inclusion of helper mutations can be beneficial in any of the tiling strategies noted above. In general each probe having a particular interrogation position has the same helper mutation(s). Thus, such probes have a segment in common which shows perfect complementarity with a reference sequence, except that the segment contains at least one helper mutation (the same in each of the probes) and at least one interrogation position (different in all of the probes). For example, in the basic tiling strategy, a probe from the first probe set comprises a segment containing an interrogation position and showing perfect complementarity with a reference sequence except for one or two helper mutations. The corresponding probes from the second, third and fourth probe sets usually comprise the same segment (or sometimes a subsequence thereof including the helper mutation(s) and interrogation position), except that the base occupying the interrogation position varies in each probe. *See* Fig. 9.

Usually, the helper mutation tiling strategy is used in conjunction with one of the tiling strategies described above. The probes containing helper mutations are used to tile regions of a reference sequence otherwise giving low hybridization signal (*e.g.*, because of self-complementarity), and the alternative tiling strategy is used to tile intervening regions.

8. Pooling Strategies

Pooling strategies also employ arrays of immobilized probes. Probes are immobilized in cells of an array, and the hybridization signal of each cell can be determined independently of any other cell. A particular cell may be occupied by pooled mixture of probes. Although the identity of each probe in the mixture is known, the individual probes in the pool are not separately addressable. Thus, the hybridization signal from a cell is the aggregate of that of the different probes occupying the cell. In general, a cell is scored as hybridizing to a target sequence if at least one probe occupying the cell comprises a segment exhibiting perfect complementarity to the target sequence.

A simple strategy to show the increased power of pooled strategies over a standard tiling is to create three cells each containing a pooled probe having a single pooled position, the pooled position being the same in each of the pooled probes. At the pooled position, there are two possible nucleotide, allowing the pooled probe to hybridize to two target sequences. In tiling terminology, the pooled position of each probe is an interrogation position. As will become apparent, comparison of the hybridization intensities of the pooled probes from the three cells reveals the identity of the nucleotide in the target sequence corresponding to the interrogation position (*i.e.*, that is matched with the interrogation position when the target sequence and pooled probes are maximally aligned for complementarity).

The three cells are assigned probe pools that are perfectly complementary to the target except at the pooled position, which is occupied by a different pooled nucleotide in each probe as follows:

[AC] = M, [GT]=K, [AG]=R

as substitutions in the probe
IUPAC standard ambiguity notation)

```
                X - interrogation position
Target:   TAACCACTCACGGGAGCA

Pool 1:    ATTGGMGAGTGCCC
          =ATTGGaGAGTGCCC     (complement to mutant 't')
          +ATTGGcGAGTGCCC     (complement to mutant 'g')

Pool 2:    ATTGGKGAGTGCCC
          =ATTGGgGAGTGCCC     (complement to mutant 'c')
          +ATTGGtGAGTGCCC     (complement to wild type 'a')

Pool 3:    ATTGGRGAGTGCCC
          =ATTGGaGAGTGCCC     (complement to mutant 't')
          +ATTGGgGAGTGCCC     (complement to mutant 'c')
```

With 3 pooled probes, all 4 possible single base pair states (wild and 3 mutants) are detected. A pool hybridizes with a target if some probe contained within that pool is complementary to that target.

| | | Hybridization? | | |
|---|---|---|---|---|
| Pool: | | 1 | 2 | 3 |
| Target: | TAACCACTCACGGGAGCA | n | y | n |
| Mutant: | TAACCcCTCACGGGAGCA | n | y | y |
| Mutant: | TAACCgCTCACGGGAGCA | y | n | n |
| Mutant: | TAACCtCTCACGGGAGCA | y | n | y |

A cell containing a pair (or more) of oligonucleotides lights up when a target complementary to any of the oligonucleotide in the cell is present. Using the simple strategy, each of the four possible targets (wild and three mutants) yields a unique hybridization pattern among the three cells.

Since a different pattern of hybridizing pools is obtained for each possible nucleotide in the target sequence corresponding to the pooled interrogation position in the probes, the identity of the nucleotide can be determined from the hybridization pattern of the pools. Whereas, a standard tiling requires four cells to detect and identify the possible single-base substitutions at one location, this simple pooled strategy only requires three cells.

A more efficient pooling strategy for sequence analysis is the 'Trellis' strategy. In this strategy, each pooled probe has a segment of perfect complementarity to a reference sequence except at three pooled positions. One pooled position is an N pool. The three pooled positions may or may not be contiguous in a probe. The other two pooled positions are selected from the group of three pools consisting of (1) M or K, (2) R or Y and (3) W or S, where the single letters are IUPAC standard ambiguity codes. The sequence of a pooled probe is thus, of the form XXXN[(M/K) or (R/Y) or (W/S)][(M/K) or (R/Y) or (W/S)]XXXXX, where XXX represents bases complementary to the reference sequence. The three pooled positions may be in any order, and may be contiguous or separated by intervening nucleotides. For, the two positions occupied by [(M/K) or (R/Y) or (W/S)], two choices must be made. First, one must select one of the following three pairs of pooled nucleotides (1) M/K, (2) R/Y and (3) W/S. The one of three pooled nucleotides selected may be the same or different at the two pooled positions. Second, supposing. for example, one selects M/K at one position, one must then chose between M or K. This choice should result in selection of a pooled nucleotide comprising a nucleotide that complements the corresponding nucleotide in a reference sequence, when the probe and reference sequence are maximally aligned. The same principle governs the selection between R and Y, and between W and S. A trellis pool probe has one pooled position with four possibilities, and two pooled positions, each with two possibilities. Thus, a trellis pool probe comprises a mixture of 16 (4 x 2 x 2) probes. Since each pooled position includes one nucleotide that complements the corresponding nucleotide from the reference sequence, one of these 16 probes has a segment that is the exact complement of the reference sequence. A target sequence that is the same as the reference sequence (*i.e.*, a wildtype target) gives a hybridization signal to each probe cell. Here, as in other tiling methods, the segment of complementarity should be sufficiently long to permit specific hybridization of a pooled probe to a reference sequence be detected relative to a variant of that reference sequence. Typically, the segment of complementarity is about 9-21 nucleotides.

A target sequence is analyzed by comparing hybridization intensities at three pooled probes, each having the structure described above. The segments complementary to the reference sequence present in the three pooled probes

show some overlap. Sometimes the segments are identical (other than at the interrogation positions). However, this need not be the case. For example, the segments can tile across a reference sequence in increments of one nucleotide (*i.e.*, one pooled probe differs from the next by the acquisition of one nucleotide at the 5' end and loss of a nucleotide at the 3' end). The three interrogation positions may or may not occur at the same relative positions within each pooled probe (*i.e.*, spacing from a probe terminus). All that is required is that one of the three interrogation positions from each of the three pooled probes aligns with the same nucleotide in the reference sequence, and that this interrogation position is occupied by a different pooled nucleotide in each of the three probes. In one of the three probes, the interrogation position is occupied by an N. In the other two pooled probes the interrogation position is occupied by one of (M/K) or (R/Y) or (W/S).

In the simplest form of the trellis strategy, three pooled probes are used to analyze a single nucleotide in the reference sequence. Much greater economy of probes is achieved when more pooled probes are included in an array. For example, consider an array of five pooled probes each having the general structure outlined above. Three of these pooled probes have an interrogation position that aligns with the same nucleotide in the reference sequence and are used to read that nucleotide. A different combination of three probes have an interrogation position that aligns with a different nucleotide in the reference sequence. Comparison of these three probe intensities allows analysis of this second nucleotide. Still another combination of three pooled probes from the set of five have an interrogation position that aligns with a third nucleotide in the reference sequence and these probes are used to analyze that nucleotide. Thus, three nucleotides in the reference sequence are fully analyzed from only five pooled probes. By comparison, the basic tiling strategy would require 12 probes for a similar analysis.

As an example, a pooled probe for analysis of a target sequence by the trellis strategy is shown below:

```
Target:   ATTAACCACTCACGGGAGCTCT
Pool:          TGGTGNKYGCCCT
```

The pooled probe actually comprises 16 individual probes:

```
 TGGTGAGcGCCCT
+TGGTGcGcGCCCT
+TGGTGgGcGCCCT
+TGGTGtGcGCCCT
+TGGTGAtcGCCCT
+TGGTGctcGCCCT
+TGGTGgtcGCCCT
+TGGTGttcGCCCT
+TGGTGAGTGCCCT
+TGGTGcGTGCCCT
+TGGTGgGTGCCCT
+TGGTGtGTGCCCT
+TGGTGAtTGCCCT
+TGGTGctTGCCCT
+TGGTGgtTGCCCT
+TGGTGttTGCCCT
```

The trellis strategy employs an array of probes having at least three cells, each of which is occupied by a pooled probe as described above.

Consider the use of three such pooled probes for analyzing a target sequence, of which one position may contain any single base substitution to the reference sequence (i.e, there are four possible target sequences to be distinguished). Three cells are occupied by pooled probes having a pooled interrogation position corresponding to the position of possible substitution in the target sequence, one cell with an 'N', one cell with one of 'M' or 'K', and one cell with 'R' or 'Y'. An interrogation position corresponds to a nucleotide in the target sequence if it aligns adjacent with that nucleotide when the probe and target sequence are aligned to maximize complementarity. Note that although each of the pooled probes has two other pooled positions, these positions are not relevant for the present illustration. The positions are only relevant when more than one position in the target sequence is to be read, a circumstance that will

be considered later. For present purposes, the cell with the 'N' in the interrogation position lights up for the wildtype sequence and any of the three single base substitutions of the target sequence. The cell with M/K in the interrogation position lights up for the wildtype sequence and one of the single-base substitutions. The cell with R/Y in the interrogation position lights up for the wildtype sequence and a second of the single-base substitutions. Thus, the four possible target sequences hybridize to the three pools of probes in four distinct patterns, and the four possible target sequences can be distinguished.

To illustrate further, consider, four possible target sequences (differing at a single position) and a pooled probe having three pooled positions, N, K and Y with the Y position as the interrogation position (*i.e.*, aligned with the variable position in the target sequence):

| | Target | |
|---|---|---|
| Wild: | ATTAACCACTCACGGGAGCTCT | (w) |
| Mutants: | ATTAACCACTCcCGGGAGCTCT | (c) |
| Mutants: | ATTAACCACTCgCGGGAGCTCT | (g) |
| Mutants: | ATTAACCACTCtCGGGAGCTCT | (t) |
| | TGGTGNKYGCCCT (pooled probe). | |

The sixteen individual component probes of the pooled probe hybridize to the four possible target sequences as follows:

| | TARGET | | | |
|---|---|---|---|---|
| | w | c | g | t |
| TGGTGAGcGCCCT | n | n | y | n |
| TGGTGcGcGCCCT | n | n | n | n |
| TGGTGgGcGCCCT | n | n | n | n |
| TGGTGtGcGCCCT | n | n | n | n |
| TGGTGAtcGCCCT | n | n | n | n |
| TGGTGctcGCCCT | n | n | n | n |
| TGGTGgtcGCCCT | n | n | n | n |
| TGGTGttcGCCCT | n | n | n | n |
| TGGTGAGTGCCCT | y | n | n | n |
| TGGTGcGTGCCCT | n | n | n | n |
| TGGTGgGTGCCCT | n | n | n | n |
| TGGTGtGTGCCCT | n | n | n | n |
| TGGTGAtTGCCCT | n | n | n | n |
| TGGTGctTGCCCT | n | n | n | n |
| TGGTGgtTGCCCT | n | n | n | n |
| TGGTGttTGCCCT | n | n | n | n |

The pooled probe hybridizes according to the aggregate of its components:

| | | | | | |
|---|---|---|---|---|---|
| Pool: | TGGTGNKYGCCCT | y | n | y | n |

Thus, as stated above, it can be seen that a pooled probe having a y at the interrogation position hybridizes to the wildtype target and one of the mutants. Similar tables can be drawn to illustrate the hybridization patterns of probe pools having other pooled nucleotides at the interrogation position.

The above strategy of using pooled probes to analyze a single base in a target sequence can readily be extended to analyze any number of bases. At this point, the purpose of including three pooled positions within each probe will become apparent. In the example that follows, ten pools of probes, each containing three pooled probe positions, can be used to analyze a each of a contiguous sequence of eight nucleotides in a target sequence.

```
ATTAACCACTCACGGGAGCTCT  Reference sequence
        --------  Readable nucleotides

Pools:
4    TAATTNKYGAGTG
5     AATTGNKRAGTGC
6      ATTGGNKRGTGCC
7       TTGGTNMRTGCCC
8        TGGTGNKYGCCCT
9         GGTGANKRCCCTC
10         GTGAGNKYCCTCG
11          TGAGTNMYCTCGA
12           GAGTGNMYTCGAG
13            AGTGCNMYCGAGA
```

In this example, the different pooled probes tile across the reference sequence, each pooled probe differing from the next by increments of one nucleotide. For each of the readable nucleotides in the reference sequence, there are three probe pools having a pooled interrogation position aligned with the readable nucleotide. For example, the 12th nucleotide from the left in the reference sequence is aligned with pooled interrogation positions in pooled probes 8, 9, and 10. Comparison of the hybridization intensities of these pooled probes reveals the identity of the nucleotide occupying position 12 in a target sequence.

| | Targets | Pools 8 | 9 | 10 |
|---|---|---|---|---|
| Wild: | ATTAACCACTCACGGGAGCTCT | Y | Y | Y |
| Mutants: | ATTAACCACTCcCGGGAGCTCT | N | Y | Y |
| Mutants: | ATTAACCACTCgCGGGAGCTCT | Y | N | Y |
| Mutants: | ATTAACCACTCtCGGGAGCTCT | N | N | Y |

| Example Intensities: | | | | | | |
|---|---|---|---|---|---|---|
| | = lit cell | Wild | | | | |
| | = blank cell | 'C' | | | | |
| | | 'G' | | | | |
| | | 'T' | | | | |
| | | None | | | | |

Thus, for example, if pools 8, 9 and 10 all light up, one knows the target sequence is wildtype, If pools, 9 and 10 light up, the target sequence has a C mutant at position 12. If pools 8 and 10 light up, the target sequence has a G mutant at position 12. If only pool 10 lights up, the target sequence has a t mutant at position 12.

The identity of other nucleotides in the target sequence is determined by a comparison of other sets of three pooled probes. For example, the identity of the 13th nucleotide in the target sequence is determined by comparing the hybridization patterns of the probe pools designated 9, 10 and 11. Similarly, the identity of the 14th nucleotide in the target sequence is determined by comparing the hybridization patterns of the probe pools designated 10, 11, and 12.

In the above example, successive probes tile across the reference sequence in increments of one nucleotide, and each probe has three interrogation positions occupying the same positions in each probe relative to the terminus of the probe (*i.e.*, the 7, 8 and 9th positions relative to the 3' terminus). However, the trellis strategy does not require that probes tile in increments of one or that the interrogation position positions occur in the same position in each probe. In a variant of trellis tiling referred to as "loop" tiling, a nucleotide of interest in a target sequence is read by comparison of pooled probes, which each have a pooled interrogation position corresponding to the nucleotide of interest, but in

which the spacing of the interrogation position in the probe differs from probe to probe. Analogously to the block tiling approach, this allows several nucleotides to be read from a target sequence from a collection of probes that are identical except at the interrogation position. The identity in sequence of probes, particularly at their 3' termini, simplifies synthesis of the array and result in more uniform probe density per cell.

To illustrate the loop strategy, consider a reference sequence of which the 4, 5, 6, 7 and 8th nucleotides (from the 3' termini are to be read. All of the four possible nucleotides at each of these positions can be read from comparison of hybridization intensities of five pooled probes. Note that the pooled positions in the probes are different (for example in probe 55, the pooled positions are 4, 5 and 6 and in probe 56, 5, 6 and 7).

| | | |
|---|---|---|
| | TAACCACTCACGGGAGCA | Reference sequence |
| 55 | ATTNKYGAGTGCC | |
| 56 | ATTGNKRAGTGCC | |
| 57 | ATTGGNKRGTGCC | |
| 58 | ATTRGTNMGTGCC | |
| 59 | ATTKRTGNGTGCC | |

Each position of interest in the reference sequence is read by comparing hybridization intensities for the three probe pools that have an interrogation position aligned with the nucleotide of interest in the reference sequence. For example, to read the fourth nucleotide in the reference sequence, probes 55, 58 and 59 provide pools at the fourth position. Similarly, to read the fifth nucleotide in the reference sequence, probes 55, 56 and 59 provide pools at the fifth position. As in the previous trellis strategy, one of the three probes being compared has an N at the pooled position and the other two have M or K, and (2) R or Y and (3) W or S.

The hybridization pattern of the five pooled probes to target sequences representing each possible nucleotide substitution at five positions in the reference sequence is shown below. Each possible substitution results in a unique hybridization pattern at three pooled probes, and the identity of the nucleotide at that position can be deduced from the hybridization pattern.

| | | Pools | | | | |
|---|---|---|---|---|---|---|
| | Targets | 55 | 56 | 57 | 58 | 59 |
| Wild: | TAACCACTCACGGGAGCA | Y | Y | Y | Y | Y |
| Mutant: | TAAgCACTCACGGGAGCA | Y | N | N | N | N |
| Mutant: | TAAtCACTCACGGGAGCA | Y | N | N | Y | N |
| Mutant: | TAAaCACTCACGGGAGCA | Y | N | N | N | Y |
| Mutant: | TAACgACTCACGGGAGCA | N | Y | N | N | N |
| Mutant: | TAACtACTCACGGGAGCA | N | Y | N | N | Y |
| Mutant: | TAACaACTCACGGGAGCA | Y | Y | N | N | N |
| Mutant: | TAACCcCTCACGGGAGCA | N | Y | Y | N | N |
| Mutant: | TAACCgCTCACGGGAGCA | Y | N | Y | N | N |
| Mutant: | TAACCtCTCACGGGAGCA | N | N | Y | N | N |
| Mutant: | TAACCAgTCACGGGAGCA | N | N | N | Y | N |
| Mutant: | TAACCAtTCACGGGAGCA | N | Y | N | Y | N |
| Mutant: | TAACCAaTCACGGGAGCA | N | N | Y | Y | N |
| Mutant: | TAACCACaCACGGGAGCA | N | N | N | N | Y |
| Mutant: | TAACCACcCACGGGAGCA | N | N | Y | N | Y |
| Mutant: | TAACCACgCACGGGAGCA | N | N | N | Y | Y |

Many variations on the loop and trellis tilings can be created. All that is required is that each position in sequence must have a probe with a 'N', a probe containing one of R/Y, M/K or W/S, and a probe containing a different pool from that set, complementary to the wild type target at that position, and at least one probe with no pool at all at that position. This combination allows all mutations at that position to be uniquely detected and identified.

A further class of strategies involving pooled probes are termed coding strategies. These strategies assign code words from some set of numbers to variants of a reference sequence. Any number of variants can be coded. The variants can include multiple closely spaced substitutions, deletions or insertions. The designation letters or other symbols assigned to each variant may be any arbitrary set of numbers, in any order. For example, a binary code is often used, but codes to other bases are entirely feasible. The numbers are often assigned such that each variant has a designation having at least one digit and at least one nonzero value for that digit. For example, in a binary system, a variant assigned the number 101, has a designation of three digits, with one possible nonzero value for each digit.

The designation of the variants are coded into an array of pooled probes comprising a pooled probe for each nonzero value of each digit in the numbers assigned to the variants. For example, if the variants are assigned successive number in a numbering system of base m, and the highest number assigned to a variant has n digits, the array would have about n x (m-1) pooled probes. In general, $\log_m (3N+1)$ probes are required to analyze all variants of N locations in a reference sequence, each having three possible mutant substitutions. For example, 10 base pairs of sequence may be analyzed with only 5 pooled probes using a binary coding system. Each pooled probe has a segment exactly complementary to the reference sequence except that certain positions are pooled. The segment should be sufficiently long to allow specific hybridization of the pooled probe to the reference sequence relative to a mutated form of the reference sequence. As in other tiling strategies, segments lengths of 9-21 nucleotides are typical. Often the probe has no nucleotides other than the 9-21 nucleotide segment. The pooled positions comprise nucleotides that allow the pooled probe to hybridize to every variant assigned a particular nonzero value in a particular digit. Usually, the pooled positions further comprises a nucleotide that allows the pooled probe to hybridize to the reference sequence. Thus, a wildtype target (or reference sequence) is immediately recognizable from all the pooled probes being lit.

When a target is hybridized to the pools, only those pools comprising a component probe having a segment that is exactly complementary to the target light up. The identity of the target is then decoded from the pattern of hybridizing pools. Each pool that lights up is correlated with a particular value in a particular digit. Thus, the aggregate hybridization patterns of each lighting pool reveal the value of each digit in the code defining the identity of the target hybridized to the array.

As an example, consider a reference sequence having four positions, each of which can be occupied by three possible mutations. Thus, in total there are 4 x 3 possible variant forms of the reference sequence. Each variant is assigned a binary number binary numbers 0001-1100 and the wildtype reference sequence is assigned the binary number 1111.

| | | | | | | |
|---|---|---|---|---|---|---|
| | X | X | X | X | - | 4 |
| Positions | | | | | | |
| Target: TAAC | C=1111 | A=1111 | C=1111 | T=1111 | | |
| CACGGGAGCA | | | | | | |
| | G=0001 | C=0010 | G=0011 | A=0100 | | |
| | T=0101 | G=0110 | T-0111 | C=1000 | | |
| | A=1001 | T=1010 | A=1011 | G=1100 | | |

A first pooled probe is designed by including probes that complement exactly each variant having a 1 in the first digit.

| | | | | | |
|---|---|---|---|---|---|
| target(1111): | TAAC | C | A | C | T CACGGGAGCA |
| Mutant(0001): | TAAC | g | A | C | T CACGGGAGCA |
| Mutant(0101): | TAAC | t | A | C | T CACGGGAGCA |
| Mutant(1001): | TAAC | a | A | C | T CACGGGAGCA |
| Mutant(0011): | TAAC | C | A | g | T CACGGGAGCA |
| Mutant(0111): | TAAC | C | A | t | T CACGGGAGCA |
| Mutant(1101): | TAAC | C | A | a | T CACGGGAGCA |

First pooled probe

| | | | | | |
|---|---|---|---|---|---|
| = | ATTG | [GCAT] | T | [GCAT] | A GTGCCC |
| = | ATTG | N | T | N | A GTGCCC |

Second, third and fourth pooled probes are then designed respectively including component probes that hybridize to each variant having a 1 in the second, third and fourth digit.

XXXX - 4 positions examined

| | | | |
|---|---|---|---|
| Target: | TAACCACTCACGGGAGCA | | |
| Pool 1(1): | ATTGnTnAGTGCCC = | 16 probes | (4x1x4x1) |
| Pool 2(2): | ATTGGnnAGTGCCC = | 16 probes | (1x4x4x1) |
| Pool 3(4): | ATTGyrydGTGCCC = | 24 probes | (2x2x2x3) |
| Pool 4(8): | ATTGmwmbGTGCCC = | 24 probes | (2x2x2x3) |

The pooled probes hybridize to variant targets as follows: Hybridization pattern:

| | Targets | Pools 1 | 2 | 3 | 4 |
|---|---|---|---|---|---|
| Wild(1111) | TAACCACTCACGGGAGCA | Y | Y | Y | Y |
| Mutant(0001): | TAACgACTCACGGGAGCA | Y | N | N | N |
| Mutant(0101): | TAACtACTCACGGGAGCA | Y | N | Y | N |
| Mutant(1001): | TAACaACTCACGGGAGCA | Y | N | N | Y |
| Mutant(0010): | TAACCcCTCACGGGAGCA | N | Y | N | N |
| Mutant(0110): | TAACCgCTCACGGGAGCA | N | Y | Y | N |
| Mutant(1010): | TAACCtCTCACGGGAGCA | N | Y | N | Y |
| Mutant(0011): | TAACCAgTCACGGGAGCA | Y | Y | N | N |
| Mutant(0111): | TAACCAtTCACGGGAGCA | Y | Y | Y | N |
| Mutant(1101): | TAACCAaTCACGGGAGCA | Y | N | Y | Y |
| Mutant(0100): | TAACCACaCACGGGAGCA | N | N | Y | N |
| Mutant(1000): | TAACCACcCACGGGAGCA | N | N | N | Y |
| Mutant(1100): | TAACCACgCACGGGAGCA | N | N | Y | Y |

The identity of a variant (i.e., mutant) target is read directly from the hybridization pattern of the pooled probes. For example the mutant assigned the number 0001 gives a hybridization pattern of NNNY with respect to probes 4, 3, 2 and 1 respectively.

In the above example, variants are assigned successive numbers in a numbering system. In other embodiments, sets of numbers can be chosen for their properties. If the codewords are chosen from an error-control code, the properties of that code carry over to sequence analysis. An error code is a numbering system in which some designations are assigned to variants and other designations serve to indicate errors that may have occurred in the hybridization process. For example, if all codewords have an odd number of nonzero digits ('binary coding+error detection'), any single error in hybridization will be detected by having an even number of pools lit.

| | | | |
|---|---|---|---|
| Wild Target: | TAACCACTCACGGGAGCA | | |
| Pool 1(1): | ATTGnAnAGTGCCC = | 16 Probes | (4x1x4x1) |
| Pool 2(2): | ATTGGnnAGTGCCC = | 16 Probes | (1X4X4X1) |
| Pool 3(4): | ATTGryrhGTGCCC = | 24 Probes | (2X2X2X3) |
| Pool 4(8): | ATTGkwkvGTGCCC = | 24 Probes | (2X2X2X3) |

A fifth probe can be added to make the number of pools that hybridize to any single mutation odd.

Pool 5(c): ATTGdhsmGTGCCC = 36 probes (2x2x3x3) Hybridization of pooled probes to targets

| | Target | Pool 1 | 2 | 3 | 4 | 5 |
|---|---|---|---|---|---|---|
| Target(11111): | TAACCACTCACGGGAGCA | Y | Y | Y | Y | Y |
| Mutant(00001): | TAACgACTCACGGGAGCA | Y | N | N | N | N |
| Mutant(10101): | TAACtACTCACGGGAGCA | Y | N | N | N | N |
| Mutant(11001): | TAACaACTCACGGGAGCA | Y | N | N | Y | Y |
| Mutant(00010): | TAACCcCTCACGGGAGCA | N | Y | N | N | N |
| Mutant(10110): | TAACCgCTCACGGGAGCA | N | Y | Y | N | Y |
| Mutant(11010): | TAACCtCTCACGGGAGCA | N | Y | N | Y | Y |
| Mutant(10011): | TAACCAgTCACGGGAGCA | Y | Y | N | N | Y |
| Mutant(00111): | TAACAtTCACGGGAGCA | Y | Y | Y | N | N |
| Mutant(01101): | TAACCAaTCACGGGAGCA | Y | N | Y | Y | N |
| Mutant(00100): | TAACCACaCACGGGAGCA | N | N | Y | N | N |
| Mutant(01000): | TAACCAcCCACGGGAGCA | N | N | N | Y | N |
| Mutant(11100): | TAACCACgCACGGGAGCA | N | N | Y | Y | Y |

9. Bridging Strategy

Probes that contain partial matches to two separate (*i.e.*, non contiguous) subsequences of a target sequence sometimes hybridize strongly to the target sequence. In certain instances, such probes have generated stronger signals than probes of the same length which are perfect matches to the target sequence. It is believed (but not necessary to the invention) that this observation results from interactions of a single target sequence with two or more probes simultaneously. This invention exploits this observation to provide arrays of probes having at least first and second segments, which are respectively complementary to first and second subsequences of a reference sequence. Optionally, the probes may have a third or more complementary segments. These probes can be employed in any of the strategies noted above. The two segments of such a probe can be complementary to disjoint subsequences of the reference sequences or contiguous subsequences. If the latter, the two segments in the probe are inverted relative to the order of the complement of the reference sequence. The two subsequences of the reference sequence each typically comprises about 3 to 30 contiguous nucleotides. The subsequences of the reference sequence are sometimes separated by 0, 1, 2 or 3 bases. Often the sequences, are adjacent and nonoverlapping.

For example, a wild-type probe is created by complementing two sections of a reference sequence (indicated by subscript and superscript) and reversing their order. The interrogation position is designated (*) and is apparent from comparison of the structure of the wildtype probe with the three mutant probes. The corresponding nucleotide in the reference sequence is the "a" in the superscripted segment.

Reference: 5' $T_{GGCTA}^{CGAGG}$AATCATCTGTTA

Probes:
3' GCTCC CCGAT (Probe from first probe set)
3' GCACC CCGAT
3' GCCCC CCGAT
3' GCGCC CCGAT

(* above the second position of the probes)

The expected hybridizations are:

**Match:**

GCTCCCCGAT
... TGGCTACGAGGAATCATCTGTTA
GCTCCCCGAT

**Mismatch:**

GCTCCCCGAT
... TGGCTACGAGGAATCATCTGTTA
GCGCCCCGAT

Bridge tilings are specified using a notation which gives the length of the two constituent segments and the relative position of the interrogation position. The designation n/m indicates a segment complementary to a region of the reference sequence which extends for n bases and is located such that the interrogation position is in the mth base from the 5' end. If m is larger than n, this indicates that the entire segment is to the 5' side of the interrogation position. If m is negative, it indicates that the interrogation position is the absolute value of m bases 5' of the first base of the segment (m cannot be zero). Probes comprising multiple segments, such as n/m + a/b + ... have a first segment at the 3' end of the probe and additional segments added 5' with respect to the first segment. For example, a 4/8 tiling consists of (from the 3' end of the probe) a 4 base complementary segment, starting 7 bases 5' of the interrogation position, followed by a 6 base region in which the interrogation position is located at the third base. Between these two segments, one base from the reference sequence is omitted. By this notation, the set shown above is a 5/3 + 5/8 tiling. Many different tilings are possible with this method, since the lengths of both segments can be varied, as well as their relative position (they may be in either order and there may be a gap between them) and their location relative to the interrogation position.

As an example, a 16 mer oligo target was hybridized to a chip containing all $4^{10}$ probes of length 10. The chip includes short tilings of both standard and bridging types. The data from a standard 10/5 tiling was compared to data from a 5/3 + 5/8 bridge tiling (*see* Table 1). Probe intensities (mean count/pixel) are displayed along with discrimination ratios (correct probe intensity / highest incorrect probe intensity). Missing intensity values are less than 50 counts. Note that for each base displayed the bridge tiling has a higher discrimination value.

TABLE 1:

| **Comparison of Standard and Bridge Tilings** | | | | | |
|---|---|---|---|---|---|
| TILING | PROBE BASE: | CORRECT PROBE BASE | | | |
| | | C | A | C | C |
| STANDARD (10/5) | A | 92 | 496 | 294 | 299 |
| | C | 536 | 148 | 532 | 534 |
| | G | 69 | 167 | 72 | 52 |
| | T | 146 | 95 | 212 | 126 |
| DISCRIMINATION: | | 3.7 | 3.0 | 1.8 | 1.8 |
| BRIDGING 5/3 + 5/8 | A | - | 404 | - | 156 |
| | C | 276 | - | 345 | 379 |
| | G | - | 80 | - | - |
| | T | - | - | - | 58 |
| DISCRIMINATION: | | >5.5 | 5.1 | 2.4 | 1.26 |

The bridging strategy offers the following advantages:

(1) Higher discrimination between matched and mismatched probes,
(2) The possibility of using longer probes in a bridging tiling, thereby increasing the specificity of the hybridization, without sacrificing discrimination,

(3) The use of probes in which an interrogation position is located very off-center relative to the regions of target complementarity. This may be of particular advantage when, for example, when a probe centered about one region of the target gives low hybridization signal. The low signal is overcome by using a probe centered about an adjoining region giving a higher hybridization signal.
(4) Disruption of secondary structure that might result in annealing of certain probes (see previous discussion of helper mutations).

10. Deletion Tiling

Deletion tiling is related to both the bridging and helper mutant strategies described above. In the deletion strategy, comparisons are performed between probes sharing a common deletion but differing from each other at an interrogation position located outside the deletion. For example, a first probe comprises first and second segments, each exactly complementary to respective first and second subsequences of a reference sequence, wherein the first and second subsequences of the reference sequence are separated by a short distance (*e.g.*, 1 or 2 nucleotides). The order of the first and second segments in the probe is usually the same as that of the complement to the first and second subsequences in the reference sequence. The interrogation position is usually separated from The comparison is performed with three other probes, which are identical to the first probe except at an interrogation position, which is different in each probe. Reference:... AGTACCAGATCTCTAA ... Probe set: CATGGNC AGAGA (N = interrogation position). Such tilings sometimes offer superior discrimination in hybridization intensities between the probe having an interrogation position complementary to the target and other probes. Thermodynamically, the difference between the hybridizations to matched and mismatched targets for the probe set shown above is the difference between a single-base bulge, and a large asymmetric loop (*e.g.*, two bases of target, one of probe). This often results in a larger difference in stability than the comparison of a perfectly matched probe with a probe showing a single base mismatch in the basic tiling strategy.
The superior discrimination offered by deletion tiling is illustrated by Table 2, which compares hybridization data from a standard 10/5 tiling with a (4/8 + 6/3) deletion tiling of the reference sequence. (The numerators indicate the length of the segments and the denominators, the spacing of the deletion from the far termini of the segments.) Probe intensities (mean count/pixel) are displayed along with discrimination ratios (correct probe intensity / highest incorrect probe intensity). Note that for each base displayed the deletion tiling has a higher discrimination value than either standard tiling shown.

TABLE 2.

| Comparison of Standard and Deletion Tilings | | | | | |
|---|---|---|---|---|---|
| TILING | PROBE BASE: | CORRECT PROBE BASE | | | |
| | | C | A | C | C |
| STANDARD (10/5) | A | 92 | 496 | 294 | 299 |
| | C | 536 | 148 | 532 | 534 |
| | G | 69 | 167 | 72 | 52 |
| | T | 146 | 95 | 212 | 126 |
| DISCRIMINATION: | | 3.7 | 3.0 | 1.8 | 1.8 |
| DELETION 4/8 + 6/3 | A | 6 | 412 | 29 | 48 |
| | C | 297 | 32 | 465 | 160 |
| | G | 8 | 77 | 10 | 4 |
| | T | 8 | 26 | 31 | 5 |
| DISCRIMINATION: | | 37.1 | 5.4 | 15 | 3.3 |

TABLE 2. (continued)

| Comparison of Standard and Deletion Tilings | | | | | |
|---|---|---|---|---|---|
| TILING | PROBE BASE: | CORRECT PROBE BASE | | | |
| | | C | A | C | C |
| STANDARD (10/7) | A | 347 | 533 | 228 | 277 |
| | C | 729 | 194 | 536 | 496 |
| | G | 232 | 231 | 102 | 89 |
| | T | 344 | 133 | 163 | 150 |
| DISCRIMINATION: | | 2.1 | 2.3 | 2.3 | 1.8 |

The use of deletion or bridging probes is quite general. These probes can be used in any of the tiling strategies of the invention. As well as offering superior discrimination, the use of deletion or bridging strategies is advantageous for certain probes to avoid self-hybridization (either within a probe or between two probes of the same sequence)

C. Preparation of Target Samples

The target polynucleotide, whose sequence is to be determined, is usually isolated from a tissue sample. If the target is genomic, the sample may be from any tissue (except exclusively red blood cells). For example, whole blood, peripheral blood lymphocytes or PBMC, skin, hair or semen are convenient sources of clinical samples. These sources are also suitable if the target is RNA. Blood and other body fluids are also a convenient source for isolating viral nucleic acids. If the target is mRNA, the sample is obtained from a tissue in which the mRNA is expressed. If the polynucleotide in the sample is RNA, it is usually reverse transcribed to DNA. DNA samples or cDNA resulting from reverse transcription are usually amplified, *e.g.,* by PCR. Depending on the selection of primers and amplifying enzyme (s), the amplification product can be RNA or DNA. Paired primers are selected to flank the borders of a target polynucleotide of interest. More than one target can be simultaneously amplified by multiplex PCR in which multiple paired primers are employed. The target can be labelled at one or more nucleotides during or after amplification. For some target polynucleotides (depending on size of sample), *e.g.*, episomal DNA, sufficient DNA is present in the tissue sample to dispense with the amplification step.

When the target strand is prepared in single-stranded form as in preparation of target RNA, the sense of the strand should of course be complementary to that of the probes on the chip. This is achieved by appropriate selection of primers. The target is preferably fragmented before application to the chip to reduce or eliminate the formation of secondary structures in the target. The average size of targets segments following hybridization is usually larger than the size of probe on the chip.

II. ILLUSTRATIVE CHIPS

A. HIV Chip

HIV has infected a large and expanding number of people, resulting in massive health care expenditures. HIV can rapidly become resistant to drugs used to treat the infection, primarily due to the action of the heterodimeric protein (51 kDa and 66 kDa) HIV reverse transcriptase (RT) both subunits of which are encoded by the 1.7 kb *pol* gene. The high error rate (5-10 per round) of the RT protein is believed to account for the hypermutability of HIV. The nucleoside analogues, *i.e*., AZT, ddI, ddC, and d4T, commonly used to treat HIV infection are converted to nucleotide analogues by sequential phosphorylation in the cytoplasm of infected cells, where incorporation of the analogue into the viral DNA results in termination of viral replication, because the 5' -> 3' phosphodiester linkage cannot be completed. However, after about 6 months to 1 year of treatment or less, HIV typically mutates the RT gene so as to become incapable of incorporating the analogue and so resistant to treatment. Several mutations known to be associated with drug resistance are shown in the table below. After a virus having drug resistance via a mutation becomes predominant, the patient suffers dramatically increased viral load, worsening symptoms (typically more frequent and difficult-to-treat infections), and ultimately death. Switching to a different treatment regimen as soon as a resistant mutant virus takes hold may be an important step in patient management which prolongs patient life and reduces morbidity during life.

TABLE 3

| SOME RT MUTATIONS ASSOCIATED WITH DRUG RESISTANCE | | | |
|---|---|---|---|
| ANTIVIRAL | CODON | aa CHANGE | nt CHANGE |
| AZT | 67 | Asp -> Asn | GAC -> AAC |
| AZT | 70 | Lys -> Arg | AAA -> AGA |
| AZT | 215 | Thr -> Phe or Tyr | ACC -> TTC or TAC |
| AZT | 219 | Lys -> Gln or Glu | AAA -> CAA or GAA |
| AZT | 41 | Met -> Leu | ATG -> TTG or CTG |
| ddI and ddC | 184 | Met -> Val | ATG -> GTG |
| ddI and ddC | 74 | Leu -> Val | |
| TIBO 82150 | 100 | Leu -> Ile | |
| ddC | 65 | Lys -> Asn | AAA -> AGA |
| ddC | 69 | Thr -> Asp | ACT -> GAT |
| 3TC | 184 | Met -> Val | ATG -> GTG or GTA |
| 3TC | 184 | Met -> Ile | ATG -> ATA |
| AZT + ddI | 62 | Ala -> Val | GCC -> GTC |
| AZT + ddI | 75 | Val -> Ile | GTA -> ATA |
| AZT + ddI | 77 | Phe -> Leu | TTC -> TTA |
| AZT + ddI | 116 | Phe -> Tyn | TTT -> TAT |
| AZT + ddI | 151 | Gln -> Met | CAG -> ATG |
| Nevaripine | 103 | Lys -> Asn | AAA -> AAT |
| | 106 | Val -> Ala | GTA -> GCA |
| | 108 | | |
| | 181 | Tyr -> Cys | TAT -> TGT |
| | 188 | Tyr -> His | TAT -> CAT |
| | 190 | Gly -> Ala | GGA -> GCA |
| N.B. Other mutations confer resistance to other drugs. | | | |

A second important therapeutic target for anti-HIV drugs is the aspartyl protease enzyme encoded by the HIV genome, whose function is required for the formation of infectious progeny. *See* Robbins & Plattner, *J. Acquired Immune Deficiency Syndromes* 6, 162-170 (1993); Kozal et al., *Curr. Op. Infect. Dis.* 7:72-81 (1994). The protease function in processing of viral precursor polypeptides to their active forms. Drugs targeted against this enzyme do not impair endogenous human proteases, thereby achieving a high degree of selective toxicity. Moreover, the protease is expressed later in the life-cycle that reverse transcriptase, thereby offering the possibility of a combined attack on HIV at two different times in its life-cycle. As for drugs targeted against the reverse transcriptase, administration of drugs to the protease can result in acquisition of drug resistance through mutation of the protease. By monitoring the protease gene from patients, it is possible to detect the occurrence of mutations, and thereby make appropriate adjustments in the drug(s) being administered.

In addition to being infected with HIV, AIDS patients are often also infected with a wide variety of other infectious agents giving rise to a complex series of symptoms. Often diagnosis and treatment is difficult because many different pathogens (some life-threatening, others routine) cause similar symptoms. Some of these infections, so-called opportunistic infections, are caused by bacterial, fungal, protozoan or viral pathogens which are normally present in small quantity in the body, but are held in check by the immune system. When the immune system in AIDS patients fails, these normally latent pathogens can grow and generate rampant infection. In treating such patients, it would be desirable simultaneously to diagnose the presence or absence of a variety of the most lethal common infections,

determine the most effective therapeutic regime against the HIV virus, and monitor the overall status of the patient's infection.

The present invention provides DNA chips for detecting the multiple mutations in HIV genes associated with resistance to different therapeutics. These DNA chips allow physicians to monitor mutations over time and to change therapeutics if resistance develops. Some chips also provide probes for diagnosis of pathogenic microorganisms that typically occur in AIDS patients.

The sequence selected as a reference sequence can be from anywhere in the HIV genome, but should preferably cover a region of the HIV genome in which mutations associated with drug resistance are known to occur. A reference sequence is usually between about 5, 10, 20, 50, 100, 5000, 1000, 5,000 or 10,000 bases in length, and preferably is about 100-1700 bases in length. Some reference sequences encompass at least part of the reverse transcriptase sequence encoded by the pol gene. Preferably, the reference sequence encompasses all, or substantially all (i.e, about 75 or 90%) of the reverse transcriptase gene. Reverse transcriptase is the target of several drugs and as noted, above, the coding sequence is the site of many mutations associated with drug resistance. In some chips, the reference sequence contains the entire region coding reverse transcriptase (850 bp), and in other chips, subfragments thereof. In some chips, the reference sequence includes other subfragments of the pol gene encoding HIV protease or endonuclease, instead of, or as well as the segment encoding reverse transcriptase. In some chips, the reference sequence also includes other HIV genes such as env or gag as well as or instead of the reverse transcriptase gene. Certain regions of the gag and env genes are relatively well conserved, and their detection provides a means for identifying and quantifying the amount of HIV virus infecting a patient. In some chips, the reference sequence comprises an entire HIV genome.

It is not critical from which strain of HIV the reference sequence is obtained. HIV strains are classified as HIV-I, HIV-II or HIV-III, and within these generic groupings there are several strains and polymorphic variants of each of these. BRU, SF2, HXB2, HXB2R are examples of HIV-1 strains, the sequences of which are available from GenBank. The reverse transcriptase genes of the BRU and SF2 strains differ at 23 nucleotides. The HXB2 and HXB2R strains have the same reverse transcriptase gene sequence, which differs from that of the BRU strain at four nucleotides, and that of SF2 by 27 nucleotides. In some chips, the reference sequence corresponds exactly to the reverse transcriptase sequence in the wildtype version of a strain. In other chips, the reference sequence corresponds to a consensus sequence of several HIV strains. In some chips, the reference sequence corresponds to a mutant form of a HIV strain.

Chips are designed in accordance with the tiling strategies noted above. The probes are designed to be complementary to either the coding or noncoding strand of the HIV reference sequence. If only one strand is to be read, it is preferable to read the coding strand. The greater percentage of A residues in this strand relative to the noncoding strand generally result in fewer regions of ambiguous sequence.

Some chips contain additional probes or groups of probes designed to be complementary to a second reference sequence. The second reference sequence is often a subsequence of the first reference sequence bearing one or more commonly occurring HIV mutations or interstrain variations (*e.g.*, within codons 67, 70, 215 or 219 of the reverse transcriptase gene). The inclusion of a second group is particularly useful for analyzing short subsequences of the primary reference sequence in which multiple mutations are expected to occur within a short distance commensurate with the length of the probes (i.e., two or more mutations within 9 to 21 bases).

The total number of probes on the chips depends on the tiling strategy, the length of the reference sequence and the options selected with respect to inclusion of multiple probe lengths and secondary groups of probes to provide confirmation of the existence of common mutations. To read much or all of the HIV reverse transcriptase gene (857 b for the BRU strain), chips tiled by the basic strategy typically contain at least 857 x 4 = 3428 probes.

The target HIV polynucleotide, whose sequence is to be determined, is usually isolated from blood samples (peripheral blood lymphocytes or PBMC) in the form of RNA. The RNA is reverse transcribed to DNA, and the DNA product is then amplified. Depending on the selection of primers and amplifying enzyme, the amplification product can be RNA or DNA. Suitable primers for amplification of target are shown in the table below.

## TABLE 4
## AMPLIFICATION OF TARGET

| TARGET SIZE | FORWARD PRIMER | REVERSE PRIMER |
|---|---|---|
| 1,742 bp | GTAGAATTCTGTTGACTCAGATTGG | GATAAGCTTGGGCCTTATCTATTCCAT |
| 535 bp | AAATCCATACAATACTCCAGTATTTGC | ACCCATCCAAAGGAATGGAGGTTCTTTC |
| 323 bp | Genbank # K02013 1889-1908 | bases 2211-2192 |
| | AATTAACCCTCACTAAAGGGAga ggaagaatctgttgactcagattggt (RT#1-T3) | AATTTAATACGACTCACTATAGGGAtttcccca ctaacttctgtatgtcattgaca-3' (89-391 T7) |
| | AATTAACCCTCACTAAAGGGAga agtatactgcatttaccatacctagta (RT#3-T3) | |
| | TAATACGACTCACTATAGGGAGA tcgacgcaggactcggcttgctgaa (HV1-T2) | |
| | AATTAACCCTCACTAAAGGGAGA ccttgtaagtcattggtcttaaaggta (HV2-T3) | |

In another aspect of the invention, chips are provided for simultaneous detection of HIV and microorganisms that commonly parasitize AIDS patients (*e.g.*, cytomegalovirus (CMV), Pneumocystis carini (PCP), fungi (candida albicans), mycobacteria). Non-HIV viral pathogens are detected and their drug resistance determined using a similar strategy as for HIV. That is groups of probes are designed to show complementarity to a target sequence from a region of the genome of a nonviral pathogen known to be associated with acquisition of drug resistance. For example, CMV and HSV viruses, which frequently co-parasitize AIDS patients, undergo mutations to acquire resistance to acyclovir.

For detection of non-viral pathogens, the chips include an array of probes which allow full-sequence determination of 16S ribosomal RNA or corresponding genomic DNA of the pathogens. The additional probes are designed by the same principles as described above except that the target sequence is a variable region from a 16S RNA (or corresponding DNA) of a pathogenic microorganism. Alternatively, the target sequence can be a consensus sequences of variable 16S rRNA regions from multiple organisms. 16S ribosomal DNA and RNA is present in all organisms (except viruses) and the sequence of the DNA or RNA is closely related to the evolutionary genetic distance between any two species. Hence, organisms which are quite close in type (*e.g.*, all mycobacteria) share a common region of 16S rDNA, and differ in other regions (variable regions) of the 16S rRNA. These differences can be exploited to allow identification of the different subtype strains. The full sequence of 16S ribosomal RNA or DNA read from the chip is compared against a database of the sequence of thousands of known pathogens to type unambiguously most nonviral pathogens infecting AIDS patients.

In a further embodiment, the invention provides chips which also contain probes for detection of bacterial genes conferring antibiotic resistance. An antibiotic resistance gene can be detected by hybridization to a single probe employed in a reverse dot blot format. Alternatively, a group of probes can be designed according to the same principles discussed above to read all or part the DNA sequence encoding an antibiotic resistance gene. Analogous probes groups are designed for reading other antibiotic resistance gene sequences. Antibiotic resistance frequently resides in one of the following genes in microorganisms coparasitizing AIDS patients: rpoB (encoding RNA polymerase), katG (encoding catalase peroxidase, and DNA gyrase A and B genes.

The inclusion of probes for combinations of tests on a single chip simulates the clinical diagnosis tree that a physician would follow based on the presentation of a given syndrome which could be caused by any number of possible pathogens. Such chips allow identification of the presence and titer of HIV in a patient, identification of the HIV strain type and drug resistance, identification of opportunistic pathogens, and identification of the drug resistance of such pathogens. Thus, the physician is simultaneously apprised of the full spectrum of pathogens infecting the patient and the most effective treatments therefor.

<u>Exemplary HIV Chips</u>

<u>(a) HV 273</u>

The HV 273 chip contains an array of oligonucleotide probes for analysis of an 857 base HIV amplicon between nucleotides 2090 and 2946 (HIVBRU strain numbering). The chip contains four groups of probes: 11 mers, 13 mers, 15 mers and 17 mers. From top to bottom, the HV 273 chip is occupied by rows of 11 mers, followed by rows of 13

mers, followed by rows of 15 mers followed by rows of 17 mers. The interrogation position is nucleotide 6, 7, 8 and 9 respectively in the different sized chips. This arrangement of the different sized probes is referred to as being "in series." Within each size group, there are four probe sets laid down in an A-lane, a C-lane a G-lane and a T-lane respectively. Each lane contains an overlapping series of probes with one probe for each nucleotide in the 2090-2946 HIV reverse transcriptase reference sequence. (*i.e.*, 857 probes per lane). The lanes also include a few column positions which are empty or occupied by control probes. These positions serve to orient the chip, determine background fluorescence and punctuate different subsequences within the target. The chip has an area of 1.28 x 1.28 cm, within which the probes form a 130 X 135 matrix (17,550 cells total). The area occupied by each probe (*i.e.*, a probe cell) is about 98 X 95 microns.

The chip was tested for its capacity to sequence a reverse transcriptase fragment from the HIV strain SF2. An 831 bp RNA fragment (designated pPol19) spanning most of the HIV reverse transcriptase coding sequence was amplified by PCR, using primers tagged with T3 and T7 promoter sequences. The primers, designated RT#1-T3 and 89-391 T7 are shown in Table 4; *see also* Gingeras et al., *J. Inf. Dis*. 164, 1066-1074 (1991). RNA was labelled by incorporation of fluorescent nucleotides. The RNA was fragmented by heating and hybridized to the chip for 40 min at 30 degrees. Hybridization signals were quantified by fluorescence imaging.

Taking the best data from the four probes sets at each position in the target sequence, 715 out of 821 bases were read correctly (87%). (comparisons are based on the sequence of pPol19 determined by the conventional dideoxy method to be identical to SF2). In general, the longer sized probes yielded more sequence than the shorter probes. Of the 21 positions at which the SF2 and BRU strains diverged within the target, 19 were read correctly.

Many of the short ambiguous regions in the target arise in segments of the target flanking the points at which the SF2 and BRU sequences diverge. These ambiguities arise because in these regions the comparison of hybridization signals is not drawn between perfectly matched and single base mismatch probes but between a single-mismatched probe and three probes having two mismatches. These ambiguities in reading an SF2 sequence would not detract from the chip's ability to read a BRU sequence either alone or in a mixture with an SF2 target sequence.

In a variation of the above procedure, the chip was treated with RNase after hybridization of the pPol19 target to the probes. Addition of RNase digests mismatched target and thereby increases the signal to noise ratio. RNase treatment increased the number of correctly read bases to 743/821 or 90% (combining the data from the four groups of probes).

In a further variation, the RNA target was replaced with a DNA target containing the same segment of the HIV genome. The DNA probe was prepared by linear amplification using Taq polymerase, RT#1-T3 primer, and fluorescein d-UTP label. The DNA probe was fragmented with uracil DNA glycosylase and heat treatment. The hybridization pattern across the array and percentage of readable sequence were similar to those obtained using an RNA target. However, there were a few regions of sequence that could be read from the RNA target that could not be read from the DNA target and vice versa.

(b) HV 407 Chip

The 407 chip was designed according to the same principles as the HV 273 chip, but differs in several respects. First, the oligonucleotide probes on this chip are designed to exhibit perfect sequence identity (with the exception of the interrogation position on each probe) to the HIV strain SF2 (rather than the BRU strain as was the case for the HV 273 chip). Second, the 407 chip contains 13 mers, 15 mers, 17 mers and 19 mers (with interrogation positions at nucleotide 7, 8, 9 and 10 respectively), rather than the 11 mers, 13 mers, 15 mers and 17 mers on the HV 273 chip. Third, the different sized groups of oligomers are arranged in parallel in place of the in-series arrangement on the HV 273 chip. In the parallel arrangement, the chip contains from top to bottom a row of 13 mers, a row of 15 mers, a row of 17 mers, a row of 19 mers, followed by a further row of 13 mers, a row of 15 mers, a row of 17 mers, a row of 19 mers, followed by a row of 13 mers, and so forth. Each row contains 4 lanes of probes, an A lane, a C lane, a G lane and a T lane, as described above. The probes in each lane tile across the reference sequence. The layout of probes on the HV 407 chip is shown in Fig. 10.

The 407 chip was separately tested for its ability to sequence two targets, pPol19 RNA and 4MUT18 RNA. pPol19 contains an 831 bp fragment from the SF2 reverse transcriptase gene which exhibits perfect complementarity to the probes on the 407 chip (except of course for the interrogation positions in three of the probes in each column). 4MUT18 differs from the reference sequence at thirty-one positions within the target, including five positions in codons 67, 70, 215 and 219 associated with acquisition of drug resistance. Target RNA was prepared, labelled and fragmented as described above and hybridized to the HV 407 chip. The hybridization pattern for the pPol19 target is shown in Fig. 11.

The sequences read off the chip for the pPol19 and 4MUT18 targets are both shown in Fig. 12 (although the two sequences were determined in different experiments). The sequence labelled wildtype in the Figure is the reference sequence. The four lanes of sequence immediately below the reference sequence are the respective sequences read from the four-sized groups of probes for the pPoll9 target (from top-to-bottom, 13 mers, 15 mers, 17 mers and 19 mers).

The next four lanes of sequence are the sequences read from the four-sized groups of probes for the 4MUT18 target (from top-to-bottom in the same order). The regions of sequences shown in normal type are those that could be read unambiguously from the chip. Regions where sequence could not be accurately read are shown highlighted. Some regions of sequence that could not be read from one sized set of probes could be read from another.

Taking the best result from the four sized groups of probes at each column position, about 97% of bases in the pPol19 sequence and about 90% of bases in the 4MUT18 sequence were read accurately. Of the 31 nucleotide differences between 4MUT18 and the reference sequence, twenty-seven were read correctly including three of the nucleotide changes associated with acquisition of drug resistance. Of the ambiguous regions in the 4MUT18 sequence determination, most occurred in the 4MUT18 segments flanking points of divergence between the 4MUT18 and reference sequences. Notably, most of the common mutations in HIV reverse transcriptase associated with drug resistance *(see* Table 3) occur at sequence positions that can be read from the chip. Thus, most of the commonly occurring mutations can be detected by a chip containing an array of probes based on a single reference sequence.

Comparison of the sequence read of the probes of different sizes is useful in determining the optimum size probe to use for different regions of the target. The strategy of customizing probe length within a single group of probe sets minimizes the total number of probes required to read a particular target sequence. This leaves ample capacity for the chip to include probes to other reference sequences (*e.g.*, 16S RNA for pathogenic microorganisms) as discussed below.

The HV 407 chip has also been tested for its capacity to detect mixtures of different HIV strains. The mixture comprises varying proportions of two target sequences; one a segment of a reverse transcriptase gene from a wildtype SF2 strain, the other a corresponding segment from an SF2 strain bearing a codon 67 mutation. *See* Fig. 13. The Figure also represents the probes on the chip having an interrogation position for reading the nucleotide in which the mutation occurs. A single probe in the Figure represents four probes on the chip with the symbol (o) indicating the interrogation position, which differs in each of the four probes. Figure 14 shows the fluorescence intensity for the four 13 mers and the four 15 mers having an interrogation position for reading the nucleotide in the target sequence in which the mutation occurs. As the percentage of mutant target is increase, the fluorescence intensity of the probe exhibiting perfect complementarity to the wildtype target decreases, and the intensity of the probe exhibiting perfect complementarity to the mutant sequence increases. The intensities of the other two probes do not change appreciably. It is concluded that the chip can be used to analyze simultaneously a mixture of strains, and that a strain comprising as little as ten percent of a mixture can be easily detected.

c. Protease Chip

A protease chip was constructed using the basic tiling strategy. The chip comprises four probes tiling across a 382 nucleotide span including 297 nucleotides from the protease coding sequence. The reference sequence was a consensus Clay-B HIV protease sequence. Different probes lengths were employed for tiling different regions of the reference sequence. Probe lengths were 11, 14, 17 and 20 nucleotides with interrogation positions at or adjacent to the center of each probe. Lengths were optimized from prior hybridization data employing a chip having multiple tilings, each with a different probe length.

The chip was hybridized to four different single-stranded DNA protease target sequences (HXB2, SF2, NY5, pPol4mut18). Both sense and antisense strands were sequenced. Data from the chip was compared with that from an ABI sequencer. The overall accuracy from sequencing the four targets is illustrated in the Table 5 below.

Table 5

| | | ABI | Protease Chip | |
|---|---|---|---|---|
| | Sense | Antisense | Sense | Antisense |
| No call | 0 | 4 | 9 | 4 |
| Ambiguous | 6 | 14 | 17 | 8 |
| Wrong call | 2 | 3 | 3 | 1 |
| TOTAL | 8 | 21 | 29 | 13 |
| ABI (sense) - 99.5%<br>Chip (sense) - 98.1%<br>ABI (antisense) - 98.6%<br>Chip (antisense) - 99.1% | | | | |

Combining the data from sense and antisense strands, both the chip and the ABI sequencer provided 100% accurate data for all of the sequence from all four clones.

In a further test, the chip was hybridized to protease target sequences from viral isolates obtained from four patients before and after ddI treatment. The sequence read from the chip is shown in Fig. 15. Several mutations (indicated by arrows) have arisen in the samples obtained posttreatment. Particularly noteworthy was the chip's capacity to read a g/a mutation at nucleotide 207, notwithstanding the presence of two additional mutations (gt) at adjacent positions.

B. Cystic Fibrosis Chips

A number of years ago, cystic fibrosis, the most common severe autosomal recessive disorder in humans, was shown to be associated with mutations in a gene thereafter named the Cystic Fibrosis Transmembrane Conductance Regulator (CFTR) gene. The CFTR gene is about 250 kb in size and has 27 exons. Wildtype genomic sequence is available for all exonic regions and exons/intron boundaries (Zielenski et al., *Genomics* 10, 214-228 (1991). The full-length wildtype cDNA sequence has also been described *(see* Riordan et al., *Science* 245, 1059-1065 (1989). Over 400 mutations have been mapped (*see* Tsui et al, *Hu. Mutat*. 1, 197-203 (1992). Many of the more common mutations are shown in Table 6. The most common cystic fibrosis mutation is a three-base deletion resulting in the omission of amino acid #508 from the CFTR protein. The frequency of mutations varies widely in populations of different geographic or ethnic origin (see column 4 of Table 6). About 90% of all mutations having phenotypic effects occur in coding regions.

Detection of CFTR mutations is useful in a number of respects. For example, screening of populations can identify asymptomatic heterozygous individuals. Such individuals are at risk of giving rise to affected offspring suffering from CF if they reproduce with other such individuals. *In utero* screening of fetuses is also useful in identifying fetuses bearing 2 CFTR mutations. Identification of such mutations offers the possibility of abortion, or gene therapy. For couples known to be at risk of giving rise to affected progeny, diagnosis can be combined with *in vitro* reproduction procedures to identify an embryo having at least one wildtype CF allele before implantation. Screening children shortly after birth is also of value in identifying those having 2 copies of the defective gene. Early detection allows administration of appropriate treatment (*e.g.*, Pulmozyme Antibiotics, Pertussive Therapy) thereby improving the quality of life and perhaps prolonging the life expectancy of an individual.

The source of target DNA for detecting of CFTR mutations is usually genomic. In adults, samples can conveniently be obtained from blood or mouthwash epithelial cells. In fetuses, samples can be obtained by several conventional techniques such as amniocentesis, chorionic villus sampling or fetal blood sampling. At birth, blood from the amniotic chord is a useful tissue source.

The target DNA is usually amplified by PCR. Some appropriate pairs of primers for amplifying segments of DNA including the sites of known mutations are listed in Tables 5 and 6.

Table 7

| OLIGO NUMBER | SEQUENCE |
|---|---|
| 787 | TCTCCTTGGATATACTTGTGTGAATCAA |
| 788 | TCACCAGATTTCGTAGTCTTTTCATA |
| 851 | GTCTTGTGTTGAAATTCTCAGGGTAT |
| 769 | CTTGTACCAGCTCACTACCTAAT |
| 887 | ACCTGAGAAGATAGTAAGCTAGATGAA |
| 888 | AACTCCGCCTTTCCAGTTGTAT |
| 934 | TTAGTTTCTAGGGGTGGAAGATACA |
| 935 | TTAATGACACTGAAGATCACTGTTCTAT |
| 789 | CCATTCCAAGATCCCTGATATTTGAA |
| 790 | GCACATTTTTGCAAAGTTCATTAGA |
| 891 | TCATGGGCCATGTGCTTTTCAA |
| 892 | ACCTTCCAGCACTACAAACTAGAA |
| 760 | CAAGTGAATCCTGAGCGTGATTT |
| 850 | GGTAGTGTGAAGGGTTCATATGCATA |
| 762 | GATTACATTAGAAGGAAGATGTGCCTTT |
| 763 | ACATGAATGACATTTACAGCAAATGCTT |
| 931 | GTGACCATATTGTAATGCATGTAGTGA |
| 932 | ATGGTGAACATATTTCTCAAGAGGTAA |
| 955 | TGT CTC TGT AAA CTG ATG GCT AAC A |
| 884 | TCGTATAGAGTTGATTGGATTGAGAA |
| 885 | CCATTAACTTAATGTGGTCTCATCACAA |
| 886 | CTACCATAATGCTTGGGAGAAATGAA |
| 782 | TCAAAGAATGGCACCAGTGTGAAA |
| 901 | TGCTTAGCTAAAGTTAATGAGTTCAT |

| OLIGO NUMBER | SEQUENCE |
|---|---|
| 784 | AATTGTGAAATTGTCTGCCATTCTTAA |
| 785 | GATTCACTTACTGAACACAGTCTAACAA |
| 791 | AGGCTTCTCAGTGATCTGTTG |
| 792 | GAATCATTCAGTGGGTATAAGCA |
| 1013 | GCCATGGTACCTATATGTCACAGAA |
| 1012 | TGCAGAGTAATATGAATTTCTTGAGTACA |
| 766 | GGGACTCCAAATATTGCTGTAGTAT |
| 1065 | GTACCTGTTGCTCCAGGTATGTT |

Other primers can be readily devised from the known genomic and cDNA sequences of CFTR. The selection of primers, of course, depends on the areas of the target sequence that are to be screened. The choice of primers also depends on the strand to be amplified. For some regions of the CFTR gene, it makes little difference to the hybridization signal whether the coding or noncoding strand is used. In other regions, one strand may give better discrimination in hybridization signals between matched and mismatched probes than the other. The upper limit in the length of a segment that can be amplified from one pair of PCR primers is about 50 kb. Thus, for analysis of mutants through all or much of the CFTR gene, it is often desirable to amplify several segments from several paired primers. The different segments may be amplified sequentially or simultaneously by multiplex PCR. Frequently, fifteen or more segments of the CFTR gene are simultaneously amplified by PCR. The primers and amplifications conditions are preferably selected to generate DNA targets. An asymmetric labelling strategy incorporating fluorescently labelled dNTPs for random labelling and dUTP for target fragmentation to an average length of less than 60 bases is preferred. The use of dUTP and fragmentation with uracil N-glycosylase has the added advantage of eliminating carry over between samples.

Mutations in the CFTR gene can be detected by any of the tiling strategies noted above. The block tiling strategy is one particularly useful approach. In this strategy, a group (or block) of probes is used to analyze a short segment of contiguous nucleotides (*e.g.*, 3, 5, 7 or 9) from a CFTR gene centered around the site of a mutation. The probes in a group are sometimes referred to as constituting a block because all probes in the group are usually identical except at their interrogation positions. As noted above, the probes may also differ in the presence of leading or trailing sequences flanking regions of complementary. However, for ease of illustration, it will be assumed that such sequences are not present. As an example, to analyze a segment of five contiguous nucleotides from the CFTR gene, including the site of a mutation (such as one of the mutations in Table 6), a block of probes usually contains at least one wildtype probe and five sets of mutant probes, each having three probes. The wildtype probe has five interrogation positions corresponding to the five nucleotides being analyzed from the reference sequence. However, the identity of the interrogation positions is only apparent when the structure of the wildtype probe is compared with that of the probes in the five mutant probe sets. The first mutant probe set comprises three probes, each being identical to the wildtype probe, except in the first interrogation position, which differs in each of the three mutant probes and the wildtype probe. The second through fifth mutant probe sets are similarly composed except that the differences from the wildtype probe occur in the second through fifth interrogation position respectively. Note that in practice, each set of mutant probes is sometimes laid down on the chip juxtaposed with an associated wildtype probe. In this situation, a block would comprise five wildtype probes, each effectively providing the same information. However, visual inspection and confidence analysis of the chip is facilitated by the largely redundant information provided by five wildtype probes.

After hybridization to labelled target, the relative hybridization signals are read from the probes. Comparison of the intensities of the three probes in the first mutant probe set with that of the wildtype probe indicates the identity of the nucleotide in the target sequence corresponding to the first interrogation position. Comparison of the intensities of the three probes in the second mutant probe set with that of the wildtype probe indicates the identity of the nucleotide

in the target sequence corresponding to the second interrogation position, and so forth. Collectively, the relative hybridization intensities indicate the identity of each of the five contiguous nucleotides in the reference sequence.

In a preferred embodiment, a first group (or block) of probes is tiled based on a wildtype reference sequence and a second group is tiled based a mutant version of the wildtype reference sequence. The mutation can be a point mutation, insertion or deletion or any combination of these. The combination of first and second groups of probes facilitates analysis when multiple target sequences are simultaneously applied to the chip, as is the case when a patient being diagnosed is heterozygous for the CFTR allele.

The above strategy is illustrated in Fig. 16, which shows two groups of probes tiled for a wildtype reference sequence and a point mutation thereof. The five mutant probe sets for the wildtype reference sequence are designated wt1-5, and the five mutant probe sets for the mutant reference sequence are designated m1-5. The letter N indicates the interrogation position, which shifts by one position in successive probe sets from the same group. The figure illustrates the hybridization pattern obtained when the chip is hybridized with a homozygous wildtype target sequence comprising nucleotides n-2 to n+2, where n is the site of a mutation. For the group of probes tiled based on the reference sequence, four probes are compared at each interrogation position. At each position, one of the four probes exhibits a perfect match with the target, and the other three exhibit a single-base mismatch. For the group of probes tiled based on the mutant reference sequence, again four probes are compared at each interrogation position. At position, n, one probe exhibits a perfect match, and three probes exhibit a single base mismatch. Hybridization to a homozygous mutant yields an analogous pattern, except that the respective hybridization patterns of probes tiled on the wildtype and mutant reference sequences are reversed.

The hybridization pattern is very different when the chip is hybridized with a sample from a patient who is heterozygous for the mutant allele (*see* Fig. 17). For the group of probes tiled based on the wildtype sequence, at all positions but n, one probe exhibits a perfect match at each interrogation position, and the other three probes exhibit a one base mismatch. At position n, two probes exhibit a perfect match (one for each allele), and the other probes exhibit single-base mismatches. For the group of probes tiled on the mutant sequence, the same result is obtained. Thus, the heterozygote point mutant is easily distinguished from both the homozygous wildtype and mutant forms by the identity of hybridization patterns from the two groups of probes.

Typically, a chip comprises several paired groups of probes, each pair for detecting a particular mutation. For example, some chips contain 5, 10, 20, 40 or 100 paired groups of probes for detecting the corresponding numbers of mutations. Some chips are customized to include paired groups of probes for detecting all mutations common in particular populations (*see* Table 6). Chips usually also contain control probes for verifying that correct amplification has occurred and that the target is properly labelled.

The goal of the tiling strategy described above is to focus on short regions of the CTFR region flanking the sites of known mutation. Other tiling strategies analyze much larger regions of the CFTR gene, and are appropriate for locating and identifying hitherto uncharacterized mutations. For example, the entire genomic CFTR gene (250 kb) can be tiled by the basic tiling strategy from an array of about one million probes. Synthesis and scanning of such an array of probes is entirely feasible. Other tiling strategies, such as the block tiling, multiplex tiling or pooling can cover the entire gene with fewer probes. Some tiling strategies analyze some or all of components of the CFTR gene, such as the cDNA coding sequence or individual exons. Analysis of exons 10 and 11 is particularly informative because these are location of many common mutations including the ΔF508 mutation.

<u>Exemplary CFTR chips</u>

One illustrative chip bears an array of 1296 probes covering the full length of exon 10 of the CFTR gene arranged in a 36 x 36 array of 356 μm elements. The probes in the array can have any length, preferably in the range of from 10 to 18 residues and can be used to detect and sequence any single-base substitution and any deletion within the 192-base exon, including the three-base deletion known as ΔF508. As described in detail below, hybridization of nanomolar concentrations of wild-type and ΔF508 oligonucleotide target nucleic acids labeled with fluorescein to these arrays produces highly specific signals (detected with confocal scanning fluorescence microscopy) that permit discrimination between mutant and wild-type target sequences in both homozygous and heterozygous cases.

Sets of probes of a selected length in the range of from 10 to 18 bases and complementary to subsequences of the known wild-type CFTR sequence are synthesized starting at a position a few bases into the intron on the 5'-side of exon 10 and ending a few bases into the intron on the 3'-side. There is a probe for each possible subsequence of the given segment of the gene, and the probes are organized into a "lane" in such a way that traversing the lane from the upper left-hand corner of the chip to the lower righthand corner corresponded to traversing the gene segment base-by-base from the 5'-end. The lane containing that set of probes is, as noted above, called the "wild-type lane."

Relative to the wild-type lane, a "substitution" lane, called the "A-lane", was synthesized on the chip. The A-lane probes were identical in sequence to an adjacent (immediately below the corresponding) wild-type probe but contained, regardless of the sequence of the wild-type probe, a dA residue at position 7 (counting from the 3'-end). In

similar fashion, substitution lanes with replacement bases dC, dG, and dT were placed onto the chip in a "C-lane," a "G-lane," and a "T-lane," respectively. A sixth lane on the chip consisted of probes identical to those in the wild-type lane but for the deletion of the base in position 7 and restoration of the original probe length by addition to the 5'-end the base complementary to the gene at that position.

The four substitution lanes enable one to deduce the sequence of a target exon 10 nucleic acid from the relative intensities with which the target hybridizes to the probes in the various lanes. Various versions of such exon 10 DNA chips were made as described above with probes 15 bases long, as well as chips with probes 10, 14, and 18 bases long. For the results described below, the probes were 15 bases long, and the position of substitution was 7 from the 3'-end.

The sequences of several important probes are shown below. In each case, the letter "X" stands for the interrogation position in a given column set, so each of the sequences actually represents four probes, with A, C, G, and T, respectively, taking the place of the "X." Sets of shorter probes derived from the sets shown below by removing up to five bases from the 5'-end of each probe and sets of longer probes made from this set by adding up to three bases from the exon 10 sequence to the 5'-end of each probe, are also useful and provided by the invention.

```
3'-TTTATAXTAGAAACC
3'- TTATAGXAGAAACCA
3'-  TATAGTXGAAACCAC
3'-   ATAGTAXAAACCACA
3'-    TAGTAGXAACCACAA
3'-     AGTAGAXACCACAAA
3'-      GTAGAAXCCACAAAG
3'-       TAGAAAXCACAAAGG
3'-        AGAAACXACAAAGGA
```

To demonstrate the ability of the chip to distinguish the ΔF508 mutation from the wild-type, two synthetic target nucleic acids were made. The first, a 39-mer complementary to a subsequence of exon 10 of the CFTR gene having the three bases involved in the ΔF508 mutation near its center, is called the "wild-type" or wt508 target, corresponds to positions 111-149 of the exon, and has the sequence shown below:

**5'-CATTAAAGAAAATATCATCTTTGGTGTTTCCTATGATGA.**

The second, a 36-mer probe derived from the wild-type target by removing those same three bases, is called the "mutant" target or mu508 target and has the sequence shown below, first with dashes to indicate the deleted bases, and then without dashes but with one base underlined (to indicate the base detected by the T-lane probe, as discussed below):

**5'-CATTAAAGAAAATATCAT---TGGTGTTTCCTATGATGA;**

**5'-CATTAAAGAAAATATC<u>A</u>TTGGTGTTTCCTATGATGA.**

Both targets were labeled with fluorescein at the 5'-end.

In three separate experiments, the wild-type target, the mutant target, and an equimolar mixture of both targets was exposed (0.1 nM wt508, 0.1 nM mu508, and 0.1 nM wt508 plus 0.1 nM mu508, respectively, in a solution compatible with nucleic acid hybridization) to a CF chip. The hybridization mixture was incubated overnight at room temperature, and then the chip was scanned on a reader (a confocal fluorescence microscope in photon-counting mode); images of the chip were constructed from the photon counts) at several successively higher temperatures while still in contact with the target solution. After each temperature change, the chip was allowed to equilibrate for approximately one-half hour before being scanned. After each set of scans, the chip was exposed to denaturing solvent and conditions to

wash, *i.e.*, remove target that had bound, the chip so that the next experiment could be done with a clean chip.

The results of the experiments are shown in Figures 18, 19, 20, and 21. Figure 18, in panels A, B, and C, shows an image made from the region of a DNA chip containing CFTR exon 10 probes; in panel A, the chip was hybridized to a wild-type target; in panel C, the chip was hybridized to a mutant ΔF508 target; and in panel B, the chip was hybridized to a mixture of the wild-type and mutant targets. Figure 19, in sheets 1 - 3, corresponding to panels A, B, and C of Figure 3, shows graphs of fluorescence intensity versus tiling position. The labels on the horizontal axis show the bases in the wild-type sequence corresponding to the position of substitution in the respective probes. Plotted are the intensities observed from the features (or synthesis sites) containing wild-type probes, the features containing the substitution probes that bound the most target ("called"), and the feature containing the substitution probes that bound the target with the second highest intensity of all the substitution probes ("2nd Highest").

These figures show that, for the wild-type target and the equimolar mixture of targets, the substitution probe with a nucleotide sequence identical to the corresponding wild-type probe bound the most target, allowing for an unambiguous assignment of target sequence as shown by letters near the points on the curve. The target wt508 thus hybridized to the probes in the wild-type lane of the chip, although the strength of the hybridization varied from probe-to-probe, probably due to differences in melting temperature. The sequence of most of the target can thus be read directly from the chip, by inference from the pattern of hybridization in the lanes of substitution probes (if the target hybridizes most intensely to the probe in the A-lane, then one infers that the target has a T in the position of substitution, and so on).

For the mutant target, the sequence could similarly be called on the 3'-side of the deletion. However, the intensity of binding declined precipitously as the point of substitution approached the site of the deletion from the 3'-end of the target, so that the binding intensity on the wild-type probe whose point of substitution corresponds to the T at the 3'-end of the deletion was very close to background. Following that pattern, the wild-type probe whose point of substitution corresponds to the middle base (also a T) of the deletion bound still less target. However, the probe in the T-lane of that column set bound the target very well. Examination of the sequences of the two targets reveals that the deletion places an A at that position when the sequences are aligned at their 3'-ends and that the T-lane probe is complementary to the mutant target with but two mismatches near an end (shown below in lower-case letters, with the position of substitution underlined):

```
Target:  5'-CATTAAAGAAAATATCATTGGTGTTTCCTATGATGA
Probe:                                  3'-TagTAGTAACCACAA
```

Thus the T-lane probe in that column set calls the correct base from the mutant sequence. Note that, in the graph for the equimolar mixture of the two targets, that T-lane probe binds almost as much target as does the A-lane probe in the same column set, whereas in the other column sets, the probes that do not have wild-type sequence do not bind target at all as well. Thus, that one column set, and in particular the T-lane probe within that set, detects the ΔF508 mutation under conditions that simulate the homozygous case and also conditions that simulate the heterozygous case.

Although in this example the sequence could not be reliably deduced near the ends of the target, where there is not enough overlap between target and probe to allow effective hybridization, and around the center of the target, where hybridization was weak for some other reason, perhaps high AT-content, the results show the method and the probes of the invention can be used to detect the mutation of interest. The mutant target gave a pattern of hybridization that was very similar to that of the wt508 target at the ends, where the two share a common sequence, and very different in the middle, where the deletion is located. As one scans the image from right to left, the intensity of hybridization of the target to the probes in the wild-type lane drops off much more rapidly near the center of the image for mu508 than for wt508; in addition, there is one probe in the T-lane that hybridizes intensely with mu508 and hardly at all with wt508. The results from the equimolar mixture of the two targets, which represents the case one would encounter in testing a heterozygous individual for the mutation, are a blend of the results for the separate targets, showing the power of the invention to distinguish a wild-type target sequence from one containing the ΔF508 mutation and to detect a mixture of the two sequences.

The results above clearly demonstrate how the DNA chips of the invention can be used to detect a deletion mutation, ΔF508; another model system was used to show that the chips can also be used to detect a point mutation as well. One mutation in the CFTR gene is G480C, which involves the replacement of the G in position 46 of exon 10 by a T, resulting in the substitution of a cysteine for the glycine normally in position #480 of the CFTR protein. The model target sequences included the 21-mer probe wt480 to represent the wild-type sequence at positions 37-55 of exon 10: 5'-CCTTCAGAGGGTAAAATTAAG and the 21-mer probe mu480 to represent the mutant sequence:

5'-CCTTCAGAGTGTAAAATTAAG.

In separate experiments, a DNA chip was hybridized to each of the targets wt480 and mu480, respectively, and then scanned with a confocal microscope. Figure 20, in panels A, B, and C, shows an image made from the region of a DNA chip containing CFTR exon 10 probes; in panel A, the chip was hybridized to the wt480 target; in panel C, the chip was hybridized to the mu480 target; and in panel B, the chip was hybridized to a mixture of the wild-type and mutant targets. Figure 21, in sheets 1 - 3, corresponding to panels A, B, and C of Figure 20, shows graphs of fluorescence intensity versus tiling position. The labels on the horizontal axis show the bases in the wild-type sequence corresponding to the position of substitution in the respective probes. Plotted are the intensities observed from the features (or synthesis sites) containing wild-type probes, the features containing the substitution probes that bound the most target ("called"), and the feature containing the substitution probes that bound the target with the second highest intensity of all the substitution probes ("2nd Highest").

These figures show that the chip could be used to sequence a 16-base stretch from the center of the target wt480 and that discrimination against mismatches is quite good throughout the sequenced region. When the DNA chip was exposed to the target mu480, only one probe in the portion of the chip shown bound the target well: the probe in the set of probes devoted to identifying the base at position 46 in exon 10 and that has an A in the position of substitution and so is fully complementary to the central portion of the mutant target. All other probes in that region of the chip have at least one mismatch with the mutant target and therefore bind much less of it. In spite of that fact, the sequence of mu480 for several positions to both sides of the mutation can be read from the chip, albeit with much-reduced intensities from those observed with the wild-type target.

The results also show that, when the two targets were mixed together and exposed to the chip, the hybridization pattern observed was a combination of the other two patterns. The wild-type sequence could easily be read from the chip, but the probe that bound the mu480 target so well when only the mu480 target was present also bound it well when both the mutant and wild-type targets were present in a mixture, making the hybridization pattern easily distinguishable from that of the wild-type target alone. These results again show the power of the DNA chips of the invention to detect point mutations in both homo- and heterozygous individuals.

To demonstrate clinical application of the DNA chips of the invention, the chips were used to study and detect mutations in nucleic acids from genomic samples. Genomic samples from a individual carrying only the wild-type gene and an individual heterozygous for ΔF508 were amplified by PCR using exon 10 primers containing the promoter for T7 RNA polymerase. Illustrative primers of the invention are shown below.

| Exon | Name | Sequence |
|---|---|---|
| 10 | CFi9-T7 | TAATACGACTCACTATAGGGAGatgacctaataatgatgggttt |
| 10 | CFi10c-T7 | TAATACGACTCACTATAGGGAGtagtgtgaagggttcatatgc |
| 10 | CFi10c-T3 | CTCGGAATTAACCCTCACTAAAGGtagtgtgaagggttcatatgc |
| 11 | CFi10-T7 | TAATACGACTCACTATAGGGAGagcatactaaaagtgactctc |
| 11 | CFi11c-T7 | TAATACGACTCACTATAGGGAGacatgaatgacatttacagcaa |
| 11 | CFi11c-T3 | CGGAATTAACCCTCACTAAAGGacatgaatgacatttacagcaa |

These primers can be used to amplify exon 10 or exon 11 sequences; in another embodiment, multiplex PCR is employed, using two or more pairs of primers to amplify more than one exon at a time.

The product of amplification was then used as a template for the RNA polymerase, with fluoresceinated UTP present to label the RNA product. After sufficient RNA was made, it was fragmented and applied to an exon 10 DNA chip for 15 minutes, after which the chip was washed with hybridization buffer and scanned with the fluorescence microscope. A useful positive control included on many CF exon 10 chips is the 8-mer 3'-CGCCGCCG-5'. Figure 22, in panels A and B, shows an image made from a region of a DNA chip containing CFTR exon 10 probes; in panel A, the chip was hybridized to nucleic acid derived from the genomic DNA of an individual with wild-type ΔF508 sequences; in panel B, the target nucleic acid originated from a heterozygous (with respect to the ΔF508 mutation) individual. Figure 23, in sheets 1 and 2, corresponding to panels A and B of Figure 22, shows graphs of fluorescence intensity versus tiling position.

These figures show that the sequence of the wild-type RNA can be called for most of the bases near the

mutation. In the case of the ΔF508 heterozygous carrier, one particular probe, the same one that distinguished so clearly between the wild-type and mutant oligonucleotide targets in the model system described above, in the T-lane binds a large amount of RNA, while the same probe binds little RNA from the wild-type individual. These results show that the DNA chips of the invention are capable of detecting the ΔF508 mutation in a heterozygous carrier.

Further chips were constructed using the block tiling strategy to provide an array of probes for analyzing a CFTR mutation. The array comprised 93 mm x 96 μm features arranged into eleven columns and four rows (44 total probes). Probes in five of these columns were from four probe sets tiled based on the wildtype CFTR sequence and having interrogation positions corresponding to the site of a mutation and two bases on either side. Five of the remaining columns contained four sets of probes tiled based on the mutant version of the CFTR sequence. These probe sets also had interrogation positions corresponding to the site of mutation and two nucleotides on either side. The eleventh column contained four cells for control probes.

Fluorescently labeled hybridization targets were prepared by PCR amplification. 100 μg of genomic DNA, 0.4 μM of each primer, 50 μM each dATP, dCTP, dCTP and dUTP (Pharmacia) n 10mM Tris-Cl, pH 8.3, 50 mM KCl, 2.5 mM $MgCl_2$ and 2 U Taq polymerase (Perkin-Elmer) were cycled 36 times using a Perkin-Elmer 9600 thermocycler and the following times and temperatures: 95°C, 10 sec., 55°C, 10 sec., 72°C, 30 sec. 10 μl of this reaction product was used as a template in a second, asymmetric PCR reaction. Conditions included 1μM asymmetric PCR primer, 50 μM each dATP, dCTP, TTP, 25 μM fluorescein-dGTP (DuPont), 10 mM Tris-Cl, pH 9.1, 75 mM KCl, 3.5 mM $MgCl_2$. The reaction was cycled 5X with the following conditions: 95°C, 10 sec, 60°C, 10 sec, 55°C, 1 min. and 72°C, 1.5 min. This was immediately followed with another 20 cycles using the following conditions: 95°C, 10 sec, 60°C, 10 sec., 72°C, 1.5 min.

Amplification products were fragmented by treating with 2 U of Uracil-N-glycosylase (Gibco) at 30°C for 30 min. followed by heat denaturation at 95°C for 5 min. Finally, the labeled, fragmented PCR product was diluted into hybridization buffer made up of 5 X SSPE and 1 mM Cetyltrimethylammonium Bromide (CTAB). The dilution factor ranged from 10x to 25x with 40 μl of sample being diluted into 0.4 ml to 1 ml of hybridization solution.

Target hybridization was generally carried out with the chip shaking in a small dish containing 500 μl to 1 ml total volume of hybridization solution. All hybridizations were done at 30°C constant temperature. Alternatively, some hybridizations were carried out with chips enclosed in a plastic package with the 1 cm x 1 cm chip glued facing a 250 μl fluid chamber. 250-350 μl of hybridization solution was introduced and mixed using a syringe pump. Temperature was controlled by interfacing the back surface of the package with a Peltier heating/cooling device. Following hybridization chips were washed with 5X SSPE, 0.1% Triton X-100 at 25°C-30°C prior to fluorescent image generation.

Hybridized, washed DNA chips were scanned for fluorescence using a stage-scanning confocal epifluorescent microscope and 488nm argon ion laser excitation. Emitted light was collected through a band pass filter centered at 530nM. The resulting fluorescence image was spatially reconstructed and intensity data were then analyzed. Features with the peak fluorescence intensity in each column were identified and compared with any signal intensity at the remaining single base mismatch probe sites in the same column. The sequences of the highest intensity features were then compared across all ten columns of each sub-array to determine whether peak intensity scores for the wild type sequence and the mutant sequence were similar or significantly different. These results were used to generate the genotype call of wild type (high intensity signals only in wild type probe columns), mutant (high intensity signals only in the mutant probe columns) or heterozygous (high intensity signals in both the wild type and mutant probe columns).

Figure 24 (panel A) shows an image of the fluorescence signals in arrays designed to detect the G551D(G>A) and Q552X(C>T) CFTR mutations. The hybridization target is an exon 11 amplicon generated from wild type genomic DNA. Wild type hybridization patterns are evident at both locations. No significant fluorescence signal resulted at any of the features with probes complementary to mutant or mismatched sequences. Relative fluorescence intensities were six fold brighter for the perfect matched wildtype features compared with the background signal intensity at mutant and mismatch features. In addition, the sequence at these loci can be confirmed as AGGTC and GTCAA, respectively, where the bold type face indicates the mutation sites. Figure 24 (panel B) shows the same probe array features after hybridization with a fluorescent target generated from DNA heterozygous for the G551D mutation. Both the wild type and mutant probe columns have features with significant fluorescence intensity, indicating the hybridization of both wild type and mutant CFTR alleles at this site. Only wildtype probes hybridized with any significant fluorescence signal in the Q552X subarray indicating a wild type target sequence. However, an additional feature that did not hybridize in the first experiment shows significant fluorescence intensity in this experiment. Because the G551D and Q552X mutations are only two bases apart, the a probe sequence in the additional feature has a perfectly matched 12-mer overlap with the mutant G551D target.

Figure 25 (panels A and B) illustrates mutation analysis for ΔF508, a three base pair deletion in Exon 10 of the CFTR gene. In contrast to the hybridization pattern seen in base change mutations, in mutations where bases are inserted or deleted, probe arrays show a different hybridization pattern. Identical probes are synthesized in the two central columns of base substitution arrays. As a result, either mutant or wild type target hybridizations always result in two side-by-side features (a doublet) with high fluorescence intensity at the center of the array. In a heterozygote

hybridization, two sets of doublets, one matched to the wild type sequence and one to the mutant sequence occur (Figure 24, panel B). In contrast, wild type and mutant probe column sequences are offset from each other for deletion or insertion mutations and hybridization doublets are not seen. Instead of the six high intensity signals with one doublet, five independent features in alternating columns characterize a homozygote and ten features, one in each column will be positive with heterozygote targets. This is evident from the ΔF508 hybridization pattern in Figure 25, panel A. Although a wildtype target has been hybridized and the highest intensity features confirm the wild type sequence (ATCTT), there is an additional hybridization in the first mutant column. Analysis of that probe sequence shows a 10 base perfect match with the mutant sequence.

The image in Figure 25, panel B resulted from hybridizing a DNA chip with a target homozygous for ΔF508. In this image five features, all with probe sequences complementary to the mutant show significant signal. The mutation sequence bridging the deletion site, ATTGG, is confirmed. Similar to what was seen in the example of the G551D mutation, there is added information in neighboring subarrays designed to detect the ΔI507 and F508C mutations. This is expected since they are in such close proximity to ΔF508 that their probe sets significantly overlap the ΔF508 probes. The ΔF508 homozygous target has no perfect matches with wild type or mutant probes in the ΔI507 and F508C subarrays. However, there are some low intensity signals within these two blocks of probes. The F508C array has a doublet that matches 11 bases of the mutant ΔF508 target. Similarly, the hybridization in the eighth column of the ΔI507 array has a probe that matches 13/14 bases with the target.

Figure 26 shows hybridization of a heterozygous double mutant ΔF508/F508C to the same array as described above. Conventional reverse dot blot would score this sample as a homozygous ΔF508 mutant. In the present assays, the ΔF508 and F508C alleles are separately detected by the respective subarrays designed to detect these mutations.

C. Chins for Cancer Diagnosis

There are at least two types of genes which are often altered in cancerous cells. The first type of gene is an oncogene such as a mismatch-repair gene, and the second type of gene is a tumor suppressor gene such as a transcription factor. Examples of mismatch repair oncogenes genes include hMSH2 (Fishel et al., *Cell* 75, 1027-1038 (1993)) and hMLH1 (Papadopoulos et al., *Science* 263, 1625-1628 (1994)). The most well-known example of a tumor suppressor gene is the p53 protein gene (Buchman et al., *Gene* 70, 245-252 (1988). By monitoring the state of both oncogenes and tumor suppressor genes (individually and in combination) in a patient, it is possible to determine individual susceptibility to a cancer, a patient's prognosis upon cancer diagnosis, and to target therapy more efficiently.

The p53 gene spans 20 kbp in humans and has 11 exons, 10 of which are protein coding (*see* Tominaga et al., 1992, *Critical Reviews in Oncogenesis* 3:257-282. The gene produces a 53 kilodalton phosphoprotein that regulates DNA replication. The protein acts to halt replication at the G1/S boundary in the cell cycle and is believed to act as a "molecular policeman," shutting down replication when the DNA is damaged or blocking the reproduction of DNA viruses (*see* Lane, 1992, *Nature* 358:15-16 . The p53 transcription factor is part of a fundamental pathway which controls cell growth. Wild-type p53 can halt cell growth, or in some cases bring about programmed cell death (apoptosis). Such tumor-suppressive effects are absent in a variety of known p53 gene mutations. Moreover, p53 mutants not only deprive a cell of wild-type p53 tumor suppression, they also may spur abnormal cell growth.

In tumor cells, p53 is the most commonly mutated gene discovered to date (see Levine et al., 1991, *Nature* 351:453-456, and Hollstein et al., 1991, *Science* 253:49-53 Over half of the 6.5 million patients diagnosed with cancer annually possess p53 mutations in their tumor cells. Among common tumors, about 70% of colorectal cancers, 50% of lung cancers and 40% of breast cancers contain p53 mutations. In all, over 51 types of human tumors have been documented to possess p53 mutations, including bladder, brain, breast, cervix, colon, esophagus, larynx, liver, lung, ovary, pancreas, prostate, skin, stomach, and thyroid tumors (Culotta & Koshland, *Science* 262, 1958-1961 (1993); Rodrigues et al., 1990, *PNAS* 87:7555-7559. According to data presented by David Sidransky (1992 San Diego Conference), over 400 mutations in p53 are known. The presence of a p53 mutation in a tumor has also been correlated with a patient's prognosis. Patients who possess p53 mutations have a lower 5-year survival rate.

Proper diagnosis of the form of p53 in tumor cells is critical to clinicians to prescribe appropriate therapeutic regimens. For instance, patients with breast cancer who show no invasion of nearby lymph nodes generally do not relapse after standard surgical treatment and chemotherapy. Of the 25% who do relapse after surgery and chemotherapy, additional chemotherapy is appropriate. At present, there is no clear way to determine which patients will benefit from such additional chemotherapy prior to relapse. However, correlating p53 mutations to tumorigenicity and metastasis provides clinicians with a means to determine whether such additional treatments are warranted.

In addition to facilitating conventional chemotherapy, appropriate diagnosis of p53 mutations provides clinicians with the ability to identify individuals who will benefit the most from gene therapy techniques, in which appropriately operative p53 copies are restored to a tumor site. Clinical p53 gene therapy trials are presently underway (Culotta & Koshland, *supra*).

The analysis of p53 mutations can also be used to identify which carcinogens lead to particular tumors (Harris,

*Science* 262, 1980-1981 (1993)). For instance, dietary aflatoxin $B_1$ exposure is associated with G:C to T:A transversions at residue 249 of p53 in hepatocellular carcinomas (Hsu et al., *Nature* 350, 427 (1991); Bressac et al., *Nature* 350, 429 (1991); Harris, *supra).*

While most described p53 mutations are somatic in origin, some types of cancer are associated with germline p53 mutation. For instance, Li-Fraumeni syndrome is a hereditary condition in which individuals receive mutant p53 alleles, resulting in the early onset of various cancers (Harris, *supra);* Frebourg et al., *PNAS* 89, 6413-6417 (1992); Malkin et al., Science 250, 1233 (1990)). These mutations are associated with instability in the rest of the genome, creating multiple genetic alterations, and eventually leading to cancer.

hMLH1 and hMSH2 are mismatch repair genes which are causal agents in hereditary nonpolyposis colorectal cancer in individuals with mutant hMLH1 or hMSH2 alleles (Fishel et al., *supra*, and Papadopoulos et al., *supra*). Hereditary nonpolyposis colorectal cancer is a common genetic disorders, affecting about 1 in 200 individuals (Lynch et al., *Gastroenterology* 104, 1535 (1993)). Detection of hMLH1 and hMSH2 mutations in the population allows diagnosis of nonpolyposis colorectal cancer prone individuals prior to the manifestation of disease. This allows for the implementation of special screening programs for cancer-prone individuals to ensure early detection of cancer, thereby enhancing survival rates of afflicted individuals. In addition, genetic counselors may use the information derived from HMLH1 and HMSH2 chips to improve family planning as described for cystic fibrosis chips. The detection of mutations in hMLH1 and hMSH2 individually or in combination with p53 can also be used by clinicians to assess cancer prognosis and treatment modality. Finally, the information can be used to target appropriate individuals for gene therapy.

The entire hMLH1 gene is less than 85 kbp in length, comprising 2268 coding nucleotides (Papadopoulos et al., *supra).* Sequences from the gene have been deposited with GenBank (accession number U07418). Mutations associated with hereditary nonpolyposis colorectal cancer include the deletion of exon 5 (codons 578-632), a 4 base pair deletion of codons 727 and 728 resulting in a shift in the reading frame of the gene, a 4 base pair insertion at codons 755 and 756 resulting in an extension of the COOH terminus, a 371 base pair deletion and frameshift mutation at position 347, and a transversion causing an alteration of codon 252 resulting in the insertion of a stop codon (*id*.).

hMSH2 is a human homologue of the bacterial *MutS* and *S. cerevisiae* MSH mismatch-repair genes. MSH2, like hMLH1 is associated with hereditary nonpolyposis cancer. Although only a few MSH2 gene samples from tumor tissue have been characterized, at least some tumor samples show a T to C transition mutation at position 2020 of the cDNA sequence, resulting in the loss of an intron-exon splice acceptor site.

In view of the role of mutations in p53, MSH2 and/or hMLH1 in hereditary predisposition to cancer, to neoplastic transformation events leading to cancer and to cancer prognosis, it is important to screen individuals to determine whether they possess mutant alleles, and to identify precisely which mutations the individuals possess. Because many mutations are point mutations, or extremely small insertions or deletions, which are generally undetectable by standard Southern analysis, accurate diagnosis requires a capacity to examine a gene nucleotide-by-nucleotide.

Mutations in the hMSH2, hMLH1 or p53 genes, irrespective of whether previously characterized, can be detected by any of the tiling strategies noted above. Reference sequences of interest include full-length genomic and cDNA sequences of each of these genes and subsequences thereof, such as exons and introns. For example, each nucleotide in the 20 kb p53 genomic sequence can be tiled using the basic strategy with an array of about 80,000 probes. As in the CFTR chip, some reference sequences are comparatively short sequences including the site of a known mutation and a few flanking nucleotides. Some chips tile reference sequences that encompass mutational "hot spots." For instance, a variety of cellular and oncoviral proteins bind to specific regions of p53, including Mdm2, SV40 T antigen, E1b from adenovirus and E6 from human papilloma virus. These binding sites correlate to some extent with observed high frequency somatic mutation regions of p53 found in tumor cells from cancer patients (*see* Harris et al., *supra).* Hot spots include exons 2, 3, 5, 6, 7 and 8 and the intronic regions between exons 2 and 3, 3 and 4 and 4 and 5. Fragments of the hMLH1 gene of particular interest include those encoding codons 578-632, 727, 728, 347, 252. Some chips are tiled to read mutations in each of the hMSH2, hMLH1 and p53 genes, both wildtype and mutant versions.

Standard or asymmetric PCR can be used to generate the target DNA used in the tiling assays described above. In general, PCR is used to amplify hMSH2, hMLH1 or p53 sequences from a tissue of interest such as a tumor. Mixed PCR reactions can also be used to generate hMSH2, hMLH1 or p53 sequences simultaneously in a single reaction mixture. Any of the coding or noncoding sequences from the genes may be amplified for use in the block tiling assays described above.

Table 8 below provides examples of primers which are useful in synthesizing specific regions of hMSH2, hMHLH1 and p53. Other primers can readily be devised from the known genomic and cDNA sequences of the genes. The primers described in Table 8 specific for p53 amplification have ends tailored to facilitate cloning into standard restriction enzyme cloning sites.

Table 8: Examples of PCR primers useful in amplifying regions of p53, hMHH1 and hMSH2.

| Region Amplified | Primer Sequence | Description |
|---|---|---|
| Exon 5 (p53) | TAA TAC GAC TCA CTA TAG GGA GA CCC TGG GCA ACC AGC CCT GTC GT | Exon 5 T7 Primer (5' T7 to p53 3'). |
| Exon 5 (p53) | ATG CAA TTA ACC CTC ACT AAA GGG AGA CAC TTG TGC CCT GAC TTT CAA C | Exon 5 T3 Primer (5' T3 to p53 3'). |
| Exon 6 (p53) | TAA TAC GAC TCA CTA TAG GGA GCC TCC TCC CAG AGA CCC | Exon 6 T7 Primer (5'T7 to p53 3'). |
| Exon 6 (p53) | ATG CAA TTA ACC CTC ACT AA GGG AGA TCC CCA GGC CTC TGA TTC CTC ACT G | Exon 6 T3 Primer (5'T3 to p53 3'). |
| Exon 7 (p53) | TAA TAC GAC TCA CTA TAG GGA CTG GGG CAC AGC CAG GCC AGT GTG CA | Exon 7 T7 Primer (5' T7 to p53 3'). |
| Exon 7 (p53) | ATG CAA TTA ACC CTC ACT AAA GGG AGA GTC TCC CCA AGG CGC ACT GGC CTC A | Exon 7 T3 Primer (5' T3 to p53 3'). |
| Exon 8 (p53) | TAA TAC GAC TCA CTA TAG GGA GGG CAT AAC TGC ACC CTT GGT CTC CTC C | Exon 8 T7 Primer (5' T7 to p53 3'). |
| Exon 8 (p53) | ATG CAA TTA ACC CTC ACT AAA GGG AGA GGA CCT GAT TTC CTT ACT GCC TCT TGC | Exon 8 T3 Primer (5' T3 to p53 3'). |
| hMSH2 | GAC ATG GCG GTG CAG CCG AAG GAG A | Primer for MSH2, 5' to 3'. If used with MSH2 primer below, a 3033 base pair amplicon will result |
| hMSH2 | CTA TGT CAA TTG CAA ACA GTG CTC AGT TAC AG | Primer for hMSH2 5'to 3'. |
| hMLH1 | CTT GGC TCT TCT GGC GCC AAA ATG TCG TTC | Primer for hMLH1, 5'to 3'. If used with hMLH1 primer below, a 2484 base pair amplicon will result. |
| hMLH1 | TAT GTT AAG ACA CAT CTA TTT ATT TAT AAT CAA TCC | Primer for hMLH1 5' to 3'. |

After PCR amplification of the target amplicon one strand of the amplicon can be isolated, *i.e.*, using a biotinylated primer that allows capture of the undesired strand on streptavidin beads. Alternatively, asymmetric PCR can be used to generate a single-stranded target. Another approach involves the generation of single stranded RNA from the PCR product by incorporating a T7 or other RNA polymerase promoter in one of the primers. The single-stranded material can optionally be fragmented to generate smaller nucleic acids with less significant secondary structure than longer nucleic acids.

In one such method, fragmentation is combined with labeling. To illustrate, degenerate 8-mers or other degenerate short oligonucleotides are hybridized to the single-stranded target material. In the next step, a DNA polymerase is added with the four different dideoxynucleotides, each labeled with a different fluorophore. Fluorophore-labeled dideoxynucleotide are available from a variety of commercial suppliers. Hybridized 8-mers are extended by a labeled dideoxynucleotide. After an optional purification step, *i.e.*, with a size exclusion column, the labeled 9-mers are hybridized to the chip. Other methods of target fragmentation can be employed. The single-stranded DNA can be fragmented by partial degradation with a DNAse or partial depurination with acid. Labeling can be accomplished in a separate step, *i.e.*, fluorophore-labeled nucleotides are incorporated before the fragmentation step or a DNA binding fluorophore, such as ethidium homodimer, is attached to the target after fragmentation.

Exemplary Chips

a. Exon VI Chip

To illustrate the value of the DNA chips of the present invention in such a method, a DNA chip was synthesized by the VLSIPS™ method to provide an array of overlapping probes which represent or tile across a 60 base region of exon 6 of the p53 gene. To demonstrate the ability to detect substitution mutations in the target, twelve different single substitution mutations (wild type and three different substitutions at each of three positions) were represented on the chip along with the wild type. Each of these mutations was represented by a series of twelve 12-mer oligonucleotide probes, which were complementary to the wild type target except at the one substituted base. Each of the twelve probes was complementary to a different region of the target and contained the mutated base at a different position, *e.g.*, if the substitution was at base 32, the set of probes would be complementary--with the exception of base 32--to regions of the target 21-32, 22-33, and 32-43). This enabled investigation of the effect of the substitution position within the probe. The alignment of some of the probes with a 12-mer model target nucleic acid is shown in Figure 27.

To demonstrate the effect of probe length, an additional series of ten 10-mer probes was included for each mutation (see Figure 28). In the vicinity of the substituted positions, the wild-type sequence was represented by every possible overlapping 12-mer and 10-mer probe. To simplify comparisons, the probes corresponding to each varied position were arranged on the chip in the rectangular regions with the following structure: each row of cells represents one substitution, with the top row representing the wild type. Each column contains probes complementary to the same region of the target, with probes complementary to the 3'-end of the target on the left and probes complementary to the 5'-end of the target on the right. The difference between two adjacent columns is a single base shift in the positioning of the probes. Whenever possible, the series of 10-mer probes were placed in four rows immediately underneath and aligned with the 4 rows of 12-mer probes for the same mutation.

To provide model targets, 5' fluoresceinated 12-mers containing all possible substitutions in the first position of codon 192 were synthesized (see the starred position in the target in Figure 27). Solutions containing 10 nM target DNA in 6X SSPE, 0.25% Triton X-100 were hybridized to the chip at room temperature for several hours. While target nucleic was hybridized to the chip, the fluorophores on the chip were excited by light from an argon laser, and the chip was scanned with an autofocusing confocal microscope. The emitted signals were processed by a PC to produce an image using image analysis software. By 1 to 3 hours, the signal had reached a plateau; to remove the hybridized target and allow hybridization to another target, the chip was stripped with 60% formamide, 2 X SSPE at 17 °C for 5 minutes. The washing buffer and temperature can vary, but the buffer typically contains 2-to-3X SSPE, 10-to-60% formamide (one can use multiple washes, increasing the formamide concentration by 10% each wash, and scanning between washes to determine when the wash is complete), and optionally a small percentage of Triton X-100, and the temperature is typically in the range of 15-to-18°C

Very distinct patterns were observed after hybridization with targets with 1 base substitutions and visualization with a confocal microscope and software analysis, as shown in Figure 29. In general, the probes which form perfect matches with the target retain the highest signal. For example, in the first image, the 12-mer probes that form perfect matches with the wild-type (WT) target are in the first row (top). The 12-mer probes with single base mismatches are located in the second, third, and fourth rows and have much lower signals. The data is also depicted graphically in Figure 30. On each graph, the X ordinate is the position of the probe in its row on the chip, and the Y ordinate is the signal at that probe site after hybridization. When a target with a different one base substitution is hybridized the complementary set of probes has the highest signal (see pictures 2, 3, and 4 in Figure 29 and graphs 2, 3, and 4 in Figure 30). In each case, the probe set with no mismatches with the target has the highest signals. Within a 12-mer probe set, the signal was highest at position 6 or 7. The graphs show that the signal difference between 12-mer probes at the same X ordinate tended to be greatest at positions 5 and 8 when the target and the complementary probes formed 10 base pairs and 11 base pairs, respectively. Because tumors often have both WT and mutant p53 genes, mixed target populations were also hybridized to the chip, as shown in Figure 31. When the hybridization solution consisted of a 1:1 mixture of WT 12-mer and a 12-mer with a substitution in position 7 of the target, the sets of probes that were perfectly matched to both targets showed higher signals than the other probe sets.

The hybridization efficiency of a 10-mer probe array as compared to a 12-mer probe array was also compared.

The 10-mer and 12-mer probe arrays gave comparable signals (see graphs 1-4 in Figure 30 and graphs 1-4 in Figure 32). However, the 10-mer probe sets, which are in rows 5-8 (see images in Figure 29), seemed to be better in this model system than the 12-mer probe sets at resolving one target from another, consistent with the expectation that one base mismatches are more destabilizing for 10-mers than 12-mers. Hybridization results within probe sets perfectly matched to target also followed the expectation that, the more matches the individual probe formed with the target, the higher the signal. However, duplexes with two 3' dangles (see Figure 30, position 6 in graphs 1-4) have about as much signal as the probes which are matched along their entire length (see Figure 30, position 7, in graphs 1-4).

This illustrative model system shows that 12-mer targets that differ by one base substitutions can be readily distinguished from one another by the novel probe array provided by the invention and that resolution of the different 12-mer targets was somewhat better with the 10-mer probe sets than with the 12-mer probe sets.

b. Exon V Chip

To analyze DNA from exon 5 of the p53 tumor suppressor gene, a set of overlapping 17-mer probes was synthesized on a chip. The probes for the WT allele were synthesized so as to tile across the entire exon with single base overlaps between probes. For each WT probe, a sets of 4 additional probes, one for each possible base substitution at position 7, were synthesized and placed in a column relative to the WT probe. Exon 5 DNA was amplified by PCR with primers flanking the exon. One of the primers was labeled with fluorescein; the other primer was labeled with biotin. After amplification, the biotinylated strand was removed by binding to streptavidin beads. The fluoresceinated strand was used in hybridization.

About 1/3 of the amplified, single-stranded nucleic acid was hybridized overnight in 5 X SSPE at 60°C to the probe chip (under a cover slip). After washing with 6 X SSPE, the chip was scanned using confocal microscopy. Figure 33 shows an image of the p53 chip hybridized to the target DNA. Analysis of the intensity data showed that 93.5% of the 184 bases of exon 5 were called in agreement with the WT sequence (see Buchman et al., 1988, Gene 70: 245-252. The miscalled bases were from positions where probe signal intensities were tied (1.6%) and where non-WT probes had the highest signal intensity (4.9%). Figure 34 illustrates how the actual sequence was read. Gaps in the sequence of letters in the WT rows correspond to control probes or sites. Positions at which bases are miscalled are represented by letters in italic type in cells corresponding to probes in which the WT bases have been substituted by other bases.

As the diagram indicates, the miscalled bases are from the low intensity areas of the image, which may be due to secondary structure in the target or probes preventing intermolecular hybridization. To diminish the effects due to secondary structure, one can employ shorter targets (i.e., by target fragmentation) or use more stringent hybridization conditions. In addition, the use of a set of probes synthesized by tiling across the other strand of a duplex target can also provide sequence information buried in secondary structure in the other strand. It should be appreciated, however, that the pattern of low intensity areas that forms as a result of secondary structure in the target itself provides a means to identify that a specific target sequence is present in a sample. Other factors that may contribute to lower signal intensities include differences in probe densities and hybridization stabilities.

These results demonstrate the advantages provided by the DNA chips of the invention to genetic analysis. As another example, heterozygous mutations are currently sequenced by an arduous process involving cloning and repurification of DNA. The cloning step is required, because the gel sequencing systems are poor at resolving even a 1:1 mixture of DNA. First, the target DNA is amplified by PCR with primers allowing easy ligation into a vector, which is taken up by transformation of E. coli, which in turn must be cultured, typically on plates overnight. After growth of the bacteria, DNA is purified in a procedure that typically takes about 2 hours; then, the sequencing reactions are performed, which takes at least another hour, and the samples are run on the gel for several hours, the duration depending on the length of the fragment to be sequenced. By contrast, the present invention provides direct analysis of the PCR amplified material after brief transcription and fragmentation steps, saving days of time and labor.

D. Mitochondrial Genome Chips

A human cell may have several hundred mitochondria, each with more than one copy of mtDNA. There is strand asymmetry in the base compositions, with one strand (Heavy) being relatively G rich, and the other strand (Light) being C rich. The L strand is 30.9% A, 31.2% C, 13.1% G, and 24.7% T. Human mtDNA is information-rich, encoding some 22 tRNAs, 12S and 16S rRNAs, and 13 polypeptides involved in oxidative phosphorylation. No introns have been detected. RNAs are processed by cleavage at tRNA sequences, and polyadenylated postranscriptionally. In some transcripts, polyadenylation also creates the stop codon, illustrating the parsimony of coding. In many individuals, mtDNA can be treated as haploid. However, some individuals are heteroplasmic (have more than one mtDNA sequence), and the degree of heteroplasmy can vary from tissue to tissue. Also, the rate of replication of mtDNAs can differ and together with random segregation during cell division, can lead to changes in heteroplasmy over time.

The human mitochondrial genome is 16,569 nucleotides long. The sequence of the L-strand is numbered arbitrarily from the MboI-5/7 boundary in the D-loop region. The complete sequence of the human mitochondrial genome has been published. *See* Anderson et al., *Nature* 290, 457-465 (1981). Mitochondrial DNA is maternally inherited, and has a mutation rate estimated to be tenfold higher than single copy nuclear DNA (Brown et al., *Proc. Natl. Acad. Sci. USA 76,* 1967-1971 (1979)). Human mtDNAs differ, on average, by about 70 base substitutions (Wallace, Ann. *Rev. Biochem.* 61, 1175-1212 (1992)). over 80% of substitutions are transitions (*i.e.*, pyrimidine-pyrimidine or purine-purine).

Analysis of mitochondrial DNA serves several purposes. Detection of mutations in the mitochondrial genome allows diagnosis of a number of diseases. The mitochondrial genome has been identified as the locus of several mutations associated with human diseases. Some of the mutations result in stop codons in structural genes. Such mutations have been mapped and associated with diseases, such as Leber's hereditary optic neuropathy, neurogenic muscular weakness, ataxia and retinitis pigmentosa. Other mutations (nucleotide substitutions) occur in tRNA coding sequences, and presumably cause conformational defects in transcribed tRNA molecules. Such mutations have also been mapped and associated with diseases such as Myoclonic Epilepsy and Ragged Red Fiber Disease. Another type of mutation commonly found is deletions and/or insertions. Some deletions span segments of several kb. Again, such mutations have been mapped and associated with diseases, for example, ocular myopathy and Person Syndrome. *See* Wallace, *Ann. Rev. Biochem*. 61-1175-1212 (1992). Early detection of such diseases allows metabolic or genetic therapy to be administered before irretrievable damage has occurred. *Id*. Analysis of mitochondrial DNA is also important for forensic screening. Because the mitochondrial genome is a locus of high variability between individuals, sequencing a substantial length of mitochondrial DNA provides a fingerprint that is highly specific to an individual. Analysis of mitochondrial DNA is also important for evolutionary and epidemiological studies.

The reference sequence can be an entire mitochondrial genome or any fragment thereof. For forensic and epidemiological studies, the reference sequence is often all or part of the D-loop region in which variability between individuals is greatest (*e.g.*, from 16024-16401 and 29-408). For detection of mutations, analysis of the entire genome is useful as a reference sequence, but shorter segments including the sites of known mutations, and about 1-20 flanking bases are also useful. Some chips have probes tiling paired reference sequences, representing wildtype and mutant versions of a sequence. Tiling a second reference sequence is particularly useful for detecting an insertion mutation occurring in 30-50% of ocular myopathy and Pearson syndrome patients, which consists of direct repeats of the sequence ACCTCCCTCACCA. Some chips include reference sequences from more than one mitochondrial genome.

Mitochondrial reference sequences can be tiled using any of the strategies noted above. The block tiling strategy is particularly useful for analyzing short reference sequences or known mutations. Either the block strategy or the basic strategy is suitable for analyzing long reference sequences. In many of the tiling strategies, it is possible to use fewer probes compared with the number used in other chips without significant loss of sequence information. As noted above, most point mutations in mitochondrial DNA are transitions, so for each wildtype nucleotide in a reference sequence, one of the three possible nucleotide substitutions is much more likely than the other two. Accordingly, in the basic tiling strategy, for example, a reference sequence can be tiled using only two probe sets. One probe sets comprises a plurality of probes, each probe having a segment exactly complementary to the reference sequence. The second probe set comprises a corresponding probe for each probe in the first set. However, a probe from the second probe set differs from the corresponding probe from the first probe set in an interrogation position, in which the probe from the second probe set includes the transition of the nucleotide present in that position in the probe from the first probe set.

Target mitochondrial DNA can be amplified, labelled and fragmented prior to hybridization using the same procedures as described for other chips. Use of at least two labelled nucleotides is desirable to achieve uniform labelling. Some exemplary primers are described below and other primers can be designed from the known sequence of mitochondrial DNA. Because mitochondrial DNA is present in multiple copies per cell, it can also be hybridized directly to a chip without prior amplification.

<u>Exemplary Chips</u>

The invention provides a DNA chip for analyzing sequences contained in a 1.3 kb fragment of human mitochondrial DNA from the "D-loop" region, the most polymorphic region of human mitochondrial DNA. one such chip comprises a set of 269 overlapping oligonucleotide probes of varying length in the range of 9-14 nucleotides with varying overlaps arranged in ~600 x 600 micron features or synthesis sites in an array 1 cm x 1 cm in size. The probes on the chip are shown in columnar form below. An illustrative mitochondrial DNA chip of the invention comprises the following probes (X, Y coordinates are shown, followed by the sequence; "DL3" represents the 3'-end of the probe, which is covalently attached to the chip surface.)

| | | |
|---|---|---|
| 0 | 0 | DL3AGTGGGGTATTT |
| 1 | 0 | DL3GGGTATTTAGTT |
| 2 | 0 | DL3TTAGTTTATCCAA |
| 3 | 0 | DL3ATCCAAACCAGG |
| 4 | 0 | DL3ACCAGGATCGGA |
| 5 | 0 | DL3CGTGTGTGTGTGG |
| 6 | 0 | DL3CGTGTGTGTGTGGC |
| 7 | 0 | DL3TCGTGTGTGTGTGG |
| 8 | 0 | DL3GTAGGATGGGTC |
| 9 | 0 | DL3AGGATGGGTCGT |
| 10 | 0 | DL3GATGGGTCGTGT |
| 11 | 0 | DL3TGGCGACGATTG |
| 12 | 0 | DL3GCGACGATTGGG |
| 13 | 0 | DL3TGGGGGGGA |
| 14 | 0 | DL3GAGGGGGCG |
| 15 | 0 | DL3GGAGGGGGCGA |
| 16 | 0 | DL3GAGGGGGCGA |
| 0 | 1 | DL3GGCTTGGTTGG |
| 1 | 1 | DL3GGTTGGTTTGGG |
| 2 | 1 | DL3TGGGGTTTCTAG |
| 3 | 1 | DL3GTTTCTAGTGGG |
| 4 | 1 | DL3AGTGGGGGGTGT |
| 5 | 1 | DL3GGGGTGTCAAAT |
| 6 | 1 | DL3GTCAAATACATCG |
| 7 | 1 | DL3ACATCGAATGGAG |
| 8 | 1 | DL3CGAATGGAGGAG |
| 9 | 1 | DL3GAGGAGTTTCGT |
| 10 | 1 | DL3TTTCGTTATGTGA |
| 11 | 1 | DL3ATGTGACTTTTAC |
| 12 | 1 | DL3GACTTTTACAAAT |
| 13 | 1 | DL3AAATCTGCCCGA |
| 14 | 1 | DL3AATCTGCCCGAG |
| 15 | 1 | DL3CCCGAGTGTAGT |
| 16 | 1 | DL3AGTGTAGTGGGG |
| 0 | 2 | DL3GGGAGGGTGAG |
| 1 | 2 | DL3GGTGAGGGTATG |

| | | | | | |
|---|---|---|---|---|---|
| 2 | 2 | DL3GGTATGATGATTAG | 8 | 5 | DL3ATTGTTAAACTTA |
| 3 | 2 | DL3GATTAGAGTAAGT | 9 | 5 | DL3AAACTTACAGACG |
| 4 | 2 | DL3TTAGAGTAAGTTA | 10 | 5 | DL3ACAGACGTGTCG |
| 5 | 2 | DL3AAGTTATGTTGGG | 11 | 5 | DL3GTGTCGGTGAAA |
| 6 | 2 | DL3GTTGGGGGCG | 12 | 5 | DL3GTGAAAGGTGTGT |
| 7 | 2 | DL3GGGGCGGGTA | 13 | 5 | DL3GGTGTGTCTGTAG |
| 8 | 2 | DL3GCGGGTAGGAT | 14 | 5 | DL3TGTGTCTGTAGTA |
| 9 | 2 | DL3GGTAGGATGGGT | 15 | 5 | DL3GTAGTATTGTTTT |
| 10 | 2 | DL3GGATGGGTCGTG | 16 | 5 | DL3AGTATTGTTTTTT |
| 11 | 2 | DL3GGTCGTGTGTGT | 0 | 6 | DL3CCTCGTGGGATA |
| 12 | 2 | DL3GTGTGTGTGGCG | 1 | 6 | DL3TGGGATACAGCG |
| 13 | 2 | DL3TGTGGCGACGAT | 2 | 6 | DL3GATACAGCGTCAT |
| 14 | 2 | DL3GACGATTGGGGT | 3 | 6 | DL3GCGTCATAGACAG |
| 15 | 2 | DL3ATTGGGGTATGG | 4 | 6 | DL3AGACAGAAACTAA |
| 16 | 2 | DL3GTATGGGGCTTG | 5 | 6 | DL3CAGAAACTAAGGA |
| 0 | 3 | DL3GGATTGTGGTCG | 6 | 6 | DL3TAAGGACGGAGT |
| 1 | 3 | DL3TGGTCGGATTGG | 7 | 6 | DL3GACGGAGTAGGA |
| 2 | 3 | DL3GGATTGGTCTAAA | 8 | 6 | DL3GTAGGATAATAAA |
| 3 | 3 | DL3TCTAAAGTTTAAA | 9 | 6 | DL3TAATAAATAGCG |
| 4 | 3 | DL3GTTTAAAATAGAA | 10 | 6 | DL3ATAGCGTAGGAT |
| 5 | 3 | DL3ATAGAAAAACCG | 11 | 6 | DL3TAGCGTAGGATG |
| 6 | 3 | DL3AGAAAAACCGC | 12 | 6 | DL3AGGATGCAAGTT |
| 7 | 3 | DL3AACCGCCATAC | 13 | 6 | DL3ATGCAAGTTATAA |
| 8 | 3 | DL3CCATACGTGAAAA | 14 | 6 | DL3GTTATAATGTCCG |
| 9 | 3 | DL3ACGTGAAAATTGT | 15 | 6 | DL3ATGTCCGCTTGT |
| 10 | 3 | DL3AATTGTCAGTGGG | 16 | 6 | DL3TCCGCTTGTATG |
| 11 | 3 | DL3TGTCAGTGGGGG | 0 | 7 | DL3GTGAGTGCCCTC |
| 12 | 3 | DL3TGGGGGGTTGA | 1 | 7 | DL3TGCCCTCGAGAG |
| 13 | 3 | DL3GGGTTGATTGTGT | 2 | 7 | DL3CCTCGAGAGGTA |
| 14 | 3 | DL3TTGTGTAATAAAA | 3 | 7 | DL3AGAGGTACGTAA |
| 15 | 3 | DL3AATAAAAGGGGA | 4 | 7 | DL3ACGTAAACCATA |
| 16 | 3 | DL3TAAAAGGGGAGG | 5 | 7 | DL3ACCATAAAAGCAG |
| 0 | 4 | DL3GTTTTTTAAAGG | 6 | 7 | DL3AAAGCAGACCC |
| 1 | 4 | DL3TTTTAAAGGTGG | 7 | 7 | DL3AGACCCCCCAT |
| 2 | 4 | DL3AGGTGGTTTGG | 8 | 7 | DL3CCCCCATACGT |
| 3 | 4 | DL3TTGGGGGGGAG | 9 | 7 | DL3CATACGTGCGCT |
| 4 | 4 | DL3GGAGGGGGCG | 10 | 7 | DL3GTGCGCTATCAG |
| 5 | 4 | DL3GGGGCGAAGAC | 11 | 7 | DL3GCGCTATCAGTA |
| 6 | 4 | DL3GAAGACCGGATG | 12 | 7 | DL3TCAGTAACGCTC |
| 7 | 4 | DL3CCGGATGTCGTG | 13 | 7 | DL3GTAACGCTCTGC |
| 8 | 4 | DL3GTCGTGAATTTGT | 14 | 7 | DL3CTCTGCGACCTC |
| 9 | 4 | DL3CGTGAATTTGTGT | 15 | 7 | DL3GACCTCGGCCT |
| 10 | 4 | DL3TTGTGTAGAGACG | 16 | 7 | DL3TCGGCCTCGTG |
| 11 | 4 | DL3TAGAGACGGTTT | 0 | 8 | DL3GATGAAGTCCCAG |
| 12 | 4 | DL3ACGGTTTGGGG | 1 | 8 | DL3AGTCCCAGTATTT |
| 13 | 4 | DL3TGGGGTTTTTGT | 2 | 8 | DL3GTATTTCGGATTT |
| 14 | 4 | DL3GGGTTTTTGTTT | 3 | 8 | DL3TCGGATTTATCG |
| 15 | 4 | DL3TTGTTTCTTGGG | 4 | 8 | DL3GATTTATCGGGT |
| 16 | 4 | DL3TCTTGGGATTGTG | 5 | 8 | DL3ATCGGGTGTGCA |
| 0 | 5 | DL3TGTATGAATGATTT | 6 | 8 | DL3TGTGCAAGGGGA |
| 1 | 5 | DL3TGATTTCACACAA | 7 | 8 | DL3CAAGGGGAATTT |
| 2 | 5 | DL3ACACAATTAATTAA | 8 | 8 | DL3GAATTTATTCTGTA |
| 3 | 5 | DL3AATTAATTACGAA | 9 | 8 | DL3TCTGTAGTGCTAC |
| 4 | 5 | DL3TACGAACATCCTG | 10 | 8 | DL3GTAGTGCTACCT |
| 5 | 5 | DL3ACGAACATCCTGT | 11 | 8 | DL3GCTACCTAGTAG |
| 6 | 5 | DL3TCCTGTATTATTA | 12 | 8 | DL3CTAGTAGTCCAGA |
| 7 | 5 | DL3GTATTATTATTGTT | 13 | 8 | DL3TCCAGATAGTGGG |

| | | |
|---|---|---|
| 14 | 8 | DL3AGATAGTGGGATA |
| 15 | 8 | DL3GGGATAATTGGT |
| 16 | 8 | DL3TAATTGGTGAGTG |
| 0 | 9 | DL3TATAGGGCGTGT |
| 1 | 9 | DL3GGCGTGTTCTCA |
| 2 | 9 | DL3GTGTTCTCACGAT |
| 3 | 9 | DL3TCACGATGAGAGG |
| 4 | 9 | DL3ATGAGAGGAGCG |
| 5 | 9 | DL3AGGAGCGAGGC |
| 6 | 9 | DL3CGAGGCCCGG |
| 7 | 9 | DL3GCCCGGGTATT |
| 8 | 9 | DL3CGGGTATTGTGA |
| 9 | 9 | DL3GTGAACCCCCAT |
| 10 | 9 | DL3CCCCATCGATTT |
| 11 | 9 | DL3ATCGATTTCACTT |
| 12 | 9 | DL3TTTCACTTGACAT |
| 13 | 9 | DL3TTGACATAGAGCT |
| 14 | 9 | DL3TAGAGCTGTAGAC |
| 15 | 9 | DL3GTAGACCAAGGA |
| 16 | 9 | DL3ACCAAGGATGAAG |
| 0 | 10 | DL3CGTGTAATGTCAG |
| 1 | 10 | DL3TGTCAGTTTAGGG |
| 2 | 10 | DL3TCAGTTTAGGGA |
| 3 | 10 | DL3TAGGGAAGAGCA |
| 4 | 10 | DL3AAGAGCAGGGGT |
| 5 | 10 | DL3CAGGGGTACCTA |
| 6 | 10 | DL3GGTACCTACTGG |
| 7 | 10 | DL3TACTGGGGGGA |
| 8 | 10 | DL3GGGGGAGTCTAT |
| 9 | 10 | DL3AGTCTATCCCCA |
| 10 | 10 | DL3ATCCCCAGGGA |
| 11 | 10 | DL3CAGGGAACTGGT |
| 12 | 10 | DL3ACTGGTGGTAGG |
| 13 | 10 | DL3CTGGTGGTAGGA |
| 14 | 10 | DL3GTAGGAGGCACA |
| 15 | 10 | DL3GGCACATTTAGT |
| 16 | 10 | DL3TTTAGTTATAGGG |
| 0 | 11 | DL3AGGTTTACGGTG |
| 1 | 11 | DL3TACGGTGGGGA |
| 2 | 11 | DL3GTGGGGAGTGG |
| 3 | 11 | DL3GGGAGTGGGTGA |
| 4 | 11 | DL3GGGTGATCCTATG |
| 5 | 11 | DL3CCTATGGTTGTTT |
| 6 | 11 | DL3GGTTGTTTGGATG |
| 7 | 11 | DL3GTTTGGATGGGT |
| 8 | 11 | DL3ATGGGTGGGAAT |
| 9 | 11 | DL3GGGAATTGTCATG |
| 10 | 11 | DL3GTCATGTATCATGT |
| 11 | 11 | DL3TCATGTATTTCGG |
| 12 | 11 | DL3TATTTCGGTAAA |
| 13 | 11 | DL3TTCGGTAAATGG |
| 14 | 11 | DL3GTAAATGGCATGT |
| 15 | 11 | DL3GCATGTAATCGTG |
| 16 | 11 | DL3GTAATCGTGTAAT |
| 5 | 12 | DL3GGGAGGGGTAC |
| 6 | 12 | DL3GGGTACGAATGT |
| 7 | 12 | DL3ACGAATGTTCGTT |
| 8 | 12 | DL3TGTTCGTTCATGT |
| 9 | 12 | DL3CGTTCATGTCGTT |
| 10 | 12 | DL3GTCGTTAGTTGG |
| 11 | 12 | DL3TAGTTGGGAGTT |
| 12 | 12 | DL3GGAGTTGATAGTG |
| 13 | 12 | DL3ATAGTGTGTAGTT |
| 14 | 12 | DL3GTGTAGTTGACGT |
| 15 | 12 | DL3TGACGTTGAGGT |
| 16 | 12 | DL3CGTTGAGGTTTA |
| 5 | 13 | DL3TATAACATGCCAT |
| 6 | 13 | DL3AACATGCCATGGT |
| 7 | 13 | DL3CCATGGTATTTAT |
| 8 | 13 | DL3ATTTATGAACTGG |
| 9 | 13 | DL3AACTGGTGGACAT |
| 10 | 13 | DL3TGGACATCATGTA |
| 11 | 13 | DL3CATGTATTTTTGG |
| 12 | 13 | DL3TTTTGGGTTAGG |
| 13 | 13 | DL3GGGTTAGGATGT |
| 14 | 13 | DL3GGATGTAGTTTTG |
| 15 | 13 | DL3TGTAGTTTTGGG |
| 16 | 13 | DL3TTTGGGGGAGG |
| 5 | 14 | DL3GGGTTCATAACTG |
| 6 | 14 | DL3ATAACTGAGTGGG |
| 7 | 14 | DL3AACTGAGTGGGT |
| 8 | 14 | DL3GTGGGTAGTTGT |
| 9 | 14 | DL3GTAGTTGTTGGC |
| 10 | 14 | DL3GTTGGCGATACA |
| 11 | 14 | DL3CGATACATAAAAG |
| 12 | 14 | DL3TAAAAGCATGTAA |
| 13 | 14 | DL3GCATGTAATGACG |
| 14 | 14 | DL3ATGACGGTCGGT |
| 15 | 14 | DL3GTCGGTGGTACT |
| 16 | 14 | DL3GGTACTTATAACA |
| 5 | 15 | DL3TCGATTCTAAGAT |
| 6 | 15 | DL3TAAGATTAAATTT |
| 7 | 15 | DL3AAATTTGAATAAG |
| 8 | 15 | DL3AATAAGAGACAAG |
| 9 | 15 | DL3AAGAGACAAGAAA |
| 10 | 15 | DL3AAGAAAGTACCC |
| 11 | 15 | DL3AAAGTACCCCTT |
| 12 | 15 | DL3CCCCTTCGTCTA |
| 13 | 15 | DL3CTTCGTCTAAAC |
| 14 | 15 | DL3CTAAACCCATGG |
| 15 | 15 | DL3AACCCATGGTGG |
| 16 | 15 | DL3TGGTGGGTTCAT |
| 5 | 16 | DL3TTGGAAAAAGGT |
| 6 | 16 | DL3AAAAGGTTCCTG |
| 7 | 16 | DL3GGTTCCTGTTTA |
| 8 | 16 | DL3CCTGTTTAGTCTC |
| 9 | 16 | DL3TTAGTCTCTTTTT |
| 10 | 16 | DL3CTTTTTCAGAAAT |
| 11 | 16 | DL3AGAAATTGAGGTG |
| 12 | 16 | DL3AAATTGAGGTGGT |
| 13 | 16 | DL3GGTGGTAATCGT |
| 14 | 16 | DL3TAATCGTGGGTT |
| 15 | 16 | DL3GTGGGTTTCGAT |
| 16 | 16 | DL3GGTTTCGATTCT |

No probes were present in positions X, Y = 0, 12 to X, Y = 4, 12; X, Y = 0, 13 to X, Y = 4, 13; X, Y = 0, 14 to X, Y = 4, 14; X, Y = 0, 15 to X, Y = 4, 15; X, Y = 0, 16 to X, Y = 4, 16;

The length of each of the probes on the chip was variable to minimize differences in melting temperature and potential for cross-hybridization. Each position in the sequence was represented by at least one probe and most positions were represented by 2 or more probes. As noted above, the amount of overlap between the oligonucleotides varied from probe to probe. Figure 35 shows the human mitochondrial genome; "$O_H$" is the H strand origin of replication, and arrows indicate the cloned unshaded sequence.

DNA was prepared from hair roots of six human donors (mtl to mt6) and then amplified by PCR and cloned into M13; the resulting clones were sequenced using chain terminators to verify that the desired specific sequences were present. DNA from the sequenced M13 clones was amplified by PCR, transcribed in vitro, and labeled with fluorescein-UTP using T3 RNA polymerase. The 1.3 kb RNA transcripts were fragmented and hybridized to the chip. The results showed that each different individual had DNA that produced a unique hybridization fingerprint on the chip and that the differences in the observed patterns could be correlated with differences in the cloned genomic DNA sequence. The results also demonstrated that very long sequences of a target nucleic acid can be represented comprehensively as a specific set of overlapping oligonucleotides and that arrays of such probe sets can be usefully applied to genetic analysis.

The sample nucleic acid was hybridized to the chip in a solution composed of 6 X SSPE, 0.1% Triton-X 100 for 60 minutes at 15°C. The chip was then scanned by confocal scanning fluorescence microscopy. The individual features on the chip were 588 x 588 microns, but the lower left 5 x 5 square features in the array did not contain probes. To quantitate the data, pixel counts were measured within each synthesis site. Pixels represent 50 x 50 microns. The fluorescence intensity for each feature was scaled to a mean determined from 27 bright features. After scanning, the chip was stripped and rehybridized; all six samples were hybridized to the same chip. Figure 36 shows the image observed from the mt4 sample on the DNA chip. Figure 37 shows the image observed from the mt5 sample on the DNA chip. Figure 38 shows the predicted difference image between the mt4 and mt5 samples on the DNA chip based on mismatches between the two samples and the reference sequence (*see* Anderson et al., *supra).* Figure 39 shows the actual difference image observed.

The results show that, in almost all cases, mismatched probe/target hybrids resulted in lower fluorescence intensity than perfectly matched hybrids. Nonetheless, some probes detected mutations (or specific sequences) better than others, and in several cases, the differences were within noise levels. Improvements can be realized by increasing the amount of overlap between probes and hence overall probe density and, for duplex DNA targets, using a second set of probes, either on the same or a separate chip, corresponding to the second strand of the target. Figure 40, in sheets 1 and 2, shows a plot of normalized intensities across rows 10 and 11 of the array and a tabulation of the mutations detected.

Figure 41 shows the discrimination between wild-type and mutant hybrids obtained with this chip. The median of the six normalized hybridization scores for each probe was taken. The graph plots the ratio of the median score to the normalized hybridization score versus mean counts. On this graph, a ratio of 1.6 and mean counts above 50 yield no false positives, and while it is clear that detection of some mutants can be improved, excellent discrimination is achieved, considering the small size of the array. Figure 42 illustrates how the identity of the base mismatch may influence the ability to discriminate mutant and wild-type sequences more than the position of the mismatch within an oligonucleotide probe. The mismatch position is expressed as % of probe length from the 3'-end. The base change is indicated on the graph. These results show that the DNA chip increases the capacity of the standard reverse dot blot format by orders of magnitude, extending the power of that approach many fold and that the methods of the invention are more efficient and easier to automate than gel-based methods of nucleic acid sequence and mutation analysis.

To illustrate further these advantages, a second chip was prepared for analyzing a longer segment from human mitochondrial DNA (mtDNA). The chip "tiles" through 648 nucleotides of a reference sequence comprising human H strand mtDNA from positions 16280 to 356, and allows analysis of each nucleotide in the reference sequence. The probes in the array are 15 nucleotides in length, and each position in the target sequence is represented by a set of 4 probes (A, C, G, T substitutions), which differed from one another at position 7 from the 3'-end. The array consists of 13 blocks of 4 x 50 probes: each block scans through 50 nucleotides of contiguous mtDNA sequence. The blocks are separated by blank rows. The 4 corner columns contain control probes; there are a total of 2600 probes in a 1.28 cm x 1.28 cm square area (feature), and each area is 256 x 197 microns.

Target RNA was prepared as above. The RNA was fragmented and hybridized to the oligonucleotide array in a solution composed of 6X SSPE, 0.1% Triton X-100 for 60 minutes at 18°C. Unhybridized material was washed away with buffer, and the chip was scanned at 25 micron pixel resolution.

Figure 43 provides a 5' to 3' sequence listing of one target corresponding to the probes on the chip. X is a control probe. Positions that differ in the target (i.e., are mismatched with the probe at the designated site) are in bold. Figure 44 shows the fluorescence image produced by scanning the chip when hybridized to this sample. About 95% of the sequence could be read correctly from only one strand of the original duplex target nucleic acid. Although some

probes did not provide excellent discrimination and some probes did not appear to hybridize to the target efficiently, excellent results were achieved. The target sequence differed from the probe set at six positions: 4 transitions and 2 insertions. All 4 transitions were detected, and specific probes could readily be incorporated into the array to detect insertions or deletions. Figure 45 illustrates the detection of 4 transitions in the target sequence relative to the wild-type probes on the chip.

A further chip was constructed comprising probes tiling across the entire D-loop region (1.3 kb) of mt DNA sequences from two humans. The probes were tiled in rows of four using the basic tiling strategy. The probes were overlapping 15 mers having an interrogation position 7 nucleotides from the 3' end. The complete group of probes tiled on the reference sequence from the first individual, designated mtl, occupied the upper half of the chip. The lower half of the chip contained a similar arrangement based on a second clone, mt2. The probes were synthesized in a 1.28 x 1.28 cm area, which contained a matrix of 115 x 120 cells. The chip contained a total of 10,488 mtDNA probes.

Six samples of target DNA was extracted form hair roots from six individuals. The 1.3 kb region spanning positions 15935 to 667 of human mtDNA was PCR amplified, cloned in bacteriophage M13 and sequenced by conventional methods. The 1.3 kb region was reamplified from the phage clone using primers, L15935-T3, 5'CTCGGAATTAACCCTCACTAAAGGAAACCTTTTTCCAAGGA and H667-T7, 5'TAATACGACTCACTATAGGGAGAGGCTAGGACCAAACCTATT tagged with T3 and T7 RNA polymerase promoter sequences. Labelled RNA was generated by *in vitro* transcription using T3 RNA polymerase and fluoresceinated nucleotides, fragmented, and hybridized to the mtDNA control region resequencing chip at room temperature for 60 min, in 6xSSPE + 0.05% triton X-100. Six washes were carried out at room temperature,using 6xSSPE + 0.005% triton X-100, and the chip was read. Signal intensities varied considerably over the chip, but the large dynamic range of the detection system allowed accurate quantitation of intensities over several orders of magnitude. Even relatively low signal intensities yielded accurate results.

Five different clones (mt1-5) were hybridized, each to a separate chip. The reference sequence was also hybridized for comparative purposes. Mean counts per probe cell were determined, and used by automated basecalling software to read the sequence. The accuracy of sequence read from the chip is summarized as follows. Combining the data from the five targets analyzed, the chip read a total of 6310 nucleotides. Of these nucleotides in the target sequences, 55 were different from the reference sequence (as judged by conventional sequencing) (41 of these 55 nucleotides were both detected and read correctly from the chip). 6 of 55 nucleotides were detected as being ambiguous but their identity could not be read. 2 of 55 nucleotides were detected as mutations, but their identity was miscalled. 6 of 55 nucleotides were incorrectly called as wildtype. Of the 6255 nucleotides in the target sequence that were identical to the reference sequence, only 36 (0.57%) were miscalled or scored as ambiguous.

A further chip was constructed comprising probes tiling across a reference sequence comprising an entire mitochondrial genome. In this chip, a block tiling, strategy was used. Each block was designed to analyze seven nucleotides from a target sequence. Each block consisted of four probe sets, the probe sets each having seven probes. A block was laid down on the chip in seven columns of four probes. The upper probe was the same in each column, this being a probe exactly complementary to a subsequence of the reference sequence. The three other probes in each column were identical to the upper probe except in an interrogation position, which was occupied by a different base in each of the four probes in the column. The interrogation position shifted by one position between successive columns. Thus, except for the seven interrogation positions, one in each of the columns of probes, all probes occupying a block were identical. The array comprised many such blocks, each tiled to successive subsequences of the mitochondrial DNA reference sequence. In all, the chip tiled 15,569 nucleotides of reference sequence with double tiling at 42 positions. 66,276 probes occupied an array of 304 x 315 cells, each cell having an area of 42 x 41 microns.

The chip was hybridized to the same target sequences as described for the D-loop region, except that hybridization was at 15°C for 2 hr. The chip was scanned at 5 micron resolution to give an image with approximately 64 pixels per cell. For blocks of probes tiling across the D-loop region, a sequence-specific hybridization pattern was obtained. For other blocks, only background hybridization was observed.

These results illustrate that longer sequences can be read using the DNA chips and methods of the invention, as compared to conventional sequencing methods, where reading length is limited by the resolution of gel electrophoresis. Hybridization and signal detection require less than an hour and can be readily shortened by appropriate choice of buffers, temperatures, probes, and reagents.

III. MODES OF PRACTICING THE INVENTTON

A. VLSIPS™ Technology

As noted above, the VLSIPS™ technology is described in a number of patent publications and is preferred for making the oligonucleotide arrays of the invention. A brief description of how this technology can be used to make and screen DNA chips is provided in this Example and the accompanying Figures. In the VLSIPS™ method, light is

shone through a mask to activate functional (for oligonucleotides, typically an -OH) groups protected with a photoremovable protecting group on a surface of a solid support. After light activation, a nucleoside building block, itself protected with a photoremovable protecting group (at the 5'-OH), is coupled to the activated areas of the support. The process can be repeated, using different masks or mask orientations and building blocks, to prepare very dense arrays of many different oligonucleotide probes. The process is illustrated in Figure 46; Figure 47 illustrates how the process can be used to prepare "nucleoside combinatorials" or oligonucleotides synthesized by coupling all four nucleosides to form dimers, trimers and so forth.

New methods for the combinatorial chemical synthesis of peptide, polycarbamate, and oligonucleotide arrays have recently been reported *(see* Fodor et al., 1991, *Science* 251: 767-773; Cho et al., 1993, *Science* 261: 1303-1305; and Southern et al., 1992, *Genomics* 13: 1008-10017. These arrays, or biological chips *(see* Fodor et al., 1993, *Nature* 364: 555-556, harbor specific chemical compounds at precise locations in a high-density, information rich format, and are a powerful tool for the study of biological recognition processes. A particularly exciting application of the array technology is in the field of DNA sequence analysis. The hybridization pattern of a DNA target to an array of shorter oligonucleotide probes is used to gain primary structure information of the DNA target. This format has important applications in sequencing by hybridization, DNA diagnostics and in elucidating the thermodynamic parameters affecting nucleic acid recognition.

Conventional DNA sequencing technology is a laborious procedure requiring electrophoretic size separation of labeled DNA fragments. An alternative approach, termed Sequencing By Hybridization (SBH), has been proposed (Lysov et al., 1988, *Dokl. Akad. Nauk SSSR* 303:1508-1511; Bains et al., 1988, J. *Theor. Biol.* 135:303-307; and Drmanac et al., 1989, *Genomics* 4:114-128. This method uses a set of short oligonucleotide probes of defined sequence to search for complementary sequences on a longer target strand of DNA. The hybridization pattern is used to reconstruct the target DNA sequence. It is envisioned that hybridization analysis of large numbers of probes can be used to sequence long stretches of DNA. In immediate applications of this hybridization methodology, a small number of probes can be used to interrogate local DNA sequence.

The strategy of SBH can be illustrated by the following example. A 12-mer target DNA sequence, AGCCTAGCTGAA, is mixed with a complete set of octanucleotide probes. If only perfect complementarity is considered, five of the 65,536 octamer probes -TCGGATCG, CGGATCGA, GGATCGAC, GATCGACT, and ATCGACTT will hybridize to the target. Alignment of the overlapping sequences from the hybridizing probes reconstructs the complement of the original 12-mer target:

```
TCGGATCG
 CGGATCGA
  GGATCGAC
   GATCGACT



    ATCGACTT
TCGGATCGACTT
```

Hybridization methodology can be carried out by attaching target DNA to a surface. The target is interrogated with a set of oligonucleotide probes, one at a time *(see* Strezoska et al., 1991, *Proc. Natl. Acad. Sci. USA* 88:10089-10093, and Drmanac et al., 1993, *Science* 260:1649-1652, each of which is incorporated herein by reference). This approach can be implemented with well established methods of immobilization and hybridization detection, but involves a large number of manipulations. For example, to probe a sequence utilizing a full set of octanucleotides, tens of thousands of hybridization reactions must be performed. Alternatively, SBH can be carried out by attaching probes to a surface in an array format where the identity of the probes at each site is known. The target DNA is then added to the array of probes. The hybridization pattern determined in a single experiment directly reveals the identity of all complementary probes.

As noted above, a preferred method of oligonucleotide probe array synthesis involves the use of light to direct the synthesis of oligonucleotide probes in high-density, miniaturized arrays. Photolabile 5'-protected N-acyl-deoxynucleoside phosphoramidites, surface linker chemistry, and versatile combinatorial synthesis strategies have been developed for this technology. Matrices of spatially-defined oligonucleotide probes have been generated, and the ability to use these arrays to identify complementary sequences has been demonstrated by hybridizing fluorescent labeled oligonucleotides to the DNA chips produced by the methods. The hybridization pattern demonstrates a high degree of base specificity and reveals the sequence of oligonucleotide targets.

The basic strategy for light-directed oligonucleotide synthesis (1) is outlined in Fig. 46. The surface of a solid support modified with photolabile protecting groups (X) is illuminated through a photolithographic mask, yielding reactive hydroxyl groups in the illuminated regions. A 3'-O-phosphoramidite activated deoxynucleoside (protected at the 5'-hydroxyl with a photolabile group) is then presented to the surface and coupling occurs at sites that were exposed to light. Following capping, and oxidation, the substrate is rinsed and the surface illuminated through a second mask, to expose additional hydroxyl groups for coupling. A second 5'-protected, 3'-O-phosphoramidite activated deoxynucleoside is presented to the surface. The selective photodeprotection and coupling cycles are repeated until the desired set of products is obtained.

Light directed chemical synthesis lends itself to highly efficient synthesis strategies which will generate a maximum number of compounds in a minimum number of chemical steps. For example, the complete set of $4^n$ polynucleotides (length n), or any subset of this set can be produced in only 4 x n chemical steps. *See* Fig. 47. The patterns of illumination and the order of chemical reactants ultimately define the products and their locations. Because photolithography is used, the process can be miniaturized to generate high-density arrays of oligonucleotide probes. For an example of the nomenclature useful for describing such arrays, an array containing all possible octanucleotides of dA and dT is written as $(A+T)^8$. Expansion of this polynomial reveals the identity of all 256 octanucleotide probes from AAAAAAAA to TTTTTTTT. A DNA array composed of complete sets of dinucleotides is referred to as having a complexity of 2. The array given by (A+T+C+G)8 is the full 65,536 octanucleotide array of complexity four. Computer-aided methods of laying down predesigned arrays of probes using VLSIPS™ technology are described in commonly-assigned co-pending application USSN 08/249,188, filed May 24, 1994 (incorporated by reference in its entirety for all purposes).

To carry out hybridization of DNA targets to the probe arrays, the arrays are mounted in a thermostatically controlled hybridization chamber. Fluorescein labeled DNA targets are injected into the chamber and hybridization is allowed to proceed for 5 min to 24 hr. The surface of the matrix is scanned in an epifluorescence microscope (Zeiss Axioscop 20) equipped with photon counting electronics using 50 - 100 µW of 488 nm excitation from an Argon ion laser (Spectra Physics Model 2020). Measurements may be made with the target solution in contact with the probe matrix or after washing. Photon counts are stored and image files are presented after conversion to an eight bit image format. *See* Fig. 51.

When hybridizing a DNA target to an oligonucleotide array, N = Lt-(Lp-1) complementary hybrids are expected, where N is the number of hybrids, Lt is the length of the DNA target, and Lp is the length of the oligonucleotide probes on the array. For example, for an 11-mer target hybridized to an octanucleotide array, N = 4. Hybridizations with mismatches at positions that are 2 to 3 residues from either end of the probes will generate detectable signals. Modifying the above expression for N, one arrives at a relationship estimating the number of detectable hybridizations (Nd) for a DNA target of length Lt and an array of complexity C. Assuming an average of 5 positions giving signals above background:

$$Nd = (1 + 5(C-1))[Lt-(Lp-1)].$$

Arrays of oligonucleotides can be efficiently generated by light-directed synthesis and can be used to determine the identity of DNA target sequences. Because combinatorial strategies are used, the number of compounds increases exponentially while the number of chemical coupling cycles increases only linearly. For example, synthesizing the complete set of $4^8$ (65,536) octanucleotides will add only four hours to the synthesis for the 16 additional cycles. Furthermore, combinatorial synthesis strategies can be implemented to generate arrays of any desired composition. For example, because the entire set of dodecamers ($4^{12}$) can be produced in 48 photolysis and coupling cycles ($b^n$ compounds requires b x n cycles), any subset of the dodecamers (including any subset of shorter oligonucleotides) can be constructed with the correct lithographic mask design in 48 or fewer chemical coupling steps. In addition, the number of compounds in an array is limited only by the density of synthesis sites and the overall array size. Recent experiments have demonstrated hybridization to probes synthesized in 25 µm sites. At this resolution, the entire set of 65,536 octanucleotides can be placed in an array measuring 0.64 cm square, and the set of 1,048,576 dodecanucleotides requires only a 2.56 cm array.

Genome sequencing projects will ultimately be limited by DNA sequencing technologies. Current sequencing methodologies are highly reliant on complex procedures and require substantial manual effort. Sequencing by hybridization has the potential for transforming many of the manual efforts into more efficient and automated formats. Light-directed synthesis is an efficient means for large scale production of miniaturized arrays for SBH. The oligonucleotide arrays are not limited to primary sequencing applications. Because single base changes cause multiple changes in the hybridization pattern, the oligonucleotide arrays provide a powerful means to check the accuracy of previously elucidated DNA sequence, or to scan for changes within a sequence. In the case of octanucleotides, a single base change in the target DNA results in the loss of eight complements, and generates eight new complements. Matching of hybridization patterns may be useful in resolving sequencing ambiguities from standard gel techniques, or for rapidly

detecting DNA mutational events. The potentially very high information content of light-directed oligonucleotide arrays will change genetic diagnostic testing. Sequence comparisons of hundreds to thousands of different genes will be assayed simultaneously instead of the current one, or few at a time format. Custom arrays can also be constructed to contain genetic markers for the rapid identification of a wide variety of pathogenic organisms.

Oligonucleotide arrays can also be applied to study the sequence specificity of RNA or protein-DNA interactions. Experiments can be designed to elucidate specificity rules of non Watson-Crick oligonucleotide structures or to investigate the use of novel synthetic nucleoside analogs for antisense or triple helix applications. Suitably protected RNA monomers may be employed for RNA synthesis. The oligonucleotide arrays should find broad application deducing the thermodynamic and kinetic rules governing formation and stability of oligonucleotide complexes.

Other than the use of photoremovable protecting groups, the nucleoside coupling chemistry is very similar to that used routinely today for oligonucleotide synthesis. Fig. 48 shows the deprotection, coupling, and oxidation steps of a solid phase DNA synthesis method. Fig. 49 shows an illustrative synthesis route for the nucleoside building blocks used in the method. Fig. 50 shows a preferred photoremovable protecting group, MeNPOC, and how to prepare the group in active form. The procedures described below show how to prepare these reagents. The nucleoside building blocks are 5'-MeNPOC-THYMIDINE-3'-OCEP; 5'-MeNPOC-$N^4$-t-BUTYL PHENOXYACETYL-DEOXYCYTIDINE-3'-OCEP; 5'-MeNPOC-$N^4$-t-BUTYL PHENOXYACETYL-DEOXYGUANOSINE-3'-OCEP; and 5'-MeNPOC-$N^4$-t-BUTYL PHENOXYACETYL-DEOXYADENOSINE-3'-OCEP.

1. Preparation of 4,5-methylenedioxy-2-nitroacetophenone

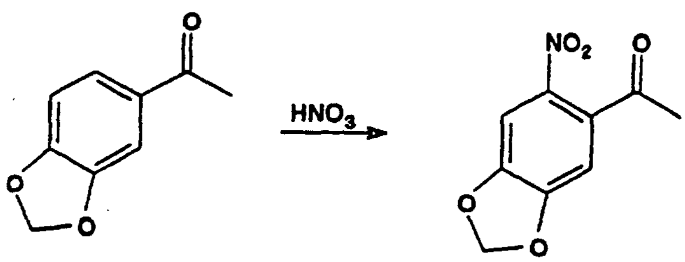


A solution of 50 g (0.305 mole) 3,4-methylenedioxy-acetophenone (Aldrich) in 200 mL glacial acetic acid was added dropwise over 30 minutes to 700 mL of cold (2-4°C) 70% $HNO_3$ with stirring (NOTE: the reaction will overheat without external cooling from an ice bath, which can be dangerous and lead to side products). At temperatures below 0°C, however, the reaction can be sluggish. A temperature of 3-5°C seems to be optimal). The mixture was left stirring for another 60 minutes at 3-5°C, and then allowed to approach ambient temperature. Analysis by TLC (25% EtOAc in hexane) indicated complete conversion of the starting material within 1-2 hr. When the reaction was complete, the mixture was poured into ~3 liters of crushed ice, and the resulting yellow solid was filtered off, washed with water and then suction-dried. Yield ~53 g (84%), used without further purification.

2. Preparation of 1-(4,5-Methylenedioxy-2-nitrophenyl) ethanol

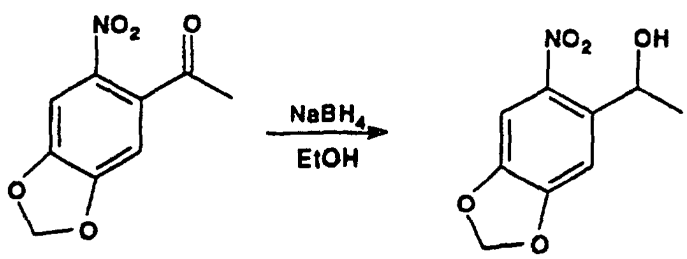

Sodium borohydride (10g; 0.27 mol) was added slowly to a cold, stirring suspension of 53g (0.25 mol) of 4,5-methylenedioxy-2-nitroacetophenone in 400 mL methanol. The temperature was kept below 10°C by slow addition of the $NaBH_4$ and external cooling with an ice bath. Stirring was continued at ambient temperature for another two hours, at which time TLC ($CH_2Cl_2$) indicated complete conversion of the ketone. The mixture was poured into one liter of ice-water and the resulting suspension was neutralized with ammonium chloride and then extracted three times with 400 mL $CH_2Cl_2$ or EtOAc (the product can be collected by filtration and washed at this point, but it is somewhat soluble in water and this results in a yield of only ~60%). The combined organic extracts were washed with brine, then dried with $MgSO_4$ and evaporated. The crude product was purified from the main byproduct by dissolving it in a minimum volume of $CH_2Cl_2$ or THF(~175 ml) and then precipitating it by slowly adding hexane (1000 ml) while stirring (yield 51g; 80% overall). It can also be recrystallized (*e.g.*, toluene-hexane), but this reduces the yield.

3. Preparation of 1-(4,5-methylenedioxy-2-nitrophenyl) ethyl chloroformate (MeNPOC-Cl)

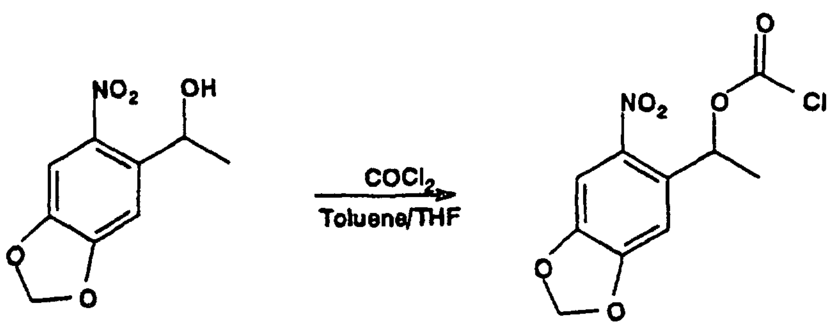


Phosgene (500 mL of 20% w/v in toluene from Fluka: 965 mmole; 4 eq.) was added slowly to a cold, stirring solution of 50g (237 mmole; 1 eq.) of 1-(4,5-methylenedioxy-2-nitrophenyl) ethanol in 400 mL dry THF. The solution was stirred overnight at ambient temperature at which point TLC (20% $Et_2O$/hexane) indicated >95% conversion. The mixture was evaporated (an oil-less pump with downstream aqueous NaOH trap is recommended to remove the excess phosgene) to afford a viscous brown oil. Purification was effected by flash chromatography on a short (9 x 13 cm) column of silica gel eluted with 20% $Et_2O$/hexane. Typically 55g (85%) of the solid yellow MeNPOC-Cl is obtained by this procedure. The crude material has also been recrystallized in 2-3 crops from 1:1 ether/hexane. On this scale, ~100ml is used for the first crop, with a few percent THF added to aid dissolution, and then cooling overnight at -20°C (this procedure has not been optimized). The product should be stored desiccated at -20°C.

4. Synthesis of 5'- Menpoc-2'-deoxynucleoside-3'-(N,N-diisopropyl 2-cyanoethyl phosphoramidites

(a.) 5'-MeNPOC-Nucleosides

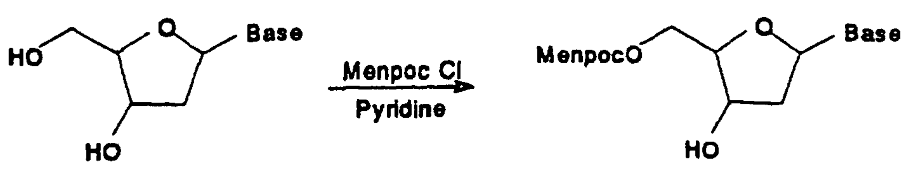


Base= THYMIDINE (T) ; N-4-ISOBUTYRYL 2'-DEOXYCYTIDINE (ibu-dC); N-2-PHENOXYACETYL 2'DEOXYGUANOSINE (PAC-dG); and N-6-PHENOXYACETYL 2'DEOXYADENOSINE (PAC-dA)

All four of the 5'-MeNPOC nucleosides were prepared from the base-protected 2'-deoxynucleosides by the following procedure. The protected 2'-deoxynucleoside (90 mmole) was dried by co-evaporating twice with 250 mL

anhydrous pyridine. The nucleoside was then dissolved in 300 mL anhydrous pyridine (or 1:1 pyridine/DMF, for the $dG^{PAC}$ nucleoside) under argon and cooled to -2°C in an ice bath. A solution of 24.6g (90 mmole) MeNPOC-Cl in 100 mL dry THF was then added with stirring over 30 minutes. The ice bath was removed, and the solution allowed to stir overnight at room temperature (TLC: 5-10% MeOH in $CH_2Cl_2$; two diastereomers). After evaporating the solvents under vacuum, the crude material was taken up in 250 mL ethyl acetate and extracted with saturated aqueous $NaHCO_3$ and brine. The organic phase was then dried over $Na_2SO_4$, filtered and evaporated to obtain a yellow foam. The crude products were finally purified by flash chromatography (9 x 30 cm silica gel column eluted with a stepped gradient of 2% - 6% MeOH in $CH_2Cl_2$). Yields of the purified diastereomeric mixtures are in the range of 65-75%.

(b.) 5'- Menpoc-2'-deoxynucleoside-3'-(N,N-diisopropyl 2-cyanoethyl phosphoramidites)

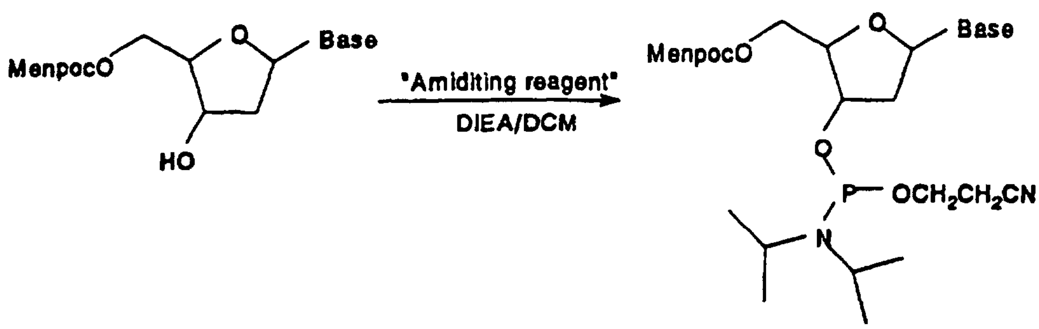


The four deoxynucleosides were phosphitylated using either 2-cyanoethyl- N,N- diisopropyl chlorophosphoramidite, or 2-cyanoethyl- N,N,N',N'- tetraisopropylphosphorodiamidite. The following is a typical procedure. Add 16.6g (17.4 ml; 55 mmole) of 2- cyanoethyl- N,N,N',N'- tetraisopropylphosphorodiamidite to a solution of 50 mmole 5'- MeNPOC-nucleoside and 4.3g (25 mmole) diisopropylammonium tetrazolide in 250 mL dry $CH_2Cl_2$ under argon at ambient temperature. Continue stirring for 4-16 hours (reaction monitored by TLC: 45:45:10 hexane/$CH_2Cl_2$/$Et_3N$). Wash the organic phase with saturated aqueous $NaHCO_3$ and brine, then dry over $Na_2SO_4$, and evaporate to dryness. Purify the crude amidite by flash chromatography (9 x 25 cm silica gel column eluted with hexane/$CH_2Cl_2$/TEA - 45:45:10 for A, C, T; or 0:90:10 for G). The yield of purified amidite is about 90%.

B. PREPARATION OF LABELED DNA/HYBRIDIZATION TO ARRAY

1. PCR

PCR amplification reactions are typically conducted in a mixture composed of, per reaction: 1 µl genomic DNA; 10 µl each primer (10 pmol/µl stocks); 10 µl 10 x PCR buffer (100 mM Tris.Cl pH8.5, 500 mM KCl, 15 mM $MgCl_2$); 10 µl 2 mM dNTPs (made from 100 mM dNTP stocks); 2.5 U Taq polymerase (Perkin Elmer AmpliTaq™, 5 U/µl); and $H_2O$ to 100 µl. The cycling conditions are usually 40 cycles (94°C 45 sec, 55°C 30 sec, 72°C 60 sec) but may need to be varied considerably from sample type to sample type. These conditions are for 0.2 mL thin wall tubes in a Perkin Elmer 9600 thermocycler. *See* Perkin Elmer 1992/93 catalogue for 9600 cycle time information. Target, primer length and sequence composition, among other factors, may also affect parameters.

For products in the 200 to 1000 bp size range, check 2 µl of the reaction on a 1.5% 0.5x TBE agarose gel using an appropriate size standard (phiX174 cut with *Hae*III is convenient). The PCR reaction should yield several picomoles of product. It is helpful to include a negative control (*i.e.*, 1 µl TE instead of genomic DNA) to check for possible contamination. To avoid contamination, keep PCR products from previous experiments away from later reactions, using filter tips as appropriate. Using a set of working solutions and storing master solutions separately is helpful, so long as one does not contaminate the master stock solutions.

For simple amplifications of short fragments from genomic DNA it is, in general, unnecessary to optimize $Mg^{2+}$ concentrations. A good procedure is the following: make a master mix minus enzyme; dispense the genomic DNA samples to individual tubes or reaction wells; add enzyme to the master mix; and mix and dispense the master solution to each well, using a new filter tip each time.

2. PURIFICATION

Removal of unincorporated nucleotides and primers from PCR samples can be accomplished using the Promega Magic PCR Preps DNA purification kit. One can purify the whole sample, following the instructions supplied with the kit (proceed from section IIIB, 'Sample preparation for direct purification from PCR reactions'). After elution of the PCR product in 50 µl of TE or $H_2O$, one centrifuges the eluate for 20 sec at 12,000 rpm in a microfuge and carefully transfers 45 µl to a new microfuge tube, avoiding any visible pellet. Resin is sometimes carried over during the elution step. This transfer prevents accidental contamination of the linear amplification reaction with 'Magic PCR' resin. Other methods, *e.g.*, size exclusion chromatography, may also be used.

3. Linear amplification

In a 0.2 mL thin-wall PCR tube mix: 4 µl purified PCR product; 2 µl primer (10 pmol/µl); 4 µl 10 x PCR buffer; 4 µl dNTPs (2 mM dA, dC, dG, 0.1 mM dT); 4 µl 0.1 mM dUTP; 1 µl 1 mM fluorescein dUTP (Amersham RPN 2121); 1 U Taq polymerase (Perkin Elmer, 5 U/µl); and add H2O to 40 µl. Conduct 40 cycles (92°C 30 sec, 55°C 30 sec, 72°C 90 sec) of PCR. These conditions have been used to amplify a 300 nucleotide mitochondrial DNA fragment but are applicable to other fragments. Even in the absence of a visible product band on an agarose gel, there should still be enough product to give an easily detectable hybridization signal. If one is not treating the DNA with uracil DNA glycosylase (see Section 4), dUTP can be omitted from the reaction.

4. Fragmentation

Purify the linear amplification product using the Promega Magic PCR Preps DNA purification kit, as per Section 2 above. In a 0.2 mL thin-wall PCR tube mix: 40 µl purified labeled DNA; 4 µl 10 x PCR buffer; and 0.5 µl uracil DNA glycosylase (BRL 1U/µl). Incubate the mixture 15 min at 37°C, then 10 min at 97°C; store at -20°C until ready to use.

5. Hybridization, Scanning & Stripping

A blank scan of the slide in hybridization buffer only is helpful to check that the slide is ready for use. The buffer is removed from the flow cell and replaced with 1 mL of (fragmented) DNA in hybridization buffer and mixed well. The scan is performed in the presence of the labeled target. Fig. 51 illustrates an illustrative detection system for scanning a DNA chip. A series of scans at 30 min intervals using a hybridization temperature of 25°C yields a very clear signal, usually in at least 30 min to two hours, but it may be desirable to hybridize longer, *i.e.*, overnight. Using a laser power of 50 µW and 50 µm pixels, one should obtain maximum counts in the range of hundreds to low thousands/pixel for a new slide. When finished, the slide can be stripped using 50% formamide. rinsing well in deionized $H_2O$, blowing dry, and storing at room temperature.

C. PREPARATION OF LABELED RNA/HYBRIDIZATION TO ARRAY

1. Tagged primers

The primers used to amplify the target nucleic acid should have promoter sequences if one desires to produce RNA from the amplified nucleic acid. Suitable promoter sequences are shown below and include:

(1) the T3 promoter sequence:

5'-CGGAATTAACCCTCACTAAAGG

5'-AATTAACCCTCACTAAAGGGAG;

(2) the T7 promoter sequence:

5' TAATACGACTCACTATAGGGAG;

and (3) the SP6 promoter sequence:

**5' ATTTAGGTGACACTATAGAA.**

The desired promoter sequence is added to the 5' end of the PCR primer. It is convenient to add a different promoter to each primer of a PCR primer pair so that either strand may be transcribed from a single PCR product.

Synthesize PCR primers so as to leave the DMT group on. DMT-on purification is unnecessary for PCR but appears to be important for transcription. Add 25 µl 0.5M NaOH to collection vial prior to collection of oligonucleotide to keep the DMT group on. Deprotect using standard chemistry -- 55°C overnight is convenient.

HPLC purification is accomplished by drying down the oligonucleotides, resuspending in 1 mL 0.1 M TEAA (dilute 2.0 M stock in deionized water, filter through 0.2 micron filter) and filter through 0.2 micron filter. Load 0.5 mL on reverse phase HPLC (column can be a Hamilton PRP-1 semi-prep, #79426). The gradient is 0 -> 50% $CH_3CN$ over 25 min (program 0.2 µmol.prep.0-50, 25 min). Pool the desired fractions, dry down, resuspend in 200 µl 80% HAc. 30 min RT. Add 200 µl EtOH; dry down. Resuspend in 200 µl $H_2O$, plus 20 µl NaAc pH5.5, 600 µl EtOH. Leave 10 min on ice; centrifuge 12,000 rpm for 10 min in microfuge. Pour off supernatant. Rinse pellet with 1 mL EtOH, dry, resuspend in 200 µl H20. Dry, resuspend in 200 µl TE. Measure A260, prepare a 10 pmol/µl solution in TE (10 mM Tris.Cl pH 8.0, 0.1 mM EDTA). Following HPLC purification of a 42 mer, a yield in the vicinity of 15 nmol from a 0.2 µmol scale synthesis is typical.

2. Genomic DNA Preparation

Add 500 µl (10 mM Tris.Cl pH8.0, 10 mM EDTA, 100 mM NaCl, 2% (w/v) SDS, 40 mM DTT, filter sterilized) to the sample. Add 1.25 µl 20 mg/ml proteinase K (Boehringer) Incubate at 55°C for 2 hours, vortexing once or twice. Perform 2x 0.5 mL 1:1 phenol:$CHCl_3$ extractions. After each extraction, centrifuge 12,000 rpm 5 min in a microfuge and recover 0.4 mL supernatant. Add 35 µl NaAc pH5.2 plus 1 mL EtOH. Place sample on ice 45 min; then centrifuge 12,000 rpm 30 min, rinse, air dry 30 min, and resuspend in 100 µl TE.

3. PCR

PCR is performed in a mixture containing, per reaction: 1 µl genomic DNA; 4 µl each primer (10 pmol/µl stocks); 4 µl 10 x PCR buffer (100 mM Tris.Cl pH8.5, 500 mM KCl, 15 mM $MgCl_2$); 4 µl 2 mM dNTPs (made from 100 mM dNTP stocks); 1 U Taq polymerase (Perkin Elmer, 5 U/µl); $H_2O$ to 40 µl. About 40 cycles (94°C 30 sec, 55°C 30 sec, 72°C 30 sec) are performed, but cycling conditions may need to be varied. These conditions are for 0.2 mL thin wall tubes in Perkin Elmer 9600. For products in the 200 to 1000 bp size range, check 2 µl of the reaction on a 1.5% 0.5xTBE agarose gel using an appropriate size standard. For larger or smaller volumes (20 - 100 µl), one can use the same amount of genomic DNA but adjust the other ingredients accordingly.

4. *In vitro* transcription

Mix: 3 µl PCR product; 4 µl 5x buffer; 2 µl DTT; 2.4 µl 10 mM rNTPs (100 mM solutions from Pharmacia); 0.48 µl 10 mM fluorescein-UTP (Fluorescein-12-UTP, 10 mM solution, from Boehringer Mannheim); 0.5 µl RNA polymerase (Promega T3 or T7 RNA polymerase); and add $H_2O$ to 20 µl. Incubate at 37°C for 3 h. Check 2 µl of the reaction on a 1.5% 0.5xTBE agarose gel using a size standard. 5x buffer is 200 mM Tris pH 7.5, 30 mM $MgCl_2$, 10 mM spermidine, 50 mM NaCl, and 100 mM DTT (supplied with enzyme). The PCR product needs no purification and can be added directly to the transcription mixture. A 20 µl reaction is suggested for an initial test experiment and hybridization; a 100 µl reaction is considered "preparative" scale (the reaction can be scaled up to obtain more target). The amount of PCR product to add is variable; typically a PCR reaction will yield several picomoles of DNA. If the PCR reaction does not produce that much target, then one should increase the amount of DNA added to the transcription reaction (as well as optimize the PCR). The ratio of fluorescein-UTP to UTP suggested above is 1:5, but ratios from 1:3 to 1:10 - all work well. One can also label with biotin-UTP and detect with streptavidin-FITC to obtain similar results as with fluorescein-UTP detection.

For nondenaturing agarose gel electrophoresis of RNA, note that the RNA band will normally migrate somewhat faster than the DNA template band, although sometimes the two bands will comigrate. The temperature of the gel can effect the migration of the RNA band. The RNA produced from *in vitro* transcription is quite stable and can be stored for months (at least) at -20°C without any evidence of degradation. It can be stored in unsterilized 6xSSPE 0.1% triton X-100 at -20°C for days (at least) and reused twice (at least) for hybridization, without taking any special precautions in preparation or during use. RNase contamination should of course be avoided. When extracting RNA from cells, it is preferable to work very rapidly and to use strongly denaturing conditions. Avoid using glassware previously contaminated with RNases. Use of new disposable plasticware (not necessarily sterilized) is preferred, as new plastic

tubes, tips, etc., are essentially RNase free. Treatment with DEPC or autoclaving is typically not necessary.

5. Fragmentation

Heat transcription mixture at 94 degrees for forty min. The extent of fragmentation is controlled by varying $Mg^{2+}$ concentration (30 mM is typical), temperature, and duration of heating.

6. Hybridization, Scanning, & Stripping

A blank scan of the slide in hybridization buffer only is helpful to check that the slide is ready for use. The buffer is removed from the flow cell and replaced with 1 mL of (hydrolysed) RNA in hybridization buffer and mixed well. Incubate for 15 - 30 min at 18°C. Remove the hybridization solution, which can be saved for subsequent experiments. Rinse the flow cell 4 - 5 times with fresh changes of 6 x SSPE / 0.1% Triton X-100, equilibrated to 18°C. The rinses can be performed rapidly, but it is important to empty the flow cell before each new rinse and to mix the liquid in the cell thoroughly. A series of scans at 30 min intervals using a hybridization temperature of 25°C yields a very clear signal, usually in at least 30 min to two hours, but it may be desirable to hybridize longer, *i.e.*, overnight. Using a laser power of 50 µW and 50 µm pixels, one should obtain maximum counts in the range of hundreds to low thousands/pixel for a new slide. When finished, the slide can be stripped using warm water.

These conditions are illustrative and assume a probe length of ~15 nucleotides. The stripping conditions suggested are fairly severe, but some signal may remain on the slide if the washing is not stringent. Nevertheless, the counts remaining after the wash should be very low in comparison to the signal in presence of target RNA. In some cases, much gentler stripping conditions are effective. The lower the hybridization temperature and the longer the duration of hybridization, the more difficult it is to strip the slide. Longer targets may be more difficult to strip than shorter targets.

7. Amplification of Signal

A variety of methods can be used to enhance detection of labelled targets bound to a probe on the array. In one embodiment, the protein MutS (from E. coli) or equivalent proteins such as yeast MSH1, MSH2, and MSH3; mouse Rep-3, and Streptococcus Hex-A, is used in conjunction with target hybridization to detect probe-target complex that contain mismatched base pairs. The protein, labeled directly or indirectly, can be added to the chip during or after hybridization of target nucleic acid, and differentially binds to homo- and heteroduplex nucleic acid. A wide variety of dyes and other labels can be used for similar purposes. For instance, the dye YOYO-1 is known to bind preferentially to nucleic acids containing sequences comprising runs of 3 or more G residues.

8. Detection of Repeat Sequences

In some circumstances, *i.e*., target nucleic acids with repeated sequences or with high G/C content, very long probes are sometimes required for optimal detection. In one embodiment for detecting specific sequences in a target nucleic acid with a DNA chip, repeat sequences are detected as follows. The chip comprises probes of length sufficient to extend into the repeat region varying distances from each end. The sample, prior to hybridization, is treated with a labelled oligonucleotide that is complementary to a repeat region but shorter than the full length of the repeat. The target nucleic is labelled with a second, distinct label. After hybridization, the chip is scanned for probes that have bound both the labelled target and the labelled oligonucleotide probe; the presence of such bound probes shows that at least two repeat sequences are present.

**Claims**

**1.** An array of oligonucleotide probes immobilized on a solid support, e.g. linked to the support via a spacer, the array comprising at least two sets of oligonucleotide probes,

(1) a first probe set comprising a plurality of probes, each probe comprising a segment of at least six nucleotides exactly complementary to a subsequence of a reference sequence, the segment including at least one interrogation position complementary to a corresponding nucleotide in the reference sequence,

(2) a second probe set comprising a corresponding probe for each probe in the first probe set, the corresponding probe in the second probe set being identical to a sequence comprising the corresponding probe from the

first probe set or a subsequence of at least six nucleotides thereof that includes the at least one interrogation position, except that the at least one interrogation position is occupied by a different nucleotide in each of the two corresponding probes from the first and second probe sets;

wherein the probes in the first probe set have at least two interrogation positions respectively corresponding to each of two contiguous nucleotides in the reference sequence.

**2.** An array of oligonucleotide probes of claim 1 further comprising third and fourth probe sets, wherein the second, third and fourth probe sets, each comprise a corresponding probe for each probe in the first probe set, the probes in the second, third and fourth probe sets being identical to a sequence comprising the corresponding probe from the first probe set or a subsequence of at least six nucleotides thereof that includes the at least one interrogation position, except that the at least one interrogation position is occupied by a different nucleotide in each of the four corresponding probes from the four probe sets.

**3.** An array of claim 1 or 2, wherein the array comprises between 100 and 100,000 probes.

**4.** An array of any of claims 1 to 3, wherein the probes of the first probe set comprise a segment of 9 to 21 nucleotides exactly complementary to a subsequence of a reference sequence, the segment including at least one interrogation position complementary to a corresponding nucleotide in the reference sequence.

**5.** An array of any of claims 1 to 4, further comprising a fifth probe set comprising a corresponding probe for each probe in the first probe set, the corresponding probe from the fifth probe set being identical to a sequence comprising the corresponding probe from the first probe set or a subsequence of at least six nucleotides thereof that includes the at least one interrogation position, except that the at least one interrogation position is deleted in the corresponding probe from the fifth probe set.

**6.** An array of any of claims 1 to 5, further comprising a sixth probe set comprising a corresponding probe for each probe in the first probe set, the corresponding probe from the sixth probe set being identical to a sequence comprising the corresponding probe from the first probe set or a subsequence of at least six nucleotides thereof that includes the at least one interrogation position, except that an additional nucleotide is inserted adjacent to the at least one interrogation position in the corresponding probe from the first probe set.

**7.** An array of any one of claims 1 to 6, wherein either:

(a) the first probe set has at least 50 interrogation positions respectively corresponding to each of 50 contiguous nucleotides in a reference sequence; or

(b) the first probe set has an interrogation position corresponding to each of the nucleotides in the reference sequence.

**8.** An array of any of claims 1 to 7, further comprising fifth, sixth and seventh probe sets, wherein:

the segment of each probe in the first set includes at least two interrogation positions each corresponding to a nucleotide in the reference sequence,

the second, third and fourth probe sets, each comprise a corresponding probe for each probe in the first probe set, the corresponding probes in the second, third and fourth probe sets being identical to a sequence comprising the corresponding probe from the first probe set or a subsequence of at least six nucleotides thereof that includes a first interrogation position except that the first interrogation position is occupied by a different nucleotide in each of the four corresponding probes from the four probe sets;

the fifth, sixth and seventh probe sets, each comprising a corresponding probe for each probe in the first probe set, the probes in the fifth, sixth and seventh probe sets being identical to a sequence comprising the corresponding probe from the first probe set or a subsequence of at least six nucleotides thereof that includes a second interrogation position, except that the second interrogation position is occupied by a different nucleotide in each of the four corresponding probes from the four probe sets.

**9.** An array of any of claims 1 to 8, wherein each probe in the first probe set further comprises a second segment of

at least six nucleotides exactly complementary to a second subsequence of the reference sequence, and the probes from the second, third and fourth probe sets comprise the corresponding probe from the first probe set or a subsequence thereof comprising the first and second segments except in the at least one interrogation position.

**10.** An array of any of claims 1 to 9, further comprising:

a fifth probe set comprising at least one probe comprising a segment of at least seven nucleotides exactly complementary to a subsequence of the reference sequence except at one or two positions, the segment including at least one interrogation position corresponding to a nucleotide in the reference sequence not at the one or two positions, wherein either (1) the first and second subsequences are noncontiguous, or (2) the first and second subsequences are contiguous and the first and second segments are inverted relative to the complement of the first and second subsequences in the reference sequence,

sixth, seventh and eighth probe sets, each comprising a probe for each probe in the fifth probe set, the corresponding probes from the sixth, seventh and eighth probe sets being identical to a sequence comprising the corresponding probe from the fifth probe set or a subsequence of at least nine nucleotides thereof including the at least one interrogation position and the one or two positions, except in the at least one interrogation position, which is occupied by a different nucleotide in each of the four probes.

**11.** An array of any of claims 1 to 10, wherein the first and second subsequences are separated by one or two nucleotides in the reference sequence.

**12.** An array of any of claims 1 to 11, wherein the probes are arranged on the substrate so that the first set of probes is arranged in a row across the substrate in an order reflecting the overlap between the probes and the reference sequence, and additional sets of probes are arranged in columns relative to the probes in said first set, so that probes with the same interrogation position are in the same column and so that each column comprises at least 4 probes.

**13.** An array of oligonucleotide probes immobilized on a solid support, the array comprising at least one pair of first and second probe groups, each group comprising a first and second sets of oligonucleotide probes as defined by claim 1;
wherein each probe in the first probe set from the first group is exactly complementary to a subsequence of a first reference sequence and each probe in the first probe set from the second group is exactly complementary to a subsequence from a second reference sequence.

**14.** An array of claim 13, wherein the second reference sequence is a mutated form of the first reference sequence.

**15.** An array of claim 13 or 14, wherein each group further comprises third and fourth probe sets, each comprising a corresponding probe for each probe in the first probe set, the probes in the second, third and fourth probe sets being identical to a sequence comprising the corresponding probe from the first probe set or a subsequence of at least six nucleotides thereof that includes the interrogation position, except that the interrogation position is occupied by a different nucleotide in each of the four corresponding probes from the four probe sets.

**16.** An array of any of claims 13 to 15, wherein the array comprises at least five pairs of first and second probe groups, wherein the probes in the first probe sets from the first groups of the five pairs are exactly complementary to subsequences from five different respective first reference sequences.

**17.** An array of oligonucleotide probes immobilized on a solid support, the array comprising at least a group of probes comprising:

a wildtype probe comprising a segment of at least six nucleotides exactly complementary to a subsequence of a reference sequence, the segment having at least first and second interrogation positions corresponding to first and second nucleotides in the reference sequence,

a first set of three mutant probes, each identical to a sequence comprising the wildtype probe or a subsequence of at least six nucleotides thereof including the first and second interrogation positions, except in the first interrogation position, which is occupied by a different nucleotide in each of the three mutant probes and the wildtype probe;

a second set of three mutant probes, each identical to a sequence comprising the wildtype probe or a subsequence of at least six nucleotides thereof including the first and second interrogation positions, except in the second interrogation position, which is occupied by a different nucleotide in each of the three mutant probes and the wildtype probe.

**18.** An array of probes immobilized to a solid support comprising two groups of probes, each group as defined in claim 17, a first group comprising a wildtype probe comprising a segment exactly complementary to a subsequence of a first reference sequence and a second group comprising a wildtype probe comprising a segment exactly complementary to a subsequence of a second reference sequence.

**19.** A method of comparing a target sequence with a reference sequence, the method comprising:

identifying variants of a reference sequence differing from the reference sequence in at least one nucleotide; assigning each variant a designation,

providing an array of pools of probes, each pool occupying a separate cell of the array, wherein each pool comprises a probe comprising a segment exactly complementary to each variant sequence assigned a particular designation,

contacting the array with a target sequence comprising a variant of the reference sequence;

determining the relative hybridization intensities of the pools in the array to the target sequence;

determining the target sequence from the relative hybridization intensities of the pools.

**20.** A pooled probe comprising a segment exactly complementary to a subsequence of a reference sequence except at a first interrogation position occupied by a pooled nucleotide N, a second interrogation position occupied by a pooled nucleotide selected from the group of three consisting of (1) M or K, (2) R or Y and (3) S or W, and a third interrogation position occupied by a second pooled nucleotide selected from the group, wherein the pooled nucleotide occupying the second interrogation position comprises a nucleotide complementary to a corresponding nucleotide from the reference sequence when the pooled probe and reference sequence are maximally aligned, and the pooled nucleotide occupying the third interrogation position comprises a nucleotide complementary to a corresponding nucleotide from the reference sequence when the pooled probe and the reference sequence are maximally aligned, wherein N is A, C, G or T(U), K is G or T(U), M is A or C, R is A or G, Y is C or T(U), W is A or T (U) and S is G or C.

**21.** An array of oligonucleotide probes immobilized on solid support, the array comprising:

first, second and third cells respectively occupied by first, second and third pooled probes as defined by claim 20,

provided that one of the three interrogation positions in the each of the three pooled probes is aligned with the same corresponding nucleotide in the reference sequence, this interrogation position being occupied by an N in one of the pooled probes, and a different pooled nucleotide in each of the other two pooled probes,

wherein N is A, C, G or T(U), K is G or T(U), M is A or C, R is A or G, Y is C or T(U), W is A or T(U) and S is G or C.

**22.** An array of claim 21, wherein the array further comprises:

fourth and fifth cells respectively occupied by fourth and fifth pooled probes, each pooled probe as defined in claim 21,

wherein one of the three interrogation position in the second, third and fourth pooled probes is aligned with the same corresponding nucleotide in the reference sequence, this interrogation position being occupied by an N in one of the pooled probes, and a different pooled nucleotide in each of the other two pooled probes,
wherein one of the three interrogation position in the third, fourth and fifth pooled probes is aligned with the same corresponding nucleotide in the reference sequence, this interrogation position being occupied by an N in one of the pooled probes, and a different pooled nucleotide in each of the other two pooled probes.

**23.** An array of oligonucleotide probes according to claim 2, wherein
the first probe set comprises a probe comprising first and second segments, each of at least six nucleotides and exactly complementary to first and second subsequences of a reference sequences, the segments including at least one interrogation position corresponding to a nucleotide in the reference sequence, wherein either (1) the first and second subsequences are noncontiguous, or (2) the first and second subsequences are contiguous and the first and second segments are inverted relative to the complement of the first and second subsequences in the reference sequence; and
the second, third and fourth probe sets comprise corresponding probes, identical to a sequence comprising the first probe or a subsequence thereof comprising at least six nucleotides from each of the first and second segments, except in the at least one interrogation position, which differs in each of the probes.

**24.** An array of oligonucleotide probes according to claim 2, wherein
the first probe set comprises a probe comprising a segment of at least 7 nucleotides exactly complementary to a subsequence of a reference sequence except at one or two positions, the segment including an interrogation position not at the one or two positions;
the second, third and fourth probe sets comprises corresponding probes, each identical to a sequence comprising the wildtype probe or a subsequence thereof including the interrogation position and the one or two positions, except in the interrogation position, which is occupied by a different nucleotide in each of the four probes.

**25.** An array of oligonucleotide probes immobilized on a solid support, the array comprising at least two sets of oligonucleotide probes,

(1) a first probe set comprising a plurality of probes, each probe comprising a segment exactly complementary to a subsequence of at least six nucleotides of a reference sequence except at an interrogation position,

(2) a second probe set comprising a corresponding probe for each probe in the first probe set, the corresponding probe in the second probe set being identical to a sequence comprising the corresponding probe from the first probe set or a subsequence of at least six nucleotides thereof that includes the interrogation position, except that the interrogation position is occupied by a different nucleotide in each of the two corresponding probes and the complement to the reference sequence,

wherein the probes in the first probe set have at least three interrogation positions respectively corresponding to each of three contiguous nucleotides in the reference sequence.

**26.** A method of comparing a target nucleic acid with a reference sequence comprising a predetermined sequence of nucleotides, the method comprising hybridizing the target nucleic acid to an array of oligonucleotide probes immobilized on a solid support as defined in any one of claims 1 to 18 or 21 to 25.

**27.** A method of claim 26, wherein the target sequence has a substituted nucleotide relative to the reference sequence in at least one undetermined position, and the relative specific binding of the probes indicates the location of the position and the nucleotide occupying the position in the target sequence.

**28.** A method of claim 26 or 27, wherein the reference sequence is from a human immunodeficiency virus.

**29.** A method of any of claim 26 to 28, wherein the target sequence is from a second human immunodeficiency virus, e.g. wherein the target sequence has a substituted nucleotide relative to the reference sequence in at least one position, the substitution conferring drug resistance to the human immunodeficiency virus, and the relative specific binding of the probes reveals the substitution.

**30.** A method of any of claims 26 to 29, wherein the determining comprises:

(1) comparing the relative specific binding of four corresponding probes from first, second, third and fourth probe sets;

(2) assigning a nucleotide in the target sequence as the complement of the interrogation position of the probe having the greatest specific binding;

(3) repeating (1) and (2) until each nucleotide of interest in the target sequence has been assigned.

**31.** A method of comparing a target nucleic acid with a reference sequence comprising a predetermined sequence of nucleotides, according to claim 26, wherein
the target sequence is hybridized to an array of claim 13 or claim 14; and the method further comprises
determining which probes in the first group, relative to one another, hybridize to the target sequence, the relative specific binding of the probes indicating whether the target sequence is the same or different from the first reference sequence;
determining which probes in the second group, relative to one another, hybridize to the target sequence, the relative specific binding of the probes indicating whether the target sequence is the same or different from the second reference sequence.

**32.** A method of comparing a target nucleic acid with a reference sequence comprising a predetermined sequence of nucleotides, according to claim 26, wherein the target sequence is hybridized to an array of oligonucleotide probes immobilized on a solid support as defined in any one of claims 1 to 18 or 21 to 25 and the method further comprises

(a) hybridizing the reference sequence to the array of oligonucleotide probes;

(b) determining which probes, relative to one another, in the array bind specifically to the reference sequence;

(c) determining which probes, relative to one another, in the array bind specifically to the target sequence;

wherein the relative specific binding of the probes to the reference and the target sequence indicates whether the reference sequence is the same or different from the target sequence.

**33.** A method of claim 32, wherein the reference sequence has a first label and a second reference sequence having a second label different from the first label, with steps (a) and (c) being performed simultaneously.

**34.** The array of any one of claims 1 to 18 or 21 to 25, wherein the reference sequence is from a human immunodeficiency virus, e.g. a reverse transcriptase gene or a protease gene of the human immunodeficiency virus.

**35.** The array of claim 34, wherein the reference sequence is a full-length reverse transcriptase gene.

**36.** An array of claim 13 or 14, wherein the first reference sequence is from a reverse transcriptase gene of a human immunodeficiency virus, and either the second reference sequence comprises a subsequence of the first reference sequence with a substitution of at least one nucleotide which confers drug resistance to a human immunodeficiency virus comprising the second reference sequence, or the second reference sequence is from a 16S RNA, or DNA encoding the 16S RNA, from a pathogenic microorganism.

**37.** An array of claim 13 or claim 14, wherein the first and second reference sequences are from a reverse transcriptase gene from first and second strains of a human immunodeficiency virus.

**38.** An array of any one of claims 1 to 18 or 21 to 25, wherein the reference sequence is from a CFTR gene, a p53 gene, an hMLH1 gene, an MSH2 gene, a mitochondrial genome.

**39.** The array of claim 38, wherein the reference sequence is a D-loop region, or a P450 gene.

**Patentansprüche**

**1.** Array aus Oligonukleotidsonden, die auf einem festen Träger immobilisiert sind, z.B. an den Träger über einen Spacer gebunden sind, **dadurch gekennzeichnet, daß** das Array mindestens zwei Sätze von Oligonukleotidsonden umfasst,

(1) ein erster Sondensatz umfassend eine Vielzahl von Sonden, wobei jede Sonde einen Abschnitt von mindestens sechs Nukleotiden umfasst, welche genau komplementär zu einer Teilsequenz einer Referenzsequenz sind, und der Abschnitt mindestens eine Abfrageposition beinhaltet, welche komplementär zu einem entsprechenden Nukleotid in der Referenzsequenz ist,

(2) ein zweiter Sondensatz umfassend eine entsprechende Sonde für jede Sonde in dem ersten Sondensatz,

wobei die entsprechende Sonde in dem zweiten Sondensatz identisch mit einer Sequenz ist, welche die entsprechende Sonde aus dem ersten Sondensatz oder eine Teilsequenz von mindestens sechs Nukleotiden davon umfasst, welche die mindestens eine Abfrageposition beinhaltet, außer daß mindestens eine Abfrageposition in jeder der beiden entsprechenden Sonden aus den ersten und zweiten Sondensätzen von einem unterschiedlichen Nukleotid besetzt ist;

wobei die Sonden in dem ersten Sondensatz jeweils mindestens zwei Abfragepositionen aufweisen, die jedem von zwei benachbarten Nukleotiden in der Referenzsequenz entsprechen.

2. Array aus Oligonukleotidsonden nach Anspruch 1, das weiterhin einen dritten und vierten Sondensatz umfasst, **dadurch gekennzeichnet, daß** der zweite, dritte und vierte Sondensatz jeweils eine entsprechende Sonde für jede Sonde in dem ersten Sondensatz umfasst, wobei die Sonden in dem zweiten, dritten und vierten Sondensatz identisch mit einer Sequenz sind, welche die entsprechende Sonde aus dem ersten Sondensatz oder eine Teilsequenz von mindestens sechs Nukleotiden davon umfasst, welche die mindestens eine Abfrageposition beinhaltet, außer dass die mindestens eine Abfrageposition durch ein abweichendes Nukleotid in jedem der vier entsprechenden Sonden aus den vier Sondensätzen besetzt ist.

3. Array nach Anspruch 1 oder 2, **dadurch gekennzeichnet, daß** das Array zwischen 100 und 100.000 Sonden umfasst.

4. Array nach einem der Ansprüche 1 bis 3, **dadurch gekennzeichnet, daß** die Sonden des ersten Sondensatzes einen Abschnitt von 9 bis 21 Nukleotiden umfassen, der genau komplementär zu einer Teilsequenz einer Referenzsequenz ist, wobei der Abschnitt mindestens eine Abfrageposition beinhaltet, welche komplementär zu einem entsprechenden Nukleotid in der Referenzsequenz ist.

5. Array nach einem der Ansprüche 1 bis 4, **dadurch gekennzeichnet, daß** es ferner einen fünften Sondensatz umfasst, der eine entsprechende Sonde für jede Sonde in dem ersten Sondensatz umfasst, wobei die entsprechende Sonde aus dem fünften Sondensatz identisch mit einer Sequenz ist, welche die entsprechende Sonde aus dem ersten Sondensatz oder eine Teilsequenz von mindestens sechs Nukleotiden davon umfasst, welche die mindestens eine Abfrageposition beinhaltet, außer wenn die mindestens eine Abfragepostion in der entsprechenden Sonde aus dem fünften Sondensatz deletiert wurde.

6. Array nach einem der Ansprüche 1 bis 5, **dadurch gekennzeichnet, daß** es ferner einen sechsten Sondensatz umfasst, der eine entsprechende Sonde für jede Sonde in dem ersten Sondensatz umfasst, wobei die entsprechende Sonde aus dem sechsten Sondensatz identisch mit einer Sequenz ist, welche die entsprechende Sonde aus dem ersten Sondensatz oder eine Teilsequenz von mindestens sechs Nukleotiden davon umfasst, welche die mindestens eine Abfrageposition beinhaltet, außer dass ein zusätzliches Nukleotid benachbart an die mindestens eine Abfrageposition in der entsprechenden Sonde aus dem ersten Sondensatz eingefügt wurde.

7. Array nach einem der Ansprüche 1 bis 6, **daduch gekennzeichnet, daß** entweder

(a) der erste Sondensatz jeweils mindestens 50 Abfragepositionen aufweist, die jedem der 50 benachbarten Nukleotide in einer Referenzsequenz entsprechen; oder

(b) der erste Sondensatz eine Abfrageposition aufweist, die jedem der Nukleotide in der Referenzsequenz entspricht.

8. Array nach einem der Ansprüche 1 bis 7, **dadurch gekennzeichnet, daß** es ferner einen fünften, sechsten und siebenten Sondensatz umfasst, wobei
der Abschnitt jeder Sonde in dem ersten Sondensatz mindestens zwei Abfragepositionen beinhaltet, die jeweils einem Nukleotid in der Referenzsequenz entsprechen,
die zweiten, dritten und vierten Sondensätze jeweils eine entsprechende Sonde für jede Sonde in dem ersten Sondensatz umfassen, wobei die entsprechenden Sonden in dem zweiten, dritten und vierten Sondensatz identisch mit einer Sequenz sind, welche die entsprechende Sonde aus dem ersten Sondensatz oder eine Teilsequenz von mindestens sechs Nukleotiden davon umfasst, welche eine erste Abfrageposition beinhaltet, außer dass die erste Abfrageposition durch ein abweichendes Nukleotid in jedem der vier entsprechenden Sonden aus den vier Sondensätzen besetzt ist;
die fünften, sechsten und siebenten Sondensätze jeweils eine entsprechende Sonde für jede Sonde in dem ersten

Sondensatz umfassen, wobei die Sonden in den fünften, sechsten und siebenten Sondensätzen identisch mit einer Sequenz sind, welche die entsprechende Sonde aus dem ersten Sondensatz oder eine Teilsequenz von mindestens sechs Nukleotiden davon umfasst, welche eine zweite Abfrageposition beinhaltet, außer dass die zweite Abfrageposition durch ein abweichendes Nukleotid in jeder der vier entsprechenden Sonden aus den vier Sondensätzen besetzt ist.

**9.** Array nach einem der Ansprüche 1 bis 8, **dadurch gekennzeichnet, daß** jede Sonde in dem ersten Sondensatz ferner einen zweiten Abschnitt von mindestens sechs Nukleotiden genau komplementär zu einer zweiten Teilsequenz der Referenzsequenz umfasst, und die Sonden aus den zweiten, dritten und vierten Sondensätzen die entsprechende Sonde aus dem ersten Sondensatz oder eine Teilsequenz davon umfassen, welche die ersten und zweiten Abschnitte außer der mindestens einen Abfrageposition umfassen.

**10.** Array nach einem der Ansprüche 1 bis 9, **dadurch gekennzeichnet, daß** es ferner
einen fünften Sondensatz, der mindestens eine Sonde umfasst, die einen Abschnitt von mindestens sieben Nukleotiden genau komplementär zu einer Teilsequenz der Referenzsequenz außer in ein oder zwei Positionen umfassen, wobei der Abschnitt mindestens eine Abfrageposition beinhaltet, welche einem Nukleotid in der Referenzsequenz entspricht, aber nicht an der einen oder den zwei Positionen in denen entweder (1) die ersten und zweiten Teilsequenzen nicht benachbart sind, oder (2) die ersten und zweiten Teilsequenzen benachbart sind und die ersten und zweiten Abschnitte relativ zu dem Gegenstück der ersten und zweiten Teilsequenzen in der Referenzsequenz invertiert vorliegen,
sechste, siebte und achte Sondensätze umfasst, die jeweils eine Sonde für jede Sonde in dem fünften Sondensatz umfassen, wobei die entsprechenden Sonden aus den sechsten, siebenten und achten Sondensätzen identisch mit einer Sequenz sind, welche die entsprechende Sonde aus dem fünften Sondensatz oder eine Teilsequenz von mindestens neun Nukleotiden davon einschließlich der mindestens einen Abfrageposition und der einen oder den zwei Positionen umfassen, außer in der mindestens einen Abfrageposition, die durch ein abweichendes Nukleotid in jeder der vier Sonden besetzt ist.

**11.** Array nach einem der Ansprüche 1 bis 10, **dadurch gekennzeichnet, daß** die ersten und zweiten Teilsequenzen durch ein oder zwei Nukleotide in der Referenzsequenz getrennt sind.

**12.** Array nach einem der Ansprüche 1 bis 11, **dadurch gekennzeichnet, daß** die Sonden auf dem Substrat so angeordnet sind, dass der erste Sondensatz in einer Reihe auf dem Substrat in einer Reihenfolge angeordnet ist, welche die Überlappung zwischen den Sonden und der Referenzsequenz widerspiegelt, und zusätzliche Sondensätze in Spalten relativ zu den Sonden in dem genannten ersten Satz angeordnet sind, so dass sich Sonden mit der gleichen Abfrageposition in der gleichen Spalte befinden und so dass jede Spalte mindestens vier Sonden umfasst.

**13.** Array aus auf einem festen Träger immobilisierten Oligonukleotidsonden, **dadurch gekennzeichnet, daß** das Array mindestens ein Paar von ersten und zweiten Sondengruppen umfasst, wobei jede Gruppe einen ersten und zweiten Satz aus Oligonukleotidsonden wie in Anspruch 1 definiert umfasst;
bei dem jede Sonde in dem ersten Sondensatz aus der ersten Gruppe genau komplementär zu einer Teilsequenz einer ersten Referenzsequenz und jede Sonde in dem ersten Sondensatz aus der zweiten Gruppe genau komplementär zu einer Teilsequenz einer zweiten Referenzsequenz ist.

**14.** Array nach Anspruch 13, **dadurch gekennzeichnet, daß** die zweite Referenzsequenz eine mutierte Form der ersten Referenzsequenz ist.

**15.** Array nach Anspruch 13 oder 14, **dadurch gekennzeichnet, daß** jede Gruppe ferner dritte und vierte Sondensätze umfasst, die jeder eine entsprechende Sonde für jede Sonde in dem ersten Sondensatz umfassen, wobei die Sonden in den zweiten, dritten und vierten Sondensätzen identisch mit einer Sequenz sind, welche die entsprechende Sonde aus dem ersten Sondensatz oder eine Teilsequenz von mindestens sechs Nukleotiden davon umfassen, welche die Abfrageposition beinhaltet, außer dass die Abfrageposition durch abweichende Nukleotide in jeder der vier entsprechenden Sonden aus den vier Sondensätzen besetzt ist.

**16.** Array nach einem der Ansprüche 13 bis 15, **dadurch gekennzeichnet, daß** das Array mindestens fünf Paare von ersten und zweiten Sondengruppen umfasst, wobei die Sonden in den ersten Sondensätzen aus den ersten Gruppen der fünf Paare genau komplementär zu Teilsequenzen von fünf unterschiedlichen respektive ersten Referenzsequenzen sind.

**17.** Array aus auf einem festen Träger immobilisierten Oligonukleotidsonden, **dadurch gekennzeichnet, daß** das Array mindestens eine Sondengruppe umfassend
eine Wildtypsonde, die einen Abschnitt von mindestens sechs Nukleotiden genau komplementär zu einer Teilsequenz einer Referenzsequenz umfasst, wobei der Abschnitt mindestens erste und zweite Abfragepositionen aufweist, welche ersten und zweiten Nukleotiden in der Referenzsequenz entsprechen,
ein erster Satz von drei mutierte Sonden, die jeweils identisch mit einer Sequenz sind, welche die Wildtypsonde oder eine Teilsequenz von mindestens sechs Nukleotiden davon einschließlich der ersten und zweiten Abfragepositionen umfasst, außer in der ersten Abfrageposition, welche durch ein abweichendes Nukleotid in jeder der drei mutierte Sonden und der Wildtypsonde besetzt ist;
ein zweiter Satz aus drei mutierte Sonden, die jeweils identisch mit einer Sequenz sind, welche die Wildtypsonde oder eine Teilsequenz von mindestens sechs Nukleotiden davon einschließlich der ersten und zweiten Abfragepositionen umfasst, außer in der zweiten Abfrageposition, die durch ein abweichendes Nukleotid in jeder der drei mutierte Sonden und der Wildtypsonde besetzt ist.

**18.** Array aus auf einem festen Träger immobilisierten Sonden, **dadurch gekennzeichnet, daß** es zwei Sondengruppen, von denen jede Gruppe wie in Anspruch 17 definiert ist, umfasst, wobei eine erste Gruppe eine Wildtypsonde, welche einen Abschnitt genau komplementär zu einer Teilsequenz von einer ersten Referenzsequenz umfasst, und eine zweite Gruppe eine Wildtypsonde umfasst, welche einen Abschnitt genau komplementär zu einer Teilsequenz von einer zweiten Referenzsequenz umfasst.

**19.** Verfahren zum Vergleichen einer Zielsequenz mit einer Referenzsequenz, **dadurch gekennzeichnet, daß** das Verfahren Schritte umfasst, bei denen man
Varianten einer Referenzsequenz identifiziert, die von der Referenzsequenz in mindestens einem Nukleotid abweichen;
jeder Variante eine Bezeichnung zuweist,
ein Array aus gepoolten Sonden bereitstellt, bei dem jeder Pool eine separate Zelle auf dem Array besetzt, wobei jeder Pool eine Sonde umfasst, die einen Abschnitt genau komplementär zu jeder Variantensequenz, der eine bestimmte Bezeichnung zugeordnet wurde, umfasst;
das Array mit einer Zielsequenz in Kontakt bringt, welche eine Variante von der Referenzsequenz umfasst;
die relativen Hybridisierungsintensitäten der Pools in dem Array gegenüber der Zielsequenz bestimmt;
die Zielsequenz aus den relativen Hybridisierungsintensitäten der Pools bestimmt.

**20.** Gepoolte Sonde, umfassend einen Abschnitt genau komplementär zu einer Teilsequenz einer Referenzsequenz außer an einer ersten Abfrageposition, die durch ein gepooltes Nukleotid N besetzt ist, einer zweiten Abfrageposition, die durch ein gepooltes Nukleotid ausgewählt aus der Gruppe von drei bestehend aus (1) M oder K; (2) R oder Y und (3) S oder W besetzt ist, und einer dritten Abfrageposition, die durch ein zweites gepooltes Nukleotid ausgewählt aus dieser Gruppe besetzt ist, umfasst, wobei das gepoolte Nukleotid, das die zweite Abfrageposition besetzt, ein Nukleotid komplementär zu einem entsprechenden Nukleotid der Referenzsequenz umfasst, wenn die gepoolte Sonde und Referenzsequenz maximal abgeglichen sind, und das gepoolte Nukleotid, das die dritte Abfrageposition besetzt, ein Nukleotid komplementär zu einem entsprechenden Nukleotid aus der Referenzsequenz umfasst, wenn die gepoolte Sonde und die Referenzsequenz maximal abgeglichen sind, worin N A, C, G oder T (U) ist, K G oder T(U) ist, M A oder C ist, R A oder G ist, Y C oder T(U) ist, W A oder T(U) ist und S G oder C ist.

**21.** Array aus auf einem festen Träger immobilisierten Oligonukleotidsonden, **dadurch gekennzeichnet, daß** das Array
erste, zweite und dritte Zellen jeweils besetzt von ersten, zweiten und dritten gepoolte Sonden wie in Anspruch 20 definiert umfasst,
vorausgesetzt, dass eine von den drei Abfragepositionen in jeder von den drei gepoolte Sonden mit dem gleichen entsprechenden Nukleotid in der Referenzsequenz abgeglichen ist, wobei diese Abfrageposition durch ein N in einer der gepoolte Sonden und einem abweichenden gepoolten Nukleotid in jeder der beiden anderen gepoolten Sonden besetzt ist,
worin N A, C, G oder T(U) ist, K G oder T(U) ist, M A oder C ist, R A oder G ist, Y C oder T(U) ist, W A oder T(U) ist und S G oder C ist.

**22.** Array nach Anspruch 21, **dadurch gekennzeichnet, daß** das Array ferner
vierte und fünfte Zellen jeweils besetzt von vierten und fünften gepoolten Sonden, wobei jede gepoolte Sonde wie in Anspruch 21 definiert ist, umfasst,
bei denen eine von den drei Abfragepositionen in den zweiten, dritten und vierten gepoolten Sonden mit dem

gleichen entsprechenden Nukleotid in der Referenzsequenz abgeglichen wurde, wobei diese Abfrageposition durch ein N in einer der gepoolten Sonden und ein abweichendes gepooltes Nukleotid in jeder der beiden anderen gepoolten Sonden besetzt ist,
bei denen eine von den drei Abfragepositionen in den dritten, vierten und fünften gepoolten Sonden mit dem gleichen entsprechenden Nukleotid in der Referenzsequenz abgeglichen ist, wobei diese Abfrageposition durch ein N in einer der gepoolten Sonden und ein abweichendes gepooltes Nukleotid in jeder der beiden anderen gepoolten Sonden besetzt ist.

**23.** Array aus Oligonukleotidsonden nach Anspruch 2, **dadurch gekennzeichnet, daß**
der erste Sondensatz eine Sonde umfasst, die erste und zweite Abschnitte umfasst, jede mit mindestens sechs Nukleotiden und genau komplementär zu ersten und zweiten Teilsequenzen der Referenzsequenzen, wobei die Abschnitte mindestens eine Abfrageposition entsprechend einem Nukleotid in der Referenzsequenz beinhalten, worin entweder (1) die ersten und zweiten Teilsequenzen nicht benachbart oder (2) die ersten und zweiten Teilsequenzen benachbart sind und die ersten und zweiten Abschnitte relativ zu dem Gegenstück der ersten und zweiten Teilsequenzen in der Referenzsequenz invertiert vorliegen; und
die zweiten, dritten und vierten Sondensätze entsprechende Sonden umfassen, die identisch mit einer Sequenz sind, welche die erste Sonde oder eine Teilsequenz davon umfassen, die mindestens sechs Nukleotide von jedem der ersten und zweiten Abschnitte umfasst, außer in der mindestens einen Abfrageposition, welche in jeder der Sonden abweicht.

**24.** Array von Oligonukleotidsonden nach Anspruch 2, **dadurch gekennzeichnet, daß**
der erste Sondensatz eine Sonde umfasst, die einen Abschnitt von mindestens sieben Nukleotiden genau komplementär zu einer Teilsequenz der Referenzsequenz außer einer oder zwei Positionen umfasst, wobei der Abschnitt eine Abfrageposition nicht an der einen oder den zwei Positionen beinhaltet;
die zweiten, dritten und vierten Sondensätze entsprechende Sonden umfassen, die jeweils identisch zu einer Sequenz sind, welche die Wildtypsonde oder eine Teilsequenz davon umfassen, welche die Abfrageposition und die eine oder die zwei Positionen beinhaltet, außer in der Abfrageposition, welche durch ein abweichendes Nukleotid in jeder der vier Sonden besetzt ist.

**25.** Array aus auf einem festen Träger immobilisierten Oligonukleotidsonden, **dadurch gekennzeichnet, daß** das Array mindestens zwei Sätze von Oligonukleotidsonden umfasst,

(1) einen ersten Sondensatz, der eine Vielzahl von Sonden umfasst, wobei jede Sonde einen Abschnitt genau komplementär zu einer Teilsequenz von mindestens sechs Nukleotiden einer Referenzsequenz außer in einer Abfrageposition umfasst,

(2) einen zweiten Sondensatz, der eine entsprechende Sonde für jede Sonde in dem ersten Sondensatz umfasst, wobei die entsprechende Sonde in dem zweiten Sondensatz identisch zu einer Sequenz ist, welche die entsprechende Sonde aus dem ersten Sondensatz oder eine Teilsequenz von mindestens sechs Nukleotiden davon umfasst, welche die Abfrageposition einschließt, außer wenn die Abfrageposition durch ein abweichendes Nukleotid in jeder der beiden entsprechenden Sonden und dem Gegenstück zu der Referenzsequenz besetzt ist,

wobei die Sonden in dem ersten Sondensatz jeweils mindestens drei Abfragepositionen entsprechend jedem von drei aufeinanderfolgenden Nukleotiden in der Referenzsequenz aufweisen.

**26.** Verfahren zum Vergleichen einer Zielnukleinsäure mit einer Referenzsequenz, die eine vorbestimmte Nukleotidsequenz umfasst, **dadurch gekennzeichnet, daß** das Verfahren die Hybridisierung der Zielnukleinsäure an ein Array aus auf einem festen Träger immobilisierten Oligonukleotidsonden wie in den Ansprüchen 1 - 18 oder 21 - 25 definiert umfasst.

**27.** Verfahren nach Anspruch 26, **dadurch gekennzeichnet, daß** die Zielsequenz ein substituiertes Nukleotid relativ zur Referenzsequenz in mindestens einer unbestimmten Position aufweist, und die relative spezifische Bindung der Sonden die Lokalisierung der Position und das Nukleotid, das diese Position in der Zielsequenz besetzt, angibt.

**28.** Verfahren nach Anspruch 26 oder 27, **dadurch gekennzeichnet, daß** die Referenzsequenz von dem humanen Immunschwäche-Virus stammt.

**29.** Verfahren nach den Ansprüchen 26 bis 28, **dadurch gekennzeichnet, daß** die Zielsequenz von einem zweiten humanen Immunschwäche-Virus stammt, z.B. worin die Zielsequenz ein substituiertes Nukleotid relativ zu der Referenzsequenz an mindestens einer Position aufweist, wobei die Substitution dem Erreger der erworbenen Immunschwäche AIDS Wirkstoffresistenz verleiht und die relative spezifische Bindung der Sonden die Substitution aufdeckt.

**30.** Verfahren einem der Ansprüche 26 bis 29, **dadurch gekennzeichnet, daß** die Bestimmung Schritte umfasst, bei denen man

(1) die relativen spezifischen Bindung von vier entsprechenden Sonden aus den ersten, zweiten, dritten und vierten Sondensätzen vergleicht;

(2) ein Nukleotides in der Zielsequenz als das Gegenstück zu der Abfrageposition von der Sonde mit der größten spezifischen Bindung bestimmt;

(3) (1) und (2) wiederholt bis jedes Nukleotid von Interesse in der Zielsequenz bestimmt worden ist.

**31.** Verfahren zum Vergleichen einer Zielnukleinsäure mit einer Referenzsequenz, die eine vorbestimmte Nukleotidsequenz umfasst, nach Anspruch 26 **dadurch gekennzeichnet, daß** man
die Zielsequenz auf ein Array nach den Ansprüchen 13 oder 14 hybridisiert und das Verfahren ferner Schritte umfasst, bei denen man
die Sonden in der ersten Gruppe bestimmt, welche relativ zueinander an die Zielsequenz hybridisieren, wobei die relative spezifische Bindung der Sonden angibt, ob die Zielsequenz gleich oder abweichend von der ersten Referenzsequenz ist;
die Sonden in der zweiten Gruppe bestimmt, welche relativ zueinander an die Zielsequenz hybridisieren, wobei die relative spezifische Bindung der Sonden angibt, ob die Zielsequenz die gleiche oder abweichend von der zweiten Referenzsequenz ist.

**32.** Verfahren zum Vergleichen einer Zielnukleinsäure mit einer Referenzsequenz, die eine vorbestimmte Nukleotidsequenz umfasst, nach Anspruch 26 dadurch gekennzeichnet, daß die Zielsequenz auf ein Array von auf einem festen Träger immobilisierten Oligonukliotidsonden wie in den Ansprüchen 1 - 18 oder 21 - 25 definiert hybridisiert wird und das Verfahren ferner Schritte umfaßt, bei denen man

(a) die Referenzsequenz an das Array von Oligonukleotidsonden hybridisiert;

(b) die Sonden bestimmt, welche relativ zu den anderen in dem Array spezifisch an die Referenzsequenz binden;

(c) die Sonden bestimmt, welche relativ zu den anderen in dem Array spezifisch an die Zielsequenz binden;

wobei die relative spezifische Bindung der Sonden an die Referenz- und Zielsequenz angibt, ob die Referenzsequenz die gleiche oder verschieden von der Zielsequenz ist.

**33.** Verfahren nach Anspruch 32, **dadurch gekennzeichnet, daß** die Referenzsequenz eine erste Markierung und eine zweite Referenzsequenz eine zweite Markierung aufweist, welche sich von der ersten Markierung unterscheidet, und die Schritte (a) und (c) gleichzeitig durchgeführt werden.

**34.** Array nach einem der Ansprüche 1 bis 18 oder 21 bis 25, **dadurch gekennzeichnet, daß** die Referenzsequenz von dem humanen Immunschwäche-Virus stammt, d. h. ein reverse Transkriptase-Gen oder ein Protease-Gen des humanen Immunschwäche-Virus ist.

**35.** Array nach Anspruch 34, **dadurch gekennzeichnet, daß** die Referenzsequenz ein Reverse-Transkriptase-Gen vollständiger Länge ist.

**36.** Array nach Anspruch 13 oder 14, **dadurch gekennzeichnet, daß** die erste Referenzsequenz von einem Reverse-Transkriptase-Gen eines humanen Immunschwäche-Virus ist, und entweder die zweite Referenzsequenz eine Teilsequenz der ersten Referenzsequenz mit einer Substitution von mindestens einem Nukleotid ist, welche einem humanen Immunschwäche-Virus, welcher die zweite Referenzsequenz umfasst, Wirkstoffresistenz verleiht, oder

die zweite Referenzsequenz aus einer 16S-RNA oder DNA, die für die 16S-RNA kodiert, aus einem pathogenen Mikroorganismus ist.

**37.** Array nach Anspruch 13 oder 14, **dadurch gekennzeichnet, daß** die ersten und zweiten Referenzsequenzen aus dem Reverse-Transkriptase-Gen aus einem ersten oder zweiten Stamm des humanen Immunschwäche-Virus sind.

**38.** Array nach einem der Ansprüche 1 - 18 oder 21 - 25, **dadurch gekennzeichnet, daß** die Referenzsequenz von einem CFTR-Gen, einem p53-Gen, einem hMLH1-Gen, einem MSH2-Gen, einem mitochondrialem Genom ist.

**39.** Array nach Anspruch 38, **dadurch gekennzeichnet, daß** die Referenzsequenz ein D-Loop-Bereich oder ein P450-Gen ist.

## Revendications

**1.** Arrangement de sondes oligonucléotidiques immobilisées sur un support solide, par exemple liées au support par l'intermédiaire d'un espaceur, l'arrangement comprenant au moins deux ensembles de sondes oligonucléotidiques,

(1) un premier ensemble de sondes comprenant une pluralité de sondes, chaque sonde comprenant un segment d'au moins six nucléotides exactement complémentaires d'une sous-séquence d'une séquence de référence, le segment incluant au moins une position d'interrogation complémentaire d'un nucléotide correspondant dans la séquence de référence,
(2) un second ensemble de sondes comprenant une sonde correspondante pour chaque sonde dans le premier ensemble de sondes, la sonde correspondante dans le second ensemble de sondes étant identique à une séquence comprenant la sonde correspondante du premier ensemble de sondes ou une sous-séquence d'au moins six de ses nucléotides qui inclut la/au moins une position d'interrogation, excepté que la/au moins une position d'interrogation est occupée par un nucléotide différent dans chacune des deux sondes correspondantes du premier et du second ensembles de sondes ;

dans lequel les sondes dans le premier ensemble de sondes ont au moins deux positions d'interrogation correspondant respectivement à chacun de deux nucléotides contigus dans la séquence de référence.

**2.** Arrangement de sondes oligonucléotidiques selon la revendication 1, comprenant en outre un troisième et un quatrième ensembles de sondes, dans lequel,
le deuxième, le troisième et le quatrième ensembles de sondes comprennent chacun une sonde correspondante pour chaque sonde dans le premier ensemble de sondes, les sondes dans le deuxième, le troisième et le quatrième ensembles de sondes étant identiques à une séquence comprenant la sonde correspondante du premier ensemble de sondes ou une sous-séquence d'au moins six de ses nucléotides qui inclut la/au moins une position d'interrogation, excepté que la/au moins une position d'interrogation est occupée par un oligonucléotide différent dans chacune des quatre sondes correspondantes des quatre ensembles de sondes.

**3.** Arrangement selon revendication 1 ou 2, l'arrangement comprenant entre 1 000 et 100 000 sondes.

**4.** Arrangement selon l'une quelconque des revendications 1 à 3, dans lequel les sondes du premier ensemble de sondes comprennent un segment de 9 à 21 nucléotides exactement complémentaire d'une sous-séquence d'une séquence de référence, le segment incluant au moins une position d'interrogation complémentaire d'un nucléotide correspondant dans la séquence de référence.

**5.** Arrangement selon l'une quelconque des revendications 1 à 4, comprenant en outre un cinquième ensemble de sondes comprenant une sonde correspondante pour chaque sonde dans le premier ensemble de sondes, la sonde correspondante du cinquième ensemble de sondes étant identique à une séquence comprenant la sonde correspondante du premier ensemble de sondes ou une sous-séquence d'au moins six de ses nucléotides qui inclut la/au moins une position d'interrogation, excepté que la/au moins une position d'interrogation est délétée dans la sonde correspondante du cinquième ensemble de sondes.

**6.** Arrangement selon l'une quelconque des revendications 1 à 5, comprenant en outre un sixième ensemble de sondes comprenant une sonde correspondante pour chaque sonde dans le premier ensemble de sondes, la sonde

correspondante du sixième ensemble de sondes étant identique à une séquence comprenant la sonde correspondante du premier ensemble de sondes ou une sous-séquence d'au moins six de ses nucléotides qui inclut la/au moins une position d'interrogation, excepté qu'un nucléotide supplémentaire est inséré à proximité de la/au moins une position d'interrogation dans la sonde correspondante du premier ensemble de sondes.

**7.** Arrangement selon l' une quelconque des revendications 1 à 6, dans lequel, soit :

(a) le premier ensemble de sondes a au moins 50 positions d'interrogation correspondant respectivement à chacun des 50 nucléotides contigus dans une séquence de référence ; soit
(b) le premier ensemble de sondes a une position d'interrogation correspondant à chacun des nucléotides dans la séquence de référence.

**8.** Arrangement selon l'une quelconque des revendications 1 à 7, comprenant en outre un cinquième, un sixième et un septième ensembles de sondes, dans lequel :

le segment de chaque sonde dans le premier ensemble inclut au moins deux positions d'interrogation correspondant chacune à un nucléotide dans la séquence de référence,
le deuxième, le troisième et le quatrième ensembles de sondes comprennent chacun une sonde correspondante pour chaque sonde dans le premier ensemble de sondes, les sondes correspondantes dans le deuxième, le troisième et le quatrième ensembles de sondes étant identiques à une séquence comprenant la sonde correspondante du premier ensemble de sondes ou une sous-séquence d'au moins six de ses nucléotides qui comprend une première position d'interrogation, excepté que la première position d'interrogation est occupée par un nucléotide différent dans chacune des quatre sondes correspondantes des quatre ensembles de sondes ;
le cinquième, le sixième et le septième ensembles de sondes comprenant chacun une sonde correspondante pour chaque sonde dans le premier ensemble de sondes, les sondes dans le cinquième, le sixième et le septième ensembles de sondes étant identiques à une séquence comprenant la sonde correspondante du premier ensemble de sondes ou une sous-séquence d'au moins six de ses nucléotides qui inclut une seconde position d'interrogation, excepté que la seconde position d'interrogation est occupée par un nucléotide différent dans chacune des quatre sondes correspondantes des quatre ensembles de sondes.

**9.** Arrangement selon l'une quelconque des revendications 1 à 8, dans lequel chaque sonde dans le premier ensemble de sondes comprend un second segment d'au moins six nucléotides exactement complémentaire d'une seconde sous-séquence de la séquence de référence, et les sondes du deuxième, du troisième et du quatrième ensembles de sondes comprennent la sonde correspondante du premier ensemble de sondes ou l'une de ses sous-séquences comprenant le premier et le second segments sauf dans la/au moins une position d'interrogation.

**10.** Arrangement selon l'une quelconque des revendications 1 à 9, comprenant en outre :

un cinquième ensemble de sondes comprenant au moins une sonde comprenant un segment d'au moins sept nucléotides exactement complémentaire d'une sous-séquence de la séquence de référence sauf en une ou deux positions, le segment incluant au moins une position d'interrogation correspondant à un nucléotide dans la séquence de référence qui ne se trouve pas à l' une ou aux deux positions, dans lequel, soit (1) la première et la seconde sous-séquences sont non contiguës, soit (2) la première et la seconde sous-séquences sont contiguës et le premier et le second segments sont inversés par rapport au complémentaire de la première et de la seconde sous-séquences dans la séquence de référence,
un sixième, un septième et un huitième ensembles de sondes, comprenant chacun une sonde pour chaque sonde dans le cinquième ensemble de sondes, les sondes correspondantes du sixième, du septième et du huitième ensembles de sondes étant identiques à une séquence comprenant la sonde correspondante du cinquième ensemble de sondes ou une sous-séquence d'au moins neuf de ses nucléotides incluant la/au moins une position d'interrogation et l'une ou les deux positions, sauf dans la/au moins une position d'interrogation, qui est occupée par un nucléotide différent dans chacune des quatre sondes.

**11.** Arrangement selon l'une quelconque des revendications 1 à 10, dans lequel la première et la seconde sous-séquences sont séparées par un ou deux nucléotides dans la séquence de référence.

**12.** Arrangement selon l'une quelconque des revendications 1 à 11, dans lequel les sondes sont arrangées sur le substrat de telle sorte que le premier ensemble de sondes soit arrangé en une rangée à travers le substrat dans

un ordre reflétant le chevauchement entre les sondes et la séquence de référence, et d'autres ensembles de sondes sont arrangés en colonnes par rapport aux sondes dans le premier ensemble, de sorte que les sondes ayant la même position d'interrogation soient dans la même colonne et de sorte que chaque colonne comprenne au moins quatre sondes.

**13.** Arrangement de sondes oligonucléotidiques immobilisées sur un support solide, l'arrangement comprenant au moins une paire de premier et second groupes de sondes, chaque groupe comprenant un premier et un second ensembles de sondes oligonucléotidiques comme les définit la revendication 1 ;
dans lequel chaque sonde dans le premier ensemble de sondes du premier groupe est exactement complémentaire d'une sous-séquence d'une première séquence de référence, et chaque sonde dans le premier ensemble de sondes du second groupe est exactement complémentaire d'une sous-séquence d'une seconde séquence de référence.

**14.** Arrangement selon la revendication 13, dans lequel la seconde séquence de référence est une forme mutée de la première séquence de référence.

**15.** Arrangement selon la revendication 13 ou 14, dans lequel chaque groupe comprend en outre un troisième et un quatrième ensembles de sondes, comprenant chacun une sonde correspondante pour chaque sonde dans le premier ensemble de sondes, les sondes dans le deuxième, le troisième et le quatrième ensembles de sondes étant identiques à une séquence comprenant la sonde correspondante du premier ensemble de sondes ou une sous-séquence d'au moins six de ses nucléotides qui inclut la position d'interrogation, excepté que la position d'interrogation est occupée par un nucléotide différent dans chacune des quatre sondes correspondantes des quatre ensembles de sondes.

**16.** Arrangement selon l'une quelconque des revendications 13 à 15, dans lequel l'arrangement comprend au moins cinq paires de premier et second groupes de sondes, dans lequel les sondes dans les premiers ensembles de sondes des premiers groupes des cinq paires sont exactement complémentaires de sous-séquences de cinq premières séquences de référence respectives différentes.

**17.** Arrangement de sondes oligonucléotidiques immobilisées sur un support solide, l'arrangement comprenant au moins un groupe de sondes comprenant :

une sonde sauvage comprenant un segment d'au moins six nucléotides exactement complémentaires d'une sous-séquence d'une séquence de référence, le segment ayant au moins une première et une seconde positions d'interrogation correspondant au premier et au second nucléotides dans la séquence de référence,
un premier ensemble de trois sondes mutantes, chacune identique à une séquence comprenant la sonde sauvage ou une sous-séquence d'au moins six de ses nucléotides incluant la première et la seconde positions d'interrogation, sauf dans la première position d'interrogation, qui est occupée par un nucléotide différent dans chacune des trois sondes mutantes et la sonde sauvage ;
un second ensemble de trois sondes mutantes, chacune identique à une séquence comprenant la séquence sauvage ou une sous-séquence d'au moins six de ses nucléotides incluant la première et la seconde positions d'interrogation, sauf dans la seconde position d'interrogation, qui est occupée par un nucléotide différent dans chacune des trois sondes mutantes et la sonde sauvage.

**18.** Arrangement de sondes immobilisées sur un support solide comprenant deux groupes de sondes, chaque groupe étant tel que défini dans la revendication 17, un premier groupe comprenant une sonde sauvage comprenant un segment exactement complémentaire d'une sous-séquence d'une première séquence de référence et un second groupe comprenant une sonde sauvage comprenant un segment exactement complémentaire d'une sous-séquence d'une seconde séquence de référence.

**19.** Procédé de comparaison d'une séquence cible avec une séquence de référence, le procédé comprenant:

l'identification des variants d'une séquence de référence différant de la séquence de référence par au moins un nucléotide ;
l'attribution à chaque variant une désignation,
la fourniture d' un arrangement de pools de sondes, chaque pool occupant une cellule séparée de l'arrangement, dans lequel chaque pool comprend une sonde comprenant un segment exactement complémentaire de chaque séquence variante à laquelle on a assigné une désignation particulière,

la mise en contact de l' arrangement avec une séquence cible comprenant un variant de la séquence de référence,
la détermination des intensités d'hybridation relatives des pools dans l'arrangement à la séquence cible,
la détermination de la séquence cible à partir des intensités d'hybridation relatives des pools.

**20.** Sonde en pool comprenant un segment exactement complémentaire d'une sous-séquence d'une séquence de référence excepté à une première position d'interrogation occupée par un nucléotide N en pool, une deuxième position d'interrogation occupée par un nucléotide en pool choisi dans le groupe de trois constitué de (1) M ou K, (2) R ou Y et (3) S ou W, et une troisième position d'interrogation occupée par un second nucléotide en pool choisi dans le groupe, dans lequel le nucléotide en pool occupant la deuxième position d'interrogation comprend un nucléotide complémentaire d'un nucléotide correspondant de la séquence de référence lorsque la sonde en pool et la séquence de référence sont alignées au maximum et le nucléotide en pool occupant la troisième position d'interrogation comprend un nucléotide complémentaire d'un nucléotide correspondant de la séquence de référence lorsque la sonde en pool et la séquence de référence sont alignées au dans laquelle, où N représente A, C, G ou T(U), K représente G ou T(U), M représente A ou C, R représente A ou G, Y représente C ou T(U), W représente A ou T(U) et S représente G ou C.

**21.** Arrangement de sondes oligonucléotidiques immobilisées sur un support solide, l'arrangement comprenant :

une première, une deuxième et une troisième cellules respectivement occupées par une première, une deuxième et
une troisième sondes en pool tel que défini par la revendication 20,
à condition que l'une des trois positions d'interrogation dans chacune des trois sondes en pool soit alignée avec le même nucléotide correspondant dans la séquence de référence, cette position d'interrogation étant occupée par un N dans l'une des sondes en pool, et un nucléotide en pool différent dans chacune des deux autres sondes en pool, où N représente A, C, G ou T(U), K représente G ou T(U), M représente A ou C, R représente A ou G, Y représente C ou T(U), W représente A ou T(U) et S représente G ou C.

**22.** Arrangement selon la revendication 21, dans lequel l'arrangement comprenant en outre :

une quatrième et une cinquième cellules respectivement occupées par une quatrième et une cinquième sondes en pool, chaque sonde en pool étant telle que définie dans la revendication 21,

dans lequel l'une des trois positions d'interrogation dans la deuxième, la troisième et la quatrième sondes en pool est alignée avec le même nucléotide correspondant dans la séquence de référence, cette position d'interrogation étant occupée par un N dans l'un des sondes en pool et par un nucléotide en pool différent dans chacune des autres sondes en pool,
dans lequel une des trois positions d'interrogation dans les troisième, quatrième et cinquième sondes en pool, est alignée avec le même nucléotide correspondant dans la séquence de référence, cette position d'interrogation étant occupée par un N dans l'une des sondes en pool, et par un nucléotide en pool différent dans chacune des deux autres sondes en pool.

**23.** Arrangement de sondes oligonucléotidiques selon la revendication 2, dans lequel
le premier ensemble de sondes comprend une sonde comprenant un premier et un second segments, chacun d'au moins six nucléotides et exactement complémentaire d'une première et d'une seconde sous-séquences d'une séquence de référence, les segments incluant au moins une position d'interrogation correspondant à un nucléotide dans la séquence de référence, où soit (1) la première et la seconde sous-séquences sont non contiguës, soit (2) la première et la seconde sous-séquences sont contiguës et le premier et le second segment sont inversés par rapport au complément de la première et de la seconde sous-séquences dans la séquence de référence ; et
le deuxième, le troisième et le quatrième ensembles de sondes comprennent des sondes correspondantes, identiques à une séquence comprenant la première sonde ou l'une de ses sous-séquences comprenant au moins six nucléotides de chacun des premier et second segments, sauf dans la/au moins une position interrogation, qui diffère dans chacune des sondes.

**24.** Arrangement de sondes oligonucléotidiques selon la revendication 2, dans lequel,
le premier ensemble de sondes comprend une sonde comprenant un segment d'au moins 7 nucléotides exactement complémentaires d'une sous-séquence d'une séquence de référence sauf en une ou deux positions, le segment incluant une position d'interrogation qui ne se trouve pas à l'une ou aux deux positions ;

les deuxième, troisième et quatrième ensembles de sondes comprennent des sondes correspondantes, chacune identique à une séquence comprenant la sonde sauvage ou l'une de ses sous-séquences incluant la position d'interrogation et l'une ou les deux positions, sauf dans la position d'interrogation, qui est occupée par un nucléotide différent dans chacune des quatre sondes.

**25.** Arrangement de sondes oligonucléotidiques immobilisées sur un support solide, l'arrangement comprenant au moins deux ensembles de sondes oligonucléotidiques,

(1) un premier ensemble de sondes comprenant une pluralité de sondes, chaque sonde comprenant un segment exactement complémentaires d'une sous-séquence d'au moins six nucléotides d'une séquence de référence sauf en une position d'interrogation ;
(2) un second ensemble de sondes comprenant une sonde correspondante pour chaque sonde dans le premier ensemble de sondes, la sonde correspondante dans le second ensemble de sondes étant identique à une séquence comprenant la sonde correspondante du premier ensemble de sondes ou à une sous-séquence d'au moins six de ses nucléotides qui inclut la/au moins une position d'interrogation, excepté au moins une position d'interrogation est occupée par un nucléotide différent dans chacune des deux sondes correspondantes le complémentaire de la séquence de référence ;

dans lequel les sondes dans le premier ensemble de sondes ont au moins trois positions d'interrogation correspondant respectivement à chacun des trois nucléotides contigus dans la séquence de référence.

**26.** Procédé de comparaison d'un acide nucléique cible avec une séquence de référence comprenant une séquence de nucléotides prédéterminée, le procédé comprenant l'hbridation de l'acide nucléotidique cible à un arrangement de sondes oligonucléotidiques immobilisée sur un support solide tel que défini dans l'une quelconque des revendications 1 à 18 ou 21 à 25.

**27.** Procédé selon la revendication 26, dans lequel la séquence cible a un nucléotide substitué par rapport à la séquence de référence en au moins une position indéterminée, et la liaison spécifique relative des sondes indique la localisation de la position et le nucléotide occupant la position dans la séquence cible.

**28.** Procédé selon la revendication 26 ou 27, dans lequel la séquence de référence provient d'un virus de l'immunodéficience humaine.

**29.** Procédé selon l'une quelconque des revendications 26 à 28, dans lequel la séquence cible provient d'un second virus de l'immunodéficience humaine, par exemple, dans lequel la séquence cible a un nucléotide substitué par rapport à la séquence de référence en au moins une position, la substitution conférant une résistance à un médicament au virus de l'immunodéficience humaine, et la liaison spécifique relative des sondes révèle la substitution.

**30.** Procédé selon l'une quelconque des revendications 26 à 29, dans lequel la détermination comprend :

(1) la comparaison de la liaison spécifique relative de quatre sondes correspondantes d'un premier, d'un deuxième, d'un troisième et quatrième ensembles de sondes ;
(2) l'attribution d' un nucléotide dans la séquence cible comme le complémentaire de la position d'interrogation de la sonde ayant la liaison spécifique la plus élevée ;
(3) la répétition (1) et (2) jusqu'à ce que chaque nucléotide d'intérêt dans la séquence cible ait été attribué.

**31.** Procédé de comparaison d'un acide nucléotidique cible avec une séquence de référence comprenant une séquence de nucléotides prédéterminée, selon la revendication 26, dans lequel :

la séquence cible est hybridée à un arrangement selon la revendication 13 ou la revendication 14, et le procédé comprend en outre
la détermination des sondes dans le premier groupe, les unes par rapport aux autres, qui s'hybrident à la séquence cible, la liaison spécifique relative des sondes indiquant si la séquence cible est la même ou différente de la première séquence de référence ;
la détermination des sondes dans le second groupe, les unes par rapport aux autres, qui s'hybrident à la séquence cible, la liaison spécifique relative des sondes indiquant si la séquence cible est la même ou différente de la seconde séquence de référence.

**32.** Procédé de comparaison d'un acide nucléique cible avec une séquence de référence comprenant une séquence de nucléotides prédéterminée, selon la revendication 26, dans lequel la séquence cible est hybridée à un arrangement de sondes oligonucléotidiques immobilisées sur un support solide tel que défini dans l'une quelconque des revendications 1 à 18 ou 21 à 25, et le procédé comprend en outre

(a) l'hybridation de la séquence de référence à l'arrangement de sondes oligonucléotidiques ;
(b) la détermination des sondes, les unes par rapport aux autre, dans l'arrangement, qui se lient spécifiquement à la séquence cible ;
(c) déterminer quelles sondes, les unes par rapport aux autres, dans l'arrangement, se lient spécifiquement à la séquence cible ;

dans lequel la liaison spécifique relative des sondes à la séquence de référence et cible indique si la séquence de référence est la même ou différente de la séquence cible.

**33.** Procédé selon la revendication 32, dans lequel la séquence de référence a un premier marqueur et une seconde séquence de référence ayant un second marqueur différent du premier marqueur, les étapes (a) et (c) étant mises en oeuvre simultanément.

**34.** Arrangement selon l'une quelconque des revendications 1 à 18 ou 21 à 25, dans lequel la séquence de référence provient d'un virus de l'immunodéficience humaine, par exemple un gène de transcriptase inverse ou un gène de protéase du virus de l'immunodéficience humaine.

**35.** Arrangement selon la revendication 34, dans lequel la séquence de référence est un gène de transcriptase inverse de longueur totale.

**36.** Arrangement selon la revendication 13 ou 14, dans lequel la première séquence de référence provient d'un gène de transcriptase inverse d'un virus de l'immunodéficience humaine, et soit la seconde séquence de référence comprend une sous-séquence de la première séquence de référence avec une substitution d'au moins un nucléotide qui confère une résistance à un médicament à un virus de l'immunodéficience humaine comprenant la seconde séquence de référence, soit la seconde de référence provient d'un ARN 16S, ou d'un ADN codant l'ARN 16S, d'un microorganisme pathogène.

**37.** Arrangement selon la revendication 13 ou la revendication 14, dans lequel la première et la seconde séquences de référence proviennent d'un gène de transcriptase inverse d'une première et d'une seconde souches de virus de l'immunodéficience humaine.

**38.** Arrangement selon l'une quelconque des revendications 1 à 18 ou 21 à 25, dans lequel la séquence de référence provient d'un gène CFTR, un gène p53, un gène hMLH1, d'un gène MSH2, un génome mitochondrial.

**39.** Arrangement selon la revendication 38, dans lequel la séquence de référence est une région de boucle D, ou un gène P450.

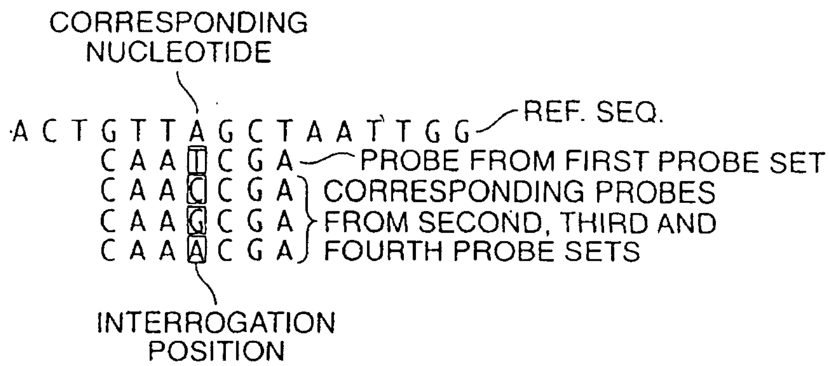
CORRESPONDING NUCLEOTIDE
A C T G T T A G C T A A T T G G
REF. SEQ.
C A A T C G A
PROBE FROM FIRST PROBE SET
C A A C C G A
C A A G C G A
C A A A C G A
CORRESPONDING PROBES FROM SECOND, THIRD AND FOURTH PROBE SETS
INTERROGATION POSITION

*FIG. 1*

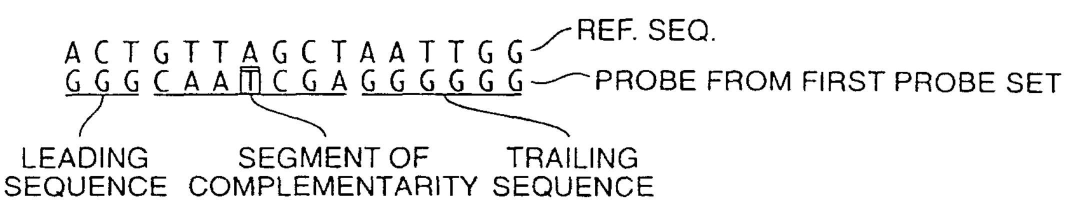
A C T G T T A G C T A A T T G G
REF. SEQ.
G G G C A A T C G A G G G G G G
PROBE FROM FIRST PROBE SET
LEADING SEQUENCE
SEGMENT OF COMPLEMENTARITY
TRAILING SEQUENCE

*FIG. 2*

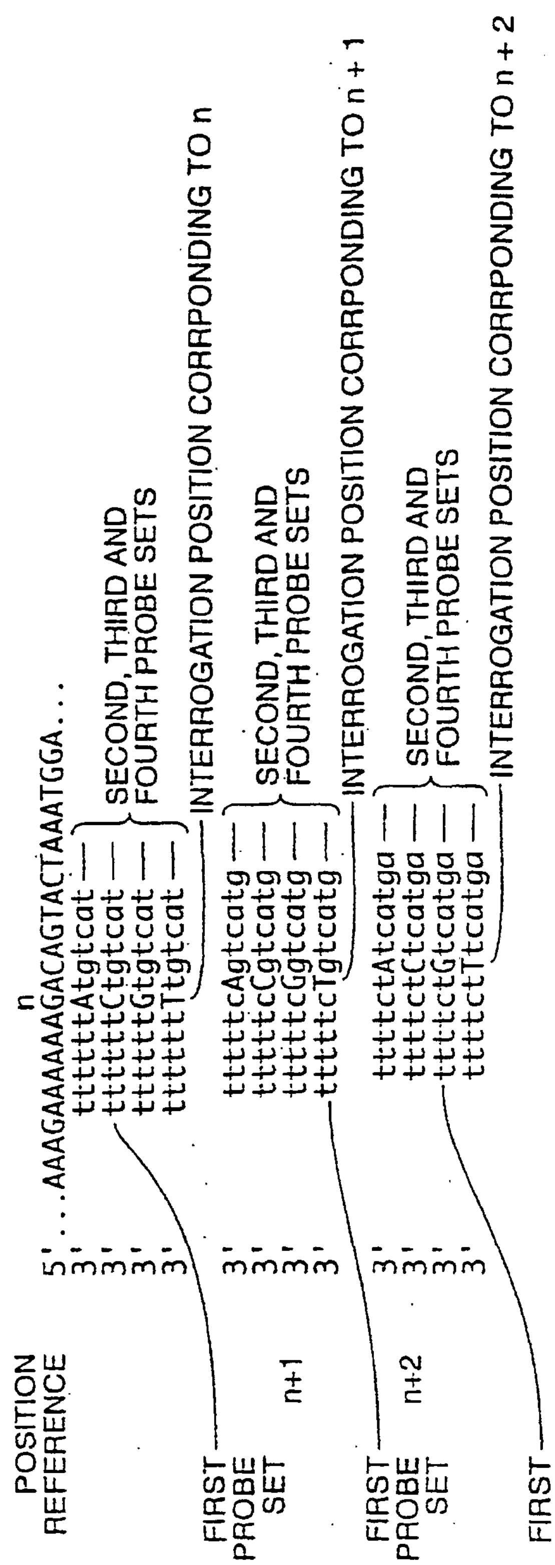
POSITION
REFERENCE
n
5' . . . AAAGAAAAAGACAGTACTAAATGGA . . .
3' ttttttAtgtcat
3' ttttttCtgtcat
3' ttttttGtgtcat
3' ttttttTtgtcat
SECOND, THIRD AND FOURTH PROBE SETS
INTERROGATION POSITION CORRPONDING TO n
FIRST PROBE SET
n+1
3' tttttcAgtcatg
3' tttttcCgtcatg
3' tttttcGgtcatg
3' tttttcTgtcatg
SECOND, THIRD AND FOURTH PROBE SETS
INTERROGATION POSITION CORRPONDING TO n + 1
FIRST PROBE SET
n+2
3' ttttctAtcatga
3' ttttctCtcatga
3' ttttctGtcatga
3' ttttctTtcatga
SECOND, THIRD AND FOURTH PROBE SETS
INTERROGATION POSITION CORRPONDING TO n + 2
FIRST PROBE SET

*FIG. 3*

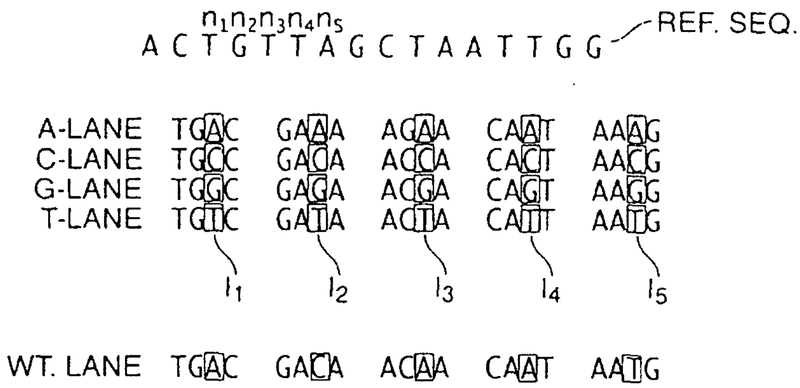

$n_1n_2n_3n_4n_5$
A C T G T T A G C T A A T T G G
REF. SEQ.
A-LANE TGAC GAAA AGAA CAAT AAAG
C-LANE TGCC GACA ACCA CACT AACG
G-LANE TGGC GAGA ACGA CAGT AAGG
T-LANE TGTC GATA ACTA CATT AATG
$l_1$ $l_2$ $l_3$ $l_4$ $l_5$
WT. LANE TGAC GACA ACAA CAAT AATG


FIG. 4

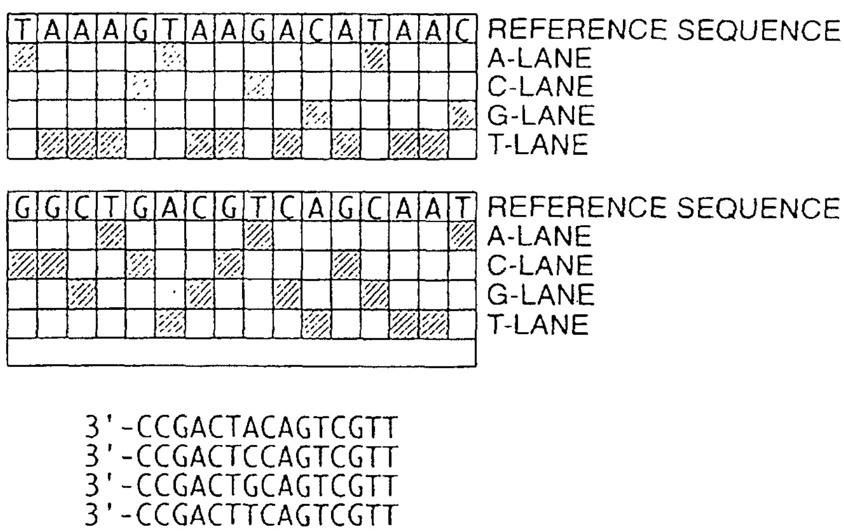

T A A A G T A A G A C A T A A C
REFERENCE SEQUENCE
A-LANE
C-LANE
G-LANE
T-LANE
G G C T G A C G T C A G C A A T
REFERENCE SEQUENCE
A-LANE
C-LANE
G-LANE
T-LANE
3'-CCGACTACAGTCGTT
3'-CCGACTCCAGTCGTT
3'-CCGACTGCAGTCGTT
3'-CCGACTTCAGTCGTT


FIG. 5

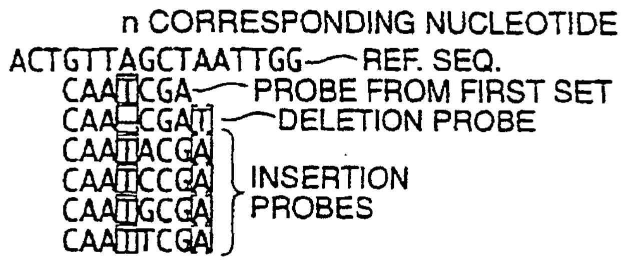
n CORRESPONDING NUCLEOTIDE
ACTGTTAGCTAATTGG
REF. SEQ.
CAATCGA
PROBE FROM FIRST SET
CAA-CGAT
DELETION PROBE
CAATACGA
CAATCCGA
CAATGCGA
CAATTCGA
INSERTION PROBES

FIG. 6

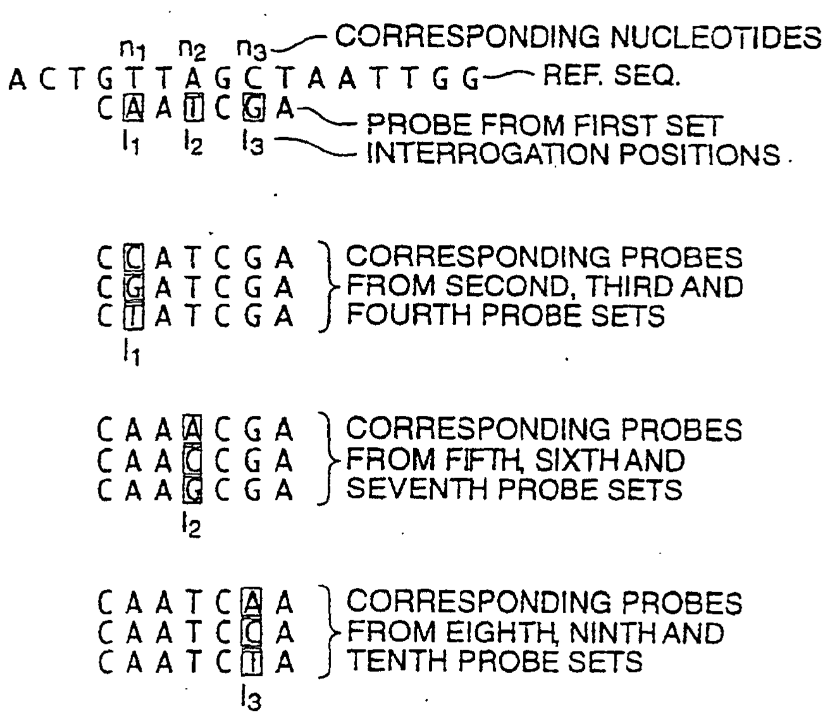
n1 n2 n3
CORRESPONDING NUCLEOTIDES
A C T G T T A G C T A A T T G G
REF. SEQ.
C A A T C G A
PROBE FROM FIRST SET
I1 I2 I3
INTERROGATION POSITIONS
C C A T C G A
C G A T C G A
C T A T C G A
CORRESPONDING PROBES FROM SECOND, THIRD AND FOURTH PROBE SETS
I1
C A A A C G A
C A A C C G A
C A A G C G A
CORRESPONDING PROBES FROM FIFTH, SIXTH AND SEVENTH PROBE SETS
I2
C A A T C A A
C A A T C C A
C A A T C T A
CORRESPONDING PROBES FROM EIGHTH, NINTH AND TENTH PROBE SETS
I3

FIG. 7

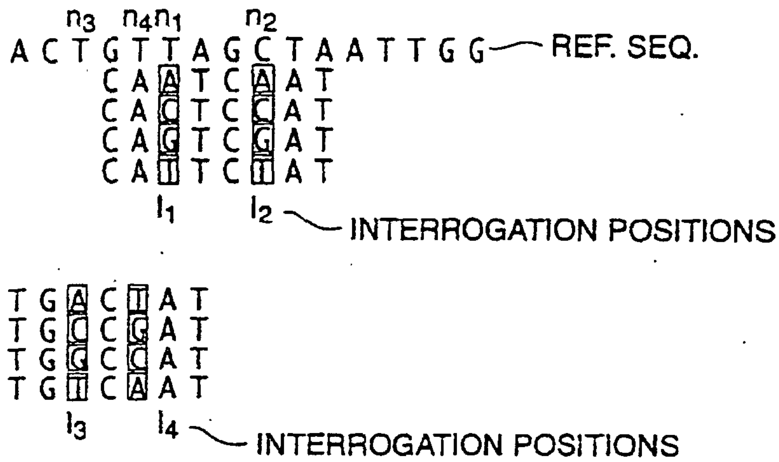
n3 n4n1 n2
A C T G T T A G C T A A T T G G
REF. SEQ.
C A A T C A A T
C A C T C C A T
C A G T C G A T
C A T T C T A T
I1 I2
INTERROGATION POSITIONS
T G A C T A T
T G C C G A T
T G G C C A T
T G T C A A T
I3 I4
INTERROGATION POSITIONS

*FIG. 8*

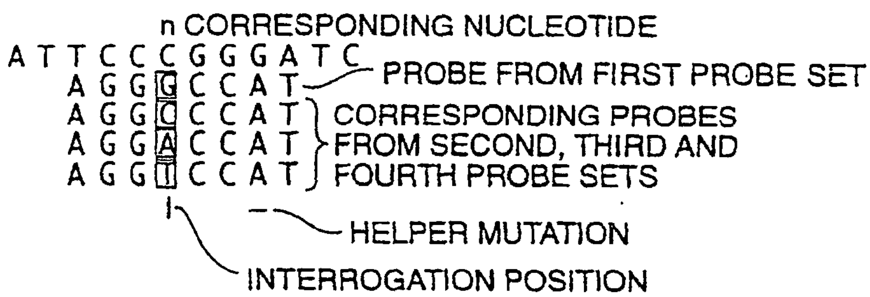
n CORRESPONDING NUCLEOTIDE
A T T C C C G G G A T C
A G G G C C A T
PROBE FROM FIRST PROBE SET
A G G C C C A T
A G G A C C A T
A G G T C C A T
CORRESPONDING PROBES FROM SECOND, THIRD AND FOURTH PROBE SETS
HELPER MUTATION
I
INTERROGATION POSITION

*FIG. 9*

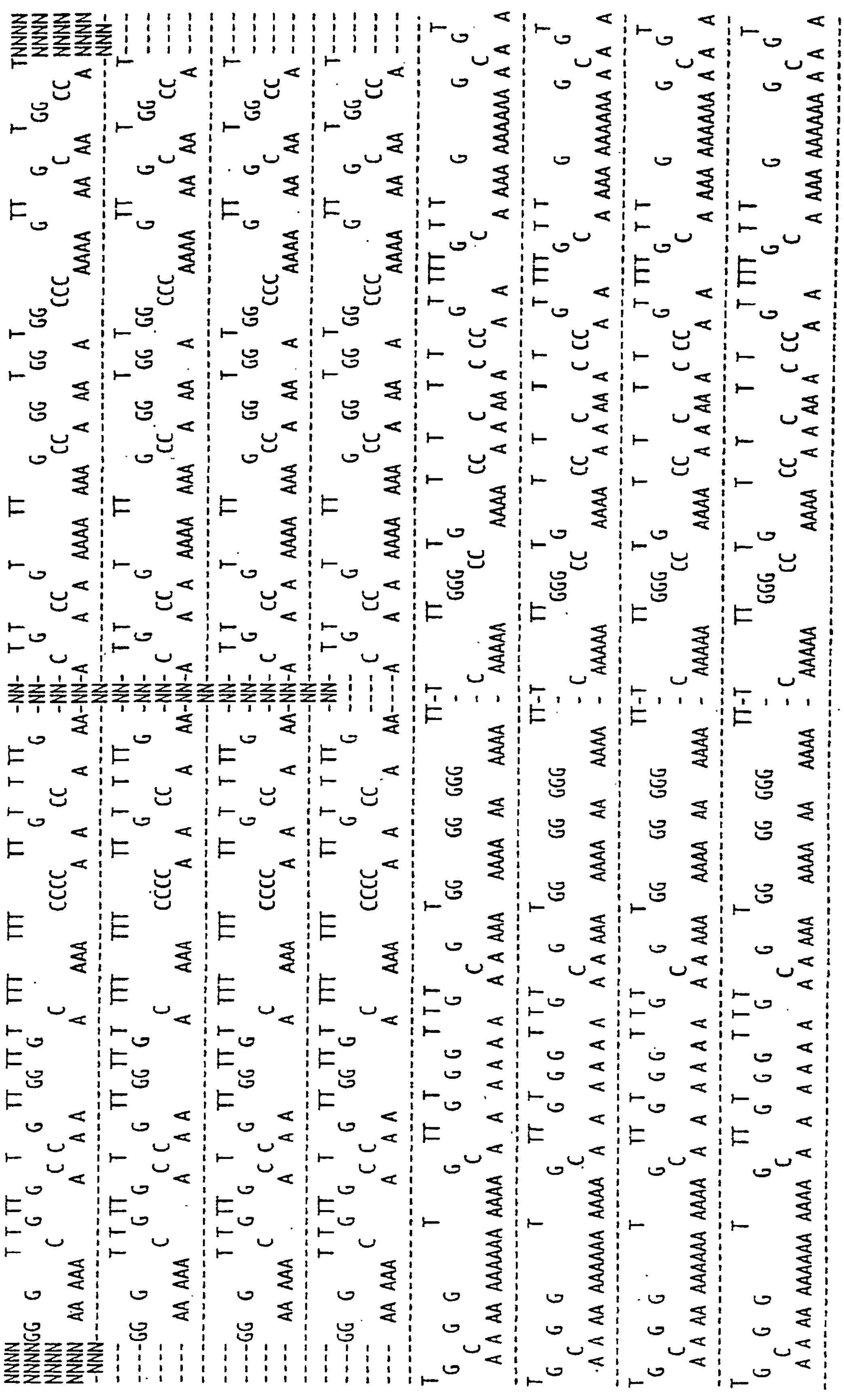

FIG. 10A

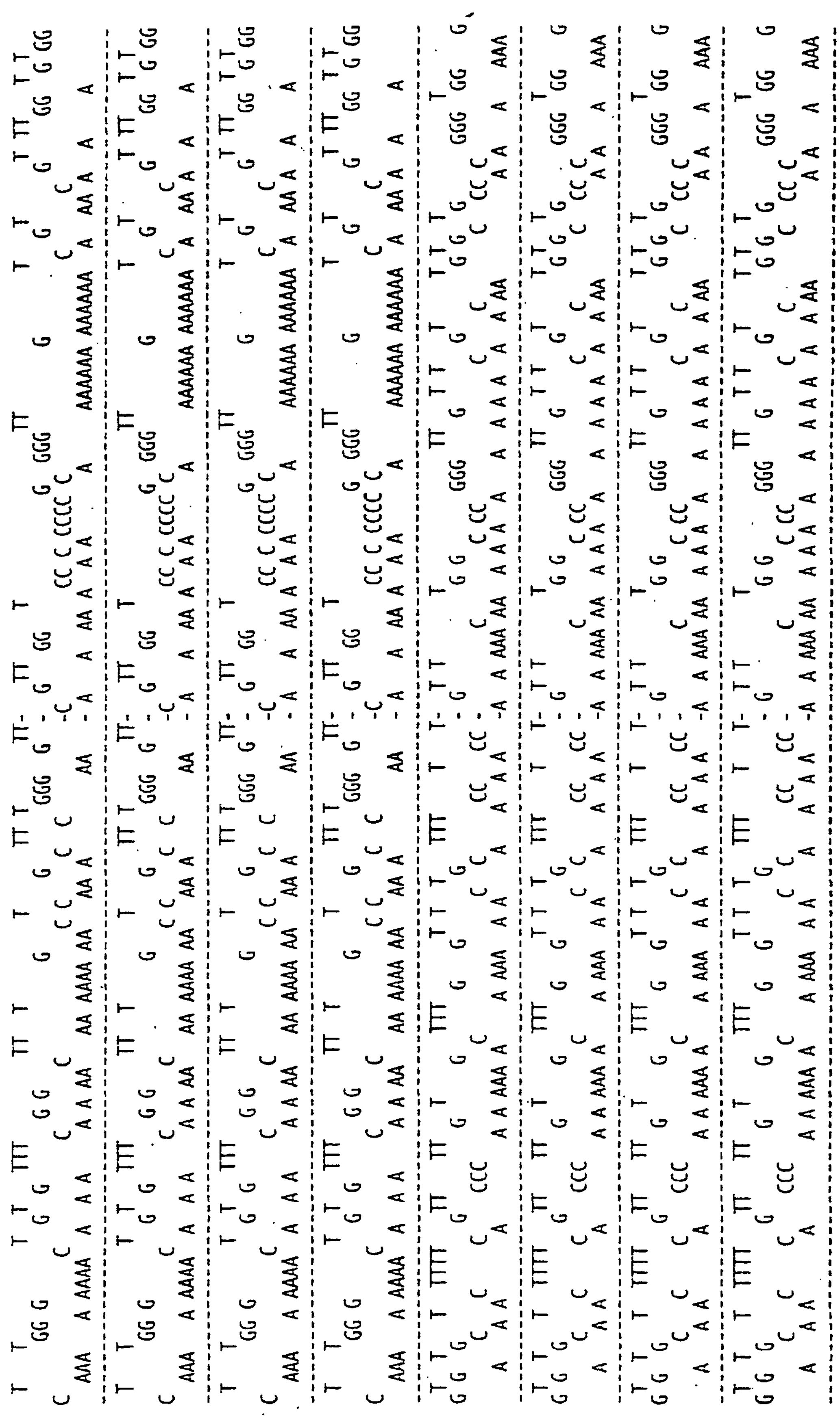

FIG. 10B

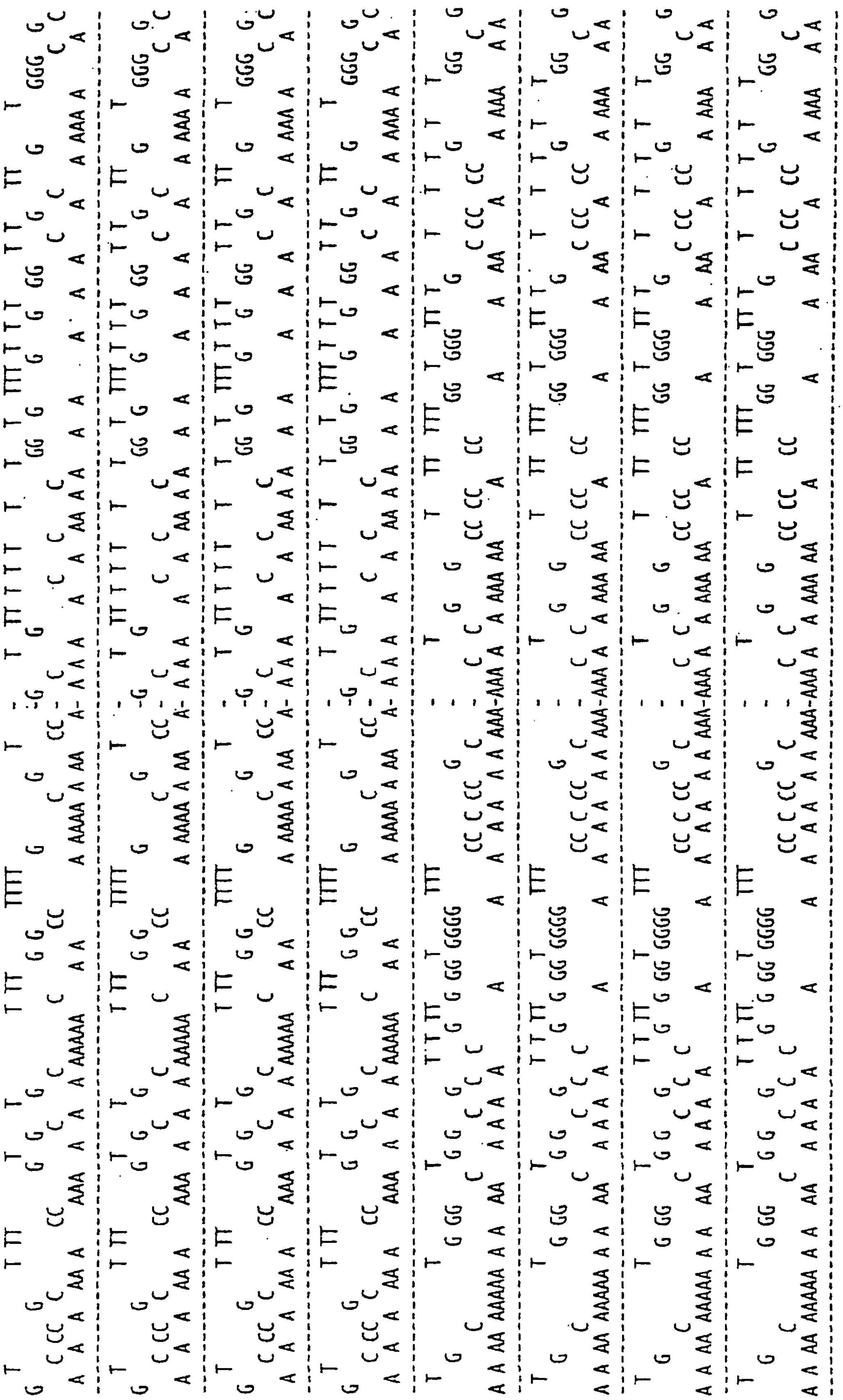

FIG. 10C

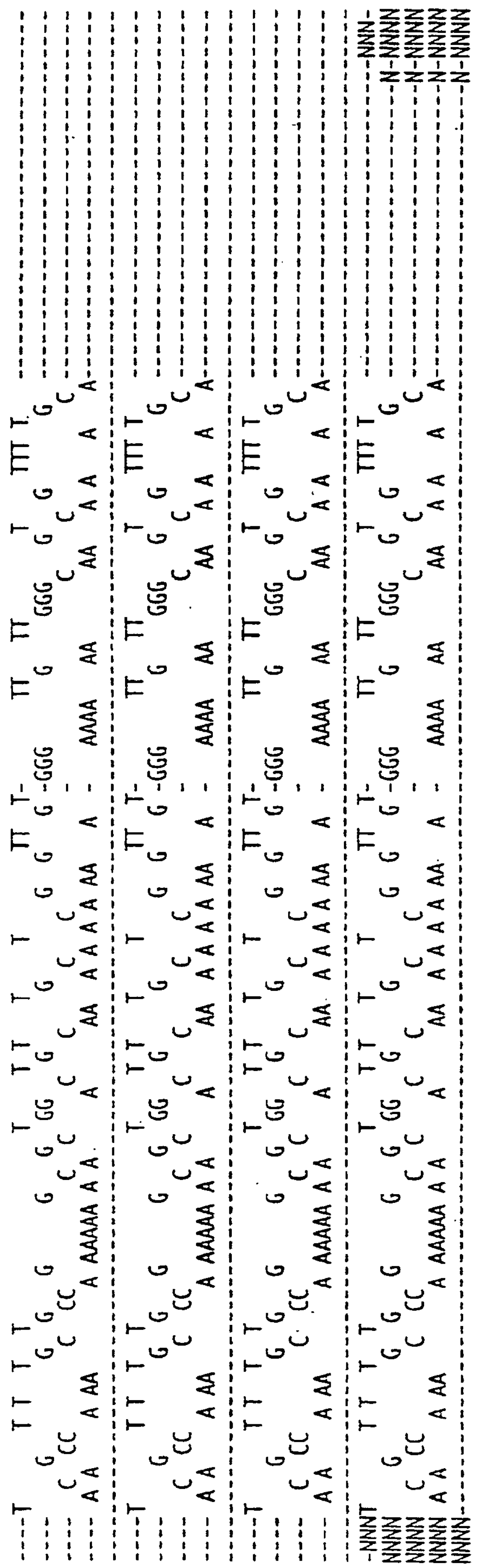

FIG. 10D

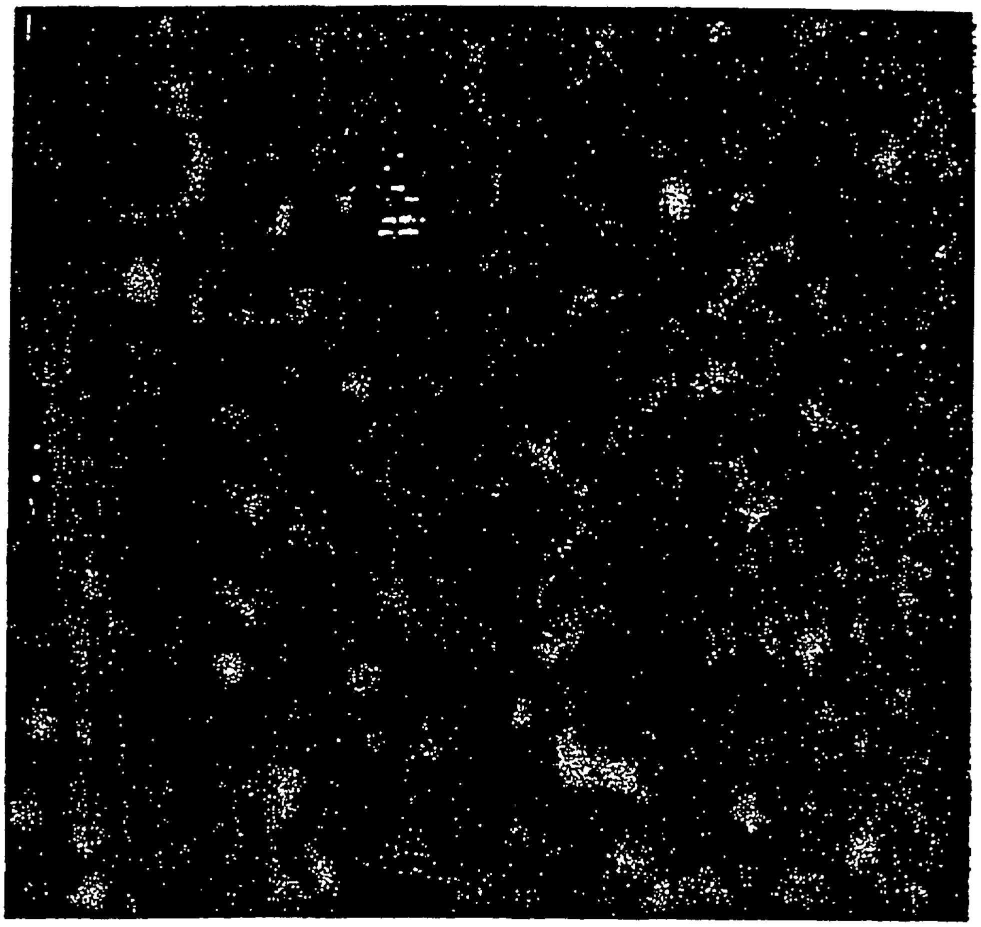


MCO7060:
=407 wafer chip hybridized with fragmented pfol19 RNA

Fig. 11

```
GGAAGAAATCTGTTGACTCAGATTGGTTGTACTTTAAATTTCCCCATTAGTCCTATTGAAACTGTACCAGTAAAATTAAAGCCAGGAATGGATGGCCCAAA
         10        20        30        40        50        60        70        80        90       100

ggRagaaatNNNNNNtctcagattggttgtNNNNBcNNNNNNNNNNNNNNNNNNNNtNNNNactgNNNcagNNNNNNaaaagccagggagggatggccNNNN
ggRagaaatNNNNNNNctcagattggttgtacNNNNNNNNNNNNNNNtNNNNNctattgaaactgtNccagtaaaataaaagccaggRagggaWggcccaaa
ggRaggaatctgttNNctcagattggttgtactNNNNNNNNNNNccattNNtcctattgaaactgtaccagKaaaataaaagccaggaagggatggcccaaa
ggaagaaatctgttgactcagattggttgtactttaaaNNNNcccattagtcctattgaaactgtaccagtaaaatWaaagccaggaaKggatggcccaaa
NgaagaaaNctNNNNNctcagattgNNNNcNNNNNNNNNNNNNNNNNNNNNNNcNNNNNNNNgNctgNNNcagtaNNataaaagccaggaagggatggcccaaa
ggRagaaatctgttgactcagattggttNcNNNNNNNNNNNNNNNNNNNNNNcNNNNNNNagactgtNccagtaaaataaaagccaggaagggatggcccaaa
ggaagaaatctgttgactcagattggttScacNNNNNNNNNNNNNNNNNNNNcNNNNNNNagactgtaccagKaaaataaaagccaggaagggaWggcccaaa
ggaagaaatctgttgactcagattggttScMctNNNNNNNNNNNNNNNNNNNcNNNNttgagactgtaccagtaaaaWaaaagccaggaaKggaWggcccaaa
                              *           0 0      *        *

                                                                     41      43 44
AGTTAAGCAATGGCCATTGACAGAAGAAAAAATAAAAGCATTAGTAGAGATATGTACAGAAATGGAAAAGGAAGGGAAAATTTCAAAAATTGGGCCTGAAA
        110       120       130       140       150       160       170       180       190       200
                                                                    C     G     T
agttaagcaaNNNNcNttgacagaagaaaaNataaaagcattagtagagatatgtaSagaaagggaaaaggaagggaaaaNNNNNNNaattgggcctgaaa
agttaagcaaWNNcNattgacagaagaaaaaataaaagcattagtagagatatgtacagaaagggaaaRggaagggaaaagNNNNNaaattgggcctgaaa
agttaagcaatgRcBMWtgacagaagaaaaaataaaagcattagtagagatatgtacagaaagggaaaRggaagggaaaaKtNNNaaaattgggcctgaaa
agttaagcaatgRMVgatgacagaagaaaaaataaaagcattagtagagatatgtacagaaaDggaaaRggaagggaaaaKttcaaaaattgggcctgaaa
NNNNNNNgNNNNNNNNttgacagaagaaaaaataaaagcattagggRgNNNNNgNSagRggggaaaaggaagggaaaagNNNNNNaattgggcctgaaa
NNNNNNagNNNNNNNattgacagaagaaaaaataaaagcattagtagRRaXNNggacagRgRgggaaaRggaagggaaaagtNNNNaaattgggcctgaaa
agNNNaaSNNNNaNtaWtgacagaagRaaaaataaaagcattagtagaaaatgggacagRgRgggaaaRggaagggXaaagttNNaaaattgggcctgaaa
agtNaaaSNNNNaMMRDtgacagaagaaaaaataaaagcattagtagaaaatKggacagRgRgggaaaRggaagggRaaaDXtcaaaaattgggcctgaaa
    *                                                *  *          *

                                        67       70             75
ATCCATACAATACTCCAGTATTTGCTATAAAGAAAAAAGACAGTACTAAATGGAGAAAACTAGTAGATTTCAGAGAACTTAATAAAAGAACTCAAGACTTC
        210       220       230       240       250       260       270       280       290       300
                                                       G              A G
NtcNNgNNNNNacNNNNNNNNNNNNNNtaaNNgaaaaagacagtaNtaaatggagaaaactagNagatttcagagaacNNNNNNRaagaactcaNNNNttc
atccagaNaNtactNNNgtattNNctataaagRaaaaagacagtactaaatggagaaaactagtagatttcagagaacttaNNaaaagaactcaaNacttc
atccagacaatactcNNgtatttgctataaagaaaaaagacagtactaaatggagaaaactagtagatttcagagaacttaataaaagaactcaagacttc
atccaKacaatactccagtatttgctataaagaaaaaagacagtactaaatggagaaaactagtagatttcagagaacttaataaaagaactcaagacttc
atcNNgacNaNacNNNNNtNNNNNNcNNNNggggRaagaacNNNNNtagaKggRgagaatNNNNNNatttcagagaactNNNNNggNNNactcaagNcttc
atccagacaatactNNNgtNNNNNNcNNNaRggaRaagaacaNNNctagatggagaaaattaNNNgatttcagagaacttNNNNNgagaactcaagacttc
atccagacaatactcNNgtNNNNNNcNNgagggaaaagaacagHactagatggagaaaattagNagatttcagagaacttaataagagaactcaagacttc
atccaKacaatactccaNtatNtgccMggaRggaaaaRaacagYactagatggagaaaattagtagatttcagagaacttaataagagaactcaagacttc
                         *          *           *         *                         *
```

FIG. 12A

```
TGGGAAGTTCAGTTAGGAATACCACACCCCGCAGGGTTAAAAAAGAAAAAATCAGTAACAGTATTGGATGTGGGTGATGCATACTTTTCAGTTCCCTTAGA
     310       320       330       340       350       360       370       380       390       400
                                                  T       G
tgggaagttcagttaggaataccacacNcNNNNgggttaaagagNRaaaaatcagtaacagtattggatgtgggtgatgcNNNNNNNNNNNNNNNcNNNga
tgggaagttcagttaggaataccacaccccNcNgggttaaagaggaaaaaatcagtaacagtattggatgtgggtgatgcaNNNNNNNNNNNNccccttaga
tgggaagttcagttaggaataccacaccccNNagggttaaaRaggaaaaaatcagtaacagtattggatgtgggtgatgcatNNNttcSNNNtcccttaga
tgggaagttcagttaggaataccacaccccgcNgggttaaaRaagaaaaaatcagtaacagtattggatgtgggtgatgcatacNNNNNNNNtcccttaga
DgggaagttNNaNgNggaataccNNNNNNNNNNgggttaaagggRaaaaatcagtaaNNNNNcNggaggtgggtgatgcNNNNNNNNNNNNNNNcNNNNN
tgggaagttcNatgaggaataccaNNtcMNNNNgggttaaagaggaaaaaatcagtaacNNtNctggaKgtgggtgatgcaNNNNNNNNNNNtccctNNNa
tgggaagttcaatKaggaataccacatcXcNcNgggttaaaRaRgaaaaaatcagtaacagtactggaKgtgggtgatgcaDNtNNNNNNNttcccttaga
tgggaagttcaattaggaataccacatXccgcNgggttaaaRaagaaaaaatcagtaacagtactggatgtgggtgatgcaDatNNNNNNNNtcccttaga
          *              *                *           *         *                  *

                                                 135
TAAAGACTTTAGAAAGTATACTGCATTTACCATACCTAGTATAAACAATGAGACACCAGGGATTAGATATCAGTACAATGTGCTGCCACAGGGATGGAAAG
   410       420       430       440       450       460       470       480       490       500
                                                 C
taaaNNNNNtNNNNagtatacNNNattNacNNtaccNNNtNNNNacaatgagacaccagggattagNNNtcagtacaatgtgctgccacagggatggaagg
taaagactttagNaagtatactgcatttaccataccNNgtataaacaatgagacaccagggattagatatcagtacaatgtgctgccacagggatggaaRg
taaagactttagaaagtatactgcatttaccatacctagtataaacaatgagacaccagggattagatatcagtacaatgtgctgccacagggatggaaag
taaagactttagaaagtatactgcatttaccatacctagtataaacaatgagacaccagggattagatatcagtacaatgtgctgccacagggatggaaag
DgRNNNNNNNNNNNNNNNNNNNNattNacNNtaccNNNtNNNaacaatgagacaccagggattagatatcagtacaatNNNNNNNNacagggatggaagg
tgaNNNNNNNNNNNNNNNNNNNNcatttaccatacctNgtataaacaatgagacaccagggattagatatcagtacaatgNNNNtccacagggatggaagg
tgaaNNNNNcNNNNNagataNNgcatttaccatacctagtataaacaatgagacaccagggattagatatcagtacaatgtNNttMcacagRgatggaaRg
tgaagaNWNcNNNNNaKatactgcatttaccatacctagtataaacaatgagacaccagggattagatatcagtacaatgtgcttHcacagggatggXaRg
 *       *  0  *                                                                     *

                                                                                 181                 188
GATCACCAGCAATATTCCAAAGTAGCATGACAAAAATCTTAGAGCCTTTTAGAAAACAGAATCCAGACATAGTTATCTATCAATACATGGATGATTTGTAT
  510       520       530       540       550       560       570       580       590       600
                                                                                  G                 C
gatcaccagcaatNNNNNaaagtagcatgacaRNNNNcttagagNcttNNNNaaaacagaatccagacataNNNNNNNNNNNNNacagggatgattNNtat
gatcaccagcaatattccaaagtagcatgacaaaaatcttagagccttttagaaaacagaatccagacatagtNNNNNNNNNatacaKggatgatttgtat
gatcaccagcaatattccaaagtagcatgacaaaaatcttagagccttttagaaaacagaatccagacatagttatNtNtcaatacatggatgatttgtat
gatcaccagcaatattccaaagtagcatgacaaaaatcttagagccttttagaaaacagaatccagacatagttatctatcaatacatggatgatttgtat
gatcaccagcaatNNNNNaaagtagcatgacaaaaNNcttagagccttNNaNNggNgNaNNgccagacatagNNNNNNNNNNNtacatggatgatttNtat
gatcaccagcaatattccaaagtagcatgacaaaaatcttagagcctttaNNggVVaaaagccagacatagttNNNtaNNNatacatggatgatttgtat
gatcaccagcaatattccaaagtagcatgacaaaaatcttagagccttttagaRRacaaaaKccagacatagttatctatcaatacatggatgatttgtat
gatcaccagcaatattccaaagtagcatgacaaaaatcttagagccttttagaaaacaaaaKccagacatagttatctatcaatacatggatgatttgtat
```

*FIG. 12B* *

```
 190                                                                        210            215           219
GTAGGATCTGACTTAGAAATAGGGCAGCATAGAACAAAAATAGAGGAACTGAGACAGCATCTGTTGAGGTGGGATTTACCACACCAGACAAAAAACATCA
 610       620       630       640       650       660       670       680       690       700
   C                                                                  G              TA         C
gtaggatctgacNtagaaatagggcagcatagaagaaaaatagaggaactgagacagcaNctgttgaggtggggatttaccacaccagacaaaaNacatca
gtaggatctgacttagaaatagggcagcatagaagaaaaatagaggaactgagacagcatctgttgaggtggggatttaccacaccagacaaaaaacatca
gtaggatctgacttagaaatagggcagcatagaacaaaaatagaggaactgagacagcatctgttgaggtggggatttaccacaccagacaaaaaacatca
gtaggatctgacttagaaatagggcagcatagaacaaaaatagaggaactgagacagcatctgttgaggtggggatttaccacaccagacaaaaaacatca
gtaggatctgacttagaaatagggcagcatagaagaaaaatagaggagctgagggaaNNNNNgttgaggtggggRccNNNNagaccagaacgNgNacatca
gtaggatctgacttagaaatagggcagcatagaagaaaaatagaggagctgagRSaMcNNNtgttgaggtggggaccNNNgNSaccagaMcVaaaacatca
gtaggatctgacttagaaatagggSagcatagaaSaaaaatagaggagctgagRcaacaNctgttgaggtggggXcVVXggacaccagaMcMaaaacatca
gtaggatctgacttagaaatagggSagcatagaacaaaaatagaggagctgagacaacatctgttgaggtggggXccXtXgacaccagaMcMaaaacatca
                                                    *        *                 *  00          *
                                                                                                 254
GAAAGAACCTCCATTCCTTTGGATGGGTTATGAACTCCATCCTGATAAATGGACAGTACAGCCTATAATGCTGCCAGAAAAAGACAGCTGGACTGTCAATG
710       720       730       740       750       760       770       780       790       800       8

gaaagaaccNNNNNNNNNNNggatgggttatgaNNtcNNNNNNNNNNaatggacagtacagNNNNNNNtgctgccagRaRaRgacagctNNactgtNNNNg
gaaagaacctcNNNNNNNNtggatgggttatgaactccaNNNNNNNaaatggacagtacagccNNNNatgctVccagaaaaagacagctgNactgtcNNNN
gaaagaacctccNNNNNNNtggatgggttatgaactccatNNNNNtaaatggacagtacagcctNNNatgctaccagaaRaagacagctggactgtcaaNg
gaaagaacctccNNNNNNttggatgggttatgaactccatcNNNataaatggacagtacagcctaNNatgctgccagaaaaagacagctggactgtcaatg
gaaagaacctcNNNNNNNNNggatgggttatgaNctccNtNNNNNtaaatggacagtacagcNNNNNNNNctgccagRaRaRgacagctggactgtcNNNg
gaaagaacctccNNNNNNNtggatgggttatgaactccatcctgNtaaatggacagtacagccNNNNgNgctgccagaaaaagaVagctggactgtcaNtg
gaaagaacctccNNNNNNttggatgggttatgaactccatNcNgataaatggacagtacagcctNNNgNgctgccagaaRaagacagctggactgtcaaNg
gaaagaacctccatNNNtttggatgggttatgaactccatcNNNataaatggacagtacagcctaNNgtgctgccagaaaaagacagctggactgtcaatg

ACATACAGAAGTTAGTGGGAAAA
10        820       830

acatacagaagttagtggggRaa
acatacagaagttagtggggaga
acatacagaagttagtggggRga
acatacagaagttagtgggggga
acatacagaagttagKggggaaa
acaKacagaagttagKggggaaa
acatacagaagttagtggggaaa
acatacagaagttagKggggaaa
```

*FIG. 12C*

```
5'Fluorescein-AAAGAAAAAAGACAGTACTAAATGGAGAAAAT wildtype
PROBE 3'          tttttt•tgtcat                13mers
PROBE 3'         ctttttt•tgtcatg               15mers
PROBE 3'        tcttttttt•tgtcatga             17mers
PROBE 3'       ttcttttttt•tgtcatgat            19mers
5'Fluorescein-AAAGAAAAAAAACAGTACTAAATGGAGAAAAT mutant
```

Fig. 13

Fig. 14

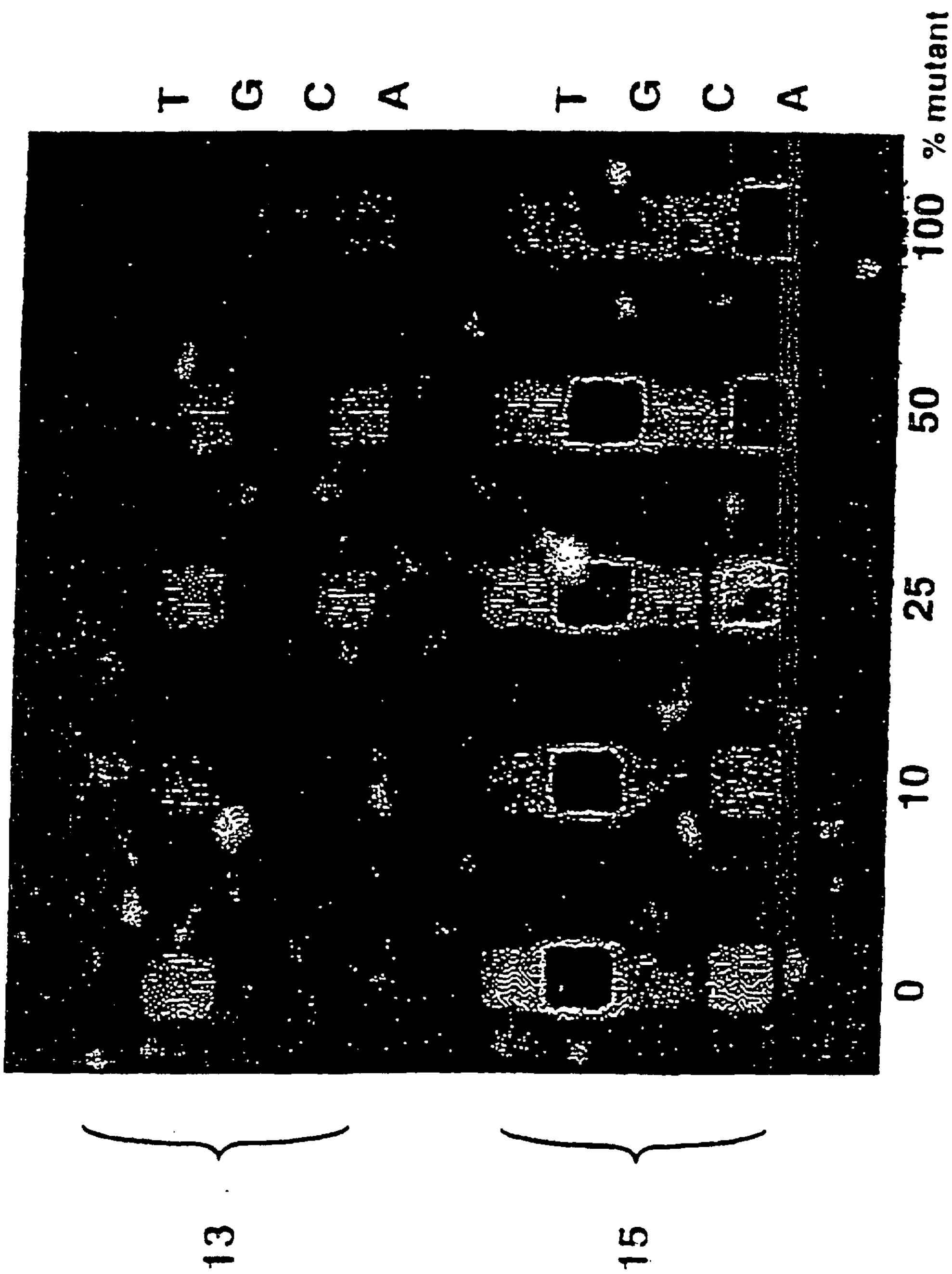

```
tcgagataat
81
nngagatann  }
tcgagataat  } PRETREATMENT
tcgagataat  }
tcgagataat  }
ncggatant   }
tcgrgataat  } POSTTREATMENT
tcgagataat  }
tcgagataat  }

ns093001wt3: ctatgtcctcgtctactatgtcataatcttctttacttaaacggtcctttacctttggttttactatccccttaacctccaaaatag
             91        101       111       121  126       136       146       156       166       176
ns093001cq2: ntatgtcctcgtcyactatgtnannnnnnnnnnnnnnnaaacggtcctnnnnnnnnnnnnnnnnnnnnwncnncntaacctccaaaatan
ns093001cq3: ctatgtcctcgtctactatgtcataatnnnnnnnacttaaacggtcctttacctttggttttactatccccttaacctccaaaatag
ns093001cq4: ctatgtcctcgtctactatgtcataatcttctttacttaaacggtcctttacctttggttttactatccmmcttaacctccaaaatag
ns093001cq5: ctatgtcctcgtctactatgtcataatcttctttacttaaacggtcctttacctttggttttactatcccmcttaacctccaaaatag
ns093002cq2: ntatgtcctcgtcyactatgtcannnnncnnncnnnncaaacggtcchccnnnnncnnnnnncnncyahaaawcycaacctccaaaatan
ns093002cq3: ctatgtcctcgtctactatgtcataatccnnncnnctcaaacggtcctyccnnnnytggttnytactatccccttaacctccaaaatag
ns093002cq4: ctatgtcctcgtctactatgtcataatcchnnctactcaaacggtccttctacctttggttttactatccmccttaacctccaaaatag
ns093002cq5: ctatgtcctcgtctactatgtcataatcttctttacycaaacggtcctxctacctttggttttactatcccmcttaacctccaaaatag

ns093001wt3: tttcattctgtcatgctagtctatggacatctttagacacctgtatttcgatatccatgt
                   186       196       206       216       226       236
ns093001cq2: nnnnnntctnnnnnannnntctannngnagnnnnaganarnccnnnnnnnnnatncatgt
ns093001cq3: tttcattctgncatannagtctatgngnngnnntagacagncnnnnntcgatatccatgt
ns093001cq4: tttcattctgtcatactagtctatgggtagctttagacamccgtatttcgatatccatgt
ns093001cq5: tttcattctgtcatactagtctatgggtagctttagacacccgtatttcgatatccatgt
ns093002cq2: nnnnnntctnnnnnannncnctnnnnnnagngnnagacacctgtatnnnnntatncaygt
ns093002cq3: tttcattctgncatacnnstctannxnnagxgttagacacctgtatttcgatatccatgt
ns093002cq4: tttcattctgtcatactagtctatgagtagctttagacacctgtatttcgatatccatgt
ns093002cq5: tttcattctgtcatactagtctatgagtagctttagacacctgtatttcgatatccatgt
```

FIG. 15

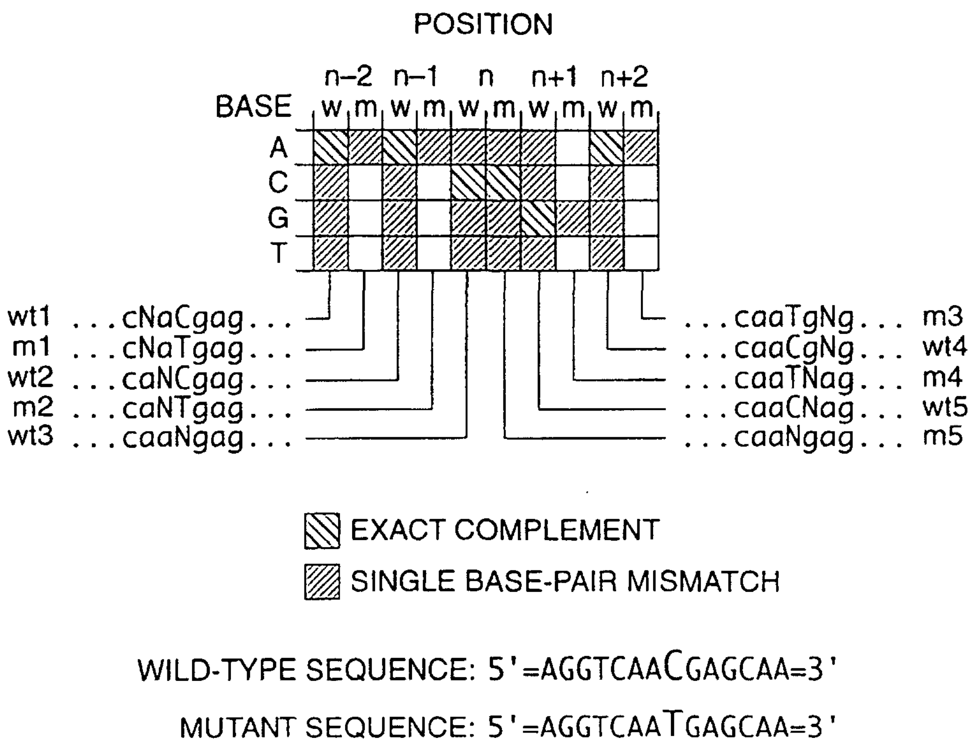
POSITION
n−2 n−1 n n+1 n+2
BASE w m w m w m w m w m
A
C
G
T
wt1 ...cNaCgag...
m1 ...cNaTgag...
wt2 ...caNCgag...
m2 ...caNTgag...
wt3 ...caaNgag...
...caaTgNg... m3
...caaCgNg... wt4
...caaTNag... m4
...caaCNag... wt5
...caaNgag... m5
EXACT COMPLEMENT
SINGLE BASE-PAIR MISMATCH
WILD-TYPE SEQUENCE: 5'=AGGTCAACGAGCAA=3'
MUTANT SEQUENCE: 5'=AGGTCAATGAGCAA=3'

*FIG. 16*

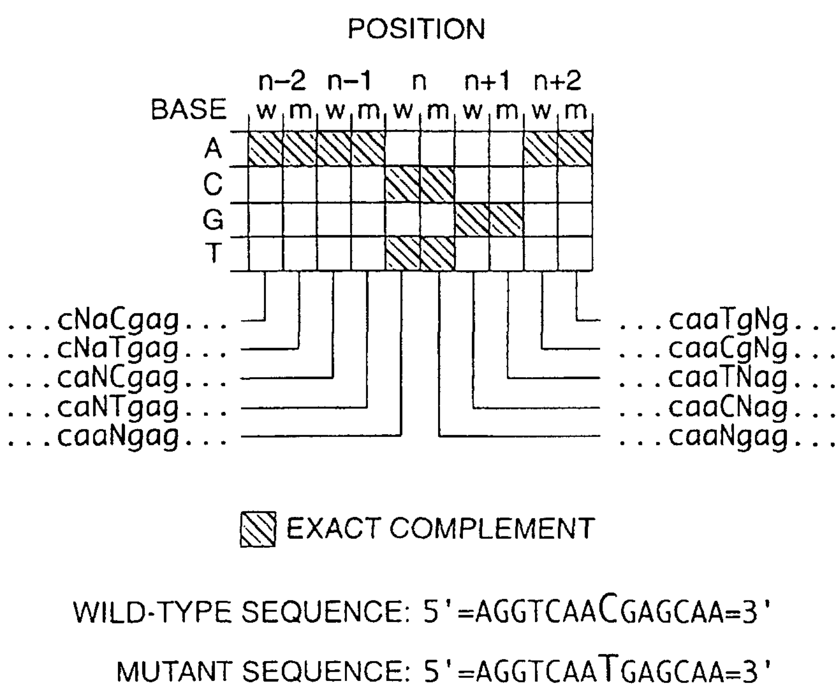
POSITION
n−2 n−1 n n+1 n+2
BASE w m w m w m w m w m
A
C
G
T
...cNaCgag...
...cNaTgag...
...caNCgag...
...caNTgag...
...caaNgag...
...caaTgNg...
...caaCgNg...
...caaTNag...
...caaCNag...
...caaNgag...
EXACT COMPLEMENT
WILD-TYPE SEQUENCE: 5'=AGGTCAACGAGCAA=3'
MUTANT SEQUENCE: 5'=AGGTCAATGAGCAA=3'

*FIG. 17*

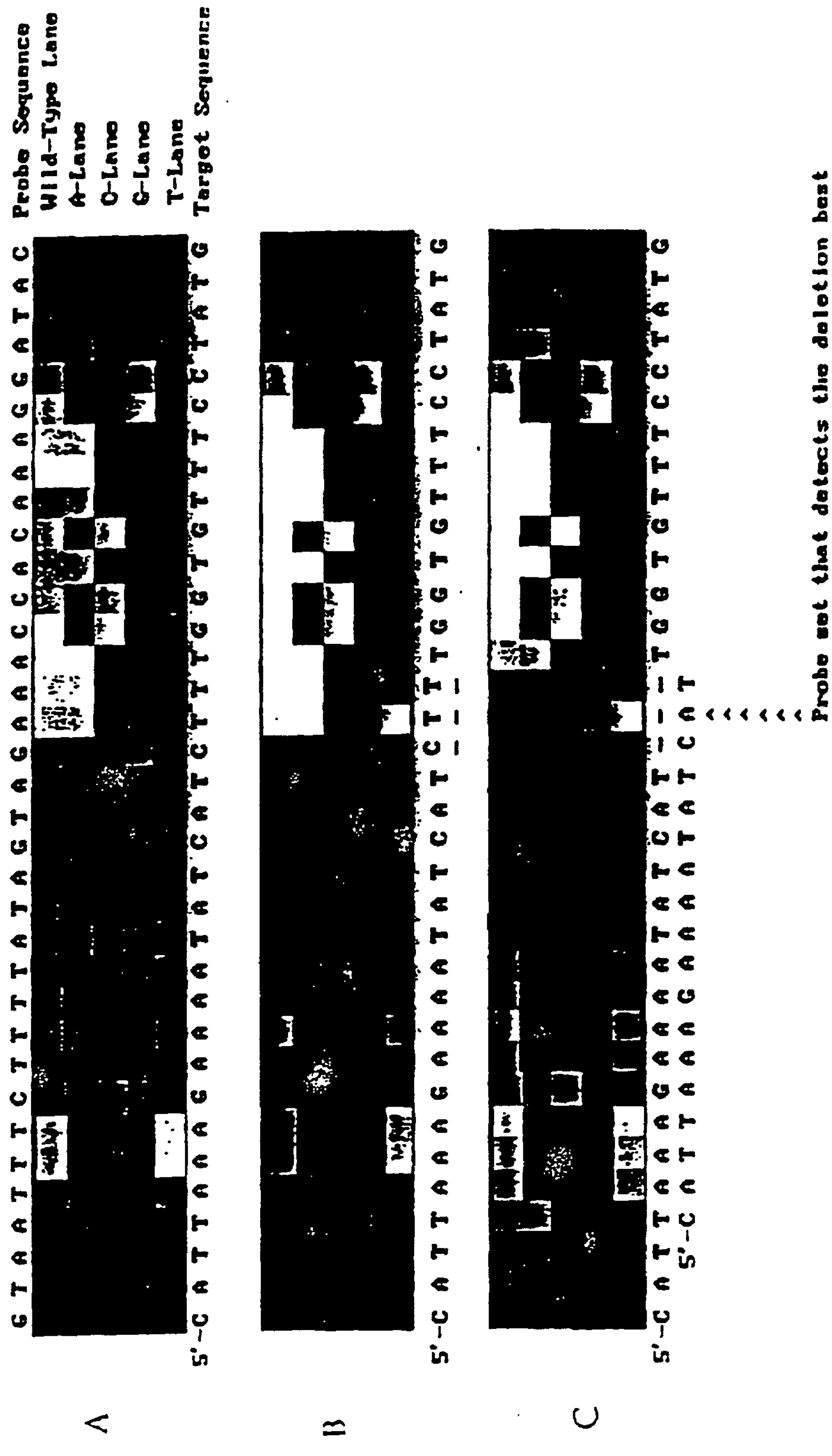
Probe Sequence
Wild-Type Lane
A-Lane
C-Lane
G-Lane
T-Lane
Target Sequence
Probe set that detects the deletion best
A
B
C

Fig. 18

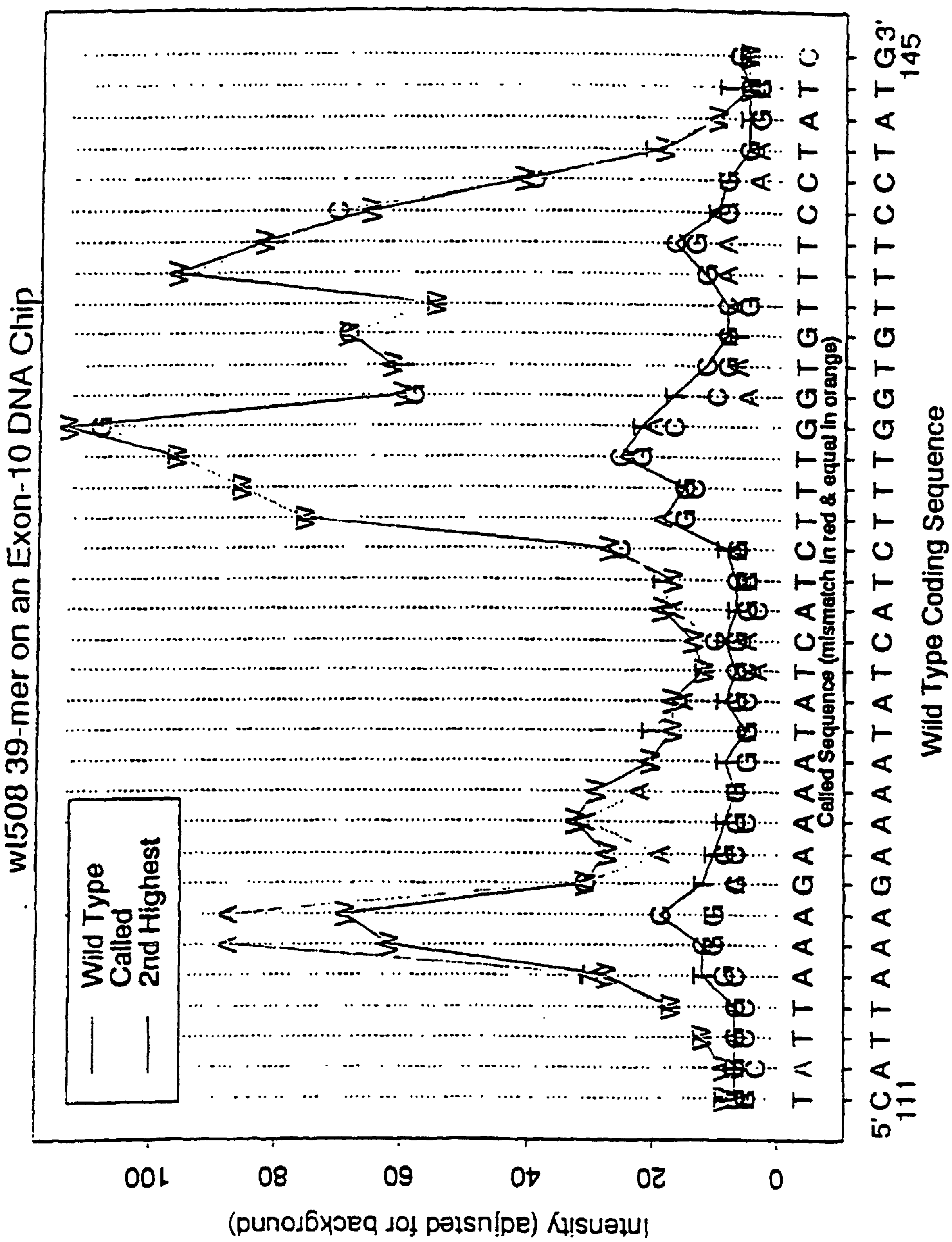
wI508 39-mer on an Exon-10 DNA Chip
Wild Type
Called
2nd Highest
Intensity (adjusted for background)
0
20
40
60
80
100
5'C A T T A A A G A A A A T A T C A T C T T T G G T G T T T C C T A T G3'
111
145
Called Sequence (mismatch in red & equal in orange)
Wild Type Coding Sequence

Fig. 19

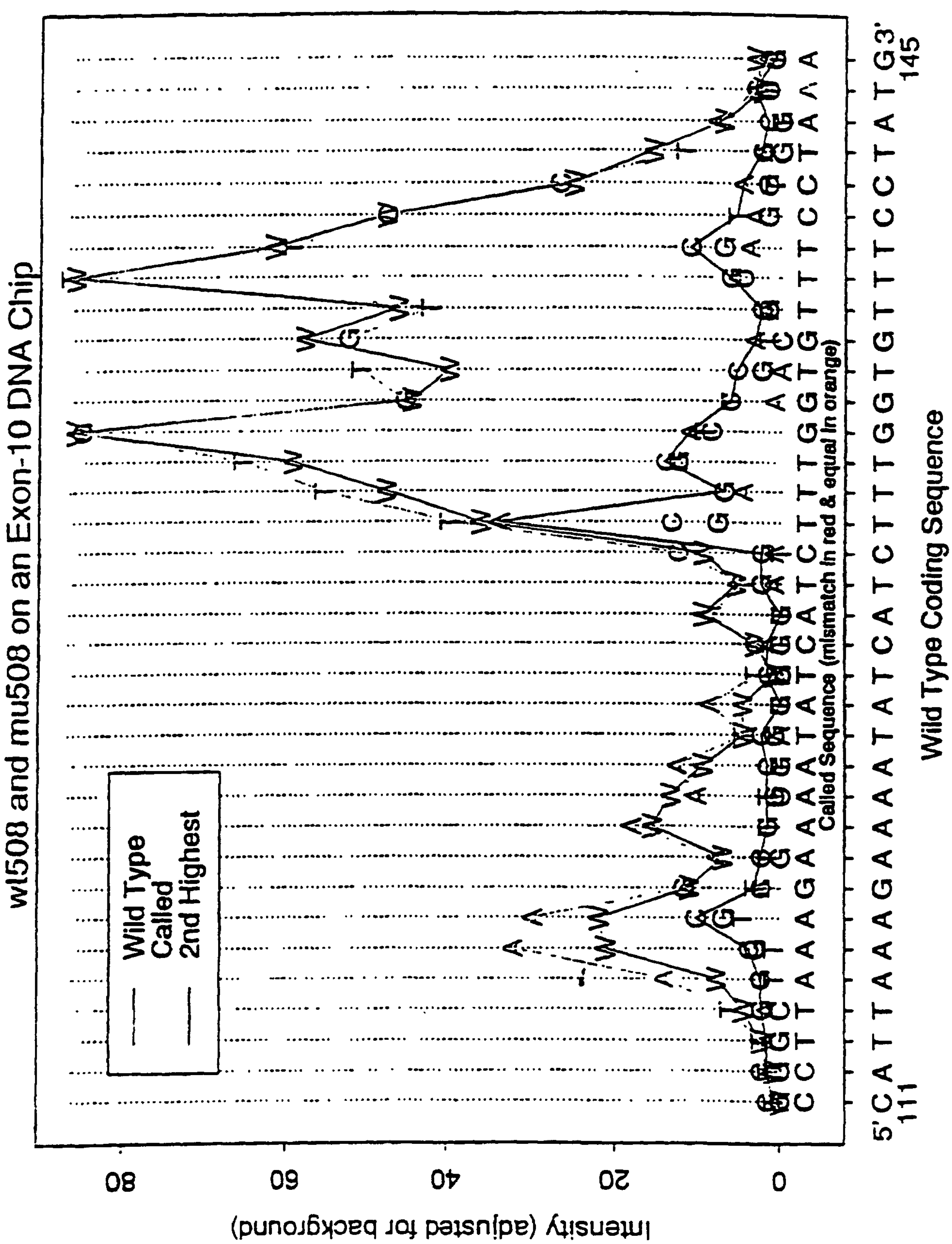
wl508 and mu508 on an Exon-10 DNA Chip
Wild Type
Called
2nd Highest
Intensity (adjusted for background)
0
20
40
60
80
5'C A T T A A A G A A A A T A T C A T C T T T G G T G T T T C C T A T G 3'
111
145
Called Sequence (mismatch in red & equal in orange)
Wild Type Coding Sequence

Fig. 19

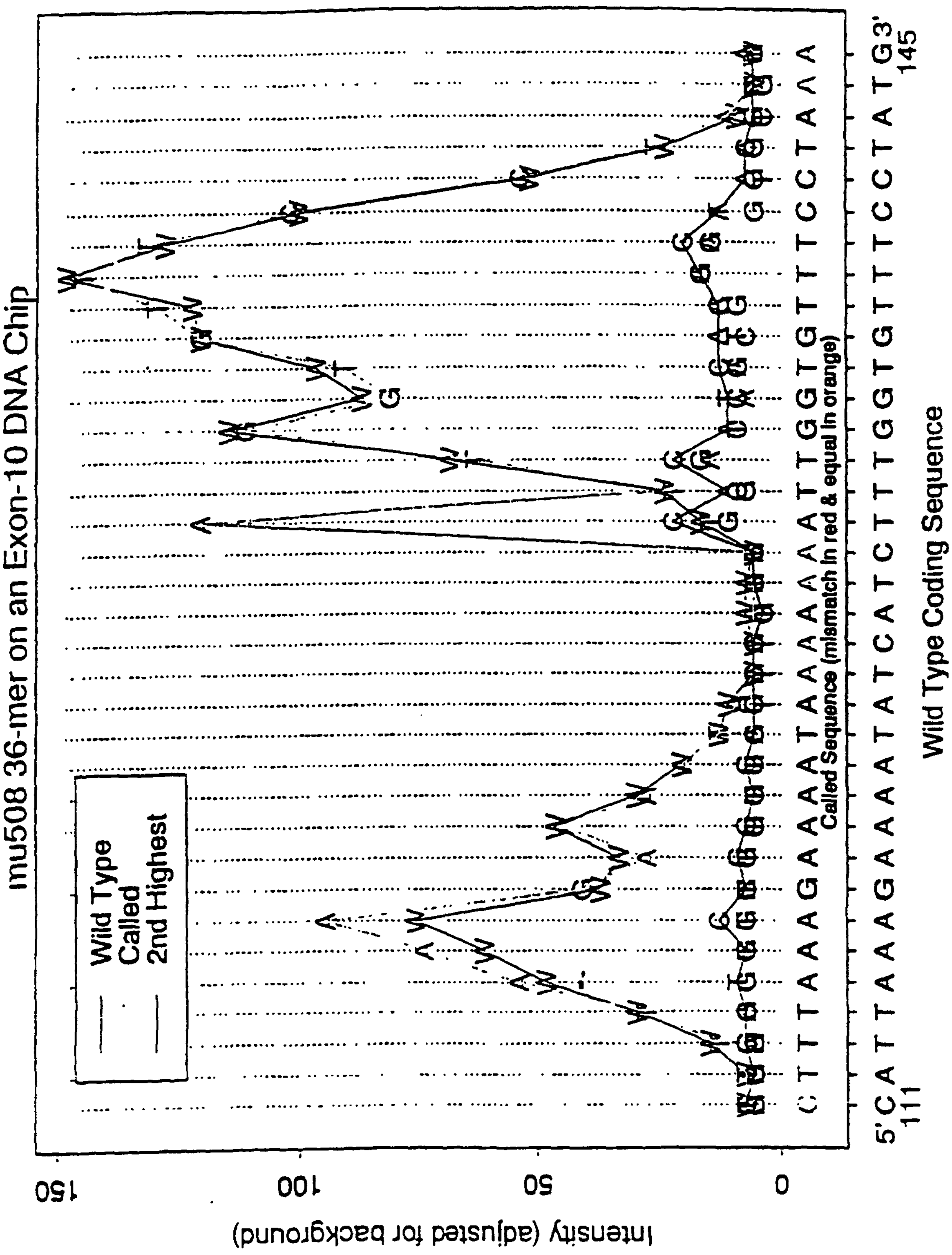
mu508 36-mer on an Exon-10 DNA Chip
Wild Type
Called
2nd Highest
Called Sequence (mismatch in red & equal in orange)
5'C A T T T A A A G A A A A T A T C A T C T T T G G T G T T T C C T A T G3'
111
145
Wild Type Coding Sequence
Intensity (adjusted for background)
0
50
100
150

Fig. 19

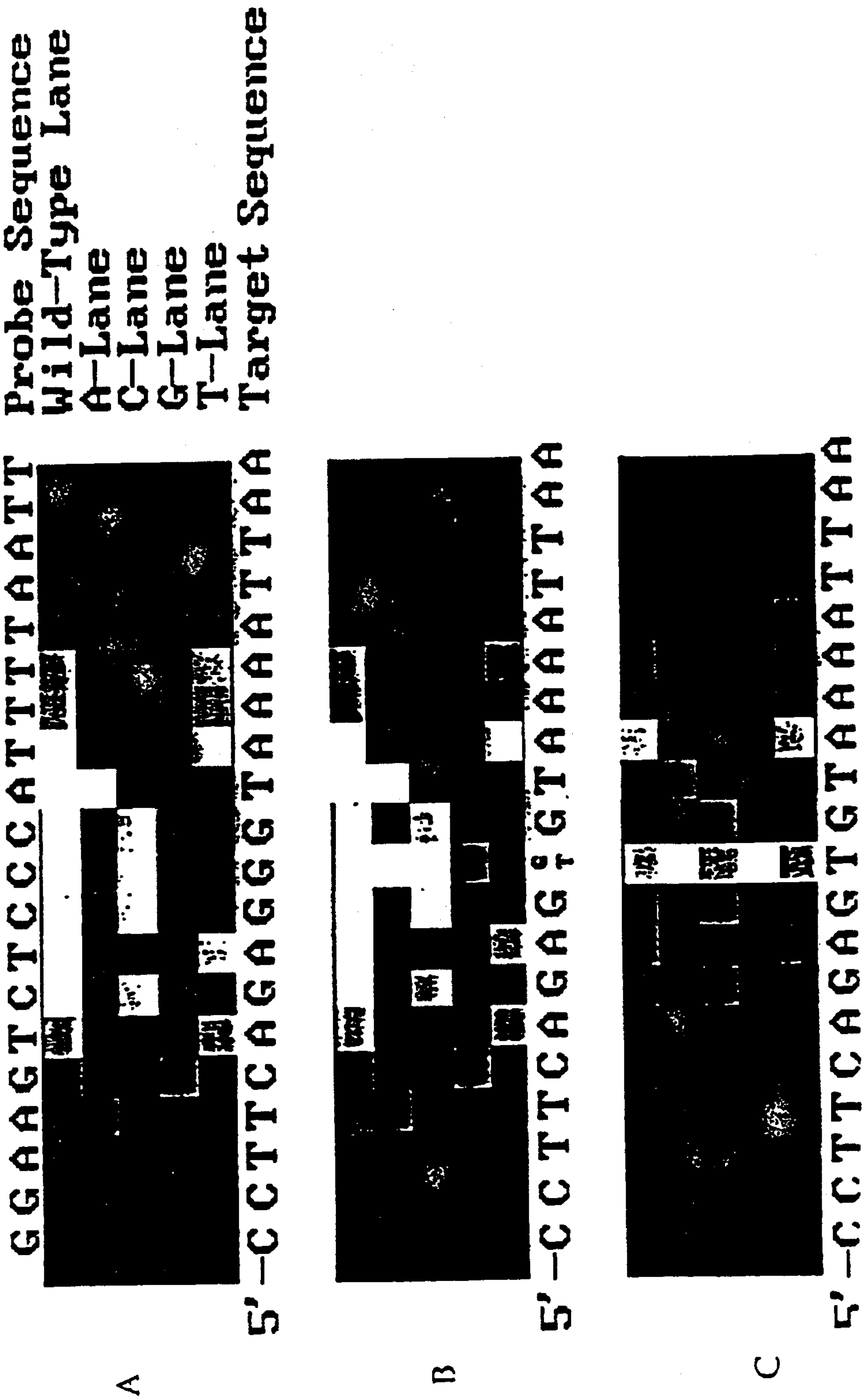
Probe Sequence
Wild-Type Lane
A-Lane
C-Lane
G-Lane
T-Lane
Target Sequence
A
B
C

Fig. 20

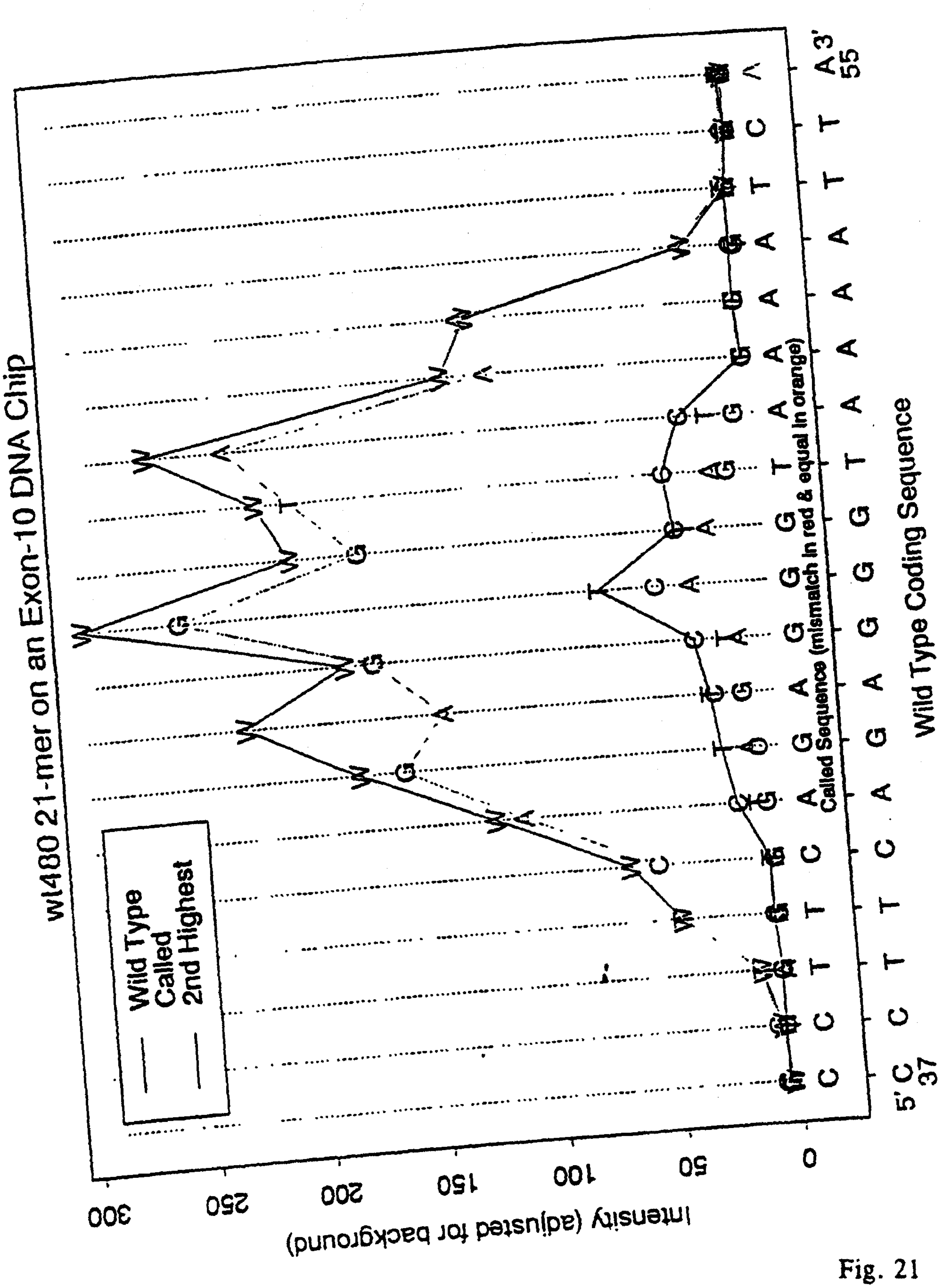
wl480 21-mer on an Exon-10 DNA Chip
Wild Type
Called
2nd Highest
Intensity (adjusted for background)
300
250
200
150
100
50
0
5' C 37
C T T C A G A G G G G T A A A A A T T A 3' 55
Called Sequence (mismatch in red & equal in orange)
Wild Type Coding Sequence

Fig. 21

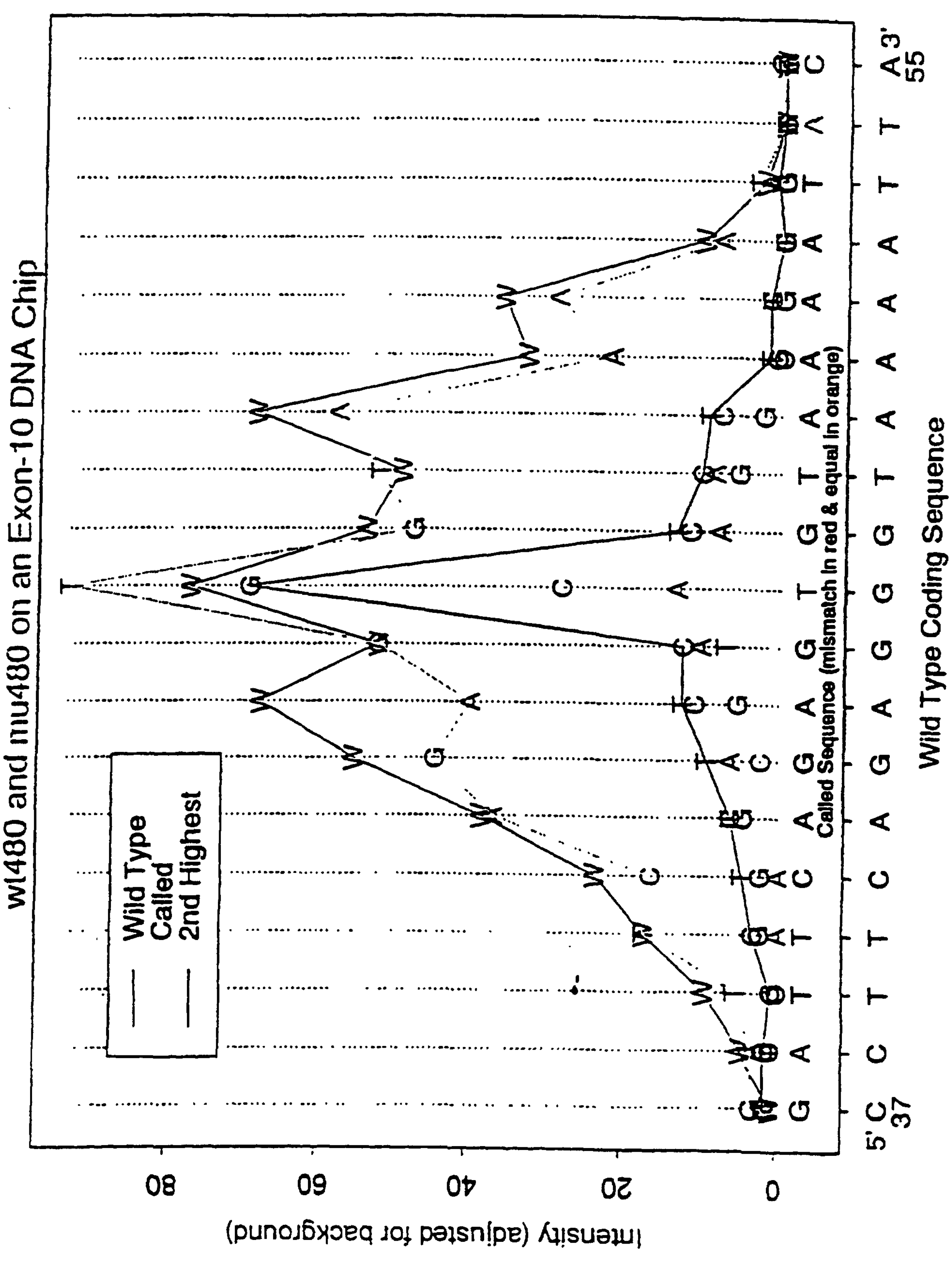
wt480 and mu480 on an Exon-10 DNA Chip
Wild Type
Called
2nd Highest
Intensity (adjusted for background)
0
20
40
60
80
5'C
37
A 3'
55
Called Sequence (mismatch in red & equal in orange)
Wild Type Coding Sequence
C T T C A G A G G G T A A A A T T A

Fig. 21

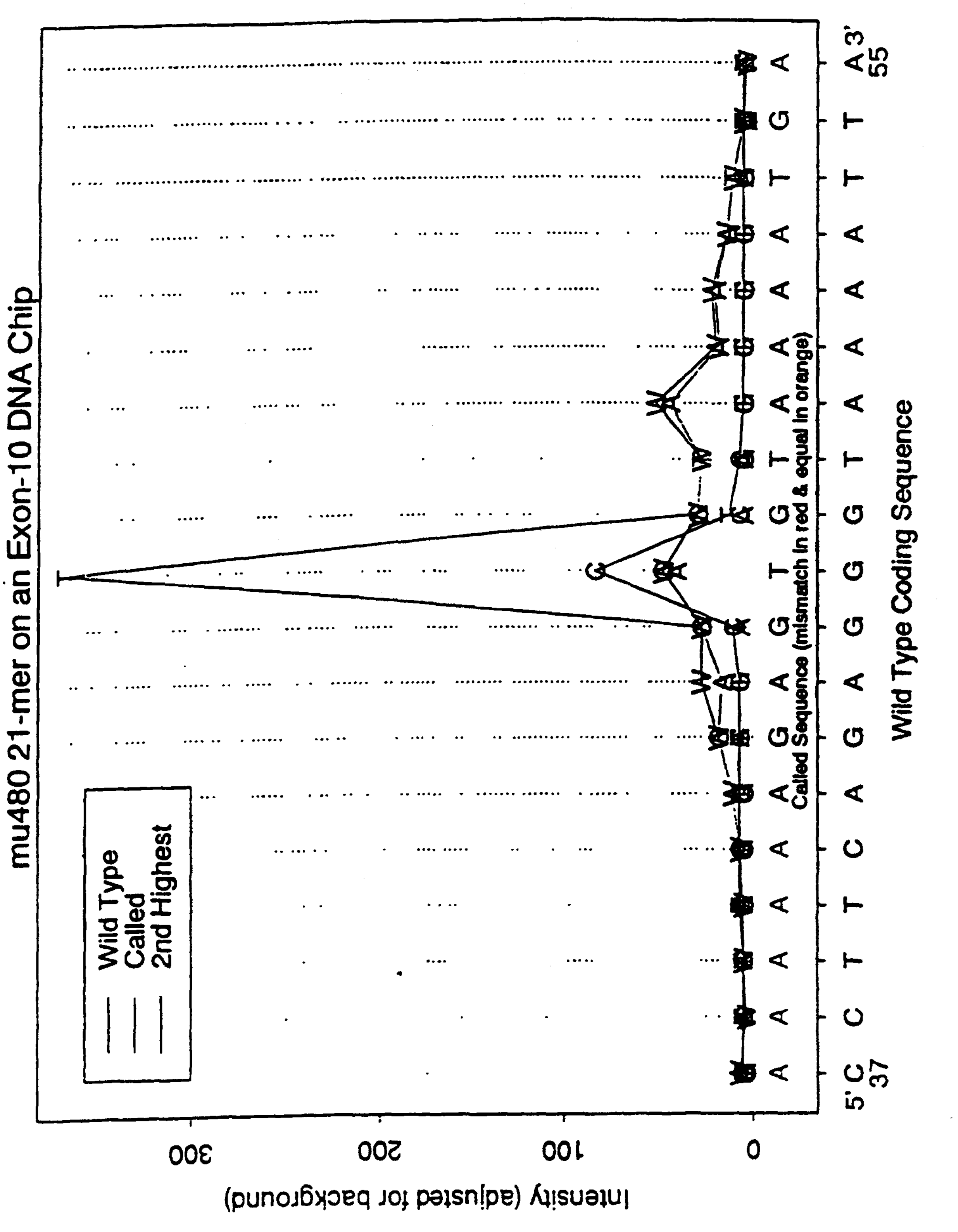
mu480 21-mer on an Exon-10 DNA Chip
Wild Type
Called
2nd Highest
Intensity (adjusted for background)
0
100
200
300
5' C 37
A 3' 55
Called Sequence (mismatch in red & equal in orange)
Wild Type Coding Sequence

Fig. 21

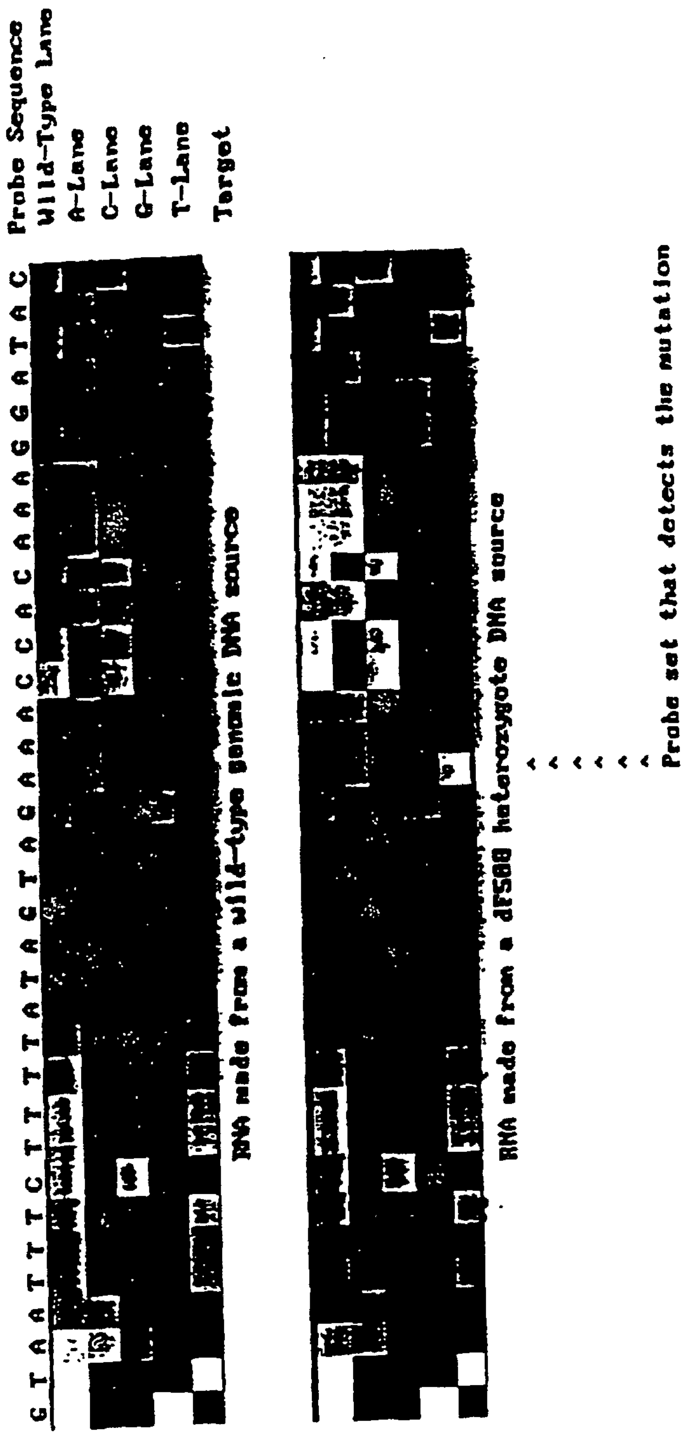
Probe Sequence
Wild-Type Lane
A-Lane
C-Lane
G-Lane
T-Lane
Target
G T A A T T T T C T T T T A T A G T A G A A A C C A C A A A G G A T A C
RNA made from a wild-type genomic DNA source
RNA made from a dF508 heterozygote DNA source
Probe set that detects the mutation

A

Fig. 22

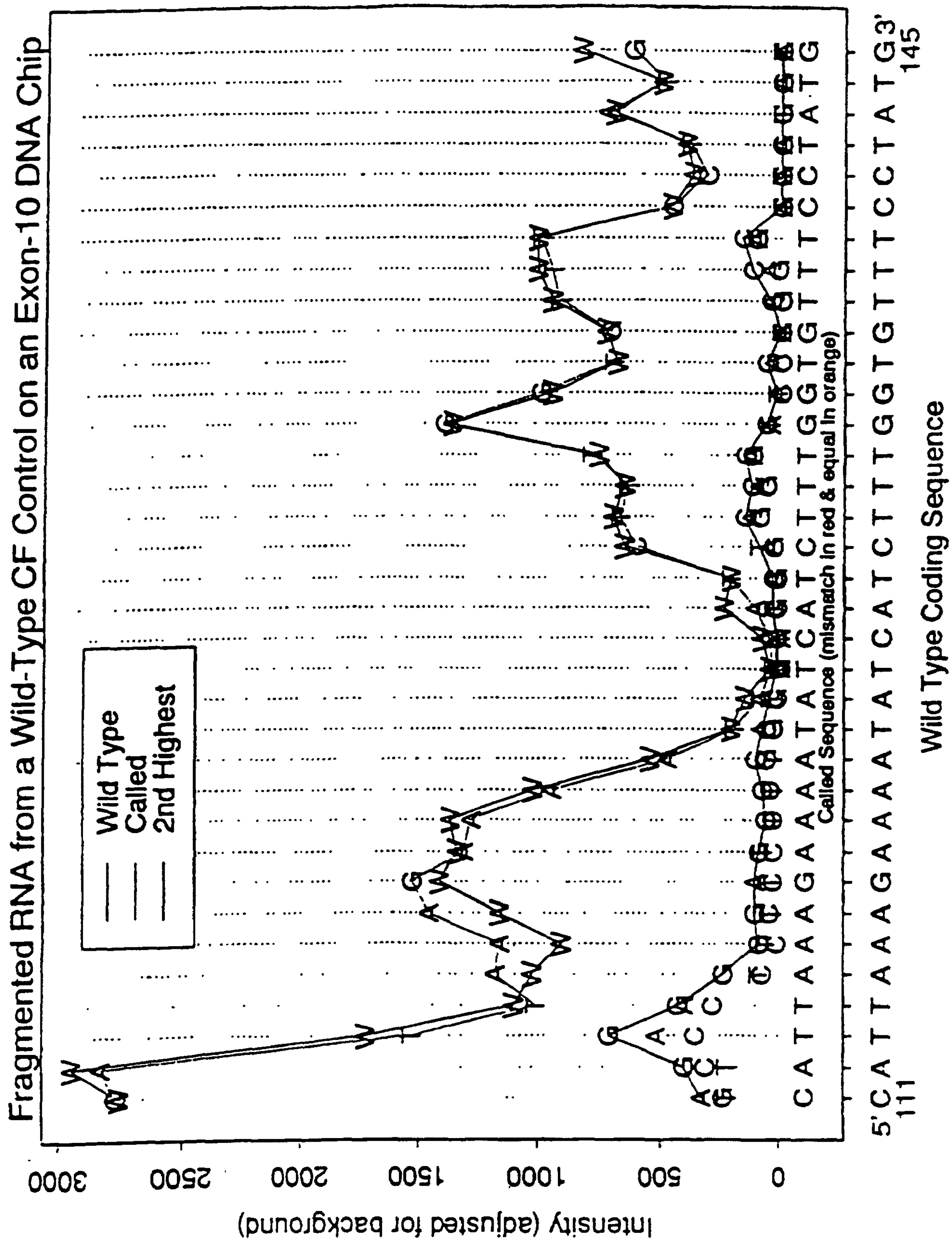
Fragmented RNA from a Wild-Type CF Control on an Exon-10 DNA Chip
Wild Type
Called
2nd Highest
Intensity (adjusted for background)
0
500
1000
1500
2000
2500
3000
Called Sequence (mismatch in red & equal in orange)
5'C A T T A A A G A A A A T A T C A T C T T T G G T G T T T C C T A T G3'
111
145
Wild Type Coding Sequence

Fig. 23

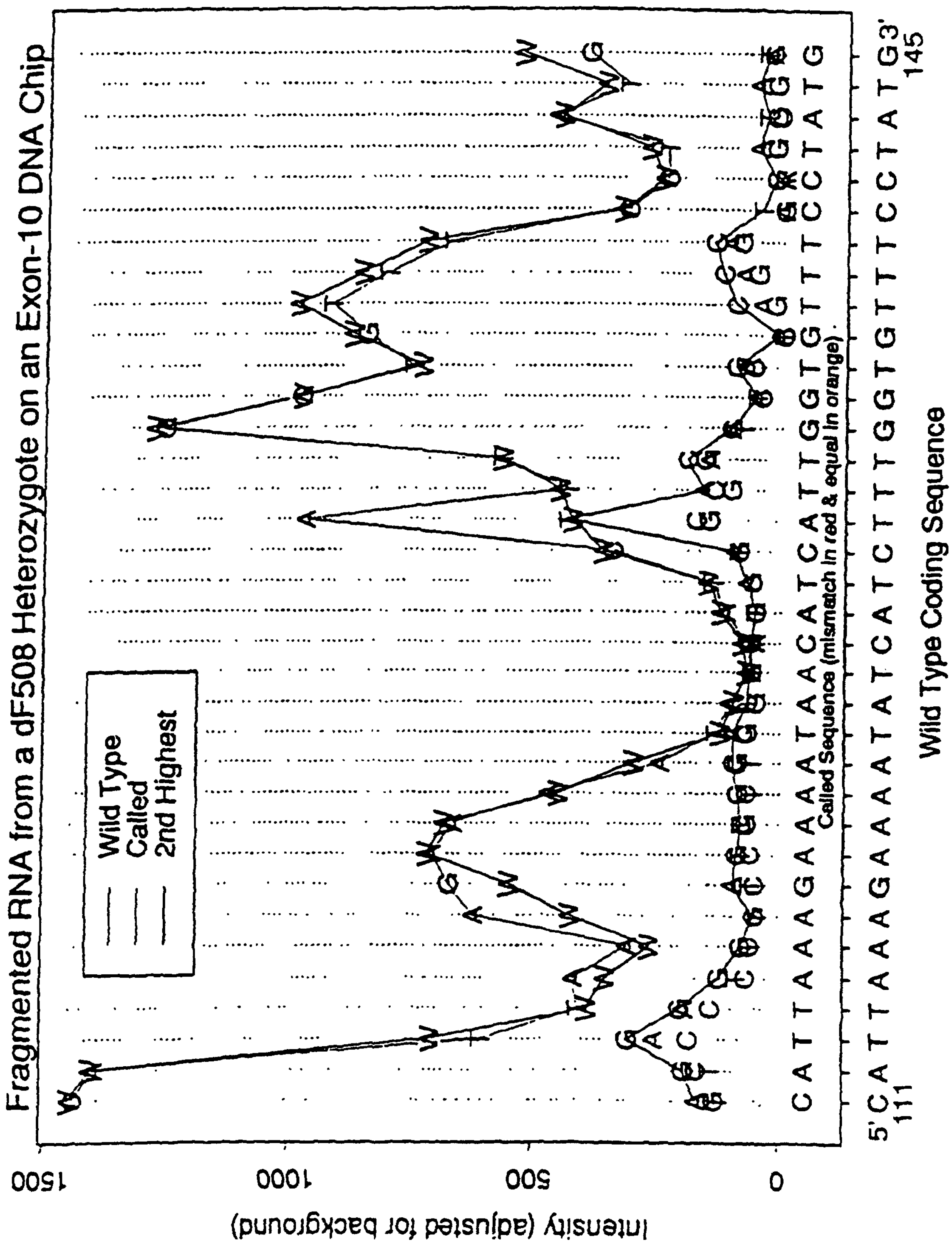
Fragmented RNA from a dF508 Heterozygote on an Exon-10 DNA Chip
Wild Type
Called
2nd Highest
Intensity (adjusted for background)
0
500
1000
1500
5'CATTAAAGAAAATATCATCTTTGGTGTTTCCTATG3'
111
145
Called Sequence (mismatch in red & equal in orange)
Wild Type Coding Sequence

Fig. 23

A B

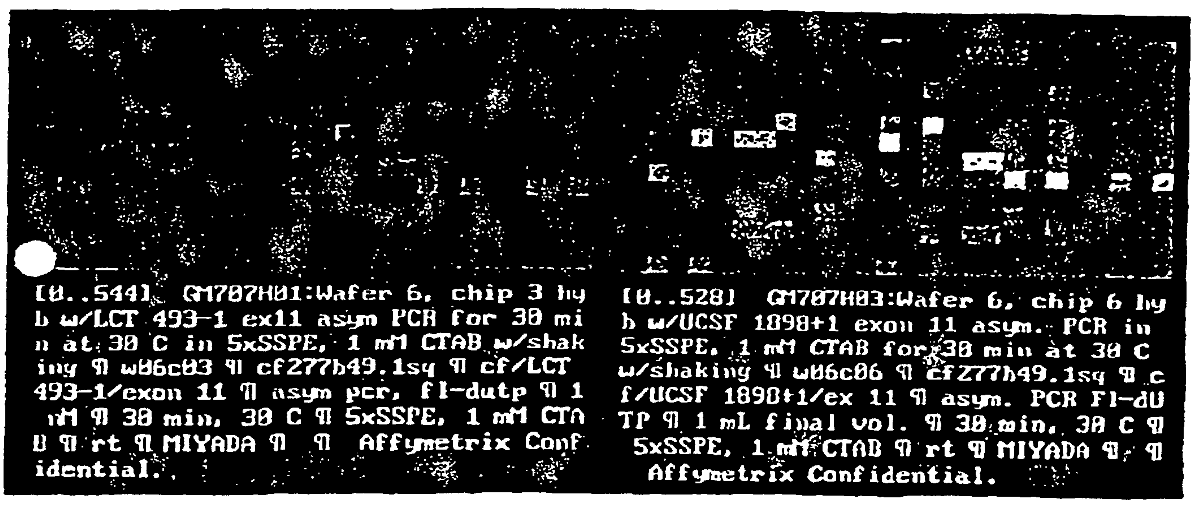
[0..544] GM707H01:Wafer 6, chip 3 hyb w/LCT 493-1 ex11 asym PCR for 30 min at 30 C in 5xSSPE, 1 mM CTAB w/shaking ¶ w06c03 ¶ cf277b49.1sq ¶ cf/LCT 493-1/exon 11 ¶ asym pcr, fl-dutp ¶ 1 mM ¶ 30 min, 30 C ¶ 5xSSPE, 1 mM CTAB ¶ rt ¶ MIYADA ¶ ¶ Affymetrix Confidential.
[0..528] GM707H03:Wafer 6, chip 6 hyb w/UCSF 1898+1 exon 11 asym. PCR in 5xSSPE, 1 mM CTAB for 30 min at 30 C w/shaking ¶ w06c06 ¶ cf277b49.1sq ¶ cf/UCSF 1898+1/ex 11 ¶ asym. PCR Fl-dUTP ¶ 1 mL final vol. ¶ 30 min, 30 C ¶ 5xSSPE, 1 mM CTAB ¶ rt ¶ MIYADA ¶ ¶ Affymetrix Confidential.

Fig. 24

A

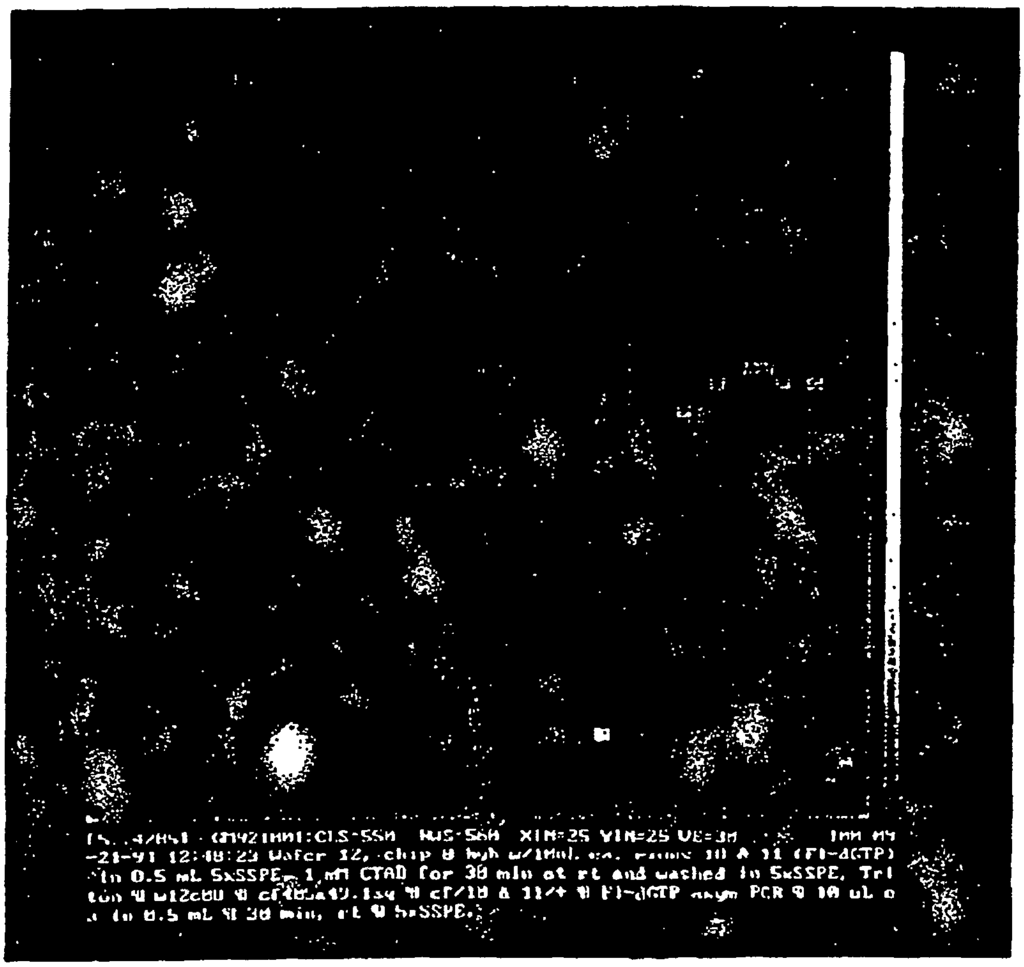

Fig. 25

B

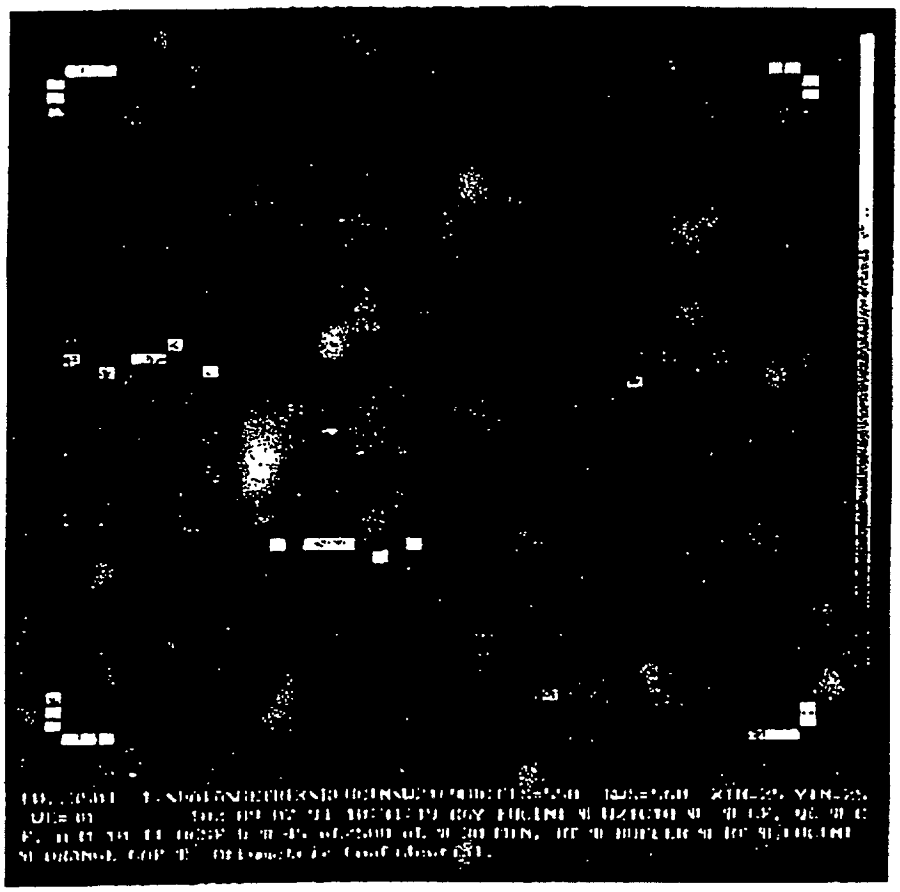

Fig. 25

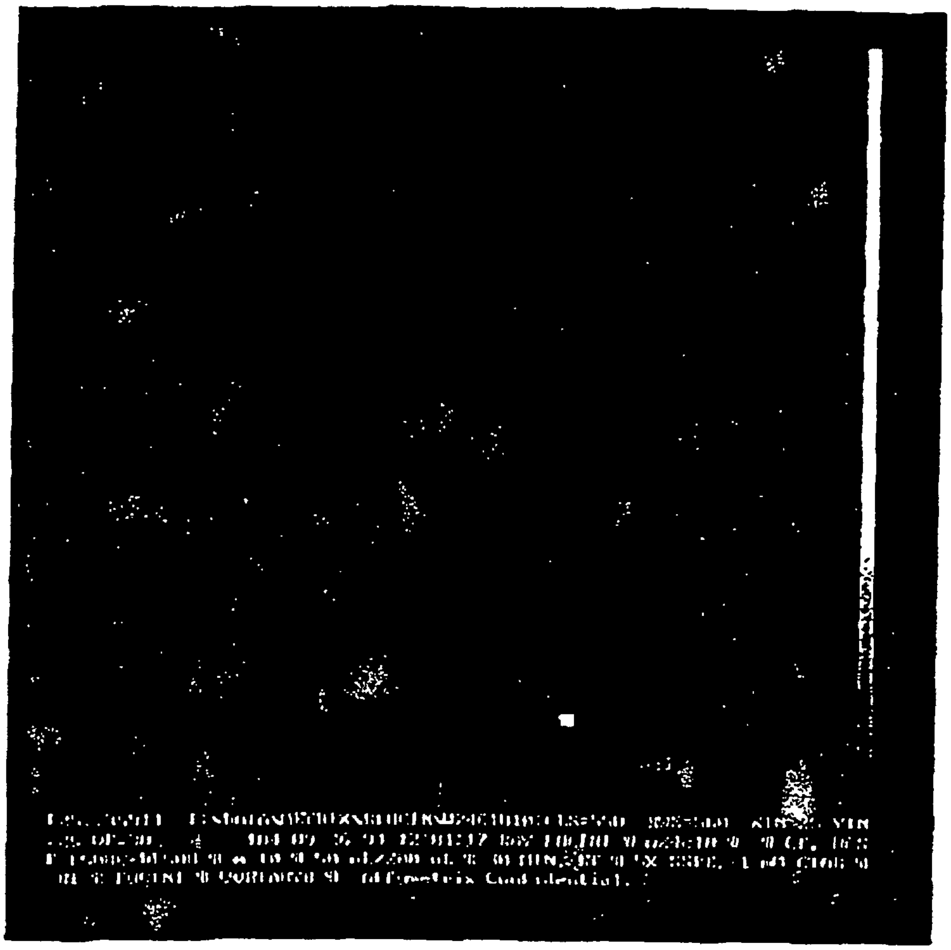

Fig. 26

## P53 EXON 6 CODON 192 REGION: 12MER PROBES

```
                             ↓
                 G A T G C T G A G G A G
0.                           C T C C T C C C C G G T
1.                         A C T C C T C C C C G G
2.                       G A C T C C T C C C C G
3.                     C G A C T C C T C C C C
4.                   A C G A C T C C T C C C
5.                 T A C G A C T C C T C C
6.               C T A C G A C T C C T C
7.             T C T A C G A C T C C T
8.           T T C T A C G A C T C C
9.         A T T C T A C G A C T C
10.      T A T T C T A C G A C T
11.    C T A T T C T A C G A C
12.  C C T A T T C T A C G A
```

Fig. 27

P53 EXON 6 CODON 192 REGION: 10MER PROBES

| | | | | | | | | | | | | | | | | | | | | | |
|---|---|---|---|---|---|---|---|---|---|---|---|---|---|---|---|---|---|---|---|---|---|
| | | | | | **G** | **A** | **T** | **G** | **C** | **T** | **G** | **A** | **G** | **G** | **A** | **G** | | | | | |
| 0. | | | | | | | | | | | | T | C | C | T | C | C | C | C | G | G |
| 1. | | | | | | | | | | | C | T | C | C | T | C | C | C | C | G | |
| 2. | | | | | | | | | | A | C | T | C | C | T | C | C | C | C | | |
| 3. | | | | | | | | | G | A | C | T | C | C | T | C | C | C | | | |
| 4. | | | | | | | | C | G | A | C | T | C | C | T | C | C | | | | |
| 5. | | | | | | | A | C | G | A | C | T | C | C | T | C | | | | | |
| 6. | | | | | | T | A | C | G | A | C | T | C | C | T | | | | | | |
| 7. | | | | | C | T | A | C | G | A | C | T | C | C | | | | | | | |
| 8. | | | | T | C | T | A | C | G | A | C | T | C | | | | | | | | |
| 9. | | | T | T | C | T | A | C | G | A | C | T | | | | | | | | | |
| 10. | | A | T | T | C | T | A | C | G | A | C | | | | | | | | | | |
| 11 | T | A | T | T | C | T | A | C | G | A | | | | | | | | | | | |

Fig. 28

# Detection of 12-mer One-Base Sustitution P53 Targets

Fig. 29

WT ("G" Substitution) Target 12-mer

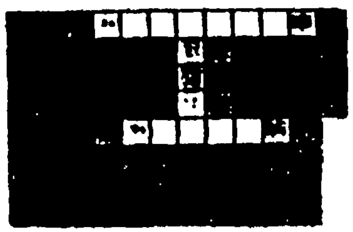

"A Substitution 12-mer Target

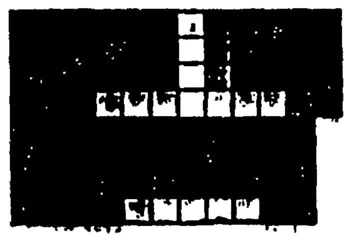

"T" Substitution Target 12-mer

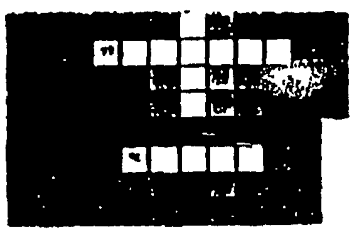

"C"Substitution Target 12-mer

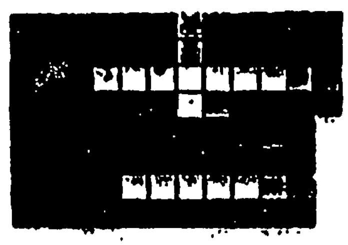

Fig. 31

4:1 Mixture of WT and "A" Substitution 12-mer Targets

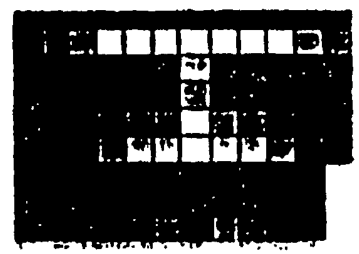

Figs. 29 and 31

P53 EXON 6 CODON 192 REGION

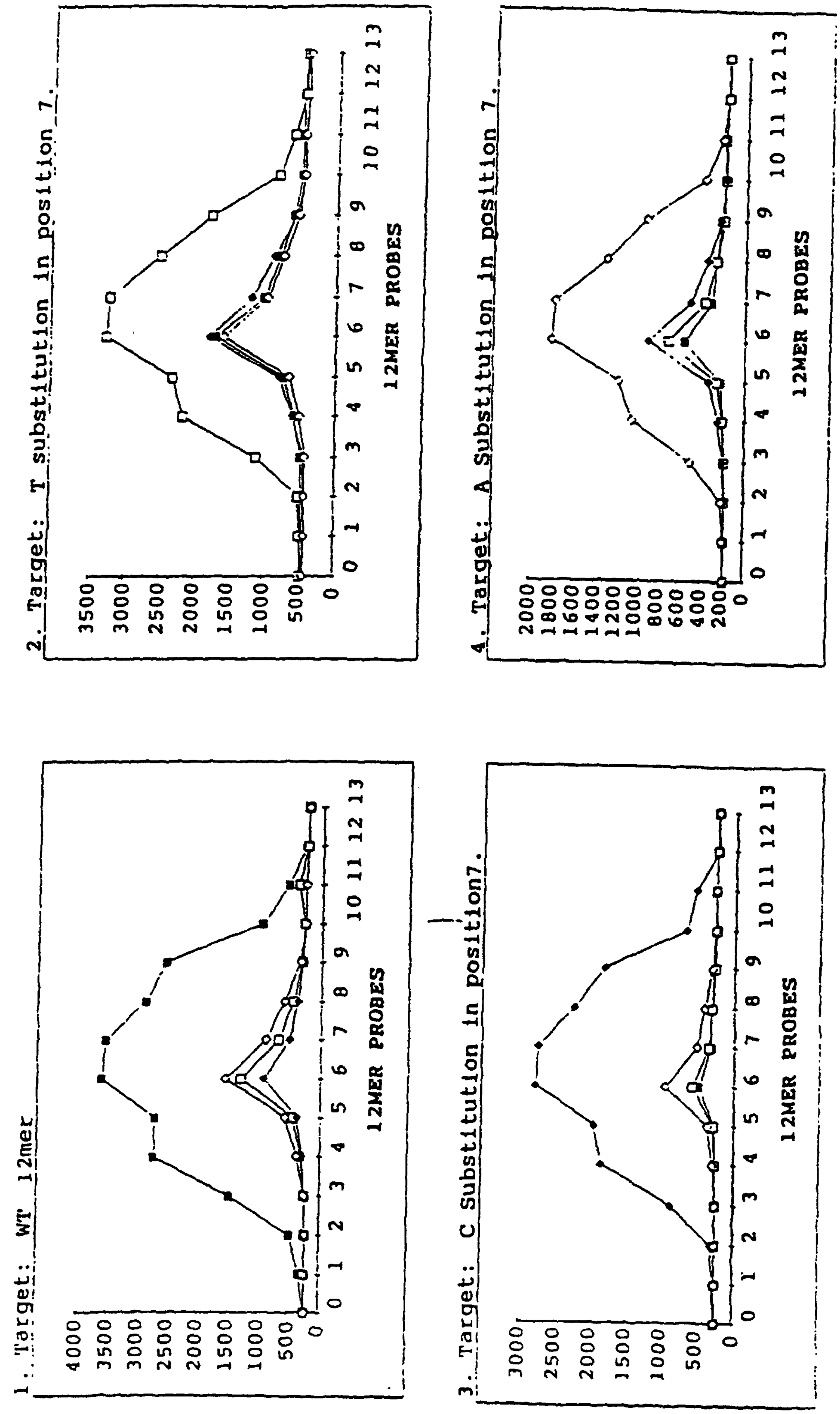


Fig. 30

P53 EXON 6 CODON 192 REGION

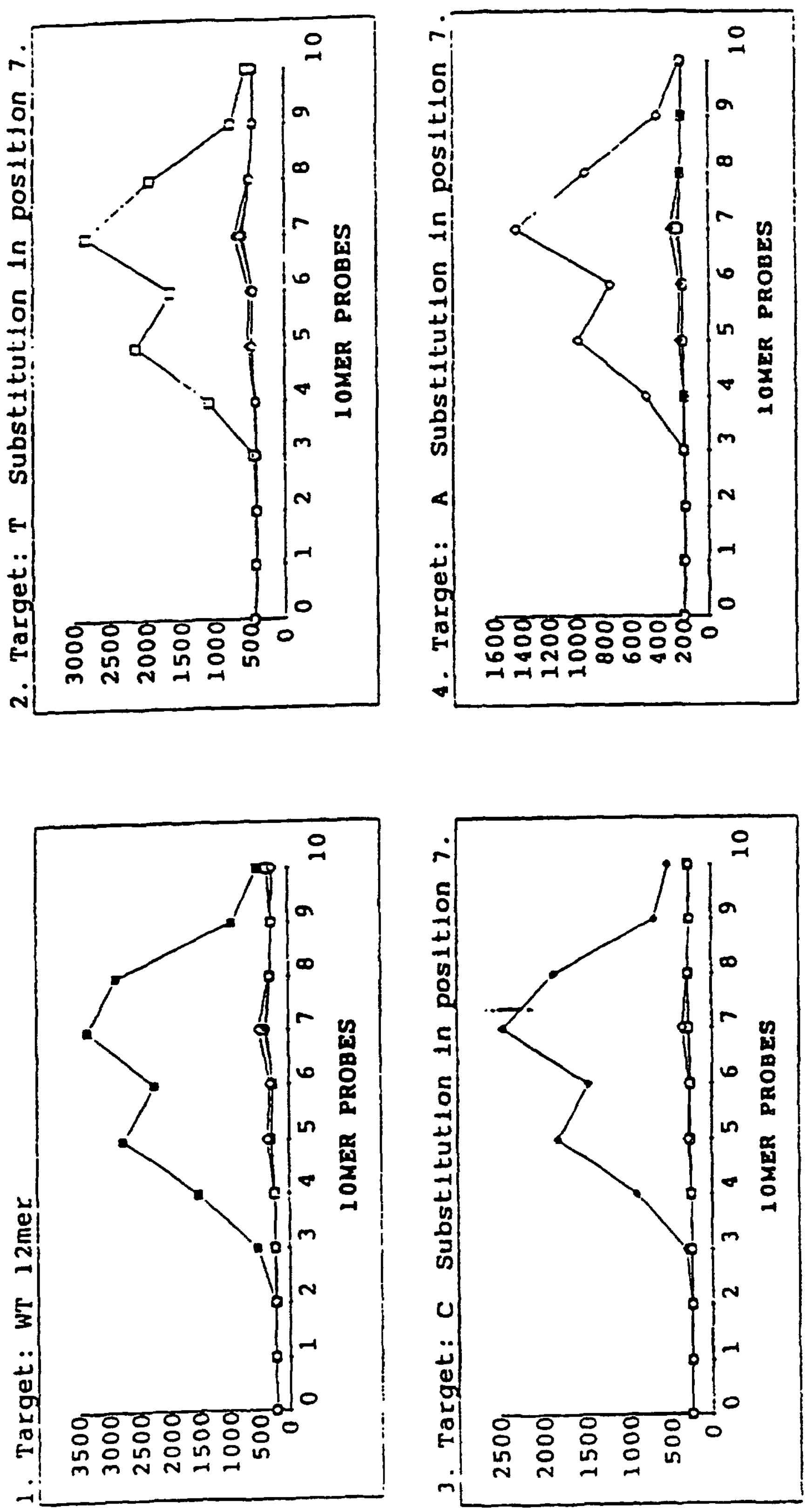


Fig. 32

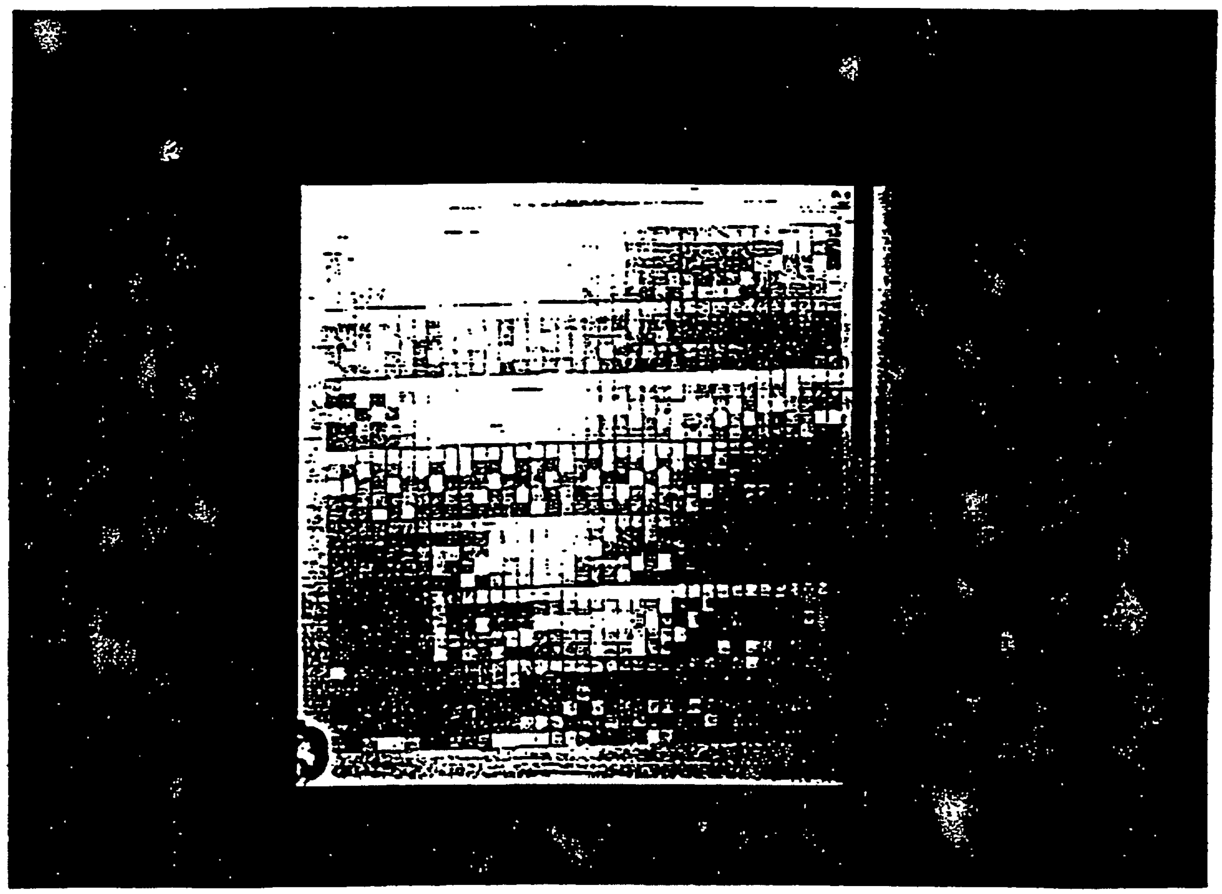

Fig. 33

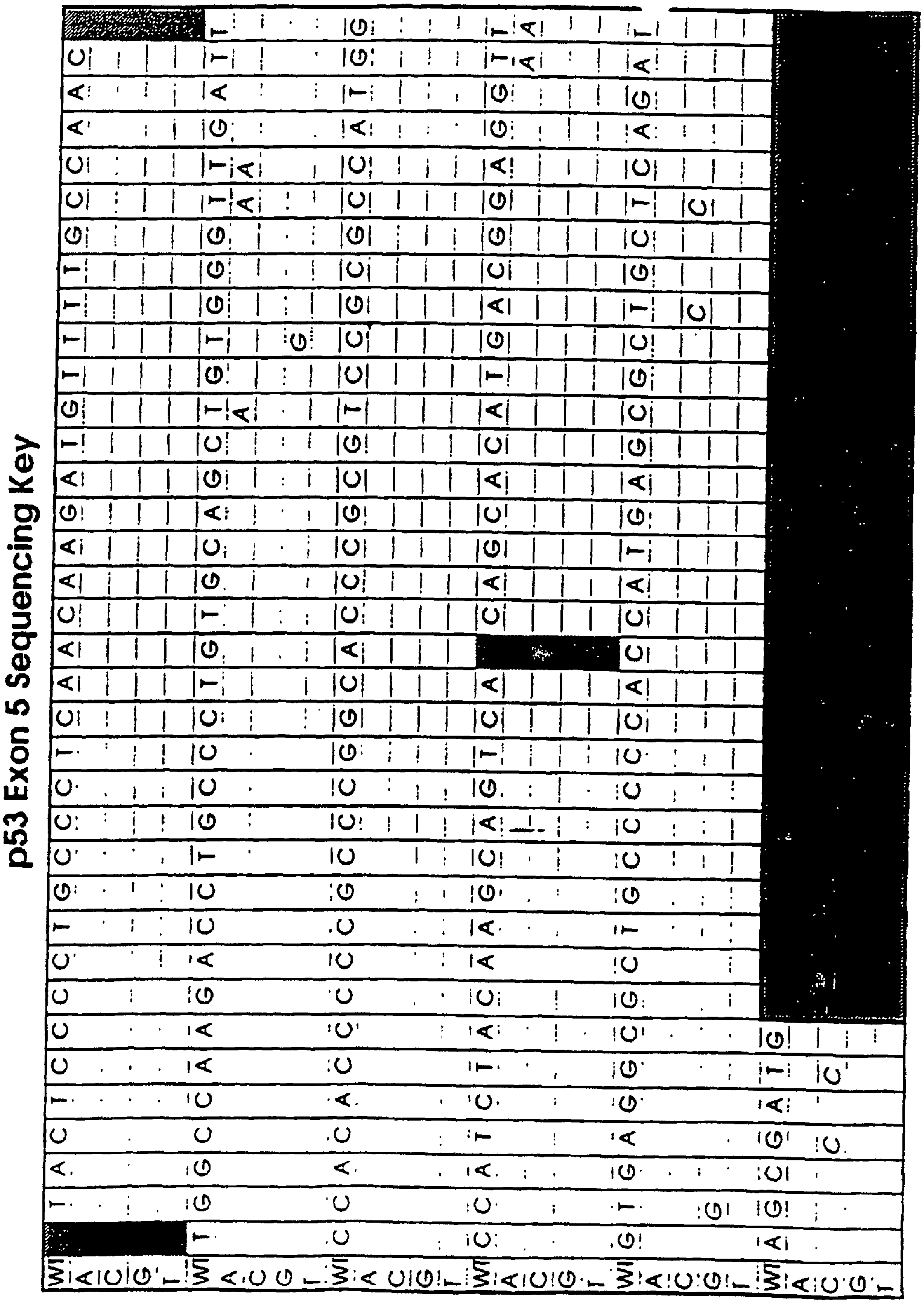


Fig. 34

# THE HUMAN MITOCHONDRIAL GENOME

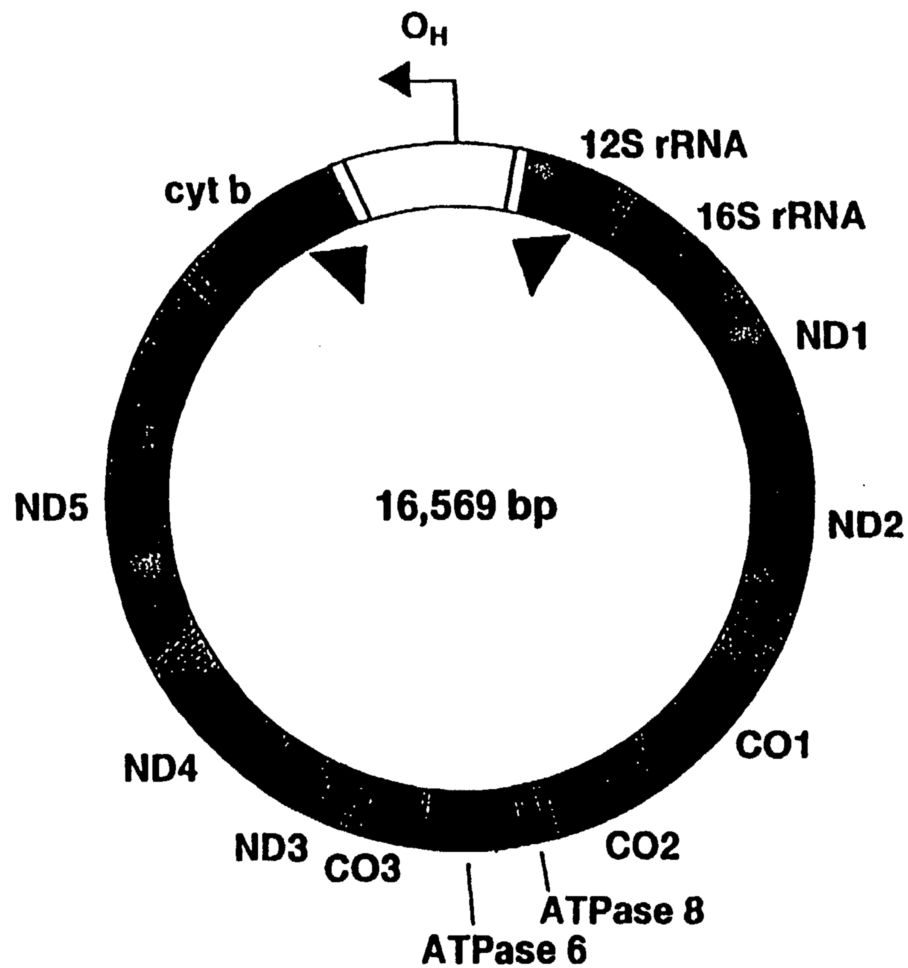


Fig. 35

## HYBRIDIZATION

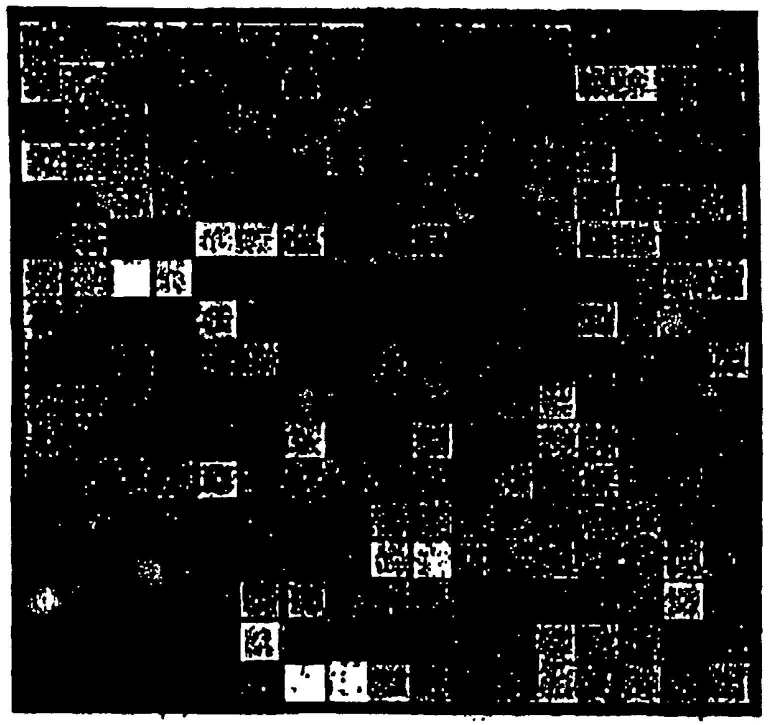

mt4

Fig. 36

HYBRIDIZATION

mt5

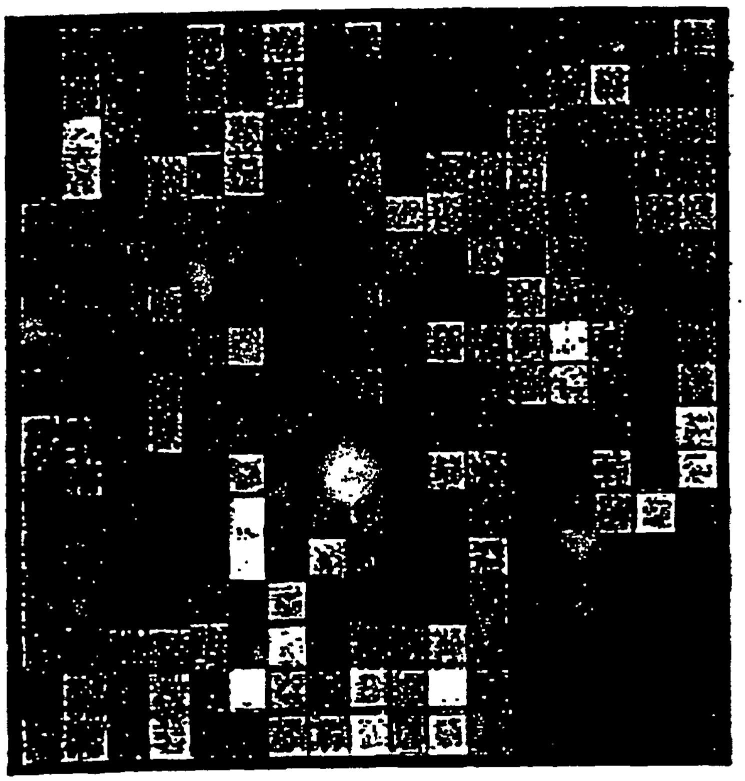

Fig. 37

# PREDICTED DIFFERENCE IMAGE

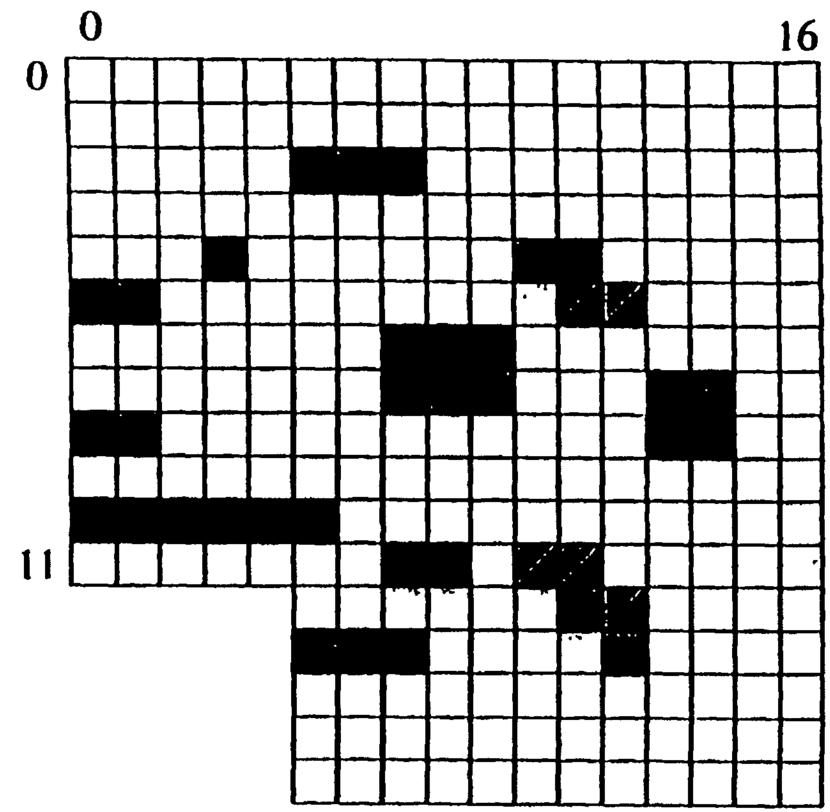


Fig. 38

DIFFERENCE IMAGE

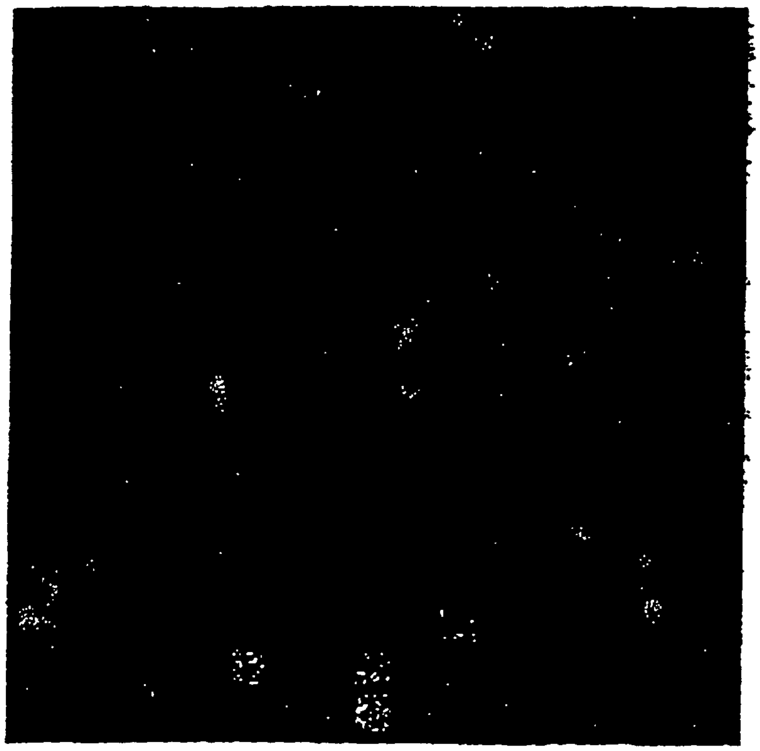

Fig. 39

# NORMALIZED INTENSITIES

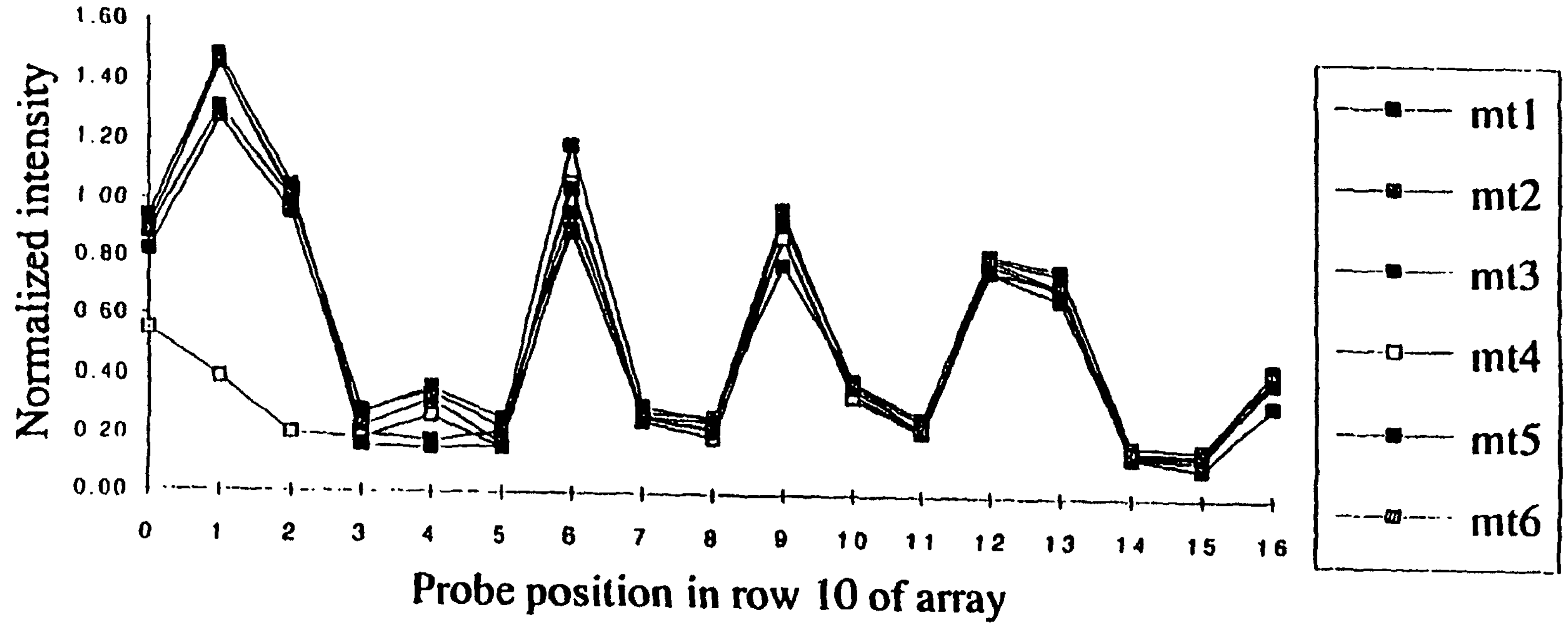


| probe position | 0 | 1 | 2 | 3 | 4 | 5 |
|---|---|---|---|---|---|---|
| probe length | 13 | 13 | 12 | 12 | 12 | 12 |
| sample (mt1 -> 6) | 4 | 4 | 4 | 2, 5 | 2, 5 | 2, 5 |
| mismatch position from 3' of probe | 12 | 5 | 3 | 12 | 7 | 2 |
| base change | t -> a | t -> a | t -> a | t -> c | t -> c | t -> c |

Fig. 40
Sheet 1 of 2

# NORMALIZED INTENSITIES

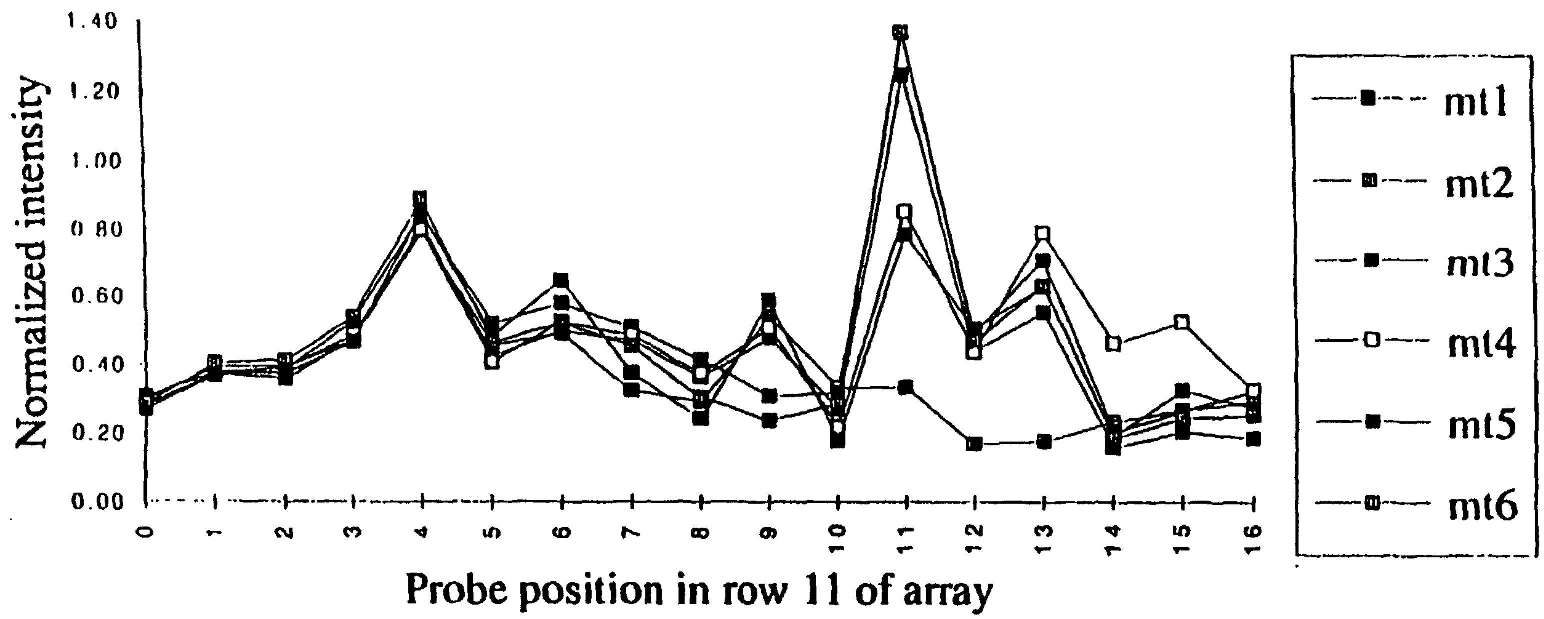


| probe position | 6 | 7 | 8 | 9 | 10 | 11 | 12 | 13 |
|---|---|---|---|---|---|---|---|---|
| probe length | 13 | 12 | 12 | 13 | 14 | 13 | 12 | 12 |
| sample (mt1 -> 6) | 2 | 2, 5 | 2, 5, 6 | 3, 6 | 3, 4, 5 | 2, 4, 5 | 2 | 2 |
| mismatch position from 3' of probe | 13 | 9, 10 | 3, 4 11 | 11, 5 | 4, 11, double | 11, 3, double | 6 | 3 |
| base change | c -> t | c -> t | c -> t t -> c | t-> c | t -> c double | g -> a t -> c double | g -> a | g -> a |

Fig. 40
Sheet 2 of 2

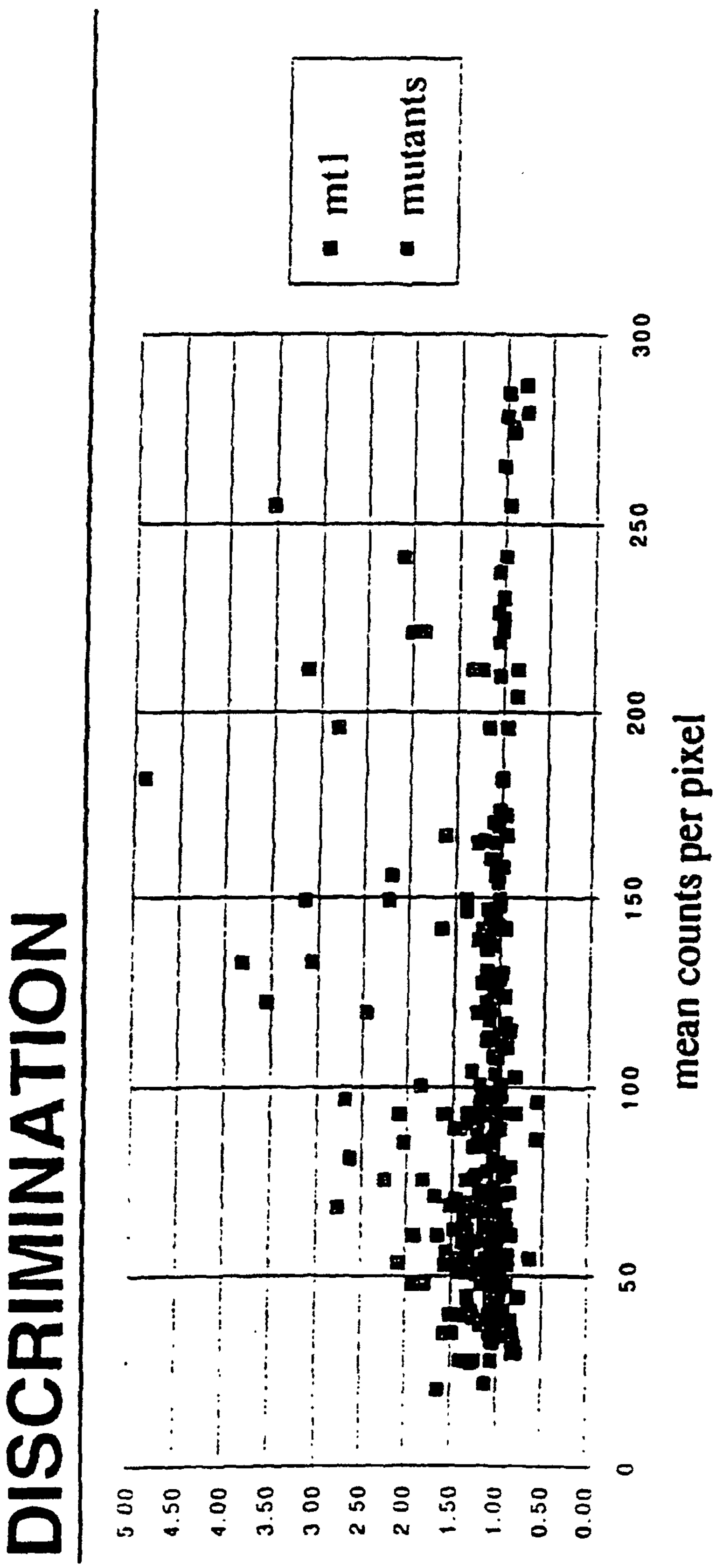
DISCRIMINATION
mt1
mutants
mean counts per pixel
0
50
100
150
200
250
300
0.00
0.50
1.00
1.50
2.00
2.50
3.00
3.50
4.00
4.50
5.00

Fig. 41

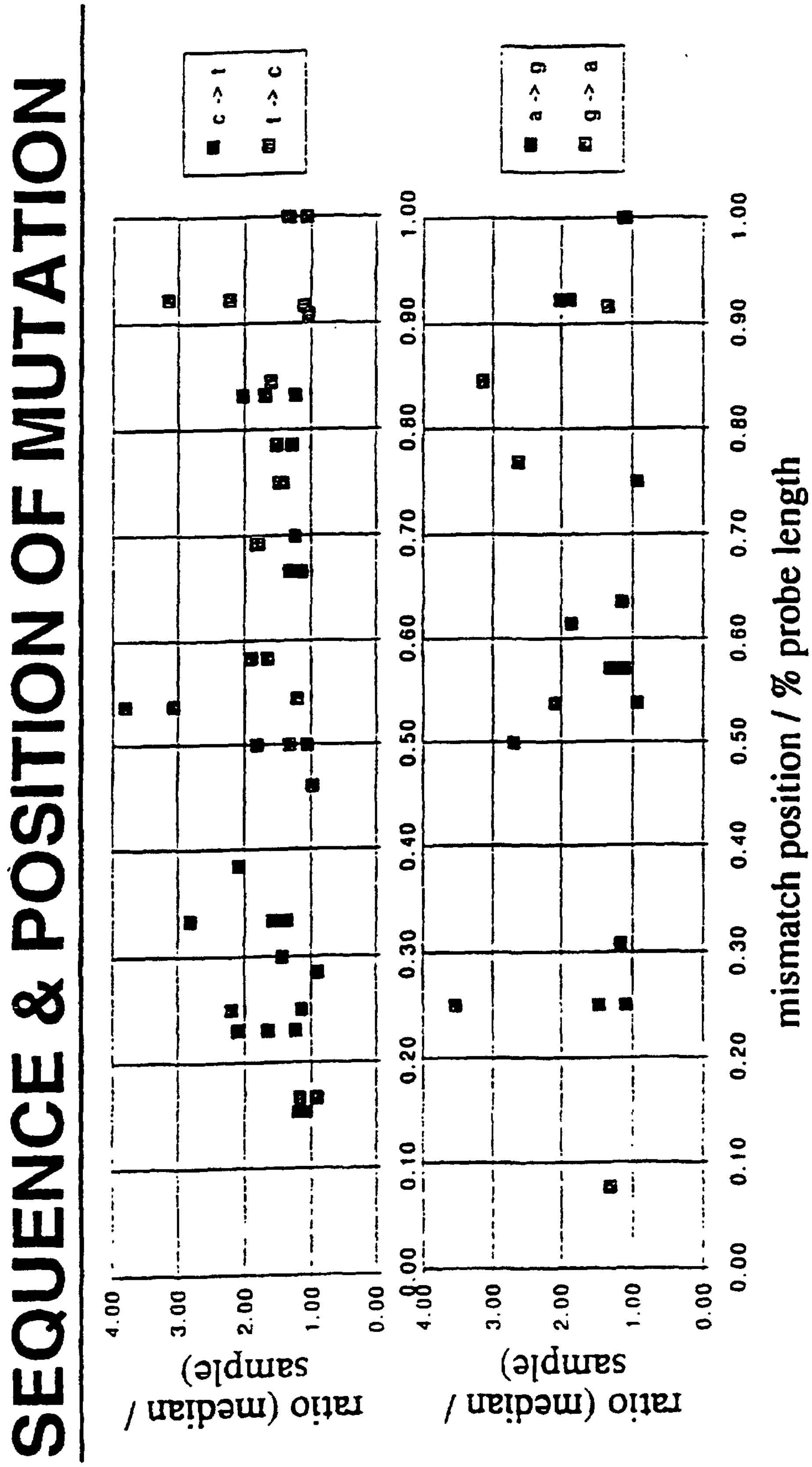
SEQUENCE & POSITION OF MUTATION
c -> t
t -> c
a -> g
g -> a
ratio (median / sample)
ratio (median / sample)
4.00
3.00
2.00
1.00
0.00
0.00
0.10
0.20
0.30
0.40
0.50
0.60
0.70
0.80
0.90
1.00
mismatch position / % probe length

Fig. 42

# SEQUENCE

```
1                                                50
XaacaaacctacccacccttaacagtacatagtacataaagccatttacX
cgtacatagcacattacagtcaaatcccttctcgtccccatggatgaccc
ccctcagataggggtcccttgaccaccatcctccgtgaaatcaatatccc
gcacaagagtgctactctcctcgctccgggcccataacacttgggggtag
ctaaagtgaactgtatccgacatctggttcctacttcagggTcataaagc
ctaaatagcccacacgttccccttaaataagacatcacgatggatcacag
gtctatcaccctattaaccactcacgggagctctccatgcatttggtatt
ttcgtctgggggtatgcacgcgatagcattgcgagacgctggagccgga
gcaccctatgtcgcagtatctgtctttgattcctgcctcatccTattatt
tatcgcacctacgttcaatattacaggcgaacatacttactaaagtgtgt
taattaattaatgcttgtaggacataataataacaattgaatgtctgcac
agccActttccacacagacatcataacaaaaatttccaccaaaccccc
XctccccccgcttctggccacagcacttaaacacatcTctgccaaaccccX
```

Fig. 43

Fig. 44

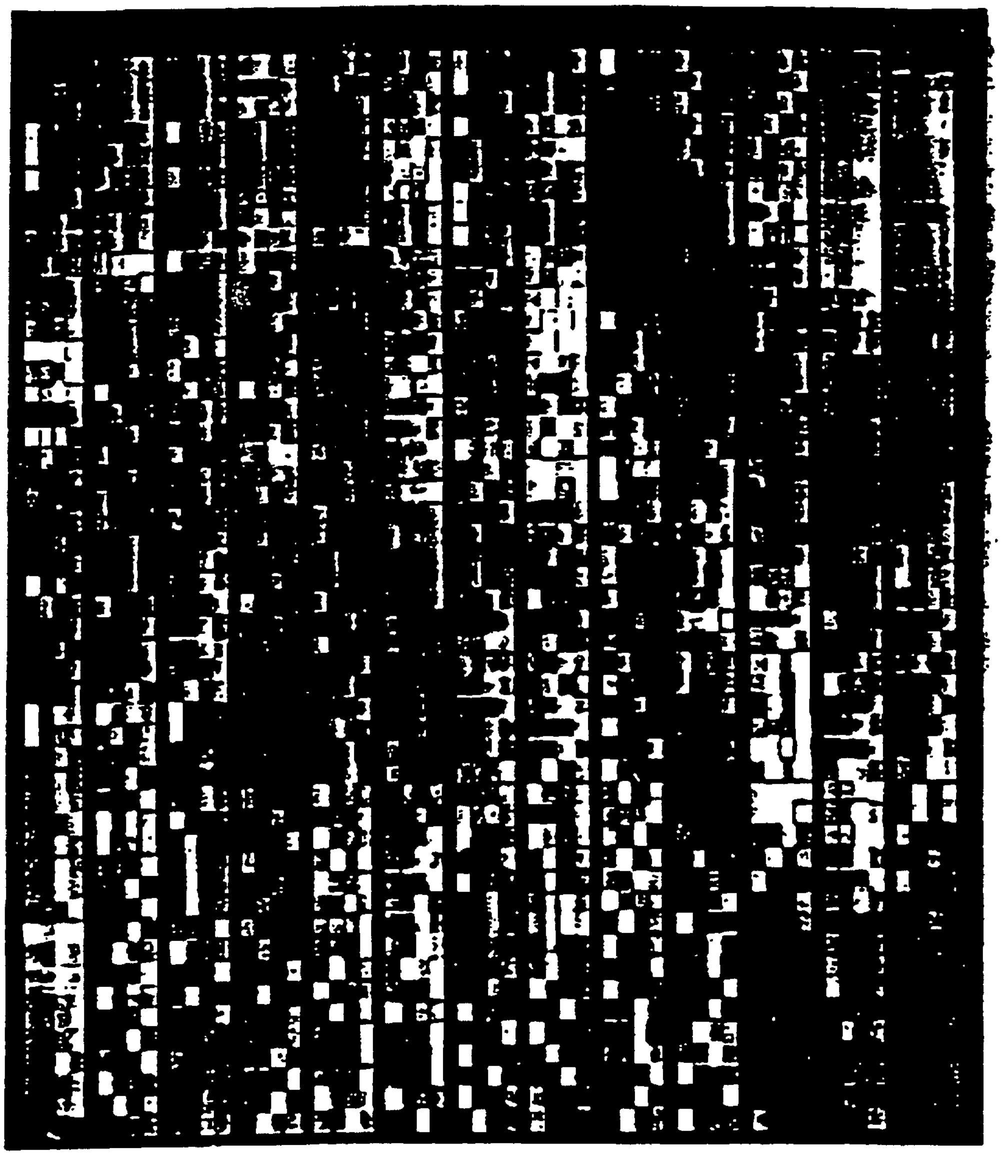

HYBRIDIZATION

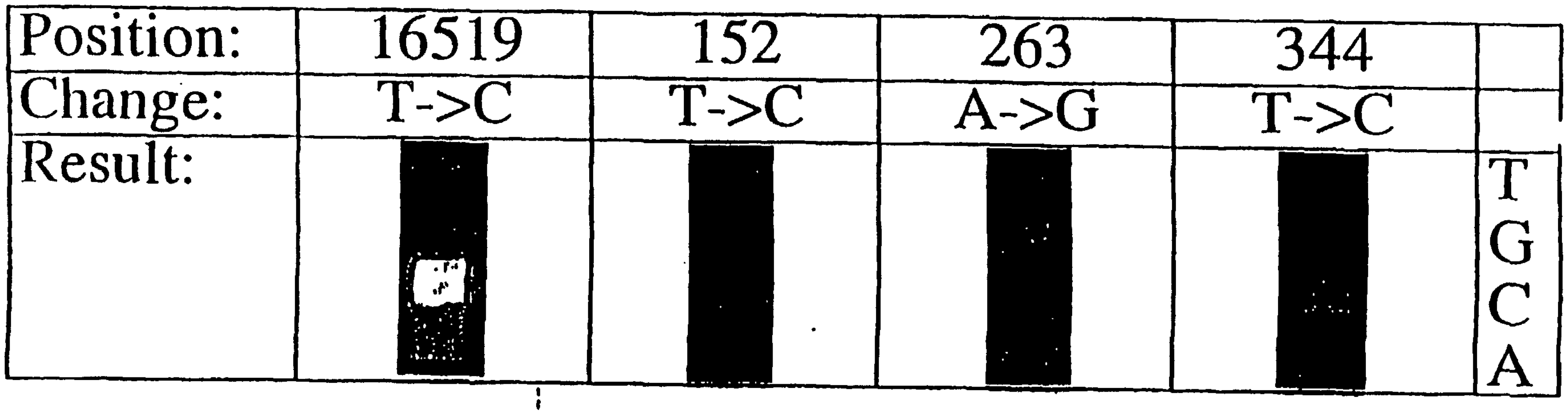


Fig. 45

Fig. 46

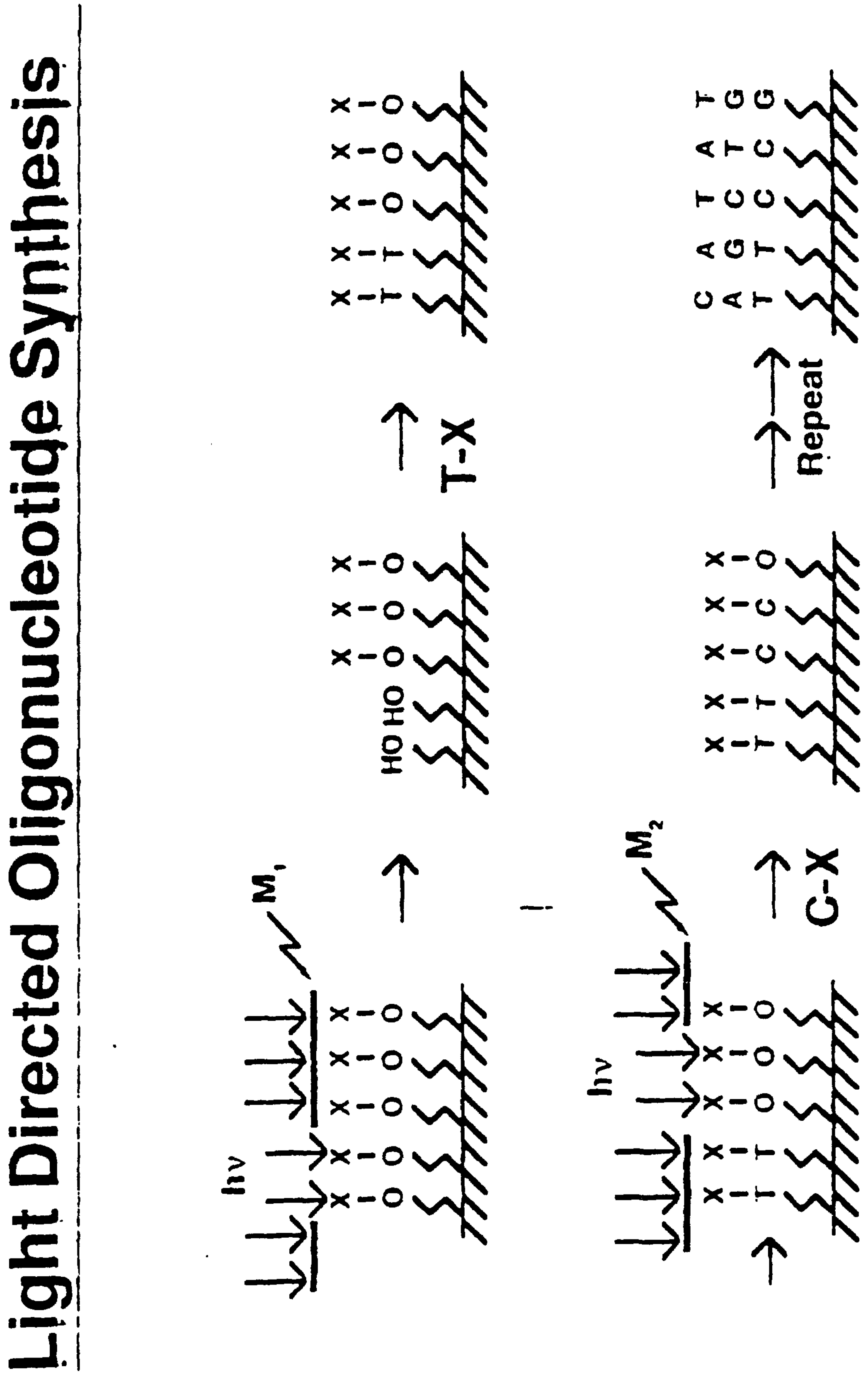
Light Directed Oligonucleotide Synthesis
hν
M1
T-X
hν
M2
C-X
Repeat

# Nucleoside Combinatorials

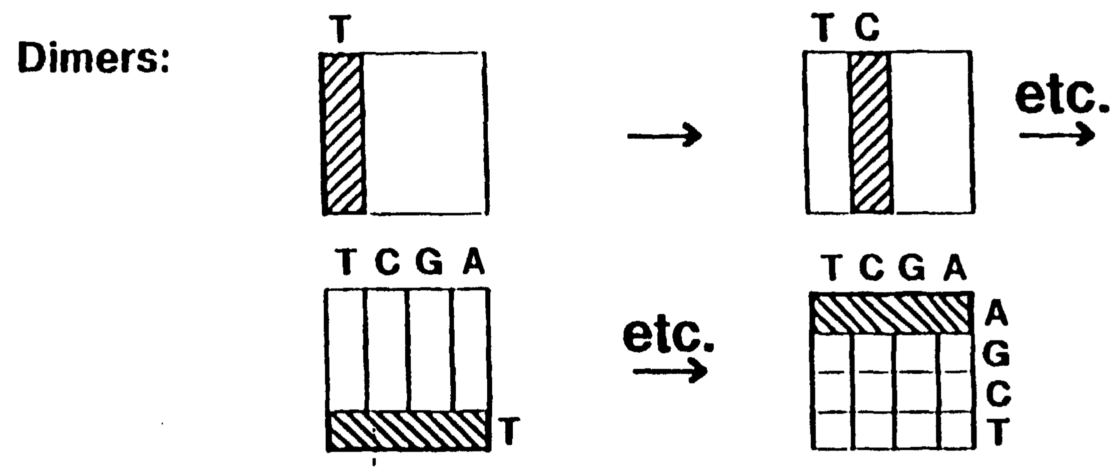


in polynomial notation:

$$(T + C + A + G)^2 = \text{All Dimers}$$

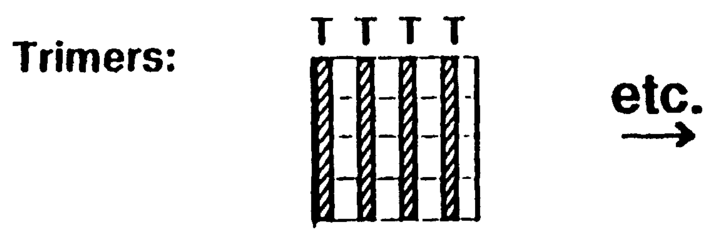


Fig. 47

Fig. 48

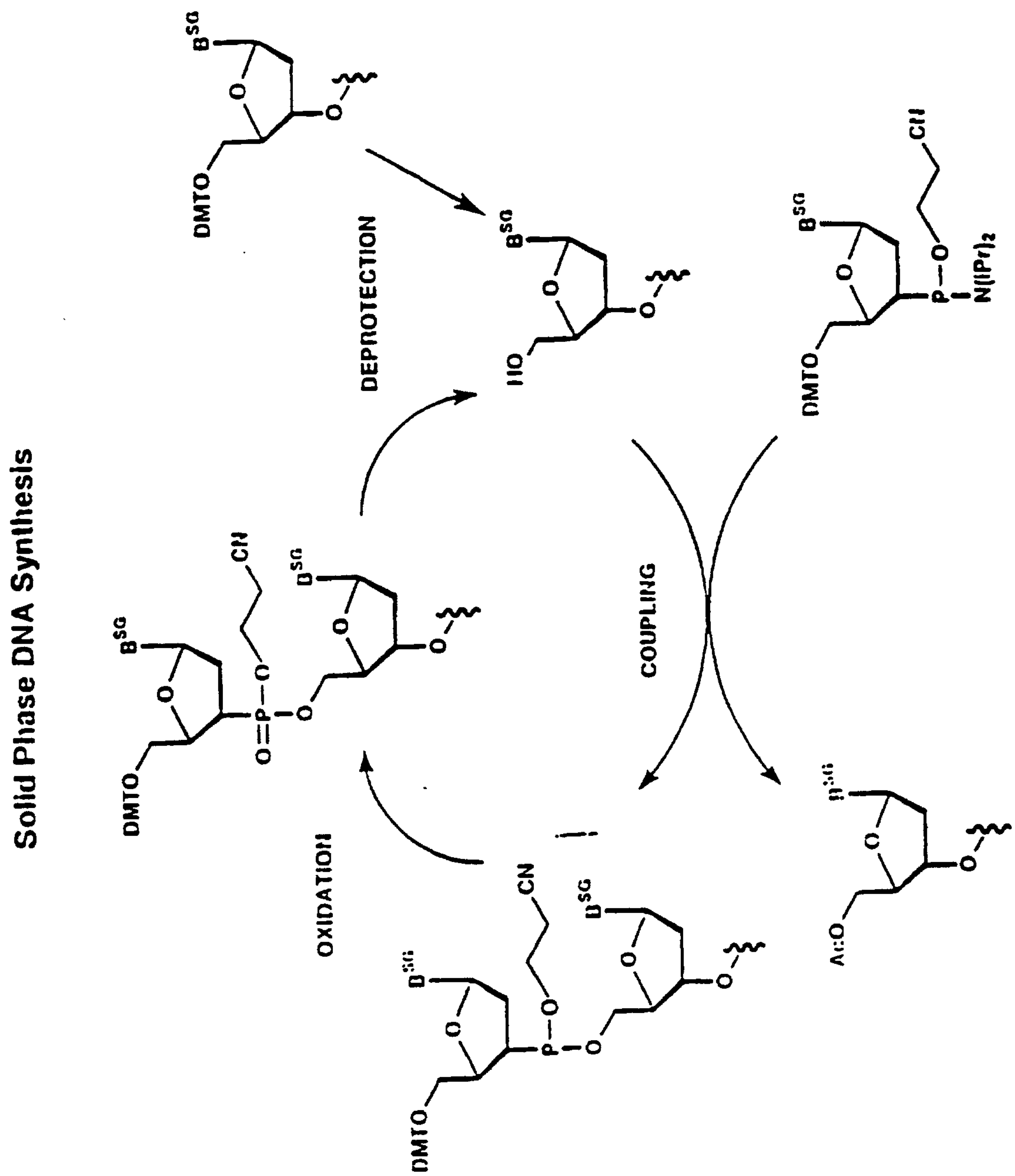
Solid Phase DNA Synthesis
DEPROTECTION
COUPLING
OXIDATION

Fig. 49

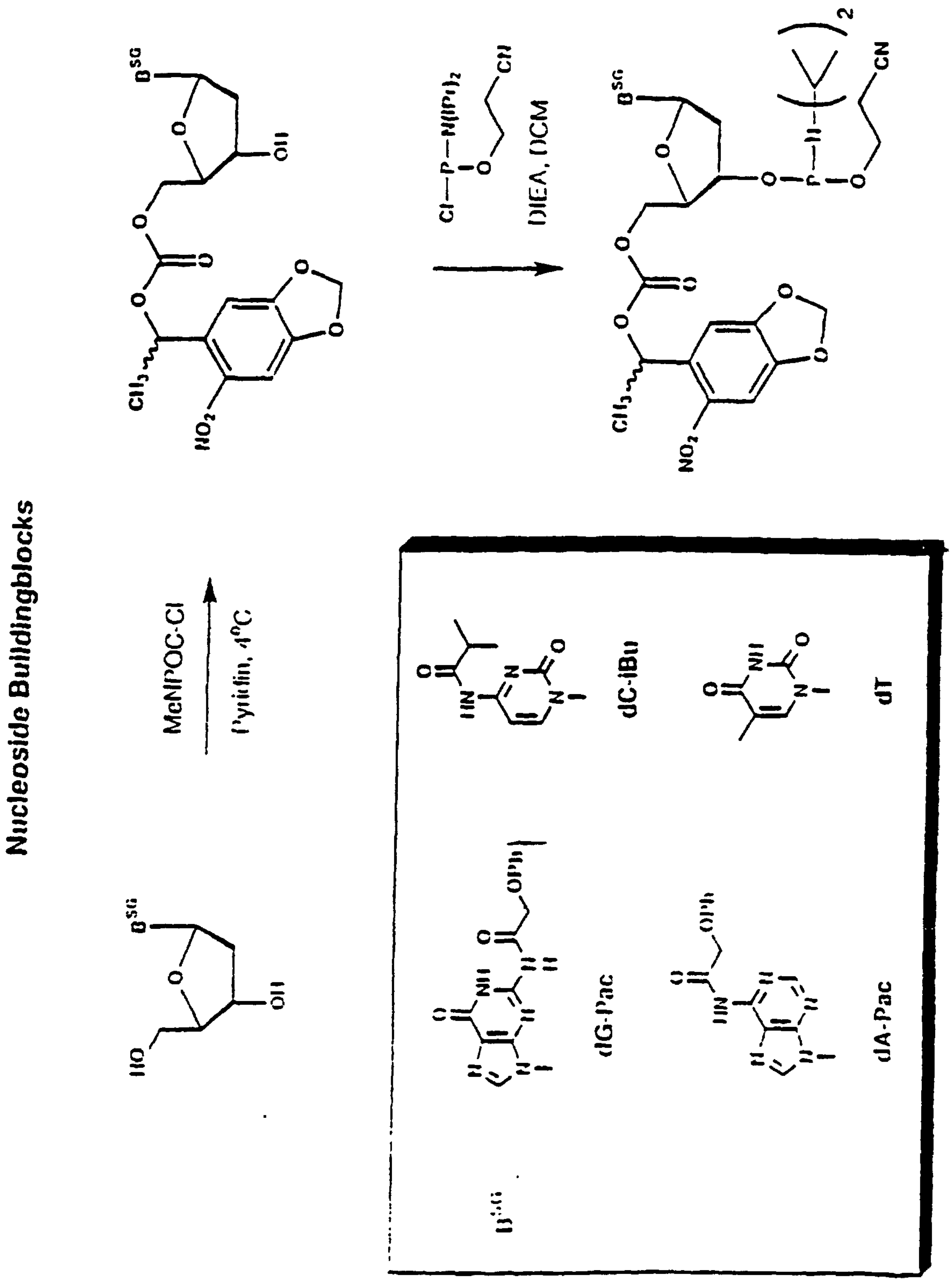
Nucleoside Buildingblocks
MeNPOC-Cl
Pyridin, 4°C
DIEA, DCM
dG-Pac
dA-Pac
dC-iBu
dT

Fig. 50

MeNPOC-Cl

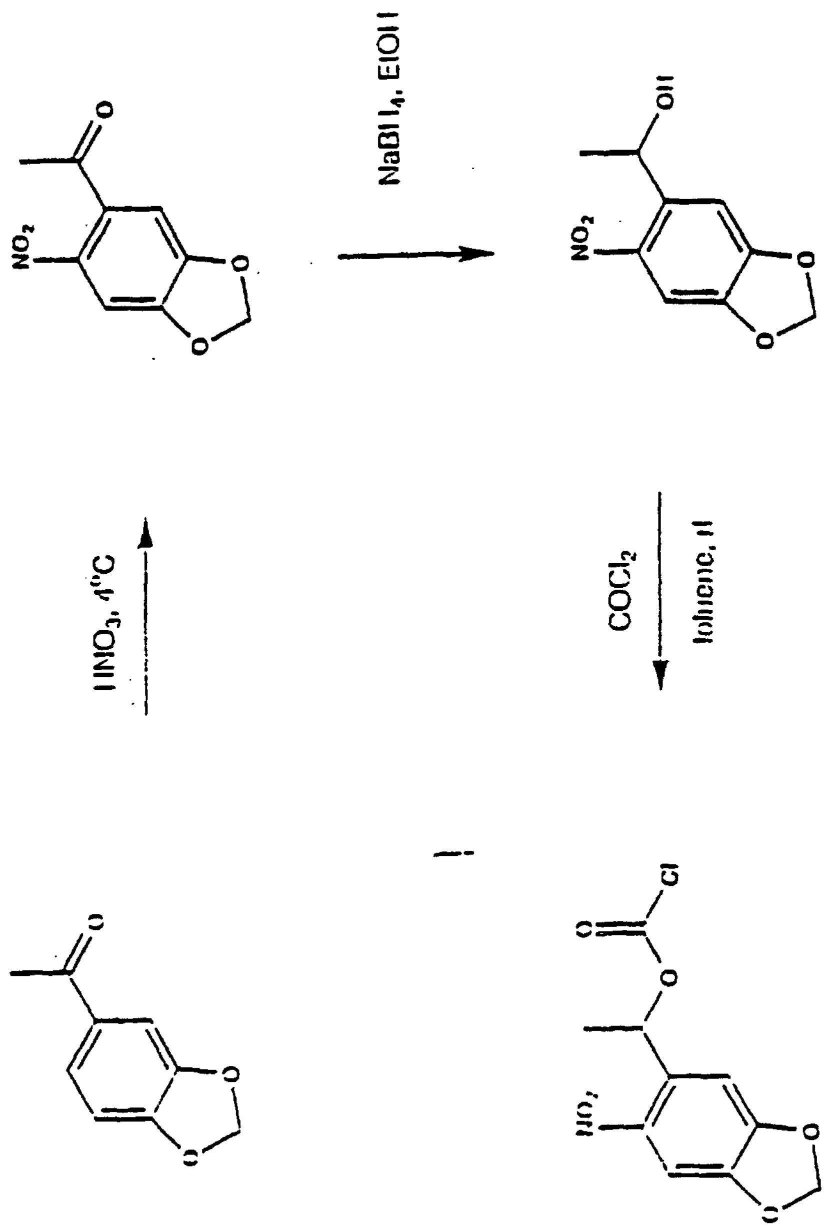

Fig. 51

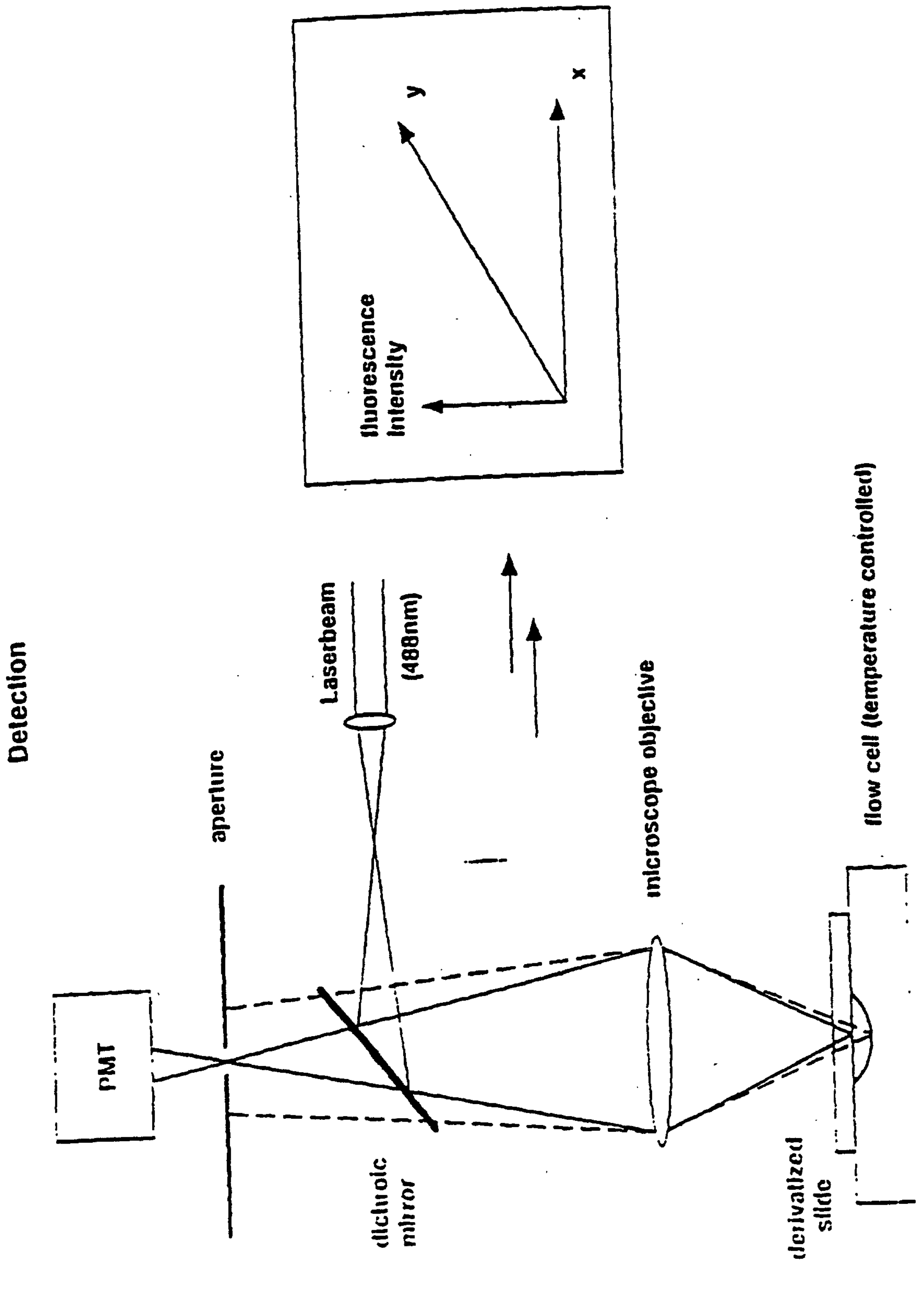
Detection
PMT
aperture
dichroic mirror
Laserbeam
(488nm)
microscope objective
derivatized slide
flow cell (temperature controlled)
fluorescence intensity
y
x